(19) Europäisches Patentamt
European Patent Office
Office européen des brevets

(11) EP 4 707 292 A2

(12) EUROPEAN PATENT APPLICATION

(43) Date of publication:
**11.03.2026 Bulletin 2026/11**

(21) Application number: **25211519.1**

(22) Date of filing: **15.03.2018**

(51) International Patent Classification (IPC):
***C07K 14/01*** *(2006.01)*

(52) Cooperative Patent Classification (CPC):
**A61P 3/08; A61K 35/76; C07K 14/005; C12N 15/86;** C12N 2750/14122; C12N 2750/14123; C12N 2750/14142; C12N 2750/14143; C12N 2750/14152

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2017 US 201762471762 P**
**16.06.2017 US 201762520901 P**
**14.02.2018 US 201862630558 P**

(62) Document number(s) of the earlier application(s) in accordance with Art. 76 EPC:
**23195775.4 / 4 303 225**
**18768025.1 / 3 610 023**

(71) Applicant: **The University of North Carolina at Chapel Hill**
**Chapel Hill, NC 27514 (US)**

(72) Inventors:
- **SAMULSKI, Richard, Jude**
  **Chapel Hill, 27514 (US)**
- **LI, Chengwen**
  **Chapel Hill, 27516 (US)**

(74) Representative: **Haseltine Lake Kempner LLP**
**Cheapside House**
**138 Cheapside**
**London EC2V 6BJ (GB)**

Remarks:
•The complete document including Reference Table(s) and the Sequence Listing(s) can be downloaded from the EPO website
•This application was filed on 27-10-2025 as a divisional application to the application mentioned under INID code 62.
•Claims filed after the date of filing of the application / after the date of receipt of the divisonal application (Rule 68(4) EPC).

# (54) POLYPLOID ADENO-ASSOCIATED VIRUS VECTORS AND METHODS OF MAKING AND USING THE SAME

(57) The present invention provides a polyploid adeno-associated virus (AAV) capsid, wherein the capsid comprises capsid protein VP1, wherein said capsid protein VP1 is from one or more than one first AAV serotype, wherein said capsid protein VP2 is from one or more than one first AAV serotype and capsid protein VP3, wherein said capsid protein VP3 is from one or more than one second AAV serotype and wherein at least one of said first AAV serotype is different from at least one of said second AAV serotype and is different from at least one of said third AAV serotype, in any combination.

EP 4 707 292 A2

## Description

## RELATED APPLICATIONS

This application claims the benefit, under 35 U.S.C. § 119(e), of U.S. Provisional Application Serial No. 62/471,762, filed March 15, 2017, U.S. Provisional Application Serial No. 62/520,901, filed June 16, 2017, and U.S. Provisional Application Serial No. 62/630,558, filed February 14, 2018, the entire contents of each of which are incorporated by reference herein.

## STATEMENT REGARDING ELECTRONIC FILING OF A SEQUENCE LISTING

A Sequence Listing in ASCII text format, submitted under 37 C.F.R. § 1.821, entitled 5470-786WO_ST25.txt, 102,196 bytes in size, generated on March 15, 2018 and filed via EFS-Web, is provided in lieu of a paper copy. This Sequence Listing is hereby incorporated herein by reference into the specification for its disclosures.

## FIELD OF THE INVENTION

The present invention relates to modified capsid proteins from adeno-associated virus (AAV) particles, virions, virus capsids and virus vectors bound with surface protein for enhanced comprising the same. In particular, the invention relates to modified AAV capsid proteins and capsids comprising the same that can be incorporated into virus vectors to combine transduction and reduced antigenicity, tropism and/or other desirable phenotypic features in the virus vector.

## BACKGROUND OF THE INVENTION

Adeno-associated virus (AAV) vector has been used in over 100 clinical trials with promising results, in particular, for the treatment of blindness and hemophilia B. AAV is non-pathogenic, has a broad tissue tropism, and can infect dividing or non-dividing cells. More importantly, AAV vector transduction has induced long-term therapeutic transgene expression in pre-clinical and clinical trials. Currently there are 12 serotypes of AAV isolated for gene delivery. Among them, AAV8 has been shown to be the best for mouse liver targeting. Due to extensive studies in pre-clinical animals with FIX deficiency, Phase I/II clinical trials have been carried out using AAV2 and studies in pre-clinical animals with FIX deficiency, Phase I/II clinical trials have been carried out using AAV2 and AAV8 in patients with hemophilia B. The results from these trials are very promising; however, the FIX expression from patients receiving AAV/FIX was not proportional to what has been achieved in animal models even though the same vector dosage/kg was used. When $1x10^{11}$ particles of AAV8 encoding FIX were used in FIX knock out mice for systemic administration, 160% of normal level FIX was detected in blood. However, when $2x10^{11}$ particles of AAV8/FIX were administered, only 40% of FIX was achieved in primates and less than 1% of FIX was found in human. The inconsistent FIX expression following AAV vector transduction among these species may be due to altered hepatocyte tropism in different species. Another interesting finding from AAV FIX clinical trials is the capsid specific cytotoxic T lymphocyte (CTL) response that eradicates AAV transduced hepatocytes, resulting in therapeutic failure. This phenomenon has not been demonstrated in animal models following AAV delivery, which points out another variation between preclinical and clinical studies. When a much higher dose of AAV/FIX vector was used, FIX expression was detected in both clinical trials using either AAV2 or AAV8; however the blood FIX level decreased at week 4 or 9 post injection, respectively. Further studies suggested that AAV vector infection elicited a capsid specific CTL response, which appeared to eliminate AAV transduced hepatocytes. Therefore, the results from these clinical trials highlight the necessity to explore effective approaches for enhancement of AAV transduction without increasing vector capsid burden. Any vector improvement that reduces AAV capsid antigen will also impact the daunting vector production concerns and be a welcome addition to viable gene therapy drug development.

Adeno-associated virus (AAV), a non-pathogenic-dependent parvovirus that needs helper viruses for efficient replication, is utilized as a virus vector for gene therapy because of its safety and simplicity. AAV has a broad host and cell type tropism capable of transducing both dividing and non-dividing cells. To date, 12 AAV serotypes and more than 100 variants have been identified. Different serotype capsids have different infectivity in tissues or culture cells, which depend on the primary receptor and co-receptors on the cell surface or the intracellular trafficking pathway itself. The primary receptors of some serotypes of AAV have been determined, such as heparin sulfate proteoglycan (HSPG) for AAV2 and AAV3, and N-linked sialic acid for AAV5, while the primary receptor of AAV7 and AAV8 has not been identified. Interestingly, AAV vector transduction efficiency in cultured cells may not always be translated into that in animals. For instance, AAV8 induces much higher transgene expression than other serotypes in mouse liver, but not in culture cell lines.

Of 12 serotypes, several AAV serotypes and variants have been used in clinical trials. As the first characterized capsid, AAV2 has been most widely used in gene delivery such as RPE 65 for Leber congenital amaurosis and Factor IX (FIX) for hemophilia B. Although the application of AAV vectors has been proven safe and therapeutic effect has been achieved in these clinical trials, one of the major challenges of AAV vector is its low infectivity that requires relatively huge numbers of virus genomes. AAV8 vector is another vector which has been used in several clinical trials in patients with hemophilia B. The results from AAV8/FIX liver-targeted delivery have demonstrated that there are distinct species-specific differences in transgene expression between mice, non-human primates and humans. While $10^{10}$ vg of AAV8 with FIX gene could reach supra-physiologic levels (>100%) of FIX expression in FIX knock-out mice, only high doses (2 x $10^{12}$ vg/kg of body weight) could induce detectable FIX expression in humans. Based on these results described above, the development of effective strategies to enhance AAV transduction is still necessary.

The majority of people have been naturally exposed to AAVs. As a result, a large portion of the population has developed neutralizing antibodies (Nabs) in the blood and other bodily fluids against AAVs. The presence of Nabs poses another major challenge for broader AAV applications in future clinical trials. Many approaches have been explored to enhance AAV transduction or evade Nab activity, especially genetic modification of the AAV capsid based on rational design and directed evolution. Although several AAV mutants have demonstrated high transduction *in vitro* or in animal models, along with the capacity to escape Nabs, the modification of the capsid composition provides an ability to alter the cell tropisms of parental AAVs.

The present invention addresses a need in the art for AAV vectors with combined desirable features.

## SUMMARY OF THE INVENTION

In one aspect, the present invention provides an adeno-associated virus (AAV) particle comprising a surface-bound protein, wherein the protein bound to the surface of the AAV particle is selected from the group consisting of: (a) fibrinogen alpha chain; (b) fibrinogen beta chain; (c) fibrinogen gamma chain; (d) fibronectin; (e) plasminogen; (f) von Willebrand factor; (g) alpha-1-acid glycoprotein; (h) platelet factor 4; (i) cryoprecipitate; (j) factor VIII; (k) factor XIII; (1) albumin (e.g., human serum albumin, or albumin from any other species such as dog, horse, cow, pig); (m) apolipoprotein B (ApoB), (n) apolipoprotein E (ApoE); (o) transferrin; (p) low density lipoprotein; (q) immunoglobulin; (r) any other serum proteins and fusion serum protein that increases AAV binding on the cell surface and/or enhances AAV intracellular trafficking; and (s) any combination of (a)-(r) above.

Our previous studies have shown that the capsids from different AAV serotypes (AAV1 to AAV5) were compatible to assemble haploid AAV capsids and most isolated AAV monoclonal antibodies recognized several sites located on different AAV subunits. Additionally, the studies from chimeric AAV capsids demonstrated that higher transduction can be achieved with introduction of a domain for a primary receptor or tissue-specific domain from other serotypes. Introduction of AAV9 glycan receptor into AAV2 capsid enhances AAV2 transduction. Substitution of a 100 amino acid (aa) domain from AAV6 into AAV2 capsid increases muscle tropism. We presumed that the polyploid AAV vectors which are composed of capsids from two or more AAV serotypes might take advantages from individual serotypes for higher transduction but not eliminate the tropism from the parents. Moreover, these polyploid viruses might have the ability to escape the neutralization by Nabs since the majority of Nab recognize conformational epitopes and polyploid virions may have changed its surface structure.

AAV2 and AAV8 have been used for clinical application. In this study, we first characterized the haploid AAV virus from AAV2 and AAV8 for transduction efficiency *in vitro* and *in vivo,* as well as Nab escape ability. We found that the virus yield of the haploid vector was not compromised and the heparin binding profile was related to the incorporation of AAV2 capsid subunit proteins. The haploid vectors AAV2/8 initiated a higher transduction in mouse muscle and liver. When applied to a mouse model with FIX deficiency, higher FIX expression and improved bleeding phenotypic correction were observed in haploid vector-treated mice compared to AAV8 group. Importantly, the haploid virus AAV2/8 had low binding affinity to A20 and was able to escape the neutralization from anti-AAV2 serum. The next haploid virus AAV2/8/9 was made from capsids of three serotypes (AAV2, 8 and 9). It was demonstrated that the neutralizing antibody escape ability of haploid AAV2/8/9 was significantly improved against sera immunized with parental serotypes.

Thus, in one embodiment, the present invention provides an adeno-associated virus (AAV) capsid, wherein the capsid comprises capsid protein VP1, wherein said capsid protein VP1 is from one or more than one first AAV serotype and capsid protein VP3, wherein said capsid protein VP3 is from one or more than one second AAV serotype and wherein at least one of said first AAV serotype is different from at least one of said second AAV serotype, in any combination.

In some embodiments, the capsid of this invention comprises capsid protein VP2, wherein said capsid protein VP2 is from one or more than one third AAV serotype, wherein at least one of said one or more than one third AAV serotype is different from said first AAV serotype and/or said second AAV serotype, in any combination.

In some embodiments, the capsid of this invention comprises capsid protein VP1.5, wherein said capsid protein VP1.5 is from one or more than one fourth AAV serotype, wherein at least one of said one or more than one fourth AAV serotype is different from said first AAV serotype and/or said second AAV serotype, in any combination. In some embodiments, the AAV capsid protein described herein can comprise capsid protein VP2.

The present invention also provides an AAV capsid wherein the capsid comprises capsid protein VP1, wherein said capsid protein VP1 is from one or more than one first AAV serotype, and capsid protein VP2, wherein said capsid protein VP2 is from one or more than one second AAV serotype, and wherein at least one of said first AAV serotype is different from at least one of said second AAV serotype, in any combination.

In some embodiments, the capsid comprises capsid protein VP3, wherein said capsid protein VP3 is from one or more than one third AAV serotype, wherein at least one of said one or more than one third AAV serotype is different from said first AAV serotype and/or said second AAV serotype, in any combination. In some embodiments, the AAV capsid described herein can comprise capsid protein VP1.5.

The present invention further provides an adeno-associated virus (AAV) capsid, wherein the capsid comprises capsid protein VP1, wherein said capsid protein VP1 is from one or more than one first AAV serotype, and capsid protein VP1.5, wherein said capsid protein VP1.5 is from one or more than one second AAV serotype, and wherein at least one of said first AAV serotype is different from at least one of said second AAV serotype, in any combination.

In additional embodiments, the present invention provides a virus vector comprising: (a) an AAV capsid of this invention; and (b) a nucleic acid comprising at least one terminal repeat sequence, wherein the nucleic acid is encapsidated by the AAV capsid. The virus vector can be an AAV particle and the capsid protein, capsid, virus vector and/or AAV particle of this invention can be present in a composition that further comprises a pharmaceutically acceptable carrier.

Further provided herein is a method of making an AAV particle comprising the AAV capsid of any preceding claim, comprising: (a) transfecting a host cell with one or more plasmids that provide, in combination all functions and genes needed to assemble AAV particles; (b) introducing one or more nucleic acid constructs into a packaging cell line or producer cell line to provide, in combination all functions and genes needed to assemble AAV particles; (c) introducing into a host cell one or more recombinant baculovirus vectors that provide in combination all functions and genes needed to assemble AAV particles; and/or (d) introducing into a host cell one or more recombinant herpesvirus vectors that provide in combination all functions and genes needed to assemble AAV particles.

In further embodiments, the present invention provides a method of administering a nucleic acid to a cell, the method comprising contacting the cell with the virus vector of this invention and/or a composition of this invention.

Also provided herein is a method of delivering a nucleic acid to a subject, the method comprising administering to the subject the virus vector and/or a composition of this invention.

Additionally, provided herein is the capsid protein, capsid, virus vector, AAV particle and/or composition of this invention for use as a medicament in the beneficial treatment of a disorder or disease.

These and other aspects of the invention are addressed in more detail in the description of the invention set forth below.

## BRIEF DESCRIPTION OF THE DRAWINGS

**Fig. 1:** The enhanced effect of serum on AAV transduction. **(a)** Human serum enhances AAV transduction from different serotypes. $1x10^8$ particles of AAV/luc vector were incubated with 1:500 diluted sera or PBS for 2hr at 4°C. The mixture of AAV vector and sera was used to transduce $1x10^5$ Huh7 cells in a 48-well plate in the presence of adenovirus d1309 at MOI of 5. After 24 hr, luciferase activity from the cell lysate was analyzed. The fold increase of transgene expression from sera incubation was calculated by comparison to PBS. **(b)** The effect of incubation time of AAV with human serum on enhanced transduction. $1x10^8$ particles of AAV8/luc were incubated with 1:100 diluted human sera or PBS for different time periods at 4°C in the presence of ad dl309. 24hr later, luciferase expression was measured from the cell lysate. **(c)** Enhanced AAV transduction after systemic administration. $1x10^{10}$ particles of AAV8/luc were incubated with human serum at different dilutions for 2hr at 4°C. The mixture was administered into adult female C57BL mice via retro-orbital injection. The imaging was performed for 5min at day 3 after AAV injection. Upper panel: Representative live animal bioluminescent images of luciferase transgene expression profiles. Bottom panel: Quantification of luciferase transgene expression for enhanced AAV transduction from 6 mice after systemic administration. **(d)** Enhanced AAV transduction after muscular injection. The mixture of AAV8/luc with human serum from **(c)** was diluted to $1x10^9$ particles/200ul in PBS and injected into mouse hind leg muscle. At week 2 post injection, the imaging was taken for 5min. Face up: left leg-AAV8 + human sera, right leg-AAV8 + PBS. Upper panel: Representative imaging. Bottom: Data of enhanced AAV transduction from 6 mice after muscular injection. The fold increase of transduction was calculated by transduction from HSA incubated AAV to that from the PBS treated one.

**Fig. 2:** The effect of human albumin on AAV8 transduction. **(a)** Transduction enhancement is related to direct interaction of AAV with serum. AAV8/luc viruses were incubated with human serum or PBS at 1:100 dilution for 2 hr at 4°C, then the mixture was used to transduce Huh7 cells either in medium with FBS, serum free medium, or serum free medium plus human serum just before addition of AAV8 pre-incubated with PBS. 24 hr later, fold increase of transgene expression was calculated. **(b)** AAV8 interaction with human albumin. $1x10^{10}$ particles of AAV8/luc were incubated with human sera or PBS for 2hr at 4 °C, then the mixture of virus and human serum or PBS was applied to pre-Ig bound column. After washing, the column binding proteins were eluted for AAV8 genome copy number analysis. **(c)** AAV8 transduction with albumin depleted serum. $1x10^8$ particles of AAV8/luc were incubated with human serum or albumin depleted serum at different dilutions or PBS for 2hr at 4°C. Then the mixture was used to infect Huh7 cells in serum free medium. Two days later, luciferase was detected from the cell lysate, and the fold increase of transgene expression was calculated while compared to PBS. **(d)** Recombinant human albumin enhances AAV8 transduction. AAV8/luc was incubated with recombinant human albumin (50mg/ml) or human serum at different dilutions or PBS. Transgene expression was detected 48hr later, and the fold increase of transgene expression was calculated when compared to PBS.

**Fig. 3:** The effect of clinical grade human albumin on AAV8 transduction. **(a)** Enhanced AAV8 transduction in Huh7 cells from clinical grade HSA. $1x10^8$ particles of AAV8/luc were incubated with 5% HSA or human serum at different dilutions or PBS for 2hr at 4°C. Then the mixture was used to transduce Huh7 cells; 48hr later, luciferase expression was assayed. **(b)** Enhanced AAV8 transduction from clinical grade HSA after systemic administration. $1x10^{10}$ particles of AAV8/luc were incubated with 25% HSA at different dilutions and then injected into adult female C57BL mice via retro-orbital. Imaging was taken at day 7. Upper panel: Representative animal image. Bottom panel: Data of enhanced AAV transduction from 6 mice after systemic administration. **(c)** Enhanced AAV transduction from clinical grade HSA after muscular injection. $1x10^9$ particles of AAV8/luc were incubated with 25% HSA at different dilutions and then injected into muscles in C57BL mice. One week later, the imaging was performed. Upper panel: Representative animal image. Bottom: Data of enhanced AAV transduction from 6 or 7 mice after muscular injection.

**Fig. 4:** Incubation of AAV vector with HSA pre-freezing or post-thawing of viruses has the similar enhanced effect. **(a)** Enhanced transduction in Huh7 cells. $1x10^8$ particles of AAV/luc were incubated with clinical grade HSA at different dilutions for 2hr at 4°C before virus freezing or after virus thawing, and then added to Huh7 cells. 48 hr later, luciferase activity in the cell lysate was measured. **(b)** and **(c)** Enhanced muscle transduction. $1x10^9$ particles of AAV8/luc were directly injected into muscles of mice. At day 7 post injection, the mouse imaging **(b)** was carried out (left panel) and the fold increase **(c)** of transgene expression was calculated (right panel, n=6). Face up: left leg-HSA, right leg-PBS.

**Fig. 5:** Addition of HSA to virus preparation before dialysis does not compromise transduction enhancement. AAV8/luc viruses purified either from CsCl or column, were mixed with 1% of 25% HAS, and then applied for dialysis against PBS. After dialysis, AAV viruses were frozen; two days later, the *in vivo* transduction assay was performed. For liver transduction, $1x10^{10}$ particles of AAV/luc were administered via retro-orbital injection; the imaging was taken at day 3 after AAV injection for 5 mice **(a).** For muscle transduction, $1x10^9$ particles of AAV/luc were used; imaging was performed at day 7 post injection for 4 mice **(a).** Face up: left leg-HSA, right leg-PBS. Quantitation of imaging **(b)** was also performed.

**Fig. 6:** Human albumin increases AAV binding ability. **(a)** HSA increases AAV virus binding to Huh7 cells. AAV viruses were incubated with HSA for 2hr at 4°C, and then added to $1x10^6$ Huh7 cells for 5 or 15 min at 4°C. After washing 5 times, total DNA was extracted for AAV genome copy number analysis by q-PCR. **(b)** Imaging of liver transduction. $1x10^{11}$ particles of AAV8/luc were administered into mice via retro-orbital vein. Twenty four hr later, the imaging was carried out and the quantitation of imaging was calculated **(c).** Forty-eight hr later, mice were euthanized and liver tissue was harvested; the luciferase activity in liver tissue lysate was measured **(d)** and the AAV genome copy number was analyzed **(e).** During the first 24hr, plasma from blood was collected at 15min, 2hr, and 24hr post AAV injection, and AAV genome copy number was analyzed **(f).** The data represented the average of 4 mice and standard deviations. (*) indicates statistically significant difference with $p<0.05$ when the AAV genome copy number in the liver with HSA treatment was compared to that with PBS.

**Fig. 7:** Interaction of human albumin with AAV doesn't block Nab activity. AAV8/luc vector was first incubated with human albumin for 2hr at 4°C, then human IVIG at different dilution was added for another 2hr at 4°C. The mixture was added to Huh7 cells. At 48hr, the transgene expression from cell lysate was measured and Nab titer was calculated. **(a)** The effect of interaction of human albumin with AAV virions on Nab activity. **(b)** The effect of IVIG on human albumin enhancement of AAV transduction.

**Fig. 8:** Improvement of phenotypic correction of hemophilia B using human albumin incubated AAV vector. $2x10^9$ particles of AAV8/FIX-opt vector were incubated with human HSA or PBS for 2 hr at 4 °C, then AAV vector was administered into adult male FIX deficient mice via tail vein injection. Post AAV injection, blood was collected at indicated time points for FIX expression **(a)** and function assay **(b).** At week 6 post AAV injection, mice were applied for *in vivo* bleeding assay **(c).** (*) indicates statistically significant difference for blood loss between HSA treated mice and PBS mice with $p<0.05$. The data are based on the average and standard deviations from 6 to 8 mice.

**Fig. 9:** Mouse serum enhances AAV8 transduction *in vivo.* **(a)** Enhanced AAV transduction after systemic administration. $1x10^{10}$ particles of AAV8/luc were incubated with mouse serum at different dilutions for 2hr at 4°C. The mixture was administered into C57BL mice via retro-orbital injection. The imaging was carried out for 5min at day 3 post AAV injection. **(b)** Enhanced AAV transduction after muscular injection. $1x10^9$ AAV8 particles incubated with mouse serum were injected into mouse hind leg muscle. At week 2 after injection, the imaging was taken for 5min.

Face up: left leg-AAV8 + human sera, right leg-AAV8 + PBS. The fold increase of transduction was calculated by transduction from HSA incubated AAV to that from PBS treated one. Upper panel: representative imaging. Bottom: Data of enhanced AAV transduction from 3 or 4 mice.

**Fig. 10:** Sera from dogs and primates enhance AAV transduction in Huh7 cells. $1x10^8$ particles of AAV/luc vector were incubated with 1:500 diluted sera from 6 dogs **(a),** or 23 primates **(b),** or fetal bovines **(c),** or PBS for 2hr at 4°C. The mixture of AAV vector and sera was applied to transduce Huh7 cells in the presence of adenovirus d1309. After 24 hr, luciferase activity from the cell lysate was analyzed. The fold increase of transgene expression from sera incubation was calculated by comparison to PBS.

**Fig. 11:** Human albumin concentration in albumin depleted serum.

**Fig. 12:** rHSA Enhances AAV8 transduction *in vivo.* **(a)** Enhanced AAV8 transduction from rHSA after systemic administration. $1x10^{10}$ particles of AAV8 pre-incubated with rHSA were administered into C57BL mice via retro-orbital injection. The image was taken at day 3 post injection. **(b)** Enhanced AAV transduction from rHSA after muscular injection. $1x10^9$ particles of AAV8/luc incubated with rHSA were injected into hind leg muscles. At week 2 post injection, imaging was carried out. Upper panel: representative imaging. Bottom: Data of enhanced AAV transduction from 3 or 4 mice.

**Fig. 13:** Long-term enhanced AAV transduction with clinical grade HSA. After AAV8 muscular administration, imaging was performed at indicated time points. Left panel: representative imaging. Right panel: Data of enhanced AAV transduction from 3 or 4 mice after muscular injection.

**Fig. 14:** The effect of clinical grade of human albumin on AAV transduction from other serotypes. **(a)** HSA enhances AAV2 transduction in Huh7 cells. $1x10^8$ particles of AAV2/luc were incubated with human serum or 5% clinical grade HSA at different dilution for 2hr at 4°C, and then added to Huh7. 48 hr later, luciferase activity in the cell lysate was detected. **(b)** HSA enhances AAV9 transduction in Huh7 cells **(c)** and **(d).** HSA enhances liver or muscle transduction in C57BL mice from AAV2 and AAV9. The imaging from AAV transduction **(c)** and quantitation of imaging **(d).** For liver transduction, $1x10^{10}$ particles of AAV/luc incubated with 1 fold of HSA were administered via retro-orbital injection (n=4), the imaging was taken at day 7 (AAV2) or day3 (AAV9) after AAV injection. For muscle transduction, $1x10^9$ particles of AAV/luc incubated with 1 fold of HSA were used (n=3); imaging was performed at day 7 post injection.

**Fig. 15:** The effect of LDL and transferrin on AAV transduction *in vitro.* 10000 particles of AAV8/luc vectors per cell were incubated with LDL or transferrin at different dilutions of normal physiological plasma concentration for 2 hr at 4°C, then added to Huh7 **(A)** or 293T **(B)** cells in a 48-well plate. Forty-eight hr later, the cells were lysed and supernatant was harvested for luciferase activity analysis. The data represented the average from three independent experiments and standard deviations.

**Fig. 16:** Blocking receptors for LDL and transferrin impact AAV8 transduction in mice. Mice were injected with 0.5 mg of LDL or 1mg of lactoferrin via retro-orbital vein, and 5 minutes later, $1x10^{10}$ particles of AAV8/luc vector were systemically administered. At week 1 after AAV injection, mouse imaging was taken **(A)** and the transgene expression in the liver was calculated **(B).** The data represented the average of 5 mice and standard deviation.

**Fig. 17:** The effect of different doses of LDL or transferrin on AAV8 liver transduction. $1x10^{10}$ particles of AAV8/luc were incubated with LDL or transferrin at different dilutions of the normal physiological concentration for 2 hr at 4 °C and then administered into C57BL mice via retro-orbital injection. At day 3 post AAV injection, mice were imaged **(A** and **C)** and the transgene luciferase expression in the liver was quantitated **(B** and **D).** The data represented the average and standard deviations from 5 mice.

**Fig. 18:** LDL and transferrin increases AAV binding ability. AAV viruses were incubated with serum proteins for 1hr at 4°C, and then added to $1x10^6$ Huh7 cells or 293 Tcells for 2 hr at 4 °C. After washing 5 times, total DNA was extracted for AAV genome copy number analysis by q-PCR.

**Fig. 19:** The kinetics of AAV vector clearance in blood after systemic administration of AAV8 incubated with LDL or transferrin. $1x10^{11}$ particles of AAV8/luc were incubated with 500ug of LDL or 1 mg of transferrin for 1 hr at 4°C and then administered into C57BL mice via retro-orbital injection. At day 2 post AAV injection, mice were imaged **(A)** and the quantitation of transgene luciferase expression in the liver was performed **(B).** At indicated time points, mouse plasma was harvested and the AAV genome copy number was detected by quantitative-PCR **(C).** The data represented the average of 5 mice and standard deviations.

**Fig. 20:** The effect of LDL or transferrin on AAV8 vector bio-distribution. Mice from Figure 5 were sacrificed at day 5 post AAV administration and the tissues were harvested for luciferase activity assay *in vitro* **(A)** and genome copy number analysis **(B).**

**Fig. 21:** The effect of the combination of serum proteins on AAV transduction *in vitro.* 10000 particles of AAV8/luc vectors per cell were incubated with the combination of LDL or transferrion or albumin with either two proteins or three proteins for 2 hr at 4°C, then applied to 293T or Huh7 cells. Forty eight hr later, supernatant from cell lysate was analyzed for luciferase activity. The data represented the average of three independent experiments and standard deviations.

**Fig. 22:** The effect of the combination of serum proteins on AAV liver transduction in mice. $1x10^{10}$ particles of AAV8/luc were incubated with individual serum protein, or in combination of all three proteins (LDL, transferrin and albumin), at 100-fold dilution of physiological concentration for 2hr at 4°C, and then injected into mice. At day 3 and day 7 post AAV injection, the imaging was carried out **(A)** and liver transgene expression was analyzed **(B).** The results represented the average and standard deviations from 5 mice.

**Fig. 23:** Competitive binding analysis of serum proteins on AAV8 virions. For competitive assay of albumin **(A),** $1x10^{10}$ particles of AAV8/luc vectors were incubated with albumin at different dilutions and either LDL, transferrin, or ApoB at dilution of 100-fold for 1 hr at 4°C. Next, the specific antibodies to ApoB and transferrin were added to corresponding tubes for immunoprecipitation. After pull-down, virus titer was determined by quantitative PCR. For blocking assay **(B),** AAV8/luc vectors were incubated with albumin at different dilutions for 30 min at 4°C, then LDL, or transferrin, or ApoB at dilution of 100 fold was added for another 1 hr. After pull-down, virus titer was determined. The results represented the average of three individual experiments and standard deviation.

**Fig. 24:** Fibrinogen increases AAV9 transduction. $1x10^{10}$ particles of AAV9/luc were incubated with 3 mg of fibrinogen for 2 hr at 4°C and then injected into C57BL mice via retro-orbital vein. At day 7 post AAV injection, mice were imaged **(A)** and the transgene luciferase expression in the liver was quantitated **(B).** The data represented the average of 4 mice and standard deviations.

**Fig. 25:** Bio-distribution of AAV vector after systemic administration of AAV9 incubated with fibrinogen. Mice from Figure 1 were sacrificed at day 10 post AAV administration, and the tissues were harvested for luciferase activity assay *in vitro* **(A)** and genome copy number analysis **(B).**

**Fig. 26:** The effect of fibrinogen doses on AAV9 transduction. $1x10^{10}$ particles of AAV9/luc were incubated with different dilutions of fibrinogen for 2 hr at 4°C and then administered into C57BL mice via retro-orbital injection. At day 5 post AAV injection, mice were imaged **(A)** and the transgene luciferase expression in the liver was quantitated **(B).** The data represented the average of 4 mice and standard deviations.

**Fig. 27:** The kinetics of AAV vector clearance in blood after systemic administration of AAV9 incubated with fibrinogen. $2x10^{11}$ particles of AAV9/luc were incubated with 1mg of fibrinogen for 2 hr at 4°C and then administered into C57BL mice via retro-orbital injection. At day 2 post AAV injection, mice imaging was performed **(A)** and the quantitation of transgene luciferase expression in the liver was carried out **(B).** At indicated time points, mouse plasma was harvested and the AAV genome copy number was detected by quantitative-PCR **(C).** The data represented the average and standard deviations of 4 mice.

**Fig. 28:** Other serum proteins enhance AAV9 liver transduction. $1x10^{10}$ particles of AAV9/luc were incubated with different proteins at the dose of physiological concentration for 2 hr at 4°C and then injected into C57BL mice via retro-orbital vein. At day 3 post AAV injection, mouse imaging was performed **(A)** and the transgene luciferase expression in the liver was quantitated **(B).** The data represented the average of 5 mice and standard deviations.

**Fig. 29:** Other serum proteins enhance AAV9 brain transduction. The mice from Figure 28 were imaged at day 7 post AAV administration **(A)** and the transgene luciferase expression in the liver **(B)** and the brain was quantitated **(C).** After imaging, mice were sacrificed. The AAV genome copy number was detected in the liver **(D)** and the brain **(E).**

**Fig. 30:** The effect other serum proteins at different dilutions of physiological blood concentration on AAV9 transduction. $1x10^{10}$ particles of AAV9/luc were incubated other serum proteins at different dilutions for 2 hr at 4°C and then administered into C57BL mice via retro-orbital injection. At day 3 post AAV injection, mice were imaged **(A)** and the transgene luciferase expression in the liver was quantitated **(B).** The data represented the average and standard deviations from 5 mice.

**Fig. 31:** Enhancement of AAV9 transduction by interaction with cryoprecipitate. $1x10^{10}$ particles of AAV9/luc were incubated with different dilutions of cryoprecipitate for 2 hr at 4°C and then systemically administered into C57BL mice. At day 3 post AAV injection, mice were imaged **(A)** and the transgene luciferase expression in the liver was quantitated **(B).** The data represented the average of 5 mice and standard deviations.

**Fig. 32:** Effect of albumin interaction with AAV virions on neutralizing antibody A20 inhibition activity.

**Fig. 33:** The stability of HSA/AAV complex. **(A)** The stability of the complex in the different concentration of NaCl. **(B)** The stability of the complex in the different pH.

**Fig. 34:** The effect of $As_2O_3$ and proteasome inhibitors on AAV2 transduction. Balb/C mice received $1x10^{11}$ particles of AAV2/luc and 5mg $As_2O_3$/kg for 5 days **(A),** or 0.5 mg bortezomib/kg, 1 mg carfilzomib/kg at the same time **(B).** Transduction was assayed by live imaging at 7 days post AAV injection.

**Fig. 35:** HSA enhances AAV transduction. **(A)** The result of mass spectrometry analysis. **(B)** Interaction of AAV2 with human albumin. **(C)** Decreased AAV transduction with albumin depleted serum. **(D)** Recombinant human albumin enhances AAV2 transduction in Huh7 cells. **(E)** Enhanced AAV8 transduction from rHSA in Huh7 cells. **(F)** Enhanced liver AAV8 transduction from rHSA after systemic administration. Upper panel: imaging. Bottom panel: Data of enhanced AAV transduction after systemic injection. **(G)** Enhanced AAV8 transduction from rHSA after muscular injection. Upper panel: imaging. Face up: left leg-rHSA, right leg-PBS. Bottom panel: Data of enhanced AAV transduction after muscular injection.

**Fig. 36:** Capsid antigen presentation after AAVOVA transduction is dose responsive *in vivo.* Various doses of AAV2OVA/AAT vector were injected intravenously into C57BL/6 mice and 3 days later, CFSE-labeled OT-1 T cells were transferred. On day 10 after transfer, OT-1 T cell proliferation in the spleen was assessed via flow cytometry. (A) Representative flow cytometric histograms. **(B)** Average T cell proliferation and standard deviation of four mice. **(C)** Average proliferation index (PI) and standard deviation. $^{**}p<0.01$, $^{*}p<0.05$ compared with control mice without AAV treatment.

**Fig. 37:** The kinetics of capsid antigen presentation after AAV8OVA transduction in mice. Particles of AAVOVA/AAT virus ($1\times10^{11}$) were injected intravenously into C57BL/6 mice, and at the indicated time points, $5\times10^6$ CFSE-labeled OT-1 T cells were transferred. Ten days after transfer, proliferation of $CD8^+$ OT-1 T cells was measured by flow cytometry. **(A)** Average T cell proliferation and standard deviation for four mice. **(B)** Average proliferation index (PI) and standard deviation. $^{**}p<0.01$, $^{*}p<0.05$ compared with control mice without AAV treatment.

**Fig. 38:** Inhibition of OVA epitope presentation by VIPRs.

**Fig. 39:** Mutants isolated from mouse liver in the presence of IVIG.

**Fig. 40**: Inhibition of peptide on Nab activity. NAb assay was performed by incubation of predetermined dilution of A20 and plasma from AAV2 immunized C57/BL or Balb/C mice with peptides, then incubated with AAV2/GFP vector. After transduction on RC32 cells, the cells were harvested and applied for flow cytometry analysis.

**Fig. 41:** The effect of human IVIG on AAV8 liver transduction. 1 × 1010 particles of AAV8/luc vectors were incubated with different concentration of IVIG or PBS, then administered via retro-orbital injection in C57BL/6 mice. One week later, imaging was performed and analyzed for luciferase expression in the liver region. (a) The imaging of luciferase expression from mice (n=4). (b) Inhibition of AAV8 systemic transduction using human IVIG. Data represent the average of four mice and standard derivation.

## DETAILED DESCRIPTION OF THE INVENTION

The present invention will now be described with reference to the accompanying drawings, in which representative embodiments of the invention are shown. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention. All publications, patent applications, patents, accession numbers and other references mentioned herein are incorporated by reference herein in their entirety.

The designation of all amino acid positions in the AAV capsid proteins in the description of the invention and the appended claims is with respect to VP1 capsid subunit numbering (native AAV2 VP1 capsid protein: GenBank Accession No. AAC03780 or YP680426). It will be understood by those skilled in the art that the modifications described herein if inserted into the AAV *cap* gene may result in modifications in the VP1, VP2 and/or VP3 capsid subunits. Alternatively, the capsid subunits can be expressed independently to achieve modification in only one or two of the capsid subunits (VP1, VP2, VP3, VP1 + VP2, VP1 +VP3, or VP2 +VP3).

### Definitions

The following terms are used in the description herein and the appended claims:

The singular forms "a," "an" and "the" are intended to include the plural forms as well, unless the context clearly indicates otherwise.

Furthermore, the term "about," as used herein when referring to a measurable value such as an amount of the length of a polynucleotide or polypeptide sequence, dose, time, temperature, and the like, is meant to encompass variations of ± 20%, ± 10%, ± 5%, ± 1%, ± 0.5%, or even ± 0.1% of the specified amount.

Also as used herein, "and/or" refers to and encompasses any and all possible combinations of one or more of the associated listed items, as well as the lack of combinations when interpreted in the alternative ("or").

As used herein, the transitional phrase "consisting essentially of" means that the scope of a claim is to be interpreted to encompass the specified materials or steps recited in the claim, "and those that do not materially affect the basic and novel characteristic(s)" of the claimed invention. *See, In re Herz,* 537 F.2d 549, 551-52, 190 USPQ 461,463 (CCPA 1976) (emphasis in the original); *see also* MPEP § 2111.03. Thus, the term "consisting essentially of" when used in a claim of this invention is not intended to be interpreted to be equivalent to "comprising." Unless the context indicates otherwise, it is specifically intended that the various features of the invention described herein can be used in any combination.

Moreover, the present invention also contemplates that in some embodiments of the invention, any feature or combination of features set forth herein can be excluded or omitted.

To illustrate further, if, for example, the specification indicates that a particular amino acid can be selected from A, G, I, L and/or V, this language also indicates that the amino acid can be selected from any subset of these amino acid(s) for example A, G, I or L; A, G, I or V; A or G; only L; etc. as if each such subcombination is expressly set forth herein. Moreover, such language also indicates that one or more of the specified amino acids can be disclaimed (e.g., by negative proviso). For example, in particular embodiments the amino acid is not A, G or I; is not A; is not G or V; etc. as if each such possible disclaimer is expressly set forth herein.

As used herein, the terms "reduce," "reduces," "reduction" and similar terms mean a decrease of at least about 25%, 35%, 50%, 75%, 80%, 85%, 90%, 95%, 97% or more.

As used herein, the terms "enhance," "enhances," "enhancement" and similar terms indicate an increase of at least about 25%, 50%, 75%, 100%, 150%, 200%, 300%, 400%, 500% or more.

The term "parvovirus" as used herein encompasses the family *Parvoviridae,* including autonomously replicating parvoviruses and dependoviruses. The autonomous parvoviruses include members of the genera *Parvovirus, Erythrovirus, Densovirus, Iteravirus,* and *Contravirus.* Exemplary autonomous parvoviruses include, but are not limited to, minute virus of mouse, bovine parvovirus, canine parvovirus, chicken parvovirus, feline panleukopenia virus, feline parvovirus, goose parvovirus, H1 parvovirus, Muscovy duck parvovirus, B19 virus, and any other autonomous parvovirus now known or later discovered. Other autonomous parvoviruses are known to those skilled in the art. *See, e.g.,* BERNARD N. FIELDS et al., VIROLOGY, volume 2, chapter 69 (4th ed., Lippincott-Raven Publishers).

As used herein, the term "adeno-associated virus" (AAV), includes but is not limited to, AAV type 1, AAV type 2, AAV type 3 (including types 3A and 3B), AAV type 4, AAV type 5, AAV type 6, AAV type 7, AAV type 8, AAV type 9, AAV type 10, AAV type 11, avian AAV, bovine AAV, canine AAV, equine AAV, ovine AAV, and any other AAV now known or later discovered. *See, e.g.,* BERNARD N. FIELDS et al., VIROLOGY, volume 2, chapter 69 (4th ed., Lippincott-Raven Publishers). A number of relatively new AAV serotypes and clades have been identified *(see, e.g.,* Gao et al., (2004) J. Virology 78:6381-6388; Moris et al., (2004) Virology 33-:375- 383; and **Table 3).**

The genomic sequences of various serotypes of AAV and the autonomous parvoviruses, as well as the sequences of the native terminal repeats (TRs), Rep proteins, and capsid subunits are known in the art. Such sequences may be found in the literature or in public databases such as GenBank. *See, e.g.,* GenBank Accession Numbers NC_002077, NC_001401, NC_001729, NC_001863, NC_001829, NC_001862, NC_000883, NC_001701, NC_001510, NC_006152, NC_006261, AF063497, U89790, AF043303, AF028705, AF028704, J02275, J01901, J02275, X01457, AF288061, AH009962, AY028226, AY028223, NC_001358, NC_001540, AF513851, AF513852, AY530579; the disclosures of which are incorporated by reference herein for teaching parvovirus and AAV nucleic acid and amino acid sequences. *See also, e.g.,* Srivistava et al., (1983) J. Virology 45:555; Chiarini et al., (1998) J. Virology 71:6823; Chiarini et al., (1999) J. Virology 73:1309; Bantel-Schaal et al., (1999) J. Virology 73:939; Xiao et al., (1999) J. Virology 73:3994; Muramatsu et al., (1996) Virology 221:208; Shade et al., (1986) J. Virol. 58:921; Gao et al., (2002) Proc. Nat. Acad. Sci. USA 99:11854; Moris et al., (2004) Virology 33-:375-383; international patent publications WO 00/28061, WO 99/61601, WO 98/11244; and U.S. Patent No. 6,156,303; the disclosures of which are incorporated by reference herein for teaching parvovirus and AAV nucleic acid and amino acid sequences. *See also* **Table 1.**

The capsid structures of autonomous parvoviruses and AAV are described in more detail in BERNARD N. FIELDS et al., VIROLOGY, volume 2, chapters 69 & 70 (4th ed., Lippincott-Raven Publishers). *See also,* description of the crystal structure of AAV2 (Xie et al., (2002) Proc. Nat. Acad. Sci. 99:10405-10), AAV4 (Padron et al., (2005) J. Virol. 79: 5047-58), AAV5 (Walters et al., (2004) J. Virol. 78: 3361-71) and CPV (Xie et al., (1996) J. Mol. Biol. 6:497-520 and Tsao et al., (1991) Science 251: 1456-64).

The term "tropism" as used herein refers to preferential entry of the virus into certain cells or tissues, optionally followed by expression (e.g., transcription and, optionally, translation) of a sequence(s) carried by the viral genome in the cell, *e.g.,* for a recombinant virus, expression of a heterologous nucleic acid(s) of interest.

As used here, "systemic tropism" and "systemic transduction" (and equivalent terms) indicate that the virus capsid or virus vector of the invention exhibits tropism for and/or transduces tissues throughout the body (e.g., brain, lung, skeletal muscle, heart, liver, kidney and/or pancreas). In embodiments of the invention, systemic transduction of the central nervous system (e.g., brain, neuronal cells, etc.) is observed. In other embodiments, systemic transduction of cardiac muscle tissues is achieved.

As used herein, "selective tropism" or "specific tropism" means delivery of virus vectors to and/or specific transduction of certain target cells and/or certain tissues.

Unless indicated otherwise, "efficient transduction" or "efficient tropism," or similar terms, can be determined by reference to a suitable control (e.g., at least about 50%, 60%, 70%, 80%, 85%, 90%, 95%, 100%, 125%, 150%, 175%, 200%, 250%, 300%, 350%, 400%, 500% or more of the transduction or tropism, respectively, of the control). In particular embodiments, the virus vector efficiently transduces or has efficient tropism for neuronal cells and cardiomyocytes. Suitable controls will depend on a variety of factors including the desired tropism and/or transduction profile.

Similarly, it can be determined if a virus "does not efficiently transduce" or "does not have efficient tropism" for a target tissue, or similar terms, by reference to a suitable control. In particular embodiments, the virus vector does not efficiently transduce *(i.e.,* has does not have efficient tropism) for liver, kidney, gonads and/or germ cells. In particular embodiments, transduction (e.g., undesirable transduction) of tissue(s) (e.g., liver) is 20% or less, 10% or less, 5% or less, 1% or less, 0.1% or less of the level of transduction of the desired target tissue(s) (e.g., skeletal muscle, diaphragm muscle, cardiac muscle and/or cells of the central nervous system).

In some embodiments of this invention, an AAV particle comprising a capsid of this invention can demonstrate multiple phenotypes of efficient transduction of certain tissues/cells and very low levels of transduction (e.g., reduced transduction) for certain tissues/cells, the transduction of which is not desirable.

As used herein, the term "polypeptide" encompasses both peptides and proteins, unless indicated otherwise.

A "polynucleotide" is a sequence of nucleotide bases, and may be RNA, DNA or DNA-RNA hybrid sequences (including both naturally occurring and non-naturally occurring nucleotides), but in representative embodiments are either single or double stranded DNA sequences.

As used herein, an "isolated" polynucleotide (*e.g.*, an "isolated DNA" or an "isolated RNA") means a polynucleotide at least partially separated from at least some of the other components of the naturally occurring organism or virus, for example, the cell or viral structural components or other polypeptides or nucleic acids commonly found associated with the polynucleotide. In representative embodiments an "isolated" nucleotide is enriched by at least about 10-fold, 100-fold, 1000-fold, 10,000-fold or more as compared with the starting material.

Likewise, an "isolated" polypeptide means a polypeptide that is at least partially separated from at least some of the other components of the naturally occurring organism or virus, for example, the cell or viral structural components or other polypeptides or nucleic acids commonly found associated with the polypeptide. In representative embodiments an "isolated" polypeptide is enriched by at least about 10-fold, 100-fold, 1000-fold, 10,000-fold or more as compared with the starting material.

An "isolated cell" refers to a cell that is separated from other components with which it is normally associated in its natural state. For example, an isolated cell can be a cell in culture medium and/or a cell in a pharmaceutically acceptable carrier of this invention. Thus, an isolated cell can be delivered to and/or introduced into a subject. In some embodiments, an isolated cell can be a cell that is removed from a subject and manipulated as described herein *ex vivo* and then returned to the subject.

As used herein, by "isolate" or "purify" (or grammatical equivalents) a virus vector or virus particle or population of virus particles, it is meant that the virus vector or virus particle or population of virus particles is at least partially separated from at least some of the other components in the starting material. In representative embodiments an "isolated" or "purified" virus vector or virus particle or population of virus particles is enriched by at least about 10-fold, 100-fold, 1000-fold, 10,000-fold or more as compared with the starting material.

A "therapeutic polypeptide" is a polypeptide that can alleviate, reduce, prevent, delay and/or stabilize symptoms that result from an absence or defect in a protein in a cell or subject and/or is a polypeptide that otherwise confers a benefit to a subject, *e.g.,* anti-cancer effects or improvement in transplant survivability or induction of an immune response.

By the terms "treat," "treating," or "treatment of" (and grammatical variations thereof) it is meant that the severity of the subject's condition is reduced, at least partially improved or stabilized and/or that some alleviation, mitigation, decrease or stabilization in at least one clinical symptom is achieved and/or there is a delay in the progression of the disease or disorder.

The terms "prevent," "preventing" and "prevention" (and grammatical variations thereof) refer to prevention and/or delay of the onset of a disease, disorder and/or a clinical symptom(s) in a subject and/or a reduction in the severity of the onset of the disease, disorder and/or clinical symptom(s) relative to what would occur in the absence of the methods of the invention. The prevention can be complete, *e.g.,* the total absence of the disease, disorder and/or clinical symptom(s). The prevention can also be partial, such that the occurrence of the disease, disorder and/or clinical symptom(s) in the subject and/or the severity of onset is substantially less than what would occur in the absence of the present invention.

A "treatment effective" amount as used herein is an amount that is sufficient to provide some improvement or benefit to the subject. Alternatively stated, a "treatment effective" amount is an amount that will provide some alleviation, mitigation, decrease or stabilization in at least one clinical symptom in the subject. Those skilled in the art will appreciate that the therapeutic effects need not be complete or curative, as long as some benefit is provided to the subject.

A "prevention effective" amount as used herein is an amount that is sufficient to prevent and/or delay the onset of a disease, disorder and/or clinical symptoms in a subject and/or to reduce and/or delay the severity of the onset of a disease, disorder and/or clinical symptoms in a subject relative to what would occur in the absence of the methods of the invention. Those skilled in the art will appreciate that the level of prevention need not be complete, as long as some preventative benefit is provided to the subject.

The terms "heterologous nucleotide sequence" and "heterologous nucleic acid molecule" are used interchangeably herein and refer to a nucleic acid sequence that is not naturally occurring in the virus. Generally, the heterologous nucleic acid molecule or heterologous nucleotide sequence comprises an open reading frame that encodes a polypeptide and/or nontranslated RNA of interest (*e.g.*, for delivery to a cell and/or subject).

As used herein, the terms "virus vector," "vector" or "gene delivery vector" refer to a virus (*e.g.*, AAV) particle that functions as a nucleic acid delivery vehicle, and which comprises the vector genome (e.g., viral DNA [vDNA]) packaged within a virion. Alternatively, in some contexts, the term "vector" may be used to refer to the vector genome/vDNA alone.

A "rAAV vector genome" or "rAAV genome" is an AAV genome (*i.e.,* vDNA) that comprises one or more heterologous nucleic acid sequences. rAAV vectors generally require only the terminal repeat(s) (TR(s)) in *cis* to generate virus. All other viral sequences are dispensable and may be supplied in *trans* (Muzyczka, (1992) Curr. Topics Microbiol. Immunol. 158:97). Typically, the rAAV vector genome will only retain the one or more TR sequence so as to maximize the size of the transgene that can be efficiently packaged by the vector. The structural and non-structural protein coding sequences may be provided in *trans* (*e.g.,* from a vector, such as a plasmid, or by stably integrating the sequences into a packaging cell). In embodiments of the invention the rAAV vector genome comprises at least one TR sequence *(e.g.,* AAV TR sequence), optionally two TRs *(e.g.,* two AAV TRs), which typically will be at the 5' and 3' ends of the vector genome and flank the heterologous nucleic acid, but need not be contiguous thereto. The TRs can be the same or different from each other.

The term "terminal repeat" or "TR" includes any viral terminal repeat or synthetic sequence that forms a hairpin structure and functions as an inverted terminal repeat (*i.e*., mediates the desired functions such as replication, virus packaging, integration and/or provirus rescue, and the like). The TR can be an AAV TR or a non-AAV TR. For example, a non-AAV TR sequence such as those of other parvoviruses (*e.g*., canine parvovirus (CPV), mouse parvovirus (MVM), human parvovirus B-19) or any other suitable virus sequence (*e.g*., the SV40 hairpin that serves as the origin of SV40 replication) can be used as a TR, which can further be modified by truncation, substitution, deletion, insertion and/or addition. Further, the TR can be partially or completely synthetic, such as the "double-D sequence" as described in United States Patent No. 5,478,745 to Samulski et al.

An "AAV terminal repeat" or "AAV TR" may be from any AAV, including but not limited to serotypes 1, 2, 3, 4, 5, 6, 7, 8, 9, 10, 11 or 12 or any other AAV now known or later discovered *(see, e.g.,* **Table 1).** An AAV terminal repeat need not have the native terminal repeat sequence (e.g., a native AAV TR sequence may be altered by insertion, deletion, truncation and/or missense mutations), as long as the terminal repeat mediates the desired functions, *e.g*., replication, virus packaging, integration, and/or provirus rescue, and the like.

AAV proteins VP1, VP2 and VP3 are capsid proteins that interact together to form an AAV capsid of an icosahedral symmetry. VP1.5 is an AAV capsid protein described in US Publication No. 2014/0037585.

The virus vectors of the invention can further be "targeted" virus vectors *(e.g.,* having a directed tropism) and/or a "hybrid" parvovirus *(i.e.,* in which the viral TRs and viral capsid are from different parvoviruses) as described in international patent publication WO 00/28004 and Chao et al., (2000) Molecular Therapy 2:619.

The virus vectors of the invention can further be duplexed parvovirus particles as described in international patent publication WO 01/92551 (the disclosure of which is incorporated herein by reference in its entirety). Thus, in some embodiments, double stranded (duplex) genomes can be packaged into the virus capsids of the invention.

Further, the viral capsid or genomic elements can contain other modifications, including insertions, deletions and/or substitutions.

A "chimeric" capsid protein as used herein means an AAV capsid protein that has been modified by substitutions in one or more *(e.g.,* 2, 3, 4, 5, 6, 7, 8, 9, 10, etc.) amino acid residues in the amino acid sequence of the capsid protein relative to wild type, as well as insertions and/or deletions of one or more *(e.g.,* 2, 3, 4, 5, 6, 7, 8, 9, 10, etc.) amino acid residues in the amino acid sequence relative to wild type. In some embodiments, complete or partial domains, functional regions, epitopes, etc., from one AAV serotype can replace the corresponding wild type domain, functional region, epitope, etc. of a different AAV serotype, in any combination, to produce a chimeric capsid protein of this invention. Production of a chimeric capsid protein can be carried out according to protocols well known in the art and a large number of chimeric capsid proteins are described in the literature as well as herein that can be included in the capsid of this invention.

As used herein, the term "amino acid" encompasses any naturally occurring amino acid, modified forms thereof, and synthetic amino acids.

Naturally occurring, levorotatory (L-) amino acids are shown in **Table 2.**

Alternatively, the amino acid can be a modified amino acid residue (nonlimiting examples are shown in **Table 4)** and/or can be an amino acid that is modified by post-translation modification (*e.g*., acetylation, amidation, formylation, hydroxylation, methylation, phosphorylation or sulfatation).

Further, the non-naturally occurring amino acid can be an "unnatural" amino acid as described by Wang et al., Annu Rev Biophys Biomol Struct. 35:225-49 (2006). These unnatural amino acids can advantageously be used to chemically link molecules of interest to the AAV capsid protein.

As used herein, the term "homologous recombination" means a type of genetic recombination in which nucleotide sequences are exchanged between two similar or identical molecules of DNA. Homologous recombination also produces new combinations of DNA sequences. These new combinations of DNA represent genetic variation. Homologous recombination is also used in horizontal gene transfer to exchange genetic material between different strains and species of viruses.

As used herein, the term "gene editing," "Genome editing," or "genome engineering" means a type of genetic engineering in which DNA is inserted, deleted or replaced in the genome of a living organism using engineered nucleases, or "molecular scissors." These nucleases create site-specific double-strand breaks (DSBs) at desired locations in the genome.

As used herein, the term "gene delivery" means a process by which foreign DNA is transferred to host cells for applications of gene therapy.

As used herein, the term "CRISPR" stands for Clustered Regularly Interspaced Short Palindromic Repeats, which are the hallmark of a bacterial defense system that forms the basis for CRISPR-Cas9 genome editing technology.

As used herein, the term "zinc finger" means a small protein structural motif that is characterized by the coordination of one or more zinc ions, in order to stabilize the fold.

Modified AAV Capsid Proteins and Virus Capsids and Virus Vectors with Surface Bound Protein for Enhanced Transduction and Reduced Antigenicity

The present invention is based on the unexpected discovery that AAV virions with protein bound to the surface have enhanced transduction properties and/or reduced antigenicity. Thus, in one embodiment, the present invention provides an adeno-associated virus (AAV) particle comprising a surface-bound protein, wherein the protein bound to the surface of the AAV particle is selected from the group consisting of: (a) fibrinogen alpha chain; (b) fibrinogen beta chain; (c) fibrinogen gamma chain; (d) fibronectin; (e) plasminogen; (f) von Willebrand factor; (g) alpha-1-acid glycoprotein; (h) platelet factor 4; (i) cryoprecipitate; (j) factor VIII; (k) factor XIII; (1) albumin (e.g., human serum albumin, and/or albumin from any other species such as dog, horse, cow, pig, etc.); (m) apolipoprotein B (ApoB); (n) apolipoprotein E (ApoE); (o) transferrin; (p) low density lipoprotein; (q) any fusion serum protein that increases AAV binding on the cell surface or enhances AAV intracellular trafficking; and (r) any combination of (a)-(q) above.

The binding of serum proteins to AAV particle is dependent on the concentration of salt concentration and pH, as exemplified in the Examples section provided herein.

The AAV particle of this invention can be an AAV of a serotype or any combination of serotypes listed in **Table 10.**

In some embodiments, the AAV particle of this invention can be, singly or in any combination, AAV8, AAV9, AAV2, AAV2i8, AAV9.45, or any AAV mutant or variant described herein, now known or later identified.

In some embodiments of the AAV particle of this invention, the protein bound to the surface of the AAV particle can be present on the AAV particle surface in an amount in a range from about 2000 protein molecules per AAV particle to about 4 X $10^7$ protein molecules per AAV particle (*e.g.*, 2000, 3000, 4000, 5000, 6000, 7000, 8000, 9000, 10,000, 11,000, 12,000, 13,000, 14,000, 15,000, 16,000, 17,000, 18,000, 19,000, 20,000, 21,000, 22,000, 23,000, 24,000, 25,000, 26,000, 27,000, 28,000, 29,000, 30,000, 20,000, 21,000, 22,000, 23,000, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 60,000, 70,000, 80,000, 90,000, 1 X $10^6$, 2 X $10^6$, 3 X $10^6$, 4 X $10^6$, 5X $10^6$, 6 X $10^6$, 7 X $10^6$, 8 X $10^6$, 9 X $10^6$, 1 X $10^7$, 2 X $10^7$, 3 X $10^7$, or 4 X $10^7$, including any number in between 2000 and 4 X $10^7$ not explicitly set forth herein). The number of protein molecules per AAV particle can be determined according to protocols known in the art and as exemplified in the Examples section herein.

In some embodiments, the AAV particle comprising the surface-bound protein has enhanced transduction activity and/or reduced antigenicity relative to an AAV particle lacking the surface-bound protein. Accordingly, the number of protein molecules attached to the AAV particle can be an amount that enhances transduction activity or reduces antigenicity of the AAV particle relative to an AAV particle lacking the surface-bound protein.

In some embodiments, the AAV particle of this invention can comprise a heterologous nucleic acid molecule.

In some embodiments, the AAV particle of this invention can be synthetic viral vector designed to display a range of desirable phenotypes that are suitable for different *in vitro* and *in vivo* applications. Thus, in one embodiment, the present invention provides an AAV particle comprising an adeno-associated virus (AAV).

The present invention provides an array of synthetic viral vectors displaying a range of desirable phenotypes that are suitable for different *in vitro* and *in vivo* applications. In particular, the present invention is based on the unexpected discovery that combining capsid proteins from different AAV serotypes in an individual capsid allows for the development of improved AAV capsids that have multiple desirable phenotypes in each individual capsid. For example, triploid AAV2/8/9 vector described herein, which is produced by co-transfection of AAV helper plasmids from serotypes 2, 8 and 9, has a much higher mouse liver transduction than AAV2, similar to AAV8. Importantly, triploid AAV2/8/9 vector has an improved ability to escape neutralizing antibodies from sera immunized with parental serotypes. Although AAV3 is less efficient in transducing the whole mouse body after systemic administration, the haploid vectors H-AAV83 or H-AAV93 or H-rh10-3 described herein, in which VP3 is from AAV3 and VP1/VP2 from AAV8, 9 or rh10, induce whole body transduction, as well as much higher transduction in the liver and other tissues, compared to AAV3.

Thus, in one embodiment, the present invention provides an adeno-associated virus (AAV) capsid, wherein the capsid comprises capsid protein VP1, wherein said capsid protein VP1 is from one or more than one first AAV serotype and capsid protein VP3, wherein said capsid protein VP3 is from one or more than one second AAV serotype and wherein at least one of said first AAV serotype is different from at least one of said second AAV serotype, in any combination.

In some embodiments, the capsid of this invention comprises capsid protein VP2, wherein said capsid protein VP2 is from one or more than one third AAV serotype, wherein at least one of said one or more than one third AAV serotype is different from said first AAV serotype and/or said second AAV serotype, in any combination. In some embodiments, the AAV capsid described herein can comprise capsid protein VP1.5. VP1.5 is described in U.S. Patent Publication No. 2014/0037585 and the amino acid sequence of VP1.5 is provided herein.

In some embodiments, the capsid of this invention comprises capsid protein VP1.5, wherein said capsid protein VP1.5 is from one or more than one fourth AAV serotype, wherein at least one of said one or more than one fourth AAV serotype is different from said first AAV serotype and/or said second AAV serotype, in any combination. In some embodiments, the AAV capsid protein described herein can comprise capsid protein VP2.

The present invention also provides an AAV capsid wherein the capsid comprises capsid protein VP1, wherein said capsid protein VP1 is from one or more than one first AAV serotype and capsid protein VP2, wherein said capsid protein VP2 is from one or more than one second AAV serotype and wherein at least one of said first AAV serotype is different from at least one of said second AAV serotype, in any combination.

In some embodiments, the AAV particle of this invention can comprise a capsid that comprises capsid protein VP3, wherein said capsid protein VP3 is from one or more than one third AAV serotype, wherein at least one of said one or more than one third AAV serotype is different from said first AAV serotype and/or said second AAV serotype, in any combination. In some embodiments, the AAV capsid described herein can comprise capsid protein VP1.5.

The present invention further provides an AAV particle that comprises an adeno-associated virus (AAV) capsid, wherein the capsid comprises capsid protein VP1, wherein said capsid protein VP1 is from one or more than one first AAV serotype and capsid protein VP1.5, wherein said capsid protein VP1.5 is from one or more than one second AAV serotype and wherein at least one of said first AAV serotype is different from at least one of said second AAV serotype, in any combination.

In some embodiments, the capsid comprises capsid protein VP3, wherein said capsid protein VP3 is from one or more than one third AAV serotype, wherein at least one of said one or more than one third AAV serotype is different from said first AAV serotype and/or said second AAV serotype, in any combination. In some embodiments, the AAV capsid described herein can comprise capsid protein VP1.5.

The present invention further provides an adeno-associated virus (AAV) capsid, wherein the capsid comprises capsid protein VP1, wherein said capsid protein VP1 is from one or more than one first AAV serotype and capsid protein VP1.5, wherein said capsid protein VP1.5 is from one or more than one second AAV serotype and wherein at least one of said first AAV serotype is different from at least one of said second AAV serotype, in any combination.

In some embodiments, the AAV capsid of this invention comprises capsid protein VP3, wherein said capsid protein VP3 is from one or more than one third AAV serotype, wherein at least one of said one or more than one third AAV serotype is different from said first AAV serotype and/or said second AAV serotype, in any combination. In some embodiments, the AAV capsid protein described herein can comprise capsid protein VP2.

In some embodiments of the capsid of this invention, said one or more than one first AAV serotype, said one or more than one second AAV serotype, said one or more than one third AAV serotype and said one or more than one fourth AAV serotype are selected from the group consisting of the AAV serotypes listed in **Table 5,** in any combination.

In some embodiments of this invention, the AAV capsid described herein lacks capsid protein VP2.

In some embodiments of the capsid of this invention comprises a chimeric capsid VP1 protein, a chimeric capsid VP2 protein, a chimeric capsid VP3 protein and/or a chimeric capsid VP1.5 protein.

In some embodiments, the AAV capsid of this invention can be AAV AAV2/8/9, H-AAV82, H-AAV92, H-AAV82G9, AAV2/8 3:1, AAV2/8 1:1, AAV2/8 1:3, or AAV8/9, all of which are described in the EXAMPLES section provided herein.

Nonlimiting examples of AAV capsid proteins that can be included in the capsid of this invention in any combination with other capsid proteins described herein and/or with other capsid proteins now known or later developed, include LK3, LK01-19, AAV-DJ, Olig001, rAAV2-retro, AAV-LiC, AAV0Keral, AAV-Kera2, AAV-Kera3, AAV 7m8, AAV1,9, AAVr3.45, AAV clone 32, AAV clone 83, AAV-U87R7-C5, AAV ShH13, AAV ShH19, AAV L1-12, AAV HAE-1, AAV HAE-2, AAV variant ShH10, AAV2.5T, AAV LS1-4, AAV Lsm, AAV1289, AAVHSC 1-17, AAV2 Rec 1-4, AAV8BP2, AAV-B1, AAV-PHP.B, AAV9.45, AAV9.61, AAV9.47, AAVM41, AAV2 displayed peptides, AAV2-GMN, AAV9-peptide displayed, AAV8 and AAV9 peptide displayed, AAVpo2.1, AAVpo4, AAVpo5, AAVpo6, AAV rh, AAV Hu, AAV-Go.1, AAV-mo.1, BAAV, AAAV, AAV8 K137R, AAV Anc80L65, AAV2G9, AAV2 265 insertion-AAV2/265D, AAV2.5, AAV3 SASTG, AAV2i8, AAV8G9, AAV2 tyrosine mutants AAV2 Y-F, AAV8 Y-F, AAV9 Y-F, AAV6 Y-F, AAV6.2 and any combination thereof.

As a nonlimiting example, the AAV capsid proteins and virus capsids of this invention can be chimeric in that they can comprise all or a portion of a capsid subunit from another virus, optionally another parvovirus or AAV, *e.g.,* as described in international patent publication WO 00/28004.

The following publications describe chimeric or variant capsid proteins that can be incorporated into the AAV capsid of this invention in any combination with wild type capsid proteins and/or other chimeric or variant capsid proteins now known or later identified.

L Lisowski, AP Dane, K Chu, Y Zhang, SC Cunninghamm, EM Wilson, et al. Selection and evaluation of clinically relevant AAV variants in a xenograft liver model. Nature, 506 (2014), pp. 382-386 **(LK03** and others **LK01-19).**

Grimm D, Lee JS, Wang L, Desai T, Akache B, Storm TA, Kay MA. In vitro and in vivo gene therapy vector evolution via multispecies interbreeding and retargeting of adeno-associated viruses. J. Virol. 2008 Jun: 82(12):5887-911. **(AAV-DJ).**

Powell SK, Khan N, Parker CL, Samulski RJ, Matsushima G, Gray SJ, McCown TJ. Characterization of a novel adeno-associated viral vector with preferential oligodendrocyte tropism. Gene Ther. 2016 Nov: 23(11):807-814. **(Olig001).**

Tervo DG, Hwang BY, Viswanathan S, Gaj T, Lavzin M, Ritola KD, Lindo S, Michael S, Kuleshova E, Ojala D, Huang CC, Gerfen CR, Schiller J, Dudman JT, Hantman AW, Looger LL, Schaffer DV, Karpova AY. A Designer AAV Variant Permits Efficient Retrograde Access to Projection Neurons. Neuron. 2016 Oct 19: 92(2):372-382. **(rAAV2-retro).**

Marsic D, Govindasamy L, Currlin S, Markusic DM, Tseng YS, Herzog RW, Agbandje-McKenna M, Zolotukhin S. Vector design Tour de Force: integrating combinatorial and rational approaches to derive novel adeno-associated virus variants. Mol Ther. 2014 Nov: 22(11):1900-9. **(AAV-LiC).**

Sallach J, Di Pasquale G, Larcher F, Niehoff N, Rübsam M, Huber A, Chiorini J, Almarza D, Eming SA, Ulus H, Nishimura S, Hacker UT, Hallek M, Niessen CM, Büning H. Tropism-modified AAV vectors overcome barriers to successful cutaneous therapy. Mol Ther. 2014 May: 22(5):929-39. **(AAV-Kera1, AAV-Kera2,** and **AAV-Kera3).**

Dalkara D, Byrne LC, Klimczak RR, Visel M, Yin L, Merigan WH, Flannery JG, Schaffer DV. In vivo-directed evolution of a new adeno-associated virus for therapeutic outer retinal gene delivery from the vitreous. Sci Transl Med. 2013 Jun 12: 5(189):189ra76. **(AAV 7m8).**

Asuri P, Bartel MA, Vazin T, Jang JH, Wong TB, Schaffer DV. Directed evolution of adeno-associated virus for enhanced gene delivery and gene targeting in human pluripotent stem cells. Mol Ther. 2012 Feb: 20(2):329-38. **(AAV1.9).**

Jang JH, Koerber JT, Kim JS, Asuri P, Vazin T, Bartel M, Keung A, Kwon I, Park KI, Schaffer DV. An evolved adeno-associated viral variant enhances gene delivery and gene targeting in neural stem cells. Mol Ther. 2011 Apr: 19(4):667-75. doi: 10.1038/mt.2010.287. **(AAV r3.45).**

Gray SJ, Blake BL, Criswell HE, Nicolson SC, Samulski RJ, McCown TJ, Li W. Directed evolution of a novel adeno-associated virus (AAV) vector that crosses the seizure-compromised blood-brain barrier (BBB). Mol Ther. 2010 Mar: 18(3):570-8. **(AAV clone 32 and 83).**

Maguire CA, Gianni D, Meijer DH, Shaket LA, Wakimoto H, Rabkin SD, Gao G, Sena-Esteves M. Directed evolution of adeno-associated virus for glioma cell transduction. J. Neurooncol. 2010 Feb: 96(3):337-47. **(AAV-U87R7-C5).**

Koerber JT, Klimczak R, Jang JH, Dalkara D, Flannery JG, Schaffer DV. Molecular evolution of adeno-associated virus for enhanced glial gene delivery. Mol Ther. 2009 Dec: 17(12):2088-95. **(AAV ShH13, AAV ShH19, AAV L1-12)**

Li W, Zhang L, Johnson JS, Zhijian W, Grieger JC, Ping-Jie X, Drouin LM, Agbandje-McKenna M, Pickles RJ, Samulski RJ. Generation of novel AAV variants by directed evolution for improved CFTR delivery to human ciliated airway epithelium. Mol Ther. 2009 Dec: 17(12):2067-77. **(AAV HAE-1, AAV HAE-2).**

Klimczak RR, Koerber JT, Dalkara D, Flannery JG, Schaffer DV. A novel adeno- associated viral variant for efficient and selective intravitreal transduction of rat Müller cells. PLoS One. 2009 Oct 14: 4(10):e7467. **(AAV variant ShH10).**

Excoffon KJ, Koerber JT, Dickey DD, Murtha M, Keshavjee S, Kaspar BK, Zabner J, Schaffer DV. Directed evolution of adeno-associated virus to an infectious respiratory virus. Proc Natl Acad Sci USA. 2009 Mar 10: 106(10):3865-70. **(AAV2.5T).**

Sellner L, Stiefelhagen M, Kleinschmidt JA, Laufs S, Wenz F, Fruehauf S, Zeller WJ, Veldwijk MR. Generation of efficient human blood progenitor-targeted recombinant adeno-associated viral vectors (AAV) by applying an AAV random peptide library on primary human hematopoietic progenitor cells. Exp Hematol. 2008 Aug: 36(8):957-64. **(AAV LS1-4, AAV Lsm).**

Li W, Asokan A, Wu Z, Van Dyke T, DiPrimio N, Johnson JS, Govindaswamy L, Agbandje-McKenna M, Leichtle S, Redmond DE Jr, McCown TJ, Petermann KB, Sharpless NE, Samulski RJ. Engineering and selection of shuffled AAV genomes: a new strategy for producing targeted biological nanoparticles. Mol Ther. 2008 Jul: 16(7):1252-60. **(AAV1289).**

Charbel Issa P, De Silva SR, Lipinski DM, Singh MS, Mouravlev A, You Q. Assessment of tropism and effectiveness of new primate-derived hybrid recombinant AAV serotypes in the mouse and primate retina. PLoS ONE. 2013: 8:e60361. **(AAVHSC 1-17).**

Huang W, McMurphy T, Liu X, Wang C, Cao L. Genetic Manipulation of Brown Fat Via Oral Administration of an Engineered Recombinant Adeno-associated Viral Serotype Vector. Mol. Ther. 2016 Jun: 24(6):1062-9. **(AAV2 Rec 1-4).**

Cronin T, Vandenberghe LH, Hantz P, et al. Efficient transduction and optogenetic stimulation of retinal bipolar cells by a synthetic adeno-associated virus capsid and promoter. EMBO Mol. Med. 2014: 6:1175-1190. **(AAV8BP2).**

Choudhury SR, Fitzpatrick Z, Harris AF, Maitland SA, Ferreira JS, Zhang Y, Ma S, Sharma RB, Gray-Edwards HL, Johnson JA, Johnson AK, Alonso LC, Punzo C, Wagner KR, Maguire CA, Kotin RM, Martin DR, Sena-Esteves M. In Vivo Selection Yields AAV-B1 Capsid for Central Nervous System and Muscle Gene Therapy. Mol Ther. 2016 Aug: 24(7):1247-57. **(AAV-B1).**

Deverman BE, Pravdo PL, Simpson BP, Kumar SR, Chan KY, Banerjee A, Wu WL, Yang B, Huber N, Pasca SP, Gradinaru V. Cre-dependent selection yields AAV variants for widespread gene transfer to the adult brain. Nat Biotechnol. 2016 Feb: 34(2):204-9. doi: 10.1038/nbt.3440. **(AAV-PHP.B).**

Pulicherla N, Shen S, Yadav S, Debbink K, Govindasamy L, Agbandje-McKenna M, Asokan A. Engineering liver-detargeted AAV9 vectors for cardiac and musculoskeletal gene transfer. Mol Ther. 2011 Jun: 19(6):1070-8. (AAV9 derived **mutants-AAV9.45, AAV9.61,** and **AAV9.47).**

Yang L, Jiang J, Drouin LM, Agbandje-McKenna M, Chen C, Qiao C, Pu D, Hu X, Wang DZ, Li J, Xiao X. A myocardium tropic adeno-associated virus (AAV) evolved by DNA shuffling and in vivo selection. Proc Natl Acad Sci USA. 2009 Mar 10: 106(10):3946-51. **(AAVM41).**

Körbelin J, Sieber T, Michelfelder S, Lunding L, Spies E, Hunger A, Alawi M, Rapti K, Indenbirken D, Müller OJ, Pasqualini R, Arap W, Kleinschmidt JA, Trepel M. Pulmonary Targeting of Adeno-associated Viral Vectors by Next-generation Sequencing-guided Screening of Random Capsid Displayed Peptide Libraries. Mol Ther. 2016 Jun: 24(6):1050-61. **(AAV2 displayed peptides).**

Geoghegan JC, Keiser NW, Okulist A, Martins I, Wilson MS, Davidson BL. Chondroitin Sulfate is the Primary Receptor for a Peptide-Modified AAV That Targets Brain Vascular Endothelium In Vivo. Mol Ther Nucleic Acids. 2014 Oct 14: 3:e202. **(AAV2-GMN).**

Varadi K, Michelfelder S, Korff T, Hecker M, Trepel M, Katus HA, Kleinschmidt JA, Müller OJ. Novel random peptide libraries displayed on AAV serotype 9 for selection of endothelial cell-directed gene transfer vectors. Gene Ther. 2012 Aug: 19(8):800-9. **(AAV9-peptide displayed).**

Michelfelder S, Varadi K, Raupp C, Hunger A, Körbelin J, Pahrmann C, Schrepfer S, Müller OJ, Kleinschmidt JA, Trepel M. Peptide ligands incorporated into the threefold spike capsid domain to re-direct gene transduction of AAV8 and AAV9 in vivo. PLoS One. 2011: 6(8):e23101. **(AAV8 and AAV9 peptide displayed).**

Yu CY, Yuan Z, Cao Z, Wang B, Qiao C, Li J, Xiao X. A muscle-targeting peptide displayed on AAV2 improves muscle tropism on systemic delivery. Gene Ther. 2009 Aug: 16(8):953-62.

Michelfelder S, Lee MK, deLima-Ilahn E, Wilmes T, Kaul F, Müller O, Kleinschmidt JA, Trepel M. Vectors selected from adeno-associated viral display peptide libraries for leukemia cell-targeted cytotoxic gene therapy. Exp Hematol. 2007 Dec: 35(12): 1766-76.

Müller OJ, Kaul F, Weitzman MD, Pasqualini R, Arap W, Kleinschmidt JA, Trepel M. Random peptide libraries displayed on adeno-associated virus to select for targeted gene therapy vectors. Nat Biotechnol. 2003 Sep: 21(9):1040-6.

Grifman M, Trepel M, Speece P, Gilbert LB, Arap W, Pasqualini R, Weitzman MD. Incorporation of tumor-targeting peptides into recombinant adeno-associated virus capsids. Mol Ther. 2001 Jun: 3(6):964-75.

Anne Girod, Martin Ried, Christiane Wobus, Harald Lahm, Kristin Leike, Jürgen Kleinschmidt, Gilbert Deléage and Michael Hallek. Genetic capsid modifications allow efficient re-targeting of adeno-associated virus type 2. Nature Medicine, 1052-1056 (1999).

Bello A, Chand A, Aviles J, Soule G, Auricchio A, Kobinger GP. Novel adeno- associated viruses derived from pig tissues transduce most major organs in mice. Sci Rep. 2014 Oct 22: 4:6644. **(AAVpo2.1, -po4, -po5, and -po6).**

Gao G, Vandenberghe LH, Alvira MR, Lu Y, Calcedo R, Zhou X, Wilson JM. Clades of Adeno-associated viruses are widely disseminated in human tissues. J. Virol. 2004 Jun: 78(12):6381-8. **(AAV rh and AAV Hu).**

Arbetman AE, Lochrie M, Zhou S, Wellman J, Scallan C, Doroudchi MM, et al. Novel caprine adeno-associated virus (AAV) capsid (AAV-Go.1) is closely related to the primate AAV-5 and has unique tropism and neutralization properties. J. Virol. 2005: 79:15238-15245. **(AAV-Go.1).**

Lochrie MA, Tatsuno GP, Arbetman AE, Jones K, Pater C, Smith PH, et al. Adeno-associated virus (AAV) capsid genes isolated from rat and mouse liver genomic DNA define two new AAV species distantly related to AAV-5. Virology. 2006: 353:68-82. **(AAV-mo.1).**

Schmidt M, Katano H, Bossis I, Chiorini JA. Cloning and characterization of a bovine adeno-associated virus. J. Virol. 2004: 78:6509-6516. (**BAAV**).

Bossis I, Chiorini JA. Cloning of an avian adeno-associated virus (AAAV) and generation of recombinant AAAV particles. J. Virol. 2003: 77:6799-6810. (**AAAV**).

Chen CL, Jensen RL, Schnepp BC, Connell MJ, Shell R, Sferra TJ, Bartlett JS, Clark KR, Johnson PR. Molecular characterization of adeno-associated viruses infecting children. J. Virol. 2005 Dec: 79(23):14781-92. **(AAV variants).**

Sen D, Gadkari RA, Sudha G, Gabriel N, Kumar YS, Selot R, Samuel R, Rajalingam S, Ramya V, Nair SC, Srinivasan N, Srivastava A, Jayandharan GR. Targeted modifications in adeno-associated virus serotype 8 capsid improves its hepatic gene transfer efficiency in vivo. Hum Gene Ther Methods. 2013 Apr: 24(2):104-16. **(AAV8 K137R).**

Li B, Ma W, Ling C, Van Vliet K, Huang LY, Agbandje-McKenna M, Srivastava A, Aslanidi GV. Site-Directed Mutagenesis of Surface-Exposed Lysine Residues Leads to Improved Transduction by AAV2, But Not AAV8, Vectors in Murine Hepatocytes In Vivo. Hum Gene Ther Methods. 2015 Dec: 26(6):211-20.

Gabriel N, Hareendran S, Sen D, Gadkari RA, Sudha G, Selot R, Hussain M, Dhaksnamoorthy R, Samuel R, Srinivasan N, et al. Bioengineering of AAV2 capsid at specific serine, threonine, or lysine residues improves its transduction efficiency in vitro and in vivo. Hum Gene Ther Methods. 2013 Apr: 24(2):80-93.

Zinn E, Pacouret S, Khaychuk V, Turunen HT, Carvalho LS, Andres-Mateos E, Shah S, Shelke R, Maurer AC, Plovie E, Xiao R, Vandenberghe LH. In Silico Reconstruction of the Viral Evolutionary Lineage Yields a Potent Gene Therapy Vector. Cell Rep. 2015 Aug 11: 12(6):1056-68. **(AAV Anc80L65).**

Shen S, Horowitz ED, Troupes AN, Brown SM, Pulicherla N, Samulski RJ, Agbandje-McKenna M, Asokan A. Engraftment of a galactose receptor footprint onto adeno-associated viral capsids improves transduction efficiency. J Biol Chem. 2013 Oct 4: 288(40):28814-23. **(AAV2G9).**

Li C, Diprimio N, Bowles DE, Hirsch ML, Monahan PE, Asokan A, Rabinowitz J, Agbandje-McKenna M, Samulski RJ. Single amino acid modification of adeno-associated virus capsid changes transduction and humoral immune profiles. J. Virol. 2012 Aug: 86(15):7752-9. **(AAV2 265 insertion-AAV2/265D).**

Bowles DE, McPhee SW, Li C, Gray SJ, Samulski JJ, Camp AS, Li J, Wang B, Monahan PE, Rabinowitz JE, et al. Phase 1 gene therapy for Duchenne muscular dystrophy using a translational optimized AAV vector. Mol. Ther. 2012 Feb: 20(2):443-55. **(AAV2.5).**

Messina EL, Nienaber J, Daneshmand M, Villamizar N, Samulski J, Milano C, Bowles DE. Adeno-associated viral vectors based on serotype 3b use components of the fibroblast growth factor receptor signaling complex for efficient transduction. Hum. Gene Ther. 2012 Oct: 23(10):1031-42. **(AAV3 SASTG).**

Asokan A, Conway JC, Phillips JL, Li C, Hegge J, Sinnott R, Yadav S, DiPrimio N, Nam HJ, Agbandje-McKenna M, McPhee S, Wolff J, Samulski RJ. Reengineering a receptor footprint of adeno-associated virus enables selective and systemic gene transfer to muscle. Nat Biotechnol. 2010 Jan: 28(1):79-82. **(AAV2i8).**

Vance M, Llanga T, Bennett W, Woodard K, Murlidharan G, Chungfat N, Asokan A, Gilger B, Kurtzberg J, Samulski RJ, Hirsch ML. AAV Gene Therapy for MPS1-associated Corneal Blindness. Sci Rep. 2016 Feb 22: 6:22131. **(AAV8G9).**

Zhong L, Li B, Mah CS, Govindasamy L, Agbandje-McKenna M, Cooper M, Herzog RW, Zolotukhin I, Warrington KH Jr, Weigel-Van Aken KA, Hobbs JA, Zolotukhin S, Muzyczka N, Srivastava A. Next generation of adeno-associated virus 2 vectors: point mutations in tyrosines lead to high-efficiency transduction at lower doses. Proc Natl Acad Sci USA. 2008 Jun 3: 105(22):7827-32. **(AAV2 tyrosine mutants AAV2 Y-F).**

Petrs-Silva H, Dinculescu A, Li Q, Min SH, Chiodo V, Pang JJ, Zhong L, Zolotukhin S, Srivastava A, Lewin AS, Hauswirth WW. High-efficiency transduction of the mouse retina by tyrosine-mutant AAV serotype vectors. Mol. Ther. 2009 Mar: 17(3):463-71. **(AAV8 Y-F and AAV9 Y-F).**

Qiao C, Zhang W, Yuan Z, Shin JH, Li J, Jayandharan GR, Zhong L, Srivastava A, Xiao X, Duan D. Adeno-associated virus serotype 6 capsid tyrosine-to-phenylalanine mutations improve gene transfer to skeletal muscle. Hum Gene Ther. 2010 Oct: 21(10):1343-8 **(AAV6 Y-F).**

Carlon M, Toelen J, Van der Perren A, Vandenberghe LH, Reumers V, Sbragia L, Gijsbers R, Baekelandt V, Himmelreich U, Wilson JM, Deprest J, Debyser Z. Efficient gene transfer into the mouse lung by fetal intratracheal injection of rAAV2/6.2. Mol. Ther. 2010 Dec: 18(12):2130-8. **(AAV6.2).**

PCT Publication No. WO2013158879A1. **(lysine mutants).**

The following biological sequence files listed in the file wrappers of USPTO issued patents and published applications describe chimeric or variant capsid proteins that can be incorporated into the AAV capsid of this invention in any combination with wild type capsid proteins and/or other chimeric or variant capsid proteins now known or later identified (for demonstrative purposes, U.S. Patent Application No. 11/486,254 corresponds to U.S. Patent Application No. 11/486,254): 11486254.raw, 11932017.raw, 12172121.raw, 12302206.raw, 12308959.raw, 12679144.raw, 13036343.raw, 13121532.raw, 13172915.raw, 13583920.raw, 13668120.raw, 13673351.raw, 13679684.raw, 14006954.raw, 14149953.raw, 14192101.raw, 14194538.raw, 14225821.raw, 14468108.raw, 14516544.raw, 14603469.raw, 14680836.raw, 14695644.raw, 14878703.raw, 14956934.raw, 15191357.raw, 15284164.raw, 15368570.raw, 15371188.raw, 15493744.raw, 15503120.raw, 15660906.raw, and 15675677.raw.

It would be understood that any combination of VP1 and VP3, and when present, VP1.5 and VP2 from any combination of AAV serotypes can be employed to produce the AAV capsids of this invention. For example, a VP1 protein from any combination of AAV serotypes can be combined with a VP3 protein from any combination of AAV serotypes and the respective VP1 proteins can be present in any ratio of different serotypes and the respective VP3 proteins can be present in any ratio of different serotypes and the VP1 and VP3 proteins can be present in any ratio of different serotypes. It would be further understood that, when present, a VP1.5 and/or VP2 protein from any combination of AAV serotypes can be combined with VP1 and VP3 protein from any combination of AAV serotypes and the respective VP1 .5 proteins can be present in any ratio of different serotypes and the respective VP2 proteins can be present in any ratio of different serotypes and the respective VP1 proteins can be present in any ratio of different serotypes and the respective VP3 proteins can be present in any ratio of different serotypes and the VP1.5 and/or VP2 proteins can be present in combination with VP1 and VP3 proteins in any ratio of different serotypes.

For example, the respective viral proteins and/or the respective AAV serotypes can be combined in any ratio, which can be a ratio of A:B, A:B:C, A:B:C:D, A:B:C:D:E, A:B:C:D:E:F, A:B:C:D:E:F:G, A:B:C:D:E:F:G:H, A:B:C:D:E:F:G:H:I or A:B:C:D:E:F:G:H:I:J, wherein A can be 1, 2, 3, 4, 5, 6, 7, 8, 9, 10, 11, 12, 13, 14, 15, 20, 25, 30, 35, 40, 50, 60, 70, 80, 90, 100, etc.; B can be 1, 2, 3, 4, 5, 6, 7, 8, 9, 10, 11, 12, 13, 14, 15, 20, 25, 30, 35, 40, 50, 60, 70, 80, 90, 100, etc.; C can be 1, 2, 3, 4, 5, 6, 7, 8, 9, 10, 11, 12, 13, 14, 15, 20, 25, 30, 35, 40, 50, 60, 70, 80, 90, 100, etc.; D can be 1, 2, 3, 4, 5, 6, 7, 8, 9, 10, 11, 12, 13, 14, 15, 20, 25, 30, 35, 40, 50, 60, 70, 80, 90, 100, etc.; E can be 1, 2, 3, 4, 5, 6, 7, 8, 9, 10, 11, 12, 13, 14, 15, 20, 25, 30, 35, 40, 50, 60, 70, 80, 90, 100, etc.; F can be 1, 2, 3, 4, 5, 6, 7, 8, 9, 10, 11, 12, 13, 14, 15, 20, 25, 30, 35, 40, 50, 60, 70, 80, 90, 100, etc.; G can be 1, 2, 3, 4, 5, 6, 7, 8, 9, 10, 11, 12, 13, 14, 15, 20, 25, 30, 35, 40, 50, 60, 70, 80, 90, 100, etc.; H can be 1, 2, 3, 4, 5, 6, 7, 8, 9, 10, 11, 12, 13, 14, 15, 20, 25, 30, 35, 40, 50, 60, 70, 80, 90, 100, etc.; I can be 1, 2, 3, 4, 5, 6, 7, 8, 9, 10, 11, 12, 13, 14, 15, 20, 25, 30, 35, 40, 50, 60, 70, 80, 90, 100, etc.; and J can be 1, 2, 3, 4, 5, 6, 7, 8, 9, 10, 11, 12, 13, 14, 15, 20, 25, 30, 35, 40, 50, 60, 70, 80, 90, 100, etc.

It would also be understood that any of the VP1, VP1.5, VP2 and/or VP3 capsid proteins can be present in a capsid of this invention as a chimeric capsid protein, in any combination and ratio relative to the same protein type and/or relative to the different capsid proteins.

In further embodiments, the present invention further provides a virus vector comprising, consisting essentially of and/or consisting of (a) the AAV capsid of this invention; and (b) a nucleic acid molecule comprising at least one terminal repeat sequence, wherein the nucleic acid molecule is encapsidated by the AAV capsid. In some embodiments, the virus vector can be an AAV particle.

In some embodiments, the virus vector of this invention can have systemic or selective tropism for skeletal muscle, cardiac muscle and/or diaphragm muscle. In some embodiments, the virus vector of this invention can have reduced tropism for liver.

The present invention further provides a composition, which can be a pharmaceutical formulation, comprising the capsid protein, capsid, virus vector, AAV particle composition and/or pharmaceutical formulation of this invention and a pharmaceutically acceptable carrier.

In some nonlimiting examples, the present invention provides AAV capsid proteins (VP1, VP1.5, VP2 and/or VP3) comprising a modification in the amino acid sequence in the three-fold axis loop 4 (Opie et al., J. Viral. 77: 6995-7006 (2003)) and virus capsids and virus vectors comprising the modified AAV capsid protein. The inventors have discovered that modifications in this loop can confer one or more desirable properties to virus vectors comprising the modified AAV capsid protein including without limitation (i) reduced transduction of liver, (ii) enhanced movement across endothelial cells, (iii) systemic transduction; (iv) enhanced transduction of muscle tissue (e.g., skeletal muscle, cardiac muscle and/or diaphragm muscle), and/or (v) reduced transduction of brain tissues (e.g., neurons). Thus, the present invention addresses some of the limitations associated with conventional AAV vectors. For example, vectors based on AAV8 and rAAV9 vectors are attractive for systemic nucleic acid delivery because they readily cross the endothelial cell barrier; however, systemic administration of rAAV8 or rAAV9 results in most of the vector being delivered to the liver, thereby reducing transduction of other important target tissues such as skeletal muscle.

In embodiments of the invention, transduction of cardiac muscle and/or skeletal muscle (determined on the basis of an individual skeletal muscle, multiple skeletal muscles, or the whole range of skeletal muscles) is at least about five-fold, ten-fold, 50-fold, 100-fold, 1000-fold or higher than transduction levels in liver.

In particular embodiments, the modified AAV capsid protein of the invention comprises one or more modifications in the amino acid sequence of the three-fold axis loop 4 *(e.g.,* amino acid positions 575 to 600 [inclusive] of the native AAV2 VP1 capsid protein or the corresponding region of a capsid protein from another AAV). As used herein, a "modification" in an amino acid sequence includes substitutions, insertions and/or deletions, each of which can involve one, two, three, four, five, six, seven, eight, nine, ten or more amino acids. In particular embodiments, the modification is a substitution. For example, in particular embodiments, 1, 2, 3, 4, 5, 6, 7, 8, 9, 10, 11, 12, 13, 14, 15, 16, 17, 18, 19, 20, 21, 22, 23, 24, 25 or more amino acids from the three-fold axis loop 4 from one AAV can be substituted into amino acid positions 575-600 of the native AAV2 capsid protein or the corresponding positions of the capsid protein from another AAV. However, the modified virus capsids of the invention are not limited to AAV capsids in which amino acids from one AAV capsid are substituted into another AAV capsid, and the substituted and/or inserted amino acids can be from any source, and can further be naturally occurring or partially or completely synthetic.

As described herein, the nucleic acid and amino acid sequences of the capsid proteins from a number of AAV are known in the art. Thus, the amino acids "corresponding" to amino acid positions 575 to 600 (inclusive) or amino acid positions 585 to 590 (inclusive) of the native AAV2 capsid protein can be readily determined for any other AAV (e.g., by using sequence alignments).

In some embodiments, the invention contemplates that the modified capsid proteins of the invention can be produced by modifying the capsid protein of any AAV now known or later discovered. Further, the AAV capsid protein that is to be modified can be a naturally occurring AAV capsid protein (e.g., an AAV2, AAV3a or 3b, AAV4, AAV5, AAV8, AAV9, AAV10, AAV11, or AAV12 capsid protein or any of the AAV shown in **Table 3)** but is not so limited. Those skilled in the art will understand that a variety of manipulations to the AAV capsid proteins are known in the art and the invention is not limited to modifications of naturally occurring AAV capsid proteins. For example, the capsid protein to be modified may already have alterations as compared with naturally occurring AAV *(e.g.,* is derived from a naturally occurring AAV capsid protein, *e.g.*, AAV2, AAV3a, AAV3b, AAV4, AAV5, AAV6, AAV7, AAV8, AAV9, AAV10, AAV11 and/or AAV12 or any other AAV now known or later discovered). Such AAV capsid proteins are also within the scope of the present invention.

For example, in some embodiments, the AAV capsid protein to be modified can comprise an amino acid insertion directly following amino acid 264 of the native AAV2 capsid protein sequence *(see, e.g.,* PCT Publication WO 2006/066066) and/or can be an AAV with an altered HI loop as described in PCT Publication WO 2009/108274 and/or can be an AAV that is modified to contain a poly-His sequence to facilitate purification. As another illustrative example, the AAV capsid protein can have a peptide targeting sequence incorporated therein as an insertion or substitution. Further, the AAV capsid protein can comprise a large domain from another AAV that has been substituted and/or inserted into the capsid protein.

Thus, in particular embodiments, the AAV capsid protein to be modified can be derived from a naturally occurring AAV but further comprise one or more foreign sequences (e.g., that are exogenous to the native virus) that are inserted and/or substituted into the capsid protein and/or has been altered by deletion of one or more amino acids.

Accordingly, when referring herein to a specific AAV capsid protein (e.g., an AAV2, AAV3, AAV4, AAV5, AAV6, AAV7, AAV8, AAV9, AAV10, AAV11 or AAV12 capsid protein or a capsid protein from any of the AAV shown in **Table 1,** *etc.),* it is intended to encompass the native capsid protein as well as capsid proteins that have alterations other than the modifications of the invention. Such alterations include substitutions, insertions and/or deletions. In particular embodiments, the capsid protein comprises 1, 2, 3, 4, 5, 6, 7, 8, 9, 10, 11, 12, 13, 14, 15, 16, 17, 18, 19 or 20, less than 20, less than 30, less than 40 less than 50, less than 60, or less than 70 amino acids inserted therein (other than the insertions of the present invention) as compared with the native AAV capsid protein sequence. In embodiments of the invention, the capsid protein comprises 1, 2, 3, 4, 5, 6, 7, 8, 9, 10, 11, 12, 13, 14, 15, 16, 17, 18, 19 or 20, less than 20, less than 30, less than 40 less than 50, less than 60, or less than 70 amino acid substitutions (other than the amino acid substitutions according to the present invention) as compared with the native AAV capsid protein sequence. In embodiments of the invention, the capsid protein comprises a deletion of 1, 2, 3, 4, 5, 6, 7, 8, 9, 10, 11, 12, 13, 14, 15, 16, 17, 18, 19 or 20, more than 20, more than 30, more than 40, more than 50, more than 60, or more than 70 amino acids (other than the amino acid deletions of the invention) as compared with the native AAV capsid protein sequence.

Thus, for example, the term "AAV2 capsid protein" includes AAV capsid proteins having the native AAV2 capsid protein sequence (*see* GenBank Accession No. AAC03780) as well as those comprising substitutions, insertions and/or deletions (as described in the preceding paragraph) in the native AAV2 capsid protein sequence.

In particular embodiments, the AAV capsid protein has the native AAV capsid protein sequence or has an amino acid sequence that is at least about 70%, 75%, 80%, 85%, 90%, 95%, 97%, 98% or 99% similar or identical to a native AAV capsid protein sequence. For example, in particular embodiments, an "AAV2" capsid protein encompasses the native AAV2 capsid protein sequence as well as sequences that are at least about 75%, 80%< 85%, 90%, 95%, 97%, 98% or 99% similar or identical to the native AAV2 capsid protein sequence.

Methods of determining sequence similarity or identity between two or more amino acid sequences are known in the art. Sequence similarity or identity may be determined using standard techniques known in the art, including, but not limited to, the local sequence identity algorithm of Smith & Waterman, Adv. Appl. Math. 2,482 (1981), by the sequence identity alignment algorithm of Needleman & Wunsch, J. Mol. Biol. 48,443 (1970), by the search for similarity method of Pearson & Lipman, Proc. Natl. Acad. Sci. USA 85, 2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12, 387-395 (1984), or by inspection.

Another suitable algorithm is the BLAST algorithm, described in Altschul et al., J. Mol. Biol. 215, 403-410, (1990) and Karlin et al., Proc. Natl. Acad. Sci. USA 90, 5873-5787 (1993). A particularly useful BLAST program is the WU-BLAST-2 program which was obtained from Altschul et al., Methods in Enzymology, 266, 460-480 (1996); http://blast.wustl/edulblast/README.html. WU-BLAST-2 uses several search parameters, which are optionally set to the default values. The parameters are dynamic values and are established by the program itself depending upon the composition of the particular sequence and composition of the particular database against which the sequence of interest is being searched; however, the values may be adjusted to increase sensitivity.

Further, an additional useful algorithm is gapped BLAST as reported by Altschul et al., (1997) Nucleic Acids Res. 25, 3389-3402.

In some embodiments of the invention, a modification can be made in the region of amino acid positions 585 to 590 (inclusive) of the native AAV2 capsid protein (using VP1 numbering) or the corresponding positions of other AAV (native AAV2 VP1 capsid protein: GenBank Accession No. AAC03780 or YP680426), *i.e.,* at the amino acids corresponding to amino acid positions 585 to 590 (VP1 numbering) of the native AAV2 capsid protein. The amino acid positions in other AAV serotypes or modified AAV capsids that "correspond to" positions 585 to 590 of the native AAV2 capsid protein will be apparent to those skilled in the art and can be readily determined using sequence alignment techniques *(see, e.g.,* Figure 7 of WO 2006/066066) and/or crystal structure analysis (Padron et al., (2005) J. Virol. 79: 5047-58).

To illustrate, the modification can be introduced into an AAV capsid protein that already contains insertions and/or deletions such that the position of all downstream sequences is shifted. In this situation, the amino acid positions corresponding to amino acid positions 585 to 590 in the AAV2 capsid protein would still be readily identifiable to those skilled in the art. To illustrate, the capsid protein can be an AAV2 capsid protein that contains an insertion following amino acid position 264 *(see, e.g.,* WO 2006/066066). The amino acids found at positions 585 through 590 (e.g., RGNRQA (SEQ ID NO:1)) in the native AAV2 capsid protein) would now be at positions 586 through 591 but would still be identifiable to those skilled in the art.

The invention also provides a virus capsid comprising, consisting essentially of, or consisting of the modified AAV capsid proteins of the invention. In particular embodiments, the virus capsid is a parvovirus capsid, which may further be an autonomous parvovirus capsid or a dependovirus capsid. Optionally, the virus capsid is an AAV capsid. In particular embodiments, the AAV capsid is an AAV1, AAV2, AAV3a, AAV3b, AAV4, AAV5, AAV6, AAV7, AAV8, AAV9, AAV10, AAV11, AAV12, or any other AAV shown in **Table 1** or otherwise known or later discovered, and/or is derived from any of the foregoing by one or more insertions, substitutions and/or deletions.

The modified virus capsids can be used as "capsid vehicles," as has been described, for example, in U.S. Patent No. 5,863,541. Molecules that can be packaged by the modified virus capsid and transferred into a cell include heterologous DNA, RNA, polypeptides, small organic molecules, metals, or combinations of the same.

Heterologous molecules are defined as those that are not naturally found in an AAV infection, *e.g.*, those not encoded by a wild-type AAV genome. Further, therapeutically useful molecules can be associated with the outside of the virus capsid for transfer of the molecules into host target cells. Such associated molecules can include DNA, RNA, small organic molecules, metals, carbohydrates, lipids and/or polypeptides. In one embodiment of the invention, the therapeutically useful molecule is covalently linked *(i.e.,* conjugated or chemically coupled) to the capsid proteins. Methods of covalently linking molecules are known by those skilled in the art.

The modified virus capsids of the invention also find use in raising antibodies against the novel capsid structures. As a further alternative, an exogenous amino acid sequence may be inserted into the modified virus capsid for antigen presentation to a cell, *e.g.,* for administration to a subject to produce an immune response to the exogenous amino acid sequence.

In other embodiments, the virus capsids can be administered to block certain cellular sites prior to and/or concurrently with (e.g., within minutes or hours of each other) administration of a virus vector delivering a nucleic acid encoding a polypeptide or functional RNA of interest. For example, the inventive capsids can be delivered to block cellular receptors on liver cells and a delivery vector can be administered subsequently or concurrently, which may reduce transduction of liver cells, and enhance transduction of other targets (e.g., skeletal, cardiac and/or diaphragm muscle).

According to representative embodiments, modified virus capsids can be administered to a subject prior to and/or concurrently with a modified virus vector according to the present invention. Further, the invention provides compositions and pharmaceutical formulations comprising the inventive modified virus capsids; optionally, the composition also comprises a modified virus vector of the invention.

The invention also provides nucleic acid molecules (optionally, isolated nucleic acid molecules) encoding the modified virus capsids and capsid proteins of the invention. Further provided are vectors, comprising the nucleic acid molecules and cells (*in vivo* or in culture), comprising the nucleic acid molecules and/or vectors of the invention. Suitable vectors include without limitation viral vectors (*e.g.,* adenovirus, AAV, herpesvirus, alphaviruses, vaccinia, poxviruses, baculoviruses, and the like), plasmids, phage, YACs, BACs, and the like. Such nucleic acid molecules, vectors and cells can be used, for example, as reagents (e.g., helper packaging constructs or packaging cells) for the production of modified virus capsids or virus vectors as described herein.

Virus capsids according to the invention can be produced using any method known in the art, *e.g.,* by expression from a baculovirus (Brown et al., (1994) Virology 198:477-488).

In some embodiments, the modifications to the AAV capsid protein of this invention are "selective" modifications. This approach is in contrast to previous work with whole subunit or large domain swaps between AAV serotypes (*see, e.g.,* international patent publication WO 00/28004 and Hauck et al., (2003) J. Virology 77:2768-2774). In particular embodiments, a "selective" modification results in the insertion and/or substitution and/or deletion of less than about 20, 18, 15, 12, 10, 9, 8, 7, 6, 5, 4, 3 or 2 contiguous amino acids.

The modified capsid proteins and capsids of the invention can further comprise any other modification, now known or later identified.

The virus capsid can be a targeted virus capsid comprising a targeting sequence (e.g., substituted or inserted in the viral capsid) that directs the virus capsid to interact with cell-surface molecules present on a desired target tissue(s) (*see, e.g.,* International Patent Publication No. WO 00/28004 and Hauck et al., (2003) J. Virology 77:2768-2774); Shi et al., Human Gene Therapy 17:353-361 (2006) [describing insertion of the integrin receptor binding motif RGD at positions 520 and/or 584 of the AAV capsid subunit]; and U.S. Patent No. 7,314,912 [describing insertion of the P1 peptide containing an RGD motif following amino acid positions 447, 534, 573 and 587 of the AAV2 capsid subunit]). Other positions within the AAV capsid subunit that tolerate insertions are known in the art (e.g., positions 449 and 588 described by Grifman et al., Molecular Therapy 3:964-975 (2001)).

For example, some of the virus capsids of the invention have relatively inefficient tropism toward most target tissues of interest (e.g., liver, skeletal muscle, heart, diaphragm muscle, kidney, brain, stomach, intestines, skin, endothelial cells, and/or lungs). A targeting sequence can advantageously be incorporated into these low-transduction vectors to thereby confer to the virus capsid a desired tropism and, optionally, selective tropism for particular tissue(s). AAV capsid proteins, capsids and vectors comprising targeting sequences are described, for example in international patent publication WO 00/28004. As another possibility one or more non-naturally occurring amino acids as described by Wang et al., Annu Rev Biophys Biomol Struct. 35:225-49 (2006)) can be incorporated into the AAV capsid subunit at an orthogonal site as a means of redirecting a low-transduction vector to a desired target tissue(s). These unnatural amino acids can advantageously be used to chemically link molecules of interest to the AAV capsid protein including without limitation: glycans (mannose-dendritic cell targeting); RGD, bombesin or a neuropeptide for targeted delivery to specific cancer cell types; RNA aptamers or peptides selected from phage display targeted to specific cell surface receptors such as growth factor receptors, integrins, and the like. Methods of chemically modifying amino acids are known in the art *(see, e.g.,* Greg T. Hermanson, Bioconjugate Techniques, 1st edition, Academic Press, 1996).

In representative embodiments, the targeting sequence may be a virus capsid sequence (e.g., an autonomous parvovirus capsid sequence, AAV capsid sequence, or any other viral capsid sequence) that directs infection to a particular cell type(s).

As another nonlimiting example, a heparin binding domain (e.g., the respiratory syncytial virus heparin binding domain) may be inserted or substituted into a capsid subunit that does not typically bind HS receptors (e.g., AAV 4, AAV5) to confer heparin binding to the resulting mutant.

B19 infects primary erythroid progenitor cells using globoside as its receptor (Brown et al., (1993) Science 262:114). The structure of B19 has been determined to 8 Å resolutions (Agbandje-McKenna et al., (1994) Virology 203:106). The region of the B19 capsid that binds to globoside has been mapped between amino acids 399-406 (Chapman et al., (1993) Virology 194:419), a looped out region between β-barrel structures E and F. (Chipman et al., (1996) Proc. Nat. Acad. Sci. USA 93:7502). Accordingly, the globoside receptor binding domain of the B19 capsid may be substituted into the AAV capsid protein to target a virus capsid or virus vector comprising the same to erythroid cells.

In representative embodiments, the exogenous targeting sequence may be any amino acid sequence encoding a peptide that alters the tropism of a virus capsid or virus vector comprising the modified AAV capsid protein. In particular embodiments, the targeting peptide or protein may be naturally occurring or, alternately, completely or partially synthetic. Exemplary targeting sequences include ligands and other peptides that bind to cell surface receptors and glycoproteins, such as RGD peptide sequences, bradykinin, hormones, peptide growth factors (*e.g.,* epidermal growth factor, nerve growth factor, fibroblast growth factor, platelet-derived growth factor, insulin-like growth factors I and II, *etc.*), cytokines, melanocyte stimulating hormone (*e.g.,* α, β or γ), neuropeptides and endorphins, and the like, and fragments thereof that retain the ability to target cells to their cognate receptors. Other illustrative peptides and proteins include substance P, keratinocyte growth factor, neuropeptide Y, gastrin releasing peptide, interleukin 2, hen egg white lysozyme, erythropoietin, gonadoliberin, corticostatin, β-endorphin, leu-enkephalin, rimorphin, α-neo-enkephalin, angiotensin, pneumadin, vasoactive intestinal peptide, neurotensin, motilin, and fragments thereof as described above. As yet a further alternative, the binding domain from a toxin (e.g., tetanus toxin or snake toxins, such as α-bungarotoxin, and the like) can be substituted into the capsid protein as a targeting sequence. In a yet further representative embodiment, the AAV capsid protein can be modified by substitution of a "nonclassical" import/export signal peptide (e.g., fibroblast growth factor-1 and -2, interleukin 1, HIV-1 Tat protein, herpes virus VP22 protein, and the like) as described by Cleves (Current Biology 7:R318 (1997)) into the AAV capsid protein. Also encompassed are peptide motifs that direct uptake by specific cells, *e.g.,* a FVFLP peptide motif triggers uptake by liver cells.

Phage display techniques, as well as other techniques known in the art, may be used to identify peptides that recognize any cell type of interest.

The targeting sequence may encode any peptide that targets to a cell surface binding site, including receptors (e.g., protein, carbohydrate, glycoprotein or proteoglycan). Examples of cell surface binding sites include, but are not limited to, heparan sulfate, chondroitin sulfate, and other glycosaminoglycans, sialic acid moieties found on mucins, glycoproteins, and gangliosides, MHC I glycoproteins, carbohydrate components found on membrane glycoproteins, including, mannose, N-acetyl-galactosamine, N-acetyl-glucosamine, fucose, galactose, and the like.

In particular embodiments, a heparan sulfate (HS) or heparin binding domain is substituted into the virus capsid (for example, in an AAV that otherwise does not bind to HS or heparin). It is known in the art that HS/heparin binding is mediated by a "basic patch" that is rich in arginines and/or lysines. In exemplary embodiments, a sequence following the motif BXXB, where "B" is a basic residue and X is neutral and/or hydrophobic. As one nonlimiting example, BXXB is RGNR. In particular embodiments, BXXB is substituted for amino acid positions 262 through 265 in the native AAV2 capsid protein or the corresponding position in the capsid protein of another AAV.

Other nonlimiting examples of suitable targeting sequences include the peptides targeting coronary artery endothelial cells identified by Müller et al., Nature Biotechnology 21:1040-1046 (2003) (consensus sequences NSVRDLG/S (SEQ ID NO:2), PRSVTVP (SEQ ID NO:3), NSVSSXS/A (SEQ ID NO:4)); tumor-targeting peptides as described by Grifman et al., Molecular Therapy 3:964-975 (2001) (*e.g.,* NGR, NGRAHA (SEQ ID NO:5)); lung or brain targeting sequences as described by Work et al., Molecular Therapy 13:683-693 (2006) (QPEHSST (SEQ ID NO:6), VNTANST (SEQ ID NO:7), HGPMQKS (SEQ ID NO:8), PHKPPLA (SEQ ID NO:9), IKNNEMW (SEQ ID NO:10), RNLDTPM (SEQ ID NO:11), VDSHRQS (SEQ ID NO:12), YDSKTKT (SEQ ID NO:13), SQLPHQK (SEQ ID NO:14), STMQQNT (SEQ ID NO:15), TERYMTQ (SEQ ID NO:16), QPEHSST (SEQ ID NO:6), DASLSTS (SEQ ID NO:17), DLPNKKT (SEQ ID NO:18), DLTAARL (SEQ ID NO:19), EPHQFNY (SEQ ID NO:20), EPQSNHT (SEQ ID NO:21), MSSWPSQ (SEQ ID NO:22), NPKHNAT (SEQ ID NO:23), PDGMRTT (SEQ ID NO:24), PNNNKTT (SEQ ID NO:25), QSTTHDS (SEQ ID NO:26), TGSKQKQ (SEQ ID NO:27), SLKHQAL (SEQ ID NO:28) and SPIDGEQ (SEQ ID NO:29)); vascular targeting sequences described by Hajitou et al., TCM 16:80-88 (2006) (WIFPWIQL (SEQ ID NO:30), CDCRGDCFC (SEQ ID NO:31), CNGRC (SEQ ID NO:32), CPRECES (SEQ ID NO:33), GSL, CTTHWGFTLC (SEQ ID NO:34), CGRRAGGSC (SEQ ID NO:35), CKGGRAKDC (SEQ ID NO:36), and CVPELGHEC (SEQ ID NO:37)); targeting peptides as described by Koivunen et al., J. Nucl. Med. 40:883-888 (1999) (CRRETAWAK (SEQ ID NO:38), KGD, VSWFSHRYSPFAVS (SEQ ID NO:39), GYRDGYAGPILYN (SEQ ID NO:40), XXXY*XXX [where Y* is phospho-Tyr] (SEQ ID NO:41), Y*E/MNW (SEQ ID NO:42), RPLPPLP (SEQ ID NO:43), APPLPPR (SEQ ID NO:44), DVFYPYPY ASGS (SEQ ID NO:45), MYWYPY (SEQ ID NO:46), DITWDQL WDLMK (SEQ ID NO:47), CWDDG/L WLC (SEQ ID NO:48), EWCEYLGGYLRCY A (SEQ ID NO:49), YXCXXGPXTWXCXP (SEQ ID NO:50), IEGPTLRQWLAARA (SEQ ID NO:51), LWXXY/W/F/H (SEQ ID NO:52), XFXXYLW (SEQ ID NO:53), SSIISHFRWGLCD (SEQ ID NO:54), MSRPACPPNDKYE (SEQ ID NO:55), CLRSGRGC (SEQ ID NO:56), CHWMFSPWC (SEQ ID NO:57), WXXF (SEQ ID NO:58), CSSRLDAC (SEQ ID NO:59), CLPVASC (SEQ ID NO:60), CGFECVRQCPERC (SEQ ID NO:61), CVALCRRACGEGC (SEQ ID NO:62), SWCEPGWCR (SEQ ID NO:63), YSGKWGW (SEQ ID NO:64), GLSGGRS (SEQ ID NO:65), LMLPRAD (SEQ ID NO:66), CSCFRDVCC (SEQ ID NO:67), CRDVVSVIC (SEQ ID NO:68), CNGRC (SEQ ID NO:32), and GSL); and tumor targeting peptides as described by Newton & Deutscher, Phage Peptide Display in Handbook of Experimental Pharmacology, pages 145-163, Springer-Verlag, Berlin (2008) (MARSGL (SEQ ID NO:69), MARAKE (SEQ ID NO:70), MSRTMS (SEQ ID NO:71), KCCYSL (SEQ ID NO:72), WRR, WKR, WVR, WVK, WIK, WTR, WVL, WLL, WRT, WRG, WVS, WVA, MYWGDSHWLQYWYE (SEQ ID NO:73), MQLPLAT (SEQ ID NO:74), EWLS (SEQ ID NO:75), SNEW (SEQ ID NO:76), TNYL (SEQ ID NO:77), WIFPWIQL (SEQ ID NO:30), WDLAWMFRLPVG (SEQ ID NO:78), CTVALPGGYVRVC (SEQ ID NO:79), CVPELGHEC (SEQ ID NO:37), CGRRAGGSC (SEQ ID NO:35), CVAYCIEHHCWTC (SEQ ID NO:80), CVFAHNYDYL VC (SEQ ID NO:81), and CVFTSNYAFC (SEQ ID NO:82), VHSPNKK (SEQ ID NO:83), CDCRGDCFC (SEQ ID NO:31), CRGDGWC (SEQ ID NO:84), XRGCDX (SEQ ID NO:85), P:XXS/T (SEQ ID NO:86), CTTHWGFTLC (SEQ ID NO:34), SGKGPRQITAL (SEQ ID NO:87), A9A/Q)(N/A)(L/Y)(TN/M/R)(R/K) (SEQ ID NO:88), VYMSPF (SEQ ID NO:89), MQLPLAT (SEQ ID NO:74), ATWLPPR (SEQ ID NO:90), HTMYYHHYQHHL (SEQ ID NO:91), SEVGCRAGPLQWLCEKYFG (SEQ ID NO:92), CGLLPVGRPDRNVWRWLC (SEQ ID NO:93), CKGQCDRFKGLPWEC (SEQ ID NO:94), SGRSA (SEQ ID NO:95), WGFP (SEQ ID NO:96), LWXXAr [Ar=Y, W, F, H) (SEQ ID NO:97), XF:XXYLW (SEQ ID NO:98), AEPMPHSLNFSQYLWYT (SEQ ID NO:99), WAY(W/F)SP (SEQ ID NO:100), IELLQAR (SEQ ID NO:101), DITWDQLWDLMK (SEQ ID NO:102), AYTKCSRQWRTCMTTH (SEQ ID NO:103), PQNSKIPGPTFLDPH (SEQ ID NO:104), SMEPALPDWWWKMFK (SEQ ID NO:105), ANTPCGPYTHDCPVKR (SEQ ID NO:106), TACHQHVRMVRP (SEQ ID NO:107), VPWMEPAYQRFL (SEQ ID NO:108), DPRATPGS (SEQ ID NO:109), FRPNRAQDYNTN (SEQ ID NO:110), CTKNSYLMC (SEQ ID NO:111), C(R/Q)L/RT(G/N)XXG(AN)GC (SEQ ID NO:112), CPIEDRPMC (SEQ ID NO:113), IIEWSYLAPYPWF (SEQ ID NO:114), MCPKHPLGC (SEQ ID NO:115), RMWPSSTVNLSAGRR (SEQ ID NO:116), SAKTAVSQRVWLPSHRGGEP (SEQ ID NO:117), KSREHVNNSACPSKRITAAL (SEQ ID NO:118), EGFR (SEQ ID NO:119), RVS, AGS, AGLGVR (SEQ ID NO:120), GGR, GGL, GSV, GVS, GTRQGHTMRLGVSDG (SEQ ID NO:121), IAGLATPGWSHWLAL (SEQ ID NO:122), SMSIARL (SEQ ID NO:123), HTFEPGV (SEQ ID NO:124), NTSLKRISNKRIRRK (SEQ ID NO:125), LRIKRKRRKRKKTRK (SEQ ID NO:126), GGG, GFS, LWS, EGG, LLV, LSP, LBS, AGG, GRR, GGH and GTV).

As yet a further alternative, the targeting sequence may be a peptide that can be used for chemical coupling (e.g., can comprise arginine and/or lysine residues that can be chemically coupled through their R groups) to another molecule that targets entry into a cell.

As another option, the AAV capsid protein or virus capsid of the invention can comprise a mutation as described in WO 2006/066066. For example, the capsid protein can comprise a selective amino acid substitution at amino acid position 263, 705, 708 and/or 716 of the native AAV2 capsid protein or a corresponding change(s) in a capsid protein from another AAV. Additionally, or alternatively, in representative embodiments, the capsid protein, virus capsid or vector comprises a selective amino acid insertion directly following amino acid position 264 of the AAV2 capsid protein or a corresponding change in the capsid protein from other AAV. By "directly following amino acid position X" it is intended that the insertion immediately follows the indicated amino acid position (for example, "following amino acid position 264" indicates a point insertion at position 265 or a larger insertion, e.g., from positions 265 to 268, etc.). The foregoing embodiments of the invention can be used to deliver a heterologous nucleic acid to a cell or subject as described herein. For example, the modified vector can be used to treat a lysosomal storage disorder such as a mucopolysaccharidosis disorder (*e.g.,* Sly syndrome [β-glucuronidase], Hurler Syndrome [α-L-iduronidase], Scheie Syndrome [α-L-iduronidase], Hurler-Scheie Syndrome [α-L-iduronidase], Hunter's Syndrome [iduronate sulfatase], Sanfilippo Syndrome A [heparan sulfamidase], B [N-acetylglucosaminidase], C [acetyl-CoA:α-glucosaminide acetyltransferase], D [N-acetylglucosamine 6-sulfatase], Morquio Syndrome A [galactose-6-sulfate sulfatase], B [β-galactosidase], Maroteaux-Lamy Syndrome [N-acetylgalactosamine-4-sulfatase], *etc.*), Fabry disease (a-galactosidase), Gaucher's disease (glucocerebrosidase), or a glycogen storage disorder (e.g., Pompe disease; lysosomal acid α-glucosidase) as described herein.

Those skilled in the art will appreciate that for some AAV capsid proteins the corresponding modification will be an insertion and/or a substitution, depending on whether the corresponding amino acid positions are partially or completely present in the virus or, alternatively, are completely absent. Likewise, when modifying AAV other than AAV2, the specific amino acid position(s) may be different than the position in AAV2 *(see, e.g.,* **Table 3).** As discussed elsewhere herein, the corresponding amino acid position(s) will be readily apparent to those skilled in the art using well-known techniques.

In representative embodiments, the insertion and/or substitution and/or deletion in the capsid protein(s) results in the insertion, substitution and/or repositioning of an amino acid that (i) maintains the hydrophilic loop structure in that region; (ii) an amino acid that alters the configuration of the loop structure; (iii) a charged amino acid; and/or (iv) an amino acid that can be phosphorylated or sulfated or otherwise acquire a charge by post-translational modification (e.g., glycosylation) following 264 in an AAV2 capsid protein or a corresponding change in a capsid protein of another AAV. Suitable amino acids for insertion/substitution include aspartic acid, glutamic acid, valine, leucine, lysine, arginine, threonine, serine, tyrosine, glycine, alanine, proline, asparagine, phenylalanine, tyrosine or glutamine. In particular embodiments, a threonine is inserted or substituted into the capsid subunit. Nonlimiting examples of corresponding positions in a number of other AAV are shown in **Table 3** (Position 2). In particular embodiments, the amino acid insertion or substitution is a threonine, aspartic acid, glutamic acid or phenylalanine (excepting AAV that have a threonine, glutamic acid or phenylalanine, respectively, at this position).

According to this aspect of the invention, in some embodiments the AAV capsid protein comprises an amino acid insertion following amino acid position 264 in an AAV2, AAV3a or AAV3b capsid protein(s) or in the corresponding position in an AAV2, AAV3a or AAV3b capsid protein that has been modified to comprise non-AAV2, AAV3a or AAV3b sequences, respectively, and/or has been modified by deletion of one or more amino acids *(i.e.,* is derived from AAV2, AAV3a or AAV3b). The amino acid corresponding to position 264 in an AAV2 (or AAV3a or AAV3b) capsid subunit(s) will be readily identifiable in the starting virus that has been derived from AAV2 (or AAV3a or AAV3b), which can then be further modified according to the present invention. Suitable amino acids for insertion include aspartic acid, glutamic acid, valine, leucine, lysine, arginine, threonine, serine, tyrosine, glycine, alanine, proline, asparagine, phenylalanine, tyrosine or glutamine.

In other embodiments, the AAV capsid protein comprises an amino acid substitution at amino acid position 265 in an AAV1 capsid protein(s), at amino acid position 266 in an AAV8 capsid protein, or an amino acid substitution at amino acid position 265 in an AAV9 capsid protein or in the corresponding position in an AAV1, AAV8 or AAV9 capsid protein that has been modified to comprise non-AAV1, non-AAV8 or non-AAV9 sequences, respectively, and/or has been modified by deletion of one or more amino acids *(i.e.,* is derived from AAV1, AAV8 or AAV9). The amino acid corresponding to position 265 in an AAV1 and AAV9 capsid subunit(s) and position 266 in the AAV8 capsid subunit(s) will be readily identifiable in the starting virus that has been derived from AAV1, AAV8 or AAV9, which can then be further modified according to the present invention. Suitable amino acids for insertion include aspartic acid, glutamic acid, valine, leucine, lysine, arginine, threonine, serine, tyrosine, glycine, alanine, proline, asparagine, phenylalanine, tyrosine or glutamine.

In representative embodiments of the invention, the capsid protein comprises a threonine, aspartic acid, glutamic acid, or phenylalanine following amino acid position 264 of the AAV2 capsid protein *(i.e.,* an insertion) or the corresponding position of another capsid protein.

In other representative embodiments, the modified capsid proteins or virus capsids of the invention further comprise one or more mutations as described in WO 2007/089632 (e.g., an E7K mutation at amino acid position 531 of the AAV2 capsid protein or the corresponding position of the capsid protein from another AAV).

In further embodiments, the modified capsid protein or capsid can comprise a mutation as described in WO 2009/108274.

As another, possibility, the AAV capsid protein can comprise a mutation as described by Zhong et al. (Virology 381: 194-202 (2008); Proc. Nat. Acad. Sci. 105: 7827-32 (2008)). For example, the AAV capsid protein can comprise an YF mutation at amino acid position 730.

The modifications described above can be incorporated into the capsid proteins or capsids of the invention in combination with each other and/or with any other modification now known or later discovered.

The invention also encompasses virus vectors comprising the modified capsid proteins and capsids of the invention. In particular embodiments, the virus vector is a parvovirus vector (e.g., comprising a parvovirus capsid and/or vector genome), for example, an AAV vector (e.g., comprising an AAV capsid and/or vector genome). In representative embodiments, the virus vector comprises a modified AA V capsid comprising a modified capsid protein subunit of the invention and a vector genome.

For example, in representative embodiments, the virus vector comprises: (a) a modified virus capsid (e.g., a modified AAV capsid) comprising a modified capsid protein of the invention; and (b) a nucleic acid comprising a terminal repeat sequence *(e.g.,* an AAV TR), wherein the nucleic acid comprising the terminal repeat sequence is encapsidated by the modified virus capsid. The nucleic acid can optionally comprise two terminal repeats (e.g., two AAV TRs).
In representative embodiments, the virus vector is a recombinant virus vector comprising a heterologous nucleic acid encoding a polypeptide or functional RNA of interest. Recombinant virus vectors are described in more detail below.
In some embodiments, the virus vectors of the invention (i) have reduced transduction of liver as compared with the level of transduction by a virus vector without the modified capsid proteins of this invention; (ii) exhibit enhanced systemic transduction by the virus vector in an animal subject as compared with the level observed by a virus vector without the modified capsid proteins of this invention; (iii) demonstrate enhanced movement across endothelial cells as compared with the level of movement by a virus vector without the modified capsid proteins of this invention, and/or (iv) exhibit a selective enhancement in transduction of muscle tissue (e.g., skeletal muscle, cardiac muscle and/or diaphragm muscle), and/or (v) reduced transduction of brain tissues (*e.g.*, neurons) as compared with the level of transduction by a virus vector without the modified capsid proteins of this invention. In some embodiments, the virus vector has systemic transduction toward muscle, *e.g.,* it transduces multiple skeletal muscle groups throughout the body and optionally transduces cardiac muscle and/or diaphragm muscle.
Further, in some embodiments of the invention, the modified virus vectors demonstrate efficient transduction of target tissues.
It will be understood by those skilled in the art that the modified capsid proteins, virus capsids, virus vectors and AAV particles of the invention exclude those capsid proteins, capsids, virus vectors and AAV particles as they would be present or found in their native state.

Methods of Producing Virus Vectors

The present invention further provides methods of producing the inventive virus vectors of this invention as AAV particles. Thus, the present invention provides a method of making an AAV particle comprising the AAV capsid of this invention, comprising: (a) transfecting a host cell with one or more plasmids that provide, in combination all functions and genes needed to assemble AAV particles; (b) introducing one or more nucleic acid constructs into a packaging cell line or producer cell line to provide, in combination, all functions and genes needed to assemble AAV particles; (c) introducing into a host cell one or more recombinant baculovirus vectors that provide in combination all functions and genes needed to assemble AAV particles; and/or (d) introducing into a host cell one or more recombinant herpesvirus vectors that provide in combination all functions and genes needed to assemble AAV particles. Nonlimiting examples of various methods of making the virus vectors of this invention are described in Clement and Grieger ("Manufacturing of recombinant adeno-associated viral vectors for clinical trials" Mol. Ther. Methods Clin Dev. 3:16002 (2016)) and in Grieger et al. ("Production of recombinant adeno-associated virus vectors using suspension HEK293 cells and continuous harvest of vector from the culture media for GMP FIX and FLT1 clinical vector" Mol Ther 24(2):287-297 (2016)), the entire contents of which are incorporated by reference herein.
In one representative embodiment, the present invention provides a method of producing a virus vector, the method comprising providing to a cell: (a) a nucleic acid template comprising at least one TR sequence (e.g., AAV TR sequence), and (b) AAV sequences sufficient for replication of the nucleic acid template and encapsidation into AAV capsids *(e.g.,* AAV *rep* sequences and AAV *cap* sequences encoding the AAV capsids of the invention). Optionally, the nucleic acid template further comprises at least one heterologous nucleic acid sequence. In particular embodiments, the nucleic acid template comprises two AAV ITR sequences, which are located 5' and 3' to the heterologous nucleic acid sequence (if present), although they need not be directly contiguous thereto.
The nucleic acid template and AAV *rep* and *cap* sequences are provided under conditions such that virus vector comprising the nucleic acid template packaged within the AAV capsid is produced in the cell. The method can further comprise the step of collecting the virus vector from the cell. The virus vector can be collected from the medium and/or by lysing the cells.
The cell can be a cell that is permissive for AAV viral replication. Any suitable cell known in the art may be employed. In particular embodiments, the cell is a mammalian cell. As another option, the cell can be a trans-complementing packaging cell line that provides functions deleted from a replication-defective helper virus, *e.g.*, 293 cells or other Ela trans-complementing cells.
The AAV replication and capsid sequences may be provided by any method known in the art. Current protocols typically express the AAV *rep*/*cap genes* on a single plasmid. The AAV replication and packaging sequences need not be provided together, although it may be convenient to do so. The AAV *rep* and/or *cap* sequences may be provided by any viral or non-viral vector. For example, the *rep*/*cap* sequences may be provided by a hybrid adenovirus or herpesvirus vector (*e.g.*, inserted into the Ela or E3 regions of a deleted adenovirus vector). EBV vectors may also be employed to express the AAV *cap* and *rep* genes. One advantage of this method is that EBV vectors are episomal, yet will maintain a high copy number throughout successive cell divisions *(i.e.,* are stably integrated into the cell as extra-chromosomal elements, designated as an "EBV based nuclear episome," see Margolski, (1992) Curr. Top. Microbiol. Immun. 158:67).

As a further alternative, the *rep*/*cap* sequences may be stably incorporated into a cell. Typically the AAV *rep*/*cap* sequences will not be flanked by the TRs, to prevent rescue and/or packaging of these sequences.

The nucleic acid template can be provided to the cell using any method known in the art. For example, the template can be supplied by a non-viral (*e.g.*, plasmid) or viral vector. In particular embodiments, the nucleic acid template is supplied by a herpesvirus or adenovirus vector (*e.g.*, inserted into the Ela or E3 regions of a deleted adenovirus). As another illustration, Palombo et al., (1998) J. Virology 72:5025, describes a baculovirus vector carrying a reporter gene flanked by the AAV TRs. EBV vectors may also be employed to deliver the template, as described above with respect to the *rep*/*cap* genes.

In another representative embodiment, the nucleic acid template is provided by a replicating rAAV virus. In still other embodiments, an AAV provirus comprising the nucleic acid template is stably integrated into the chromosome of the cell.

To enhance virus titers, helper virus functions (*e.g*., adenovirus or herpesvirus) that promote a productive AAV infection can be provided to the cell. Helper virus sequences necessary for AAV replication are known in the art. Typically, these sequences will be provided by a helper adenovirus or herpesvirus vector. Alternatively, the adenovirus or herpesvirus sequences can be provided by another non-viral or viral vector, *e.g.,* as a non-infectious adenovirus miniplasmid that carries all of the helper genes that promote efficient AAV production as described by Ferrari et al., (1997) Nature Med. 3:1295, and U.S. Patent Nos. 6,040,183 and 6,093,570.

Further, the helper virus functions may be provided by a packaging cell with the helper sequences embedded in the chromosome or maintained as a stable extrachromosomal element. Generally, the helper viruses sequences cannot be packaged into AAV virions, *e.g*., are not flanked by TRs.

Those skilled in the art will appreciate that it may be advantageous to provide the AAV replication and capsid sequences and the helper virus sequences (*e.g.*, adenovirus sequences) on a single helper construct. This helper construct may be a non-viral or viral construct. As one nonlimiting illustration, the helper construct can be a hybrid adenovirus or hybrid herpesvirus comprising the AAV *rep*/*cap* genes.

In one particular embodiment, the AAV *rep*/*cap* sequences and the adenovirus helper sequences are supplied by a single adenovirus helper vector. This vector further can further comprise the nucleic acid template. The AAV *rep*/*cap* sequences and/or the rAAV template can be inserted into a deleted region (e.g., the E1a or E3 regions) of the adenovirus.

In a further embodiment, the AAV *rep*/*cap* sequences and the adenovirus helper sequences are supplied by a single adenovirus helper vector. According to this embodiment, the rAAV template can be provided as a plasmid template.

In another illustrative embodiment, the AAV *rep*/*cap* sequences and adenovirus helper sequences are provided by a single adenovirus helper vector, and the rAAV template is integrated into the cell as a provirus. Alternatively, the rAAV template is provided by an EBV vector that is maintained within the cell as an extrachromosomal element *(e.g.,* as an EBV based nuclear episome).

In a further exemplary embodiment, the AAV *rep*/*cap* sequences and adenovirus helper sequences are provided by a single adenovirus helper. The rAAV template can be provided as a separate replicating viral vector. For example, the rAAV template can be provided by a rAAV particle or a second recombinant adenovirus particle.

According to the foregoing methods, the hybrid adenovirus vector typically comprises the adenovirus 5' and 3' cis sequences sufficient for adenovirus replication and packaging *(i.e.,* the adenovirus terminal repeats and PAC sequence). The AAV *rep*/*cap* sequences and, if present, the rAAV template are embedded in the adenovirus backbone and are flanked by the 5' and 3' *cis* sequences, so that these sequences may be packaged into adenovirus capsids. As described above, the adenovirus helper sequences and the AAV *rep*/*cap* sequences are generally not flanked by TRs so that these sequences are not packaged into the AAV virions.

Zhang et al., ((2001) Gene Ther. 18:704-12) describe a chimeric helper comprising both adenovirus and the AAV *rep* and *cap* genes.

Herpesvirus may also be used as a helper virus in AAV packaging methods.

Hybrid herpesviruses encoding the AAV Rep protein(s) may advantageously facilitate scalable AAV vector production schemes. A hybrid herpes simplex virus type I (HSV-1) vector expressing the AAV-2 *rep* and *cap* genes has been described (Conway et al., (1999) Gene Therapy 6:986 and WO 00/17377.

As a further alternative, the virus vectors of the invention can be produced in insect cells using baculovirus vectors to deliver the *rep*/*cap* genes and rAAV template as described, for example, by Urabe et al., (2002) Human Gene Therapy 13:1935-43.

AAV vector stocks free of contaminating helper virus may be obtained by any method known in the art. For example, AAV and helper virus may be readily differentiated based on size. AAV may also be separated away from helper virus based on affinity for a heparin substrate (Zolotukhin et al. (1999) Gene Therapy 6:973). Deleted replication-defective helper viruses can be used so that any contaminating helper virus is not replication competent. As a further alternative, an adenovirus helper lacking late gene expression may be employed, as only adenovirus early gene expression is required to mediate packaging of AAV virus. Adenovirus mutants defective for late gene expression are known in the art (e.g., ts100K and ts149 adenovirus mutants).

Recombinant Virus Vectors

The present invention provides a method of administering a nucleic acid molecule to a cell, the method comprising contacting the cell with the virus vector, the AAV particle and/or the composition or pharmaceutical formulation of this invention.

The present invention further provides a method of delivering a nucleic acid to a subject, the method comprising administering to the subject the virus vector, the AAV particle and/or the composition or pharmaceutical formulation of this invention.

In particular embodiments, the subject is human, and in some embodiments, the subject has or is at risk for a disorder that can be treated by gene therapy protocols. Nonlimiting examples of such disorders include a muscular dystrophy including Duchenne or Becker muscular dystrophy, hemophilia A, hemophilia B, multiple sclerosis, diabetes mellitus, Gaucher disease, Fabry disease, Pompe disease, cancer, arthritis, muscle wasting, heart disease including congestive heart failure or peripheral artery disease, intimal hyperplasia, a neurological disorder including: epilepsy, Huntington's disease, Parkinson's disease or Alzheimer's disease, an autoimmune disease, cystic fibrosis, thalassemia, Hurler's Syndrome, Sly syndrome, Scheie Syndrome, Hurler-Scheie Syndrome, Hunter's Syndrome, Sanfilippo Syndrome A, B, C, D, Morquio Syndrome, Maroteaux-Lamy Syndrome, Krabbe's disease, phenylketonuria, Batten's disease, spinal cerebral ataxia, LDL receptor deficiency, hyperammonemia, anemia, arthritis, a retinal degenerative disorder including macular degeneration, adenosine deaminase deficiency, a metabolic disorder, and cancer including tumor-forming cancers.

In some embodiments of the methods of this invention, the virus vector, the AAV particle and/or the composition or pharmaceutical formulation of this invention can be administered to skeletal muscle, cardiac muscle and/or diaphragm muscle.

In the methods described herein, the virus vector, the AAV particle and/or the composition or pharmaceutical formulation of this invention can be administered/delivered to a subject of this invention via a systemic route *(e.g.,* intravenously, intraarterially, intraperitoneally, etc.). In some embodiments, the virus vector and/or composition can be administered to the subject via an intracerebroventrical, intracistemal, intraparenchymal, intracranial and/or intrathecal route. In particular embodiments, the virus vector and/or pharmaceutical formulation of this invention are administered intravenously.

The virus vectors of the present invention are useful for the delivery of nucleic acid molecules to cells *in vitro, ex vivo,* and *in vivo.* In particular, the virus vectors can be advantageously employed to deliver or transfer nucleic acid molecules to animal cells, including mammalian cells.

Any heterologous nucleic acid sequence(s) of interest may be delivered in the virus vectors of the present invention. Nucleic acid molecules of interest include nucleic acid molecules encoding polypeptides, including therapeutic (e.g., for medical or veterinary uses) and/or immunogenic (*e.g.*, for vaccines) polypeptides.

Therapeutic polypeptides include, but are not limited to, cystic fibrosis transmembrane regulator protein (CFTR), dystrophin (including mini- and micro-dystrophins, *see, e.g.,* Vincent et al., (1993) Nature Genetics 5:130; U.S. Patent Publication No. 2003/017131; International Patent Publication No. WO/2008/088895, Wang et al., Proc. Natl. Acad. Sci. USA 97:13714-13719 (2000); and Gregorevic et al., Mol. Ther. 16:657-64 (2008)), myostatin propeptide, follistatin, activin type II soluble receptor, IGF-1, anti-inflammatory polypeptides such as the I kappa B dominant mutant, sarcospan, utrophin (Tinsley et al., (1996) Nature 384:349), mini-utrophin, clotting factors *(e.g.,* Factor VIII, Factor IX, Factor X, *etc.),* erythropoietin, angiostatin, endostatin, catalase, tyrosine hydroxylase, superoxide dismutase, leptin, the LDL receptor, lipoprotein lipase, ornithine transcarbamylase, $\beta$-globin, $\alpha$-globin, spectrin, $\alpha_1$-antitrypsin, adenosine deaminase, hypoxanthine guanine phosphoribosyl transferase, glucocerebrosidase, sphingomyelinase, lysosomal hexosaminidase A, branched-chain keto acid dehydrogenase, RP65 protein, cytokines (*e.g.*, $\alpha$-interferon, $\beta$-interferon, interferon-y, interleukin-2, interleukin-4, granulocyte-macrophage colony stimulating factor, lymphotoxin, and the like), peptide growth factors, neurotrophic factors and hormones (*e.g.*, somatotropin, insulin, insulin-like growth factors 1 and 2, platelet derived growth factor, epidermal growth factor, fibroblast growth factor, nerve growth factor, neurotrophic factor-3 and -4, brain-derived neurotrophic factor, bone morphogenic proteins [including RANKL and VEGF], glial derived growth factor, transforming growth factor-$\alpha$ and -$\beta$, and the like), lysosomal acid $\alpha$-glucosidase, $\alpha$-galactosidase A, receptors (*e.g.*, the tumor necrosis growth factor-a soluble receptor), S100A1, parvalbumin, adenylyl cyclase type 6, a molecule that modulates calcium handling (*e.g.*, $SERCA_{2A}$, Inhibitor 1 of PP1 and fragments thereof [*e.g.*, WO 2006/029319 and WO 2007/100465]), a molecule that effects G-protein coupled receptor kinase type 2 knockdown such as a truncated constitutively active bARKct, anti-inflammatory factors such as IRAP, anti-myostatin proteins, aspartoacylase, monoclonal antibodies (including single chain monoclonal antibodies; an exemplary Mab is the Herceptin® Mab), neuropeptides and fragments thereof *(e.g.,* galanin, Neuropeptide Y *(see,* U.S. Patent No. 7,071,172), angiogenesis inhibitors such as Vasohibins and other VEGF inhibitors (e.g., Vasohibin 2 [*see,* WO JP2006/073052]). Other illustrative heterologous nucleic acid sequences encode suicide gene products *(e.g.,* thymidine kinase, cytosine deaminase, diphtheria toxin, and tumor necrosis factor), proteins conferring resistance to a drug used in cancer therapy, tumor suppressor gene products *(e.g.,* p53, Rb, Wt-1), TRAIL, FAS-ligand, and any other polypeptide that has a therapeutic effect in a subject in need thereof. AAV vectors can also be used to deliver monoclonal antibodies and antibody fragments, for example, an antibody or antibody fragment directed against myostatin *(see, e.g.,* Fang et al., Nature Biotechnology 23:584-590 (2005)).

Heterologous nucleic acid sequences encoding polypeptides include those encoding reporter polypeptides (*e.g.*, an enzyme). Reporter polypeptides are known in the art and include, but are not limited to, Green Fluorescent Protein (GFP), luciferase, β-galactosidase, alkaline phosphatase, luciferase, and chloramphenicol acetyltransferase gene.

Optionally, the heterologous nucleic acid molecule encodes a secreted polypeptide *(e.g.,* a polypeptide that is a secreted polypeptide in its native state or that has been engineered to be secreted, for example, by operable association with a secretory signal sequence as is known in the art).

Alternatively, in particular embodiments of this invention, the heterologous nucleic acid molecule may encode an antisense nucleic acid molecule, a ribozyme *(e.g.,* as described in U.S. Patent No. 5,877,022), RNAs that effect spliceosome-mediated *trans-splicing (see,* Puttaraju et al., (1999) Nature Biotech. 17:246; U.S. Patent No. 6,013,487; U.S. Patent No. 6,083,702), interfering RNAs (RNAi) including siRNA, shRNA or miRNA that mediate gene silencing *(see,* Sharp et al., (2000) Science 287:2431), and other non-translated RNAs, such as "guide" RNAs (Gorman et al., (1998) Proc. Nat. Acad. Sci. USA 95:4929; U.S. Patent No. 5,869,248 to Yuan et al.*),* and the like. Exemplary untranslated RNAs include RNAi against a multiple drug resistance (MDR) gene product *(e.g.,* to treat and/or prevent tumors and/or for administration to the heart to prevent damage by chemotherapy), RNAi against myostatin *(e.g.,* for Duchenne muscular dystrophy), RNAi against VEGF *(e.g.,* to treat and/or prevent tumors), RNAi against phospholamban *(e.g.,* to treat cardiovascular disease, *see, e.g.,* Andino et al., J. Gene Med. 10:132-142 (2008) and Li et al., Acta Pharmacol Sin. 26:51-55 (2005)); phospholamban inhibitory or dominant-negative molecules such as phospholamban S16E *(e.g.,* to treat cardiovascular disease, *see, e.g.,* Hoshijima et al. Nat. Med. 8:864-871 (2002)), RNAi to adenosine kinase *(e.g.,* for epilepsy), and RNAi directed against pathogenic organisms and viruses *(e.g.,* hepatitis B and/or C virus, human immunodeficiency virus, CMV, herpes simplex virus, human papilloma virus, etc.).

Further, a nucleic acid sequence that directs alternative splicing can be delivered. To illustrate, an antisense sequence (or other inhibitory sequence) complementary to the 5' and/or 3' splice site of dystrophin exon 51 can be delivered in conjunction with a U1 or U7 small nuclear (sn) RNA promoter to induce skipping of this exon. For example, a DNA sequence comprising a U1 or U7 snRNA promoter located 5' to the antisense/inhibitory sequence(s) can be packaged and delivered in a modified capsid of the invention.

The virus vector may also comprise a heterologous nucleic acid molecule that shares homology with and recombines with a locus on a host cell chromosome. This approach can be utilized, for example, to correct a genetic defect in the host cell.

The present invention also provides virus vectors that express an immunogenic polypeptide, peptide and/or epitope, *e.g.*, for vaccination. The nucleic acid molecule may encode any immunogen of interest known in the art including, but not limited to, immunogens from human immunodeficiency virus (HIV), simian immunodeficiency virus (SIV), influenza virus, HIV or SIV gag proteins, tumor antigens, cancer antigens, bacterial antigens, viral antigens, and the like.

The use of parvoviruses as vaccine vectors is known in the art *(see, e.g.,* Miyamura et al., (1994) Proc. Nat. Acad. Sci USA 91:8507; U.S. Patent No. 5,916,563 to Young et al., U.S. Patent No. 5,905,040 to Mazzara et al., U.S. Patent No. 5,882,652, and U.S. Patent No. 5,863,541 to Samulski et al.*).* The antigen may be presented in the parvovirus capsid. Alternatively, the immunogen or antigen may be expressed from a heterologous nucleic acid molecule introduced into a recombinant vector genome. Any immunogen or antigen of interest as described herein and/or as is known in the art can be provided by the virus vector of the present invention.

An immunogenic polypeptide can be any polypeptide, peptide, and/or epitope suitable for eliciting an immune response and/or protecting the subject against an infection and/or disease, including, but not limited to, microbial, bacterial, protozoal, parasitic, fungal and/or viral infections and diseases. For example, the immunogenic polypeptide can be an orthomyxovirus immunogen (e.g., an influenza virus immunogen, such as the influenza virus hemagglutinin (HA) surface protein or the influenza virus nucleoprotein, or an equine influenza virus immunogen) or a lentivirus immunogen *(e.g.,* an equine infectious anemia virus immunogen, a Simian Immunodeficiency Virus (SIV) immunogen, or a Human Immunodeficiency Virus (HIV) immunogen, such as the HIV or SIV envelope GP160 protein, the IIIV or SIV matrix/capsid proteins, and the HIV or SIV *gag, pol* and *env* gene products). The immunogenic polypeptide can also be an arenavirus immunogen *(e.g.,* Lassa fever virus immunogen, such as the Lassa fever virus nucleocapsid protein and the Lassa fever envelope glycoprotein), a poxvirus immunogen *(e.g.,* a vaccinia virus immunogen, such as the vaccinia L1 or L8 gene products), a flavivirus immunogen *(e.g.,* a yellow fever virus immunogen or a Japanese encephalitis virus immunogen), a filovirus immunogen *(e.g.,* an Ebola virus immunogen, or a Marburg virus immunogen, such as NP and GP gene products), a bunyavirus immunogen *(e.g.,* RVFV, CCHF, and/or SFS virus immunogens), or a coronavirus immunogen *(e.g.,* an infectious human coronavirus immunogen, such as the human coronavirus envelope glycoprotein, or a porcine transmissible gastroenteritis virus immunogen, or an avian infectious bronchitis virus immunogen). The immunogenic polypeptide can further be a polio immunogen, a herpes immunogen *(e.g.,* CMV, EBV, HSV immunogens) a mumps immunogen, a measles immunogen, a rubella immunogen, a diphtheria toxin or other diphtheria immunogen, a pertussis antigen, a hepatitis *(e.g.,* hepatitis A, hepatitis B, hepatitis C, etc.) immunogen, and/or any other vaccine immunogen now known in the art or later identified as an immunogen.

Alternatively, the immunogenic polypeptide can be any tumor or cancer cell antigen. Optionally, the tumor or cancer antigen is expressed on the surface of the cancer cell. Exemplary cancer and tumor cell antigens are described in S.A. Rosenberg (Immunity 10:281 (1991)). Other illustrative cancer and tumor antigens include, but are not limited to: BRCA1 gene product, BRCA2 gene product, gp100, tyrosinase, GAGE-1/2, BAGE, RAGE, LAGE, NY-ESO-1, CDK-4, β-catenin, MUM-1, Caspase-8, KIAA0205, HPVE, SART-1, PRAME, p15, melanoma tumor antigens (Kawakami et al., (1994) Proc. Natl. Acad. Sci. USA 91:3515; Kawakami et al., (1994) J. Exp. Med., 180:347; Kawakami et al., (1994) Cancer Res. 54:3124), MART-1, gp100 MAGE-1, MAGE-2, MAGE-3, CEA, TRP-1, TRP-2, P-15, tyrosinase (Brichard et al., (1993) J. Exp . Med. 178:489); HER-2/neu gene product (U.S. Pat. No. 4,968,603), CA 125, LK26, FB5 (endosialin), TAG 72, AFP, CA19-9, NSE, DU-PAN-2, CA50, SPan-1, CA72-4, HCG, STN (sialyl Tn antigen), c-erbB-2 proteins, PSA, L-CanAg, estrogen receptor, milk fat globulin, p53 tumor suppressor protein (Levine, (1993) Ann. Rev. Biochem. 62:623); mucin antigens (International Patent Publication No. WO 90/05142); telomerases; nuclear matrix proteins; prostatic acid phosphatase; papilloma virus antigens; and/or antigens now known or later discovered to be associated with the following cancers: melanoma, adenocarcinoma, thymoma, lymphoma (e.g., non-Hodgkin's lymphoma, Hodgkin's lymphoma), sarcoma, lung cancer, liver cancer, colon cancer, leukemia, uterine cancer, breast cancer, prostate cancer, ovarian cancer, cervical cancer, bladder cancer, kidney cancer, pancreatic cancer, brain cancer and any other cancer or malignant condition now known or later identified *(see, e.g.,* Rosenberg, (1996) Ann. Rev. Med. 47:481-91).

As a further alternative, the heterologous nucleic acid molecule can encode any polypeptide, peptide and/or epitope that is desirably produced in a cell *in vitro, ex vivo,* or *in vivo.* For example, the virus vectors may be introduced into cultured cells and the expressed gene product isolated therefrom.

It will be understood by those skilled in the art that the heterologous nucleic acid molecule(s) of interest can be operably associated with appropriate control sequences. For example, the heterologous nucleic acid molecule can be operably associated with expression control elements, such as transcription/translation control signals, origins of replication, polyadenylation signals, internal ribosome entry sites (IRES), promoters, and/or enhancers, and the like.

Further, regulated expression of the heterologous nucleic acid molecule(s) of interest can be achieved at the post-transcriptional level, *e.g*., by regulating selective splicing of different introns by the presence or absence of an oligonucleotide, small molecule and/or other compound that selectively blocks splicing activity at specific sites *(e.g.,* as described in WO 2006/119137).

Those skilled in the art will appreciate that a variety of promoter/enhancer elements can be used depending on the level and tissue-specific expression desired. The promoter/enhancer can be constitutive or inducible, depending on the pattern of expression desired. The promoter/enhancer can be native or foreign and can be a natural or a synthetic sequence. By foreign, it is intended that the transcriptional initiation region is not found in the wild-type host into which the transcriptional initiation region is introduced.

In particular embodiments, the promoter/enhancer elements can be native to the target cell or subject to be treated. In representative embodiments, the promoters/enhancer element can be native to the heterologous nucleic acid sequence.

The promoter/enhancer element is generally chosen so that it functions in the target cell(s) of interest. Further, in particular embodiments the promoter/enhancer element is a mammalian promoter/enhancer element. The promoter/-enhancer element may be constitutive or inducible.

Inducible expression control elements are typically advantageous in those applications in which it is desirable to provide regulation over expression of the heterologous nucleic acid sequence(s). Inducible promoters/enhancer elements for gene delivery can be tissue-specific or -preferred promoter/enhancer elements, and include muscle specific or preferred (including cardiac, skeletal and/or smooth muscle specific or preferred), neural tissue specific or preferred (including brain-specific or preferred), eye specific or preferred (including retina-specific and cornea-specific), liver specific or preferred, bone marrow specific or preferred, pancreatic specific or preferred, spleen specific or preferred, and lung specific or preferred promoter/enhancer elements. Other inducible promoter/enhancer elements include hormone-inducible and metal-inducible elements. Exemplary inducible promoters/enhancer elements include, but are not limited to, a Tet on/off element, a RU486-inducible promoter, an ecdysone-inducible promoter, a rapamycin-inducible promoter, and a metallothionein promoter.

In embodiments wherein the heterologous nucleic acid sequence(s) is transcribed and then translated in the target cells, specific initiation signals are generally included for efficient translation of inserted protein coding sequences. These exogenous translational control sequences, which may include the ATG initiation codon and adjacent sequences, can be of a variety of origins, both natural and synthetic.

The virus vectors according to the present invention provide a means for delivering heterologous nucleic acid molecules into a broad range of cells, including dividing and non-dividing cells. The virus vectors can be employed to deliver a nucleic acid molecule of interest to a cell *in vitro, e.g.,* to produce a polypeptide *in vitro* or for *ex vivo* or *in vivo* gene therapy. The virus vectors are additionally useful in a method of delivering a nucleic acid to a subject in need thereof, *e.g.*, to express an immunogenic or therapeutic polypeptide or a functional RNA. In this manner, the polypeptide or functional RNA can be produced *in vivo* in the subject. The subject can be in need of the polypeptide because the subject has a deficiency of the polypeptide.

Further, the method can be practiced because the production of the polypeptide or functional RNA in the subject may impart some beneficial effect.

The virus vectors can also be used to produce a polypeptide of interest or functional RNA in cultured cells or in a subject (e.g., using the subject as a bioreactor to produce the polypeptide or to observe the effects of the functional RNA on the subject, for example, in connection with screening methods).

In general, the virus vectors of the present invention can be employed to deliver a heterologous nucleic acid molecule encoding a polypeptide or functional RNA to treat and/or prevent any disorder or disease state for which it is beneficial to deliver a therapeutic polypeptide or functional RNA. Illustrative disease states include, but are not limited to: cystic fibrosis (cystic fibrosis transmembrane regulator protein) and other diseases of the lung, hemophilia A (Factor VIII), hemophilia B (Factor IX), thalassemia (β-globin), anemia (erythropoietin) and other blood disorders, Alzheimer's disease (GDF; neprilysin), multiple sclerosis (β-interferon), Parkinson's disease (glial-cell line derived neurotrophic factor [GDNF]), Huntington's disease (RNAi to remove repeats), amyotrophic lateral sclerosis, epilepsy (galanin, neurotrophic factors), and other neurological disorders, cancer (endostatin, angiostatin, TRAIL, FAS-ligand, cytokines including interferons; RNAi including RNAi against VEGF or the multiple drug resistance gene product, mir-26a *[e.g.,* for hepatocellular carcinoma]), diabetes mellitus (insulin), muscular dystrophies including Duchenne (dystrophin, mini-dystrophin, insulin-like growth factor I, a sarcoglycan *[e.g.,* α, β, γ], RNAi against myostatin, myostatin propeptide, follistatin, activin type II soluble receptor, anti-inflammatory polypeptides such as the Ikappa B dominant mutant, sarcospan, utrophin, mini-utrophin, antisense or RNAi against splice junctions in the dystrophin gene to induce exon skipping *[see, e.g.,* WO 2003/095647], antisense against U7 snRNAs to induce exon skipping *[see, e.g.,* WO 2006/021724], and antibodies or antibody fragments against myostatin or myostatin propeptide) and Becker, Gaucher disease (glucocerebrosidase), Hurler's disease (α-L-iduronidase), adenosine deaminase deficiency (adenosine deaminase), glycogen storage diseases *(e.g.,* Fabry disease [α-galactosidase] and Pompe disease [lysosomal acid α-glucosidase]) and other metabolic disorders, congenital emphysema (α1-antitrypsin), Lesch-Nyhan Syndrome (hypoxanthine guanine phosphoribosyl transferase), Niemann-Pick disease (sphingomyelinase), Tay Sachs disease (lysosomal hexosaminidase A), Maple Syrup Urine Disease (branched-chain keto acid dehydrogenase), retinal degenerative diseases (and other diseases of the eye and retina; *e.g.*, PDGF for macular degeneration and/or vasohibin or other inhibitors of VEGF or other angiogenesis inhibitors to treat/prevent retinal disorders, *e.g.*, in Type I diabetes), diseases of solid organs such as brain (including Parkinson's Disease [GDNF], astrocytomas [endostatin, angiostatin and/or RNAi against VEGF], glioblastomas [endostatin, angiostatin and/or RNAi against VEGF]), liver, kidney, heart including congestive heart failure or peripheral artery disease (PAD) *(e.g.,* by delivering protein phosphatase inhibitor I (I-1) and fragments thereof *(e.g.,* I1C), serca2a, zinc finger proteins that regulate the phospholamban gene, Barket, β2-adrenergic receptor, β2-adrenergic receptor kinase (BARK), phosphoinositide-3 kinase (PI3 kinase), S100A1S100A1, parvalbumin, adenylyl cyclase type 6, a molecule that effects G-protein coupled receptor kinase type 2 knockdown such as a truncated constitutively active bARKct; calsarcin, RNAi against phospholamban; phospholamban inhibitory or dominant-negative molecules such as phospholamban S16E, etc.), arthritis (insulin-like growth factors), joint disorders (insulin-like growth factor 1 and/or 2), intimal hyperplasia *(e.g.,* by delivering enos, inos), improve survival of heart transplants (superoxide dismutase), AIDS (soluble CD4), muscle wasting (insulin-like growth factor I), kidney deficiency (erythropoietin), anemia (erythropoietin), arthritis (anti-inflammatory factors such as IRAP and TNFα soluble receptor), hepatitis (α-interferon), LDL receptor deficiency (LDL receptor), hyperammonemia (ornithine transcarbamylase), Krabbe's disease (galactocerebrosidase), Batten's disease, spinal cerebral ataxias including SCA1, SCA2 and SCA3, phenylketonuria (phenylalanine hydroxylase), autoimmune diseases, and the like. The invention can further be used following organ transplantation to increase the success of the transplant and/or to reduce the negative side effects of organ transplantation or adjunct therapies *(e.g.,* by administering immunosuppressant agents or inhibitory nucleic acids to block cytokine production). As another example, bone morphogenic proteins (including BNP 2, 7, etc., RANKL and/or VEGF) can be administered with a bone allograft, for example, following a break or surgical removal in a cancer patient.

The invention can also be used to produce induced pluripotent stem cells (iPS). For example, a virus vector of the invention can be used to deliver stem cell associated nucleic acid(s) into a non-pluripotent cell, such as adult fibroblasts, skin cells, liver cells, renal cells, adipose cells, cardiac cells, neural cells, epithelial cells, endothelial cells, and the like. Nucleic acids encoding factors associated with stem cells are known in the art. Nonlimiting examples of such factors associated with stem cells and pluripotency include Oct-3/4, the SOX family (e.g., SOX1, SOX2, SOX3 and/or SOX15), the Klf family *(e.g.,* K1f1, Klf2, Klf4 and/or Klf5), the Myc family *(e.g.,* C-myc, L-myc and/or N-myc), NANOG and/or LIN28.

The invention can also be practiced to treat and/or prevent a metabolic disorder such as diabetes *(e.g.,* insulin),

hemophilia *(e.g.,* Factor IX or Factor VIII), a lysosomal storage disorder such as a mucopolysaccharidosis disorder (*e.g.*, Sly syndrome [β-glucuronidase], Hurler Syndrome [α-L-iduronidase], Scheie Syndrome [α-L-iduronidase], Hurler-Scheie Syndrome [α-L-iduronidase], Hunter's Syndrome [iduronate sulfatase], Sanfilippo Syndrome A [heparan sulfamidase], B [N-acetylglucosaminidase], C [acetyl-CoA:α-glucosaminide acetyltransferase], D [N-acetylglucosamine 6-sulfatase], Morquio Syndrome A [galactose-6-sulfate sulfatase], B [β-galactosidase], Maroteaux-Lamy Syndrome [N-acetylgalactosamine-4-sulfatase], *etc.),* Fabry disease (α-galactosidase), Gaucher's disease (glucocerebrosidase), or a glycogen storage disorder *(e.g.,* Pompe disease; lysosomal acid α-glucosidase).

Gene transfer has substantial potential use for understanding and providing therapy for disease states. There are a number of inherited diseases in which defective genes are known and have been cloned. In general, the above disease states fall into two classes: deficiency states, usually of enzymes, which are generally inherited in a recessive manner, and unbalanced states, which may involve regulatory or structural proteins, and which are typically inherited in a dominant manner. For deficiency state diseases, gene transfer can be used to bring a normal gene into affected tissues for replacement therapy, as well as to create animal models for the disease using antisense mutations. For unbalanced disease states, gene transfer can be used to create a disease state in a model system, which can then be used in efforts to counteract the disease state. Thus, virus vectors according to the present invention permit the treatment and/or prevention of genetic diseases.

The virus vectors according to the present invention may also be employed to provide a functional RNA to a cell *in vitro* or *in vivo.* Expression of the functional RNA in the cell, for example, can diminish expression of a particular target protein by the cell. Accordingly, functional RNA can be administered to decrease expression of a particular protein in a subject in need thereof. Functional RNA can also be administered to cells *in vitro* to regulate gene expression and/or cell physiology, *e.g.,* to optimize cell or tissue culture systems or in screening methods.

In addition, virus vectors according to the instant invention find use in diagnostic and screening methods, whereby a nucleic acid of interest is transiently or stably expressed in a cell culture system, or alternatively, a transgenic animal model.

The virus vectors of the present invention can also be used for various non-therapeutic purposes, including but not limited to use in protocols to assess gene targeting, clearance, transcription, translation, *etc.,* as would be apparent to one skilled in the art. The virus vectors can also be used for the purpose of evaluating safety (spread, toxicity, immunogenicity, etc.). Such data, for example, are considered by the United States Food and Drug Administration as part of the regulatory approval process prior to evaluation of clinical efficacy.

As a further aspect, the virus vectors of the present invention may be used to produce an immune response in a subject. According to this embodiment, a virus vector comprising a heterologous nucleic acid sequence encoding an immunogenic polypeptide can be administered to a subject, and an active immune response is mounted by the subject against the immunogenic polypeptide. Immunogenic polypeptides are as described hereinabove. In some embodiments, a protective immune response is elicited.

Alternatively, the virus vector may be administered to a cell *ex vivo* and the altered cell is administered to the subject. The virus vector comprising the heterologous nucleic acid is introduced into the cell, and the cell is administered to the subject, where the heterologous nucleic acid encoding the immunogen can be expressed and induce an immune response in the subject against the immunogen. In particular embodiments, the cell is an antigen-presenting cell *(e.g.,* a dendritic cell).

An "active immune response" or "active immunity" is characterized by "participation of host tissues and cells after an encounter with the immunogen. It involves differentiation and proliferation of immunocompetent cells in lymphoreticular tissues, which lead to synthesis of antibody or the development of cell-mediated reactivity, or both." Herbert B. Herscowitz, Immunophysiology: Cell Function and Cellular Interactions in Antibody Formation, in IMMUNOLOGY: BASIC PROCESSES 117 (Joseph A. Bellanti ed., 1985). Alternatively stated, an active immune response is mounted by the host after exposure to an immunogen by infection or by vaccination. Active immunity can be contrasted with passive immunity, which is acquired through the "transfer of preformed substances (antibody, transfer factor, thymic graft, and interleukin-2) from an actively immunized host to a non-immune host." *Id.*

A "protective" immune response or "protective" immunity as used herein indicates that the immune response confers some benefit to the subject in that it prevents or reduces the incidence of disease. Alternatively, a protective immune response or protective immunity may be useful in the treatment and/or prevention of disease, in particular cancer or tumors *(e.g.,* by preventing cancer or tumor formation, by causing regression of a cancer or tumor and/or by preventing metastasis and/or by preventing growth of metastatic nodules). The protective effects may be complete or partial, as long as the benefits of the treatment outweigh any disadvantages thereof.

In particular embodiments, the virus vector or cell comprising the heterologous nucleic acid molecule can be administered in an immunogenically effective amount, as described below.

The virus vectors of the present invention can also be administered for cancer immunotherapy by administration of a virus vector expressing one or more cancer cell antigens (or an immunologically similar molecule) or any other immunogen that produces an immune response against a cancer cell. To illustrate, an immune response can be produced against a cancer cell antigen in a subject by administering a virus vector comprising a heterologous nucleic acid encoding the cancer cell antigen, for example to treat a patient with cancer and/or to prevent cancer from developing in the subject. The virus vector may be administered to a subject in *vivo* or by using *ex vivo* methods, as described herein. Alternatively, the cancer antigen can be expressed as part of the virus capsid or be otherwise associated with the virus capsid *(e.g.,* as described above).

As another alternative, any other therapeutic nucleic acid (e.g., RNAi) or polypeptide *(e.g.,* cytokine) known in the art can be administered to treat and/or prevent cancer.

As used herein, the term "cancer" encompasses tumor-forming cancers.

Likewise, the term "cancerous tissue" encompasses tumors. A "cancer cell antigen" encompasses tumor antigens.

The term "cancer" has its understood meaning in the art, for example, an uncontrolled growth of tissue that has the potential to spread to distant sites of the body *(i.e.,* metastasize). Exemplary cancers include, but are not limited to melanoma, adenocarcinoma, thymoma, lymphoma *(e.g.,* non-Hodgkin's lymphoma, Hodgkin's lymphoma), sarcoma, lung cancer, liver cancer, colon cancer, leukemia, uterine cancer, breast cancer, prostate cancer, ovarian cancer, cervical cancer, bladder cancer, kidney cancer, pancreatic cancer, brain cancer and any other cancer or malignant condition now known or later identified. In representative embodiments, the invention provides a method of treating and/or preventing tumor-forming cancers.

The term "tumor" is also understood in the art, for example, as an abnormal mass of undifferentiated cells within a multicellular organism. Tumors can be malignant or benign. In representative embodiments, the methods disclosed herein are used to prevent and treat malignant tumors.

By the terms "treating cancer," "treatment of cancer" and equivalent terms it is intended that the severity of the cancer is reduced or at least partially eliminated and/or the progression of the disease is slowed and/or controlled and/or the disease is stabilized. In particular embodiments, these terms indicate that metastasis of the cancer is prevented or reduced or at least partially eliminated and/or that growth of metastatic nodules is prevented or reduced or at least partially eliminated.

By the terms "prevention of cancer" or "preventing cancer" and equivalent terms it is intended that the methods at least partially eliminate or reduce and/or delay the incidence and/or severity of the onset of cancer. Alternatively stated, the onset of cancer in the subject may be reduced in likelihood or probability and/or delayed.

In particular embodiments, cells may be removed from a subject with cancer and contacted with a virus vector expressing a cancer cell antigen according to the instant invention. The modified cell is then administered to the subject, whereby an immune response against the cancer cell antigen is elicited. This method can be advantageously employed with immunocompromised subjects that cannot mount a sufficient immune response *in vivo (i.e.,* cannot produce enhancing antibodies in sufficient quantities).

It is known in the art that immune responses may be enhanced by immunomodulatory cytokines (*e.g.*, α-interferon, β-interferon, γ-interferon, ω-interferon, τ-interferon, interleukin-1α, interleukin-1β, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-18, B cell Growth factor, CD40 Ligand, tumor necrosis factor-α, tumor necrosis factor-β, monocyte chemoattractant protein-1, granulocyte-macrophage colony stimulating factor, and lymphotoxin). Accordingly, immunomodulatory cytokines (preferably, CTL inductive cytokines) may be administered to a subject in conjunction with the virus vector.

Cytokines may be administered by any method known in the art. Exogenous cytokines may be administered to the subject, or alternatively, a nucleic acid encoding a cytokine may be delivered to the subject using a suitable vector, and the cytokine produced *in vivo.*

## Subjects, Pharmaceutical Formulations, and Modes of Administration

Virus vectors, AAV particles and capsids according to the present invention find use in both veterinary and medical applications. Suitable subjects include both avians and mammals. The term "avian" as used herein includes, but is not limited to, chickens, ducks, geese, quail, turkeys, pheasant, parrots, parakeets, and the like. The term "mammal" as used herein includes, but is not limited to, humans, non-human primates, bovines, ovines, captines, equines, felines, canines, lagomorphs, etc.

Human subjects include neonates, infants, juveniles, adults and geriatric subjects.

In representative embodiments, the subject is "in need of" the methods of the invention.

In particular embodiments, the present invention provides a pharmaceutical composition comprising a virus vector and/or capsid and/or AAV particle of the invention in a pharmaceutically acceptable carrier and, optionally, other medicinal agents, pharmaceutical agents, stabilizing agents, buffers, carriers, adjuvants, diluents, etc. For injection, the carrier will typically be a liquid. For other methods of administration, the carrier may be either solid or liquid. For inhalation administration, the carrier will be respirable, and optionally can be in solid or liquid particulate form. For administration to a subject or for other pharmaceutical uses, the carrier will be sterile and/or physiologically compatible.

By "pharmaceutically acceptable" it is meant a material that is not toxic or otherwise undesirable, *i.e.,* the material may be administered to a subject without causing any undesirable biological effects.

One aspect of the present invention is a method of transferring a nucleic acid molecule to a cell *in vitro.* The virus vector may be introduced into the cells at the appropriate multiplicity of infection according to standard transduction methods suitable for the particular target cells. Titers of virus vector to administer can vary, depending upon the target cell type and number, and the particular virus vector, and can be determined by those of skill in the art without undue experimentation. In representative embodiments, at least about $10^3$ infectious units, optionally at least about $10^5$ infectious units are introduced to the cell.

The cell(s) into which the virus vector is introduced can be of any type, including but not limited to neural cells (including cells of the peripheral and central nervous systems, in particular, brain cells such as neurons and oligodendricytes), lung cells, cells of the eye (including retinal cells, retinal pigment epithelium, and corneal cells), epithelial cells *(e.g.,* gut and respiratory epithelial cells), muscle cells *(e.g.,* skeletal muscle cells, cardiac muscle cells, smooth muscle cells and/or diaphragm muscle cells), dendritic cells, pancreatic cells (including islet cells), hepatic cells, myocardial cells, bone cells *(e.g.,* bone marrow stem cells), hematopoietic stem cells, spleen cells, keratinocytes, fibroblasts, endothelial cells, prostate cells, germ cells, and the like. In representative embodiments, the cell can be any progenitor cell. As a further possibility, the cell can be a stem cell *(e.g.,* neural stem cell, liver stem cell). As still a further alternative, the cell can be a cancer or tumor cell. Moreover, the cell can be from any species of origin, as indicated above.

The virus vector can be introduced into cells *in vitro* for the purpose of administering the modified cell to a subject. In particular embodiments, the cells have been removed from a subject, the virus vector is introduced therein, and the cells are then administered back into the subject. Methods of removing cells from subject for manipulation *ex vivo,* followed by introduction back into the subject are known in the art *(see, e.g.,* U.S. patent No. 5,399,346). Alternatively, the recombinant virus vector can be introduced into cells from a donor subject, into cultured cells, or into cells from any other suitable source, and the cells are administered to a subject in need thereof *(i.e.,* a "recipient" subject).

Suitable cells for *ex vivo* nucleic acid delivery are as described above. Dosages of the cells to administer to a subject will vary upon the age, condition and species of the subject, the type of cell, the nucleic acid being expressed by the cell, the mode of administration, and the like. Typically, at least about $10^2$ to about $10^8$ cells or at least about $10^3$ to about $10^6$ cells will be administered per dose in a pharmaceutically acceptable carrier. In particular embodiments, the cells transduced with the virus vector are administered to the subject in a treatment effective or prevention effective amount in combination with a pharmaceutical carrier.

In some embodiments, the virus vector is introduced into a cell and the cell can be administered to a subject to elicit an immunogenic response against the delivered polypeptide (e.g., expressed as a transgene or in the capsid). Typically, a quantity of cells expressing an immunogenically effective amount of the polypeptide in combination with a pharmaceutically acceptable carrier is administered. An "immunogenically effective amount" is an amount of the expressed polypeptide that is sufficient to evoke an active immune response against the polypeptide in the subject to which the pharmaceutical formulation is administered. In particular embodiments, the dosage is sufficient to produce a protective immune response (as defined above).

The degree of protection conferred need not be complete or permanent, as long as the benefits of administering the immunogenic polypeptide outweigh any disadvantages thereof.

A further aspect of the invention is a method of administering the virus vector and/or virus capsid to subjects. Administration of the virus vectors and/or capsids according to the present invention to a human subject or an animal in need thereof can be by any means known in the art. Optionally, the virus vector and/or capsid is delivered in a treatment effective or prevention effective dose in a pharmaceutically acceptable carrier.

The virus vectors and/or capsids of the invention can further be administered to elicit an immunogenic response (e.g., as a vaccine). Typically, immunogenic compositions of the present invention comprise an immunogenically effective amount of virus vector and/or capsid in combination with a pharmaceutically acceptable carrier. Optionally, the dosage is sufficient to produce a protective immune response (as defined above). The degree of protection conferred need not be complete or permanent, as long as the benefits of administering the immunogenic polypeptide outweigh any disadvantages thereof. Subjects and immunogens are as described above.

Dosages of the virus vector and/or capsid to be administered to a subject depend upon the mode of administration, the disease or condition to be treated and/or prevented, the individual subject's condition, the particular virus vector or capsid, and the nucleic acid to be delivered, and the like, and can be determined in a routine manner. Exemplary doses for achieving therapeutic effects are titers of at least about $10^5$, $10^6$, $10^7$, $10^8$, $10^9$, $10^{10}$, $10^{11}$, $10^{12}$, $10^{13}$, $10^{14}$, $10^{15}$ transducing units, optionally about $10^8$ to about $10^{13}$ transducing units.

In particular embodiments, more than one administration (e.g., two, three, four, five, six, seven, eight, nine, ten, etc., or more administrations) may be employed to achieve the desired level of gene expression over a period of various intervals, *e.g.*, hourly, daily, weekly, monthly, yearly, *etc.* Dosing can be single dosage or cumulative (serial dosing), and can be readily determined by one skilled in the art. For instance, treatment of a disease or disorder may comprise a one-time administration of an effective dose of a pharmaceutical composition virus vector disclosed herein. Alternatively, treatment of a disease or disorder may comprise multiple administrations of an effective dose of a virus vector carried out over a range of time periods, such as, *e.g.,* once daily, twice daily, trice daily, once every few days, or once weekly. The timing of administration can vary from individual to individual, depending upon such factors as the severity of an individual's symptoms. For example, an effective dose of a virus vector disclosed herein can be administered to an individual once every six months for an indefinite period of time, or until the individual no longer requires therapy. A person of ordinary skill in the art will recognize that the condition of the individual can be monitored throughout the course of treatment and that the effective amount of a virus vector disclosed herein that is administered can be adjusted accordingly.

In an embodiment, the period of administration of a virus vector is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

Exemplary modes of administration include oral, rectal, transmucosal, intranasal, inhalation *(e.g.,* via an aerosol), buccal *(e.g.,* sublingual), vaginal, intrathecal, intraocular, transdermal, *in utero* (or *in ovo),* parenteral *(e.g.,* intravenous, subcutaneous, intradermal, intramuscular [including administration to skeletal, diaphragm and/or cardiac muscle], intradermal, intrapleural, intracerebral, and intraarticular), topical (e.g., to both skin and mucosal surfaces, including airway surfaces, and transdermal administration), intralymphatic, and the like, as well as direct tissue or organ injection (e.g., to liver, skeletal muscle, cardiac muscle, diaphragm muscle or brain). Administration can also be to a tumor (e.g., in or near a tumor or a lymph node). The most suitable route in any given case will depend on the nature and severity of the condition being treated and/or prevented and on the nature of the particular vector that is being used.

Administration to skeletal muscle according to the present invention includes but is not limited to administration to skeletal muscle in the limbs (e.g., upper arm, lower arm, upper leg, and/or lower leg), back, neck, head (e.g., tongue), thorax, abdomen, pelvis/perineum, and/or digits. Suitable skeletal muscles include but are not limited to abductor digiti minimi (in the hand), abductor digiti minimi (in the foot), abductor hallucis, abductor ossis metatarsi quinti, abductor pollicis brevis, abductor pollicis longus, adductor brevis, adductor hallucis, adductor longus, adductor magnus, adductor pollicis, anconeus, anterior scalene, articularis genus, biceps brachii, biceps femoris, brachialis, brachioradialis, buccinator, coracobrachialis, corrugator supercilii, deltoid, depressor anguli oris, depressor labii inferioris, digastric, dorsal interossei (in the hand), dorsal interossei (in the foot), extensor carpi radialis brevis, extensor carpi radialis longus, extensor carpi ulnaris, extensor digiti minimi, extensor digitorum, extensor digitorum brevis, extensor digitorum longus, extensor hallucis brevis, extensor hallucis longus, extensor indicis, extensor pollicis brevis, extensor pollicis longus, flexor carpi radialis, flexor carpi ulnaris, flexor digiti minimi brevis (in the hand), flexor digiti minimi brevis (in the foot), flexor digitorum brevis, flexor digitorum longus, flexor digitorum profundus, flexor digitorum superficialis, flexor hallucis brevis, flexor hallucis longus, flexor pollicis brevis, flexor pollicis longus, frontalis, gastrocnemius, geniohyoid, gluteus maximus, gluteus medius, gluteus minimus, gracilis, iliocostalis cervicis, iliocostalis lumborum, iliocostalis thoracis, illiacus, inferior gemellus, inferior oblique, inferior rectus, infraspinatus, interspinalis, intertransversi, lateral pterygoid, lateral rectus, latissimus dorsi, levator anguli oris, levator labii superioris, levator labii superioris alaeque nasi, levator palpebrae superioris, levator scapulae, long rotators, longissimus capitis, longissimus cervicis, longissimus thoracis, longus capitis, longus colli, lumbricals (in the hand), lumbricals (in the foot), masseter, medial pterygoid, medial rectus, middle scalene, multifidus, mylohyoid, obliquus capitis inferior, obliquus capitis superior, obturator externus, obturator internus, occipitalis, omohyoid, opponens digiti minimi, opponens pollicis, orbicularis oculi, orbicularis oris, palmar interossei, palmaris brevis, palmaris longus, pectineus, pectoralis major, pectoralis minor, peroneus brevis, peroneus longus, peroneus tertius, piriformis, plantar interossei, plantaris, platysma, popliteus, posterior scalene, pronator quadratus, pronator teres, psoas major, quadratus femoris, quadratus plantae, rectus capitis anterior, rectus capitis lateralis, rectus capitis posterior major, rectus capitis posterior minor, rectus femoris, rhomboid major, rhomboid minor, risorius, sartorius, scalenus minimus, semimembranosus, semispinalis capitis, semispinalis cervicis, semispinalis thoracis, semitendinosus, serratus anterior, short rotators, soleus, spinalis capitis, spinalis cervicis, spinalis thoracis, splenius capitis, splenius cervicis, sternocleidomastoid, sternohyoid, sternothyroid, stylohyoid, subclavius, subscapularis, superior gemellus, superior oblique, superior rectus, supinator, supraspinatus, temporalis, tensor fascia lata, teres major, teres minor, thoracis, thyrohyoid, tibialis anterior, tibialis posterior, trapezius, triceps brachii, vastus intermedius, vastus lateralis, vastus medialis, zygomaticus major, and zygomaticus minor, and any other suitable skeletal muscle as known in the art.

The virus vector and/or capsid can be delivered to skeletal muscle by intravenous administration, intra-arterial administration, intraperitoneal administration, limb perfusion, (optionally, isolated limb perfusion of a leg and/or arm; *see, e.g.* Arruda et al., (2005) Blood 105: 3458-3464), and/or direct intramuscular injection. In particular embodiments, the virus vector and/or capsid is administered to a limb (arm and/or leg) of a subject (e.g., a subject with muscular dystrophy such as DMD) by limb perfusion, optionally isolated limb perfusion (e.g., by intravenous or intra-articular administration). In embodiments of the invention, the virus vectors and/or capsids of the invention can advantageously be administered without employing "hydrodynamic" techniques. Tissue delivery (e.g., to muscle) of prior art vectors is often enhanced by hydrodynamic techniques (e.g., intravenous/intravenous administration in a large volume), which increase pressure in the vasculature and facilitate the ability of the vector to cross the endothelial cell barrier. In particular embodiments, the viral vectors and/or capsids of the invention can be administered in the absence of hydrodynamic techniques such as high volume infusions and/or elevated intravascular pressure *(e.g.,* greater than normal systolic pressure, for example, less than or equal to a 5%, 10%, 15%, 20%, 25% increase in intravascular pressure over normal systolic pressure). Such methods may reduce or avoid the side effects associated with hydrodynamic techniques such as edema, nerve damage and/or compartment syndrome.

Administration to cardiac muscle includes administration to the left atrium, right atrium, left ventricle, right ventricle and/or septum. The virus vector and/or capsid can be delivered to cardiac muscle by intravenous administration, intra-arterial administration such as intra-aortic administration, direct cardiac injection (e.g., into left atrium, right atrium, left ventricle, right ventricle), and/or coronary artery perfusion.

Administration to diaphragm muscle can be by any suitable method including intravenous administration, intra-arterial administration, and/or intraperitoneal administration.

Delivery to a target tissue can also be achieved by delivering a depot comprising the virus vector and/or capsid. In representative embodiments, a depot comprising the virus vector and/or capsid is implanted into skeletal, cardiac and/or diaphragm muscle tissue or the tissue can be contacted with a film or other matrix comprising the virus vector and/or capsid. Such implantable matrices or substrates are described in U.S. Patent No. 7,201,898.

In particular embodiments, a virus vector and/or virus capsid according to the present invention is administered to skeletal muscle, diaphragm muscle and/or cardiac muscle (e.g., to treat and/or prevent muscular dystrophy, heart disease [for example, PAD or congestive heart failure]).

In representative embodiments, the invention is used to treat and/or prevent disorders of skeletal, cardiac and/or diaphragm muscle.

In a representative embodiment, the invention provides a method of treating and/or preventing muscular dystrophy in a subject in need thereof, the method comprising: administering a treatment or prevention effective amount of a virus vector of the invention to a mammalian subject, wherein the virus vector comprises a heterologous nucleic acid encoding dystrophin, a mini-dystrophin, a micro-dystrophin, myostatin propeptide, follistatin, activin type II soluble receptor, IGF-1, anti-inflammatory polypeptides such as the Ikappa B dominant mutant, sarcospan, utrophin, a micro-dystrophin, laminin-$\alpha$2, $\alpha$-sarcoglycan, $\beta$-sarcoglycan, $\gamma$-sarcoglycan, $\delta$-sarcoglycan, IGF-1, an antibody or antibody fragment against myostatin or myostatin propeptide, and/or RNAi against myostatin. In particular embodiments, the virus vector can be administered to skeletal, diaphragm and/or cardiac muscle as described elsewhere herein.

Alternatively, the invention can be practiced to deliver a nucleic acid to skeletal, cardiac or diaphragm muscle, which is used as a platform for production of a polypeptide *(e.g.,* an enzyme) or functional RNA *(e.g.,* RNAi, microRNA, antisense RNA) that normally circulates in the blood or for systemic delivery to other tissues to treat and/or prevent a disorder *(e.g.,* a metabolic disorder, such as diabetes *[e.g.,* insulin], hemophilia *[e.g.,* Factor IX or Factor VIII], a mucopolysaccharide disorder *[e.g.,* Sly syndrome, Hurler Syndrome, Scheie Syndrome, Hurler-Scheie Syndrome, Hunter's Syndrome, Sanfilippo Syndrome A, B, C, D, Morquio Syndrome, Maroteaux-Lamy Syndrome, *etc.*] or a lysosomal storage disorder such as Gaucher's disease [glucocerebrosidase] or Fabry disease [$\alpha$-galactosidase A] or a glycogen storage disorder such as Pompe disease [lysosomal acid $\alpha$ glucosidase]). Other suitable proteins for treating and/or preventing metabolic disorders are described herein. The use of muscle as a platform to express a nucleic acid of interest is described in U.S. Patent publication US 2002/0192189.

Thus, as one aspect, the invention further encompasses a method of treating and/or preventing a metabolic disorder in a subject in need thereof, the method comprising: administering a treatment or prevention effective amount of a virus vector of the invention to skeletal muscle of a subject, wherein the virus vector comprises a heterologous nucleic acid encoding a polypeptide, wherein the metabolic disorder is a result of a deficiency and/or defect in the polypeptide. Illustrative metabolic disorders and heterologous nucleic acids encoding polypeptides are described herein. Optionally, the polypeptide is secreted *(e.g.,* a polypeptide that is a secreted polypeptide in its native state or that has been engineered to be secreted, for example, by operable association with a secretory signal sequence as is known in the art). Without being limited by any particular theory of the invention, according to this embodiment, administration to the skeletal muscle can result in secretion of the polypeptide into the systemic circulation and delivery to target tissue(s). Methods of delivering virus vectors to skeletal muscle are described in more detail herein.

The invention can also be practiced to produce antisense RNA, RNAi or other functional RNA *(e.g.,* a ribozyme) for systemic delivery.

The invention also provides a method of treating and/or preventing congenital heart failure or PAD in a subject in need thereof, the method comprising administering a treatment or prevention effective amount of a virus vector of the invention to a mammalian subject, wherein the virus vector comprises a heterologous nucleic acid encoding, for example, a sarcoplasmic endoreticulum $Ca^{2+}$-ATPase (SERCA2a), an angiogenic factor, phosphatase inhibitor I (I-1) and fragments thereof *(e.g.,* 11C), RNAi against phospholamban; a phospholamban inhibitory or dominant-negative molecule such as phospholamban S16E, a zinc finger protein that regulates the phospholamban gene, β2-adrenergic receptor, β2-adrenergic receptor kinase (BARK), PI3 kinase, calsarcan, a β-adrenergic receptor kinase inhibitor (βARKct), inhibitor 1 of protein phosphatase 1 and fragments thereof *(e.g.,* I1C), S100A1, parvalbumin, adenylyl cyclase type 6, a molecule that effects G-protein coupled receptor kinase type 2 knockdown such as a truncated constitutively active bARKct, Pim-1, PGC-1α, SOD-1, SOD-2, EC-SOD, kallikrein, HIF, thymosin-β4, mir-1, mir-133, mir-206, mir-208 and/or mir-26a.

Injectables can be prepared in conventional forms, either as liquid solutions or suspensions, solid forms suitable for solution or suspension in liquid prior to injection, or as emulsions. Alternatively, one may administer the virus vector and/or virus capsids of the invention in a local rather than systemic manner, for example, in a depot or sustained-release formulation. Further, the virus vector and/or virus capsid can be delivered adhered to a surgically implantable matrix (*e.g.*, as described in U.S. Patent Publication No. US2004/0013645. The virus vectors and/or virus capsids disclosed herein can be administered to the lungs of a subject by any suitable means, optionally by administering an aerosol suspension of respirable particles comprised of the virus vectors and/or virus capsids, which the subject inhales. The respirable particles can be liquid or solid. Aerosols of liquid particles comprising the virus vectors and/or virus capsids may be produced by any suitable means, such as with a pressure-driven aerosol nebulizer or an ultrasonic nebulizer, as is known to those of skill in the art. *See, e.g.,* U.S. Patent No. 4,501,729. Aerosols of solid particles comprising the virus vectors and/or capsids may likewise be produced with any solid particulate medicament aerosol generator, by techniques known in the pharmaceutical art.

The virus vectors and virus capsids can be administered to tissues of the CNS *(e.g.,* brain, eye) and may advantageously result in broader distribution of the virus vector or capsid than would be observed in the absence of the present invention.

In particular embodiments, the delivery vectors of the invention may be administered to treat diseases of the CNS, including genetic disorders, neurodegenerative disorders, psychiatric disorders and tumors. Illustrative diseases of the CNS include, but are not limited to Alzheimer's disease, Parkinson's disease, Huntington's disease, Canavan disease, Leigh's disease, Refsum disease, Tourette syndrome, primary lateral sclerosis, amyotrophic lateral sclerosis, progressive muscular atrophy, Pick's disease, muscular dystrophy, multiple sclerosis, myasthenia gravis, Binswanger's disease, trauma due to spinal cord or head injury, Tay Sachs disease, Lesch-Nyan disease, epilepsy, cerebral infarcts, psychiatric disorders including mood disorders *(e.g.,* depression, bipolar affective disorder, persistent affective disorder, secondary mood disorder), schizophrenia, drug dependency *(e.g.,* alcoholism and other substance dependencies), neuroses *(e.g.,* anxiety, obsessional disorder, somatoform disorder, dissociative disorder, grief, post-partum depression), psychosis *(e.g.,* hallucinations and delusions), dementia, paranoia, attention deficit disorder, psychosexual disorders, sleeping disorders, pain disorders, eating or weight disorders *(e.g.,* obesity, cachexia, anorexia nervosa, and bulemia) and cancers and tumors *(e.g.,* pituitary tumors) of the CNS.

Disorders of the CNS include ophthalmic disorders involving the retina, posterior tract, and optic nerve *(e.g.,* retinitis pigmentosa, diabetic retinopathy and other retinal degenerative diseases, uveitis, age-related macular degeneration, glaucoma).

Most, if not all, ophthalmic diseases and disorders are associated with one or more of three types of indications: (1) angiogenesis, (2) inflammation, and (3) degeneration. The delivery vectors of the present invention can be employed to deliver anti-angiogenic factors; anti-inflammatory factors; factors that retard cell degeneration, promote cell sparing, or promote cell growth and combinations of the foregoing.

Diabetic retinopathy, for example, is characterized by angiogenesis. Diabetic retinopathy can be treated by delivering one or more anti-angiogenic factors either intraocularly *(e.g.,* in the vitreous) or periocularly *(e.g.,* in the sub-Tenon's region). One or more neurotrophic factors may also be co-delivered, either intraocularly *(e.g.,* intravitreally) or periocularly.

Uveitis involves inflammation. One or more anti-inflammatory factors can be administered by intraocular *(e.g.,* vitreous or anterior chamber) administration of a delivery vector of the invention.

Retinitis pigmentosa, by comparison, is characterized by retinal degeneration. In representative embodiments, retinitis pigmentosa can be treated by intraocular *(e.g.,* vitreal administration) of a delivery vector encoding one or more neurotrophic factors.

Age-related macular degeneration involves both angiogenesis and retinal degeneration. This disorder can be treated by administering the inventive deliver vectors encoding one or more neurotrophic factors intraocularly *(e.g.,* vitreous) and/or one or more anti-angiogenic factors intraocularly or periocularly *(e.g.,* in the sub-Tenon's region).

Glaucoma is characterized by increased ocular pressure and loss of retinal ganglion cells. Treatments for glaucoma include administration of one or more neuroprotective agents that protect cells from excitotoxic damage using the inventive delivery vectors. Such agents include N-methyl-D-aspartate (NMDA) antagonists, cytokines, and neurotrophic factors, delivered intraocularly, optionally intravitreally.

In other embodiments, the present invention may be used to treat seizures, *e.g.,* to reduce the onset, incidence or severity of seizures. The efficacy of a therapeutic treatment for seizures can be assessed by behavioral *(e.g.,* shaking, ticks of the eye or mouth) and/or electrographic means (most seizures have signature electrographic abnormalities). Thus, the invention can also be used to treat epilepsy, which is marked by multiple seizures over time.

In one representative embodiment, somatostatin (or an active fragment thereof) is administered to the brain using a delivery vector of the invention to treat a pituitary tumor. According to this embodiment, the delivery vector encoding somatostatin (or an active fragment thereof) is administered by microinfusion into the pituitary. Likewise, such treatment can be used to treat acromegaly (abnormal growth hormone secretion from the pituitary). The nucleic acid (e.g., GenBank Accession No. J00306) and amino acid (e.g., GenBank Accession No. P01166; contains processed active peptides somatostatin-28 and somatostatin-14) sequences of somatostatins are known in the art.

In particular embodiments, the vector can comprise a secretory signal as described in U.S. Patent No. 7,071,172.

In representative embodiments of the invention, the virus vector and/or virus capsid is administered to the CNS *(e.g.,* to the brain or to the eye). The virus vector and/or capsid may be introduced into the spinal cord, brainstem (medulla oblongata, pons), midbrain (hypothalamus, thalamus, epithalamus, pituitary gland, substantia nigra, pineal gland), cerebellum, telencephalon (corpus striatum, cerebrum including the occipital, temporal, parietal and frontal lobes, cortex, basal ganglia, hippocampus and portaamygdala), limbic system, neocortex, corpus striatum, cerebrum, and inferior colliculus . The virus vector and/or capsid may also be administered to different regions of the eye such as the retina, cornea and/or optic nerve.

The virus vector and/or capsid may be delivered into the cerebrospinal fluid *(e.g.,* by lumbar puncture) for more disperse administration of the delivery vector.

The virus vector and/or capsid may further be administered intravascularly to the CNS in situations in which the blood-brain barrier has been perturbed (e.g., brain tumor or cerebral infarct).

The virus vector and/or capsid can be administered to the desired region(s) of the CNS by any route known in the art, including but not limited to, intrathecal, intra- ocular, intracerebral, intraventricular, intravenous (e.g., in the presence of a sugar such as mannitol), intranasal, intra-aural, intra-ocular (e.g., intra-vitreous, sub-retinal, anterior chamber) and peri-ocular (e.g., sub-Tenon's region) delivery as well as intramuscular delivery with retrograde delivery to motor neurons.

In particular embodiments, the virus vector and/or capsid is administered in a liquid formulation by direct injection (e.g., stereotactic injection) to the desired region or compartment in the CNS. In other embodiments, the virus vector and/or capsid may be provided by topical application to the desired region or by intra-nasal administration of an aerosol formulation. Administration to the eye may be by topical application of liquid droplets. As a further alternative, the virus vector and/or capsid may be administered as a solid, slow-release formulation *(see, e.g.,* U.S. Patent No. 7,201,898).

In yet additional embodiments, the virus vector can used for retrograde transport to treat and/or prevent diseases and disorders involving motor neurons (e.g., amyotrophic lateral sclerosis (ALS); spinal muscular atrophy (SMA), etc.). For example, the virus vector can be delivered to muscle tissue from which it can migrate into neurons.

In other aspects of this embodiment, a virus vector reduces the severity of a disease or disorder by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In yet other aspects of this embodiment, a virus vector reduces the severity of a disease or disorder from, *e.g.*, about 5% to about 100%, about 10% to about 100%, about 20% to about 100%, about 30% to about 100%, about 40% to about 100%, about 50% to about 100%, about 60% to about 100%, about 70% to about 100%, about 80% to about 100%, about 10% to about 90%, about 20% to about 90%, about 30% to about 90%, about 40% to about 90%, about 50% to about 90%, about 60% to about 90%, about 70% to about 90%, about 10% to about 80%, about 20% to about 80%, about 30% to about 80%, about 40% to about 80%, about 50% to about 80%, or about 60% to about 80%, about 10% to about 70%, about 20% to about 70%, about 30% to about 70%, about 40% to about 70%, or about 50% to about 70%.

A virus vector disclosed herein may comprise a solvent, emulsion or other diluent in an amount sufficient to dissolve a virus vector disclosed herein. In other aspects of this embodiment, a virus vector disclosed herein may comprise a solvent, emulsion or a diluent in an amount of, e.g., less than about 90% (v/v), less than about 80% (v/v), less than about 70% (v/v), less than about 65% (v/v), less than about 60% (v/v), less than about 55% (v/v), less than about 50% (v/v), less than about 45% (v/v), less than about 40% (v/v), less than about 35% (v/v), less than about 30% (v/v), less than about 25% (v/v), less than about 20% (v/v), less than about 15% (v/v), less than about 10% (v/v), less than about 5% (v/v), or less than about 1% (v/v). In other aspects of this embodiment, a virus vector disclosed herein may comprise a solvent, emulsion or other diluent in an amount in a range of, e.g., about 1% (v/v) to 90% (v/v), about 1% (v/v) to 70% (v/v), about 1% (v/v) to 60% (v/v), about 1% (v/v) to 50% (v/v), about 1% (v/v) to 40% (v/v), about 1% (v/v) to 30% (v/v), about 1% (v/v) to 20% (v/v), about 1% (v/v) to 10% (v/v), about 2% (v/v) to 50% (v/v), about 2% (v/v) to 40% (v/v), about 2% (v/v) to 30% (v/v), about 2% (v/v) to 20% (v/v), about 2% (v/v) to 10% (v/v), about 4% (v/v) to 50% (v/v), about 4% (v/v) to 40% (v/v), about 4% (v/v) to 30% (v/v), about 4% (v/v) to 20% (v/v), about 4% (v/v) to 10% (v/v), about 6% (v/v) to 50% (v/v), about 6% (v/v) to 40% (v/v), about 6% (v/v) to 30% (v/v), about 6% (v/v) to 20% (v/v), about 6% (v/v) to 10% (v/v), about 8% (v/v) to 50% (v/v), about 8% (v/v) to 40% (v/v), about 8% (v/v) to 30% (v/v), about 8% (v/v) to 20% (v/v), about 8% (v/v) to 15% (v/v), or about 8% (v/v) to 12% (v/v).

Aspects of the present specification disclose, in part, treating an individual suffering from a disease or disorder.

As used herein, the term "treating," refers to reducing or eliminating in an individual a clinical symptom of the disease or disorder; or delaying or preventing in an individual the onset of a clinical symptom of a disease or disorder. For example, the term "treating" can mean reducing a symptom of a condition characterized by a disease or disorder, by, *e.g.,* at least 20%, at least 25%, at least 30%, at least 35%, at least 40%, at least 45%, at least 50%, at least 55%, at least 60%, at least 65%, at least 70%, at least 75%, at least 80%, at least 85%, at least 90% at least 95%, or at least 100%. The actual symptoms associated with a specific disease or disorder are well known and can be determined by a person of ordinary skill in the art by taking into account factors, including, without limitation, the location of the disease or disorder, the cause of the disease or disorder, the severity of the disease or disorder, and/or the tissue or organ affected by the disease or disorder. Those of skill in the art will know the appropriate symptoms or indicators associated with a specific type of disease or disorder and will know how to determine if an individual is a candidate for treatment as disclosed herein.

In aspects of this embodiment, a therapeutically effective amount of a virus vector disclosed herein reduces a symptom associated with a disease or disorder by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 100%. In other aspects of this embodiment, a therapeutically effective amount of a virus vector disclosed herein reduces a symptom associated with a disease or disorder by, e.g., at most 10%, at most 15%, at most 20%, at most 25%, at most 30%, at most 35%, at most 40%, at most 45%, at most 50%, at most 55%, at most 60%, at most 65%, at most 70%, at most 75%, at most 80%, at most 85%, at most 90%, at most 95% or at most 100%. In yet other aspects of this embodiment, a therapeutically effective amount of a virus vector disclosed herein reduces a symptom associated with disease or disorder by, *e.g.,* about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%.

In one embodiment, a virus vector disclosed herein is capable of increasing the level and/or amount of a protein encoded in the virus vector that is administered to a patient by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% as compared to a patient not receiving the same treatment. In other aspects of this embodiment, virus vector is capable of reducing the severity of a disease or disorder in an individual suffering from the disease or disorder by, *e.g.*, about 10% to about 100%, about 20% to about 100%, about 30% to about 100%, about 40% to about 100%, about 50% to about 100%, about 60% to about 100%, about 70% to about 100%, about 80% to about 100%, about 10% to about 90%, about 20% to about 90%, about 30% to about 90%, about 40% to about 90%, about 50% to about 90%, about 60% to about 90%, about 70% to about 90%, about 10% to about 80%, about 20% to about 80%, about 30% to about 80%, about 40% to about 80%, about 50% to about 80%, or about 60% to about 80%, about 10% to about 70%, about 20% to about 70%, about 30% to about 70%, about 40% to about 70%, or about 50% to about 70% as compared to a patient not receiving the same treatment.

In aspects of this embodiment, a therapeutically effective amount of a virus vector disclosed herein increases the amount of protein that is encoded within the virus vector in an individual by, *e.g.,* at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 100% as compared to an individual not receiving the same treatment. In other aspects of this embodiment, a therapeutically effective amount of a virus vector disclosed herein reduces the severity of a disease or disorder or maintains the severity of a disease or disorder in an individual by, *e.g.,* at most 10%, at most 15%, at most 20%, at most 25%, at most 30%, at most 35%, at most 40%, at most 45%, at most 50%, at most 55%, at most 60%, at most 65%, at most 70%, at most 75%, at most 80%, at most 85%, at most 90%, at most 95% or at most 100%. In yet other aspects of this embodiment, a therapeutically effective amount of a virus vector disclosed herein reduces or maintains the severity of a disease or disorder in an individual by, *e.g.*, about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%.

A virus vector is administered to an individual or a patient. An individual or a patient is typically a human being, but can be an animal, including, but not limited to, dogs, cats, birds, cattle, horses, sheep, goats, reptiles and other animals, whether domesticated or not.

In an embodiment, a virus vector of the present invention can be used to create an AAV that targets a specific tissue including, but not limited to, the central nervous system, retina, heart, lung, skeletal muscle and liver. These targeted virus vectors can be used to treat diseases that are tissue specific, or for the production of proteins that are endogenously produced in a specific normal tissue, such as a Factor IX (FIX), Factor VIII, FVIII and other proteins known in the art.

Diseases of the Central Nervous System

In an embodiment, diseases of the central nervous system can be treated using an AAV, wherein the AAV comprises a recipient AAV that can be any AAV serotype and a donor capsid that is selected from one or more of AAV1, AAV2, AAV3, AAV4, AAV5, AAV7, AAV8, AAV9 or AAV10. In one embodiment, the recipient AAV is an AAV2 and the donor capsid that is selected from one or more of AAV1, AAV2, AAV3, AAV4, AAV5, AAV7, AAV8, AAV9 or AAV10. In another embodiment, the recipient AAV is AAV3 and the donor capsid that is selected from one or more of AAV1, AAV2, AAV3, AAV4, AAV5, AAV7, AAV8, AAV9 or AAV10.

Diseases of the Retina

In an embodiment, diseases of the retina can be treated using an AAV, wherein the AAV comprises a recipient AAV that can be any AAV serotype and a donor capsid that is selected from one or more of AAV1, AAV2, AAV3, AAV4, AAV5, AAV7, AAV8, AAV9 or AAV10. In one embodiment, the recipient AAV is an AAV2 and the donor capsid that is selected from one or more of AAV1, AAV2, AAV3, AAV4, AAV5, AAV7, AAV8, AAV9 or AAV10. In another embodiment, the recipient AAV is AAV3 and the donor capsid is selected from AAV1, AAV2, AAV3, AAV4, AAV5, AAV7, AAV8, AAV9 or AAV10.

Diseases of the Heart

In a further embodiment, diseases of the heart can be treated using an AAV, wherein the AAV comprises a recipient AAV that can be any AAV serotype and the donor capsid that is selected from one or more of AAV1, AAV3, AAV4, AAV6 or AAV9. In an additional embodiment, the recipient AAV is an AAV2 and the donor capsid that is selected from one or more of AAV1, AAV3, AAV4, AAV6 or AAV9. In another embodiment, the recipient AAV is an AAV3, and the donor capsid that is selected from one or more of AAV1, AAV3, AAV4, AAV6 or AAV9.

Diseases of the Lung

In an embodiment, diseases of the lung can be treated using an AAV, wherein the AAV serotype comprises a recipient AAV that can be any AAV serotype and the donor capsid that is selected from one or more of AAV1, AAV5, AAV6, AAV9 or AAV10. In another embodiment, the recipient AAV is AAV2 and the donor capsid that is selected from one or more of AAV1, AAV5, AAV6, AAV9 or AAV10. In a further embodiment, the recipient AAV is AAV3 and the donor capsid is selected from that is selected from one or more of AAV1, AAV5, AAV6, AAV9 or AAV10.

Diseases of the Skeletal Muscle

In a further embodiment, diseases of the skeletal muscles can be treated using an AAV, wherein the AAV serotype comprises a recipient AAV that can be any AAV serotype and the donor capsid that is selected from one or more of AAV1, AAV2, AAV6, AAV7, AAV8, or AAV9. In another embodiment, the recipient AAV is AAV2 and the donor capsid that is selected from one or more of AAV1, AAV2, AAV6, AAV7, AAV8, or AAV9. In an embodiment, the recipient AAV is AAV3 and the donor capsid that is selected from one or more of AAV1, AAV2, AAV6, AAV7, AAV8, or AAV9.

Diseases of the Liver

In an embodiment, diseases of the liver can be treated using an AAV, wherein the AAV serotype comprises a recipient AAV that can be any AAV and the donor capsid that is selected from one or more of AAV2, AAV3, AAV6, AAV7, AAV8, or AAV9. In an additional embodiment, the recipient AAV is AAV2 and the donor capsid that is selected from one or more of AAV2, AAV3, AAV6, AAV7, AAV8, or AAV9. In a further embodiment, the recipient AAV is AAV3 and the donor capsid that is selected from one or more of AAV2, AAV3, AAV6, AAV7, AAV8, or AAV9.

## EXAMPLES

Example 1

Adeno-associated virus (AAV) vector has been used in over 100 clinical trials with promising results, in particular, for the treatment of blindness and hemophilia B. AAV is non-pathogenic, has a broad tissue tropism, and can infect dividing or non-dividing cells. More importantly, AAV vector transduction has induced long-term therapeutic transgene expression in pre-clinical and clinical trials. As of today, there are 12 serotypes of AAV isolated for gene delivery. Among them, AAV8 has been shown to be the best one for mouse liver targeting. Due to extensive studies in pre-clinical animals with FIX deficiency, Phase I/II clinical trials have been carried out using AAV2 and AAV8 in patients with hemophilia B. The results from these trials are very promising; however, the FIX expression from patients receiving AAV/FIX was not proportional to what has been achieved in animal models even though the same vector dosage/kg was used. When $1x10^{11}$ particles of AAV8 encoding FIX were used in FIX knockout mice for systemic administration, 160% of normal level FIX was detected in blood. However, when $2x10^{11}$ particles of AAV8/FIX were administered, only 40% of FIX was achieved in primates and less than 1% of FIX were found in human. The inconsistent FIX expression following AAV vector transduction among these species may be due to altered hepatocyte tropism in different species. Another interesting finding from AAV FIX clinical trials is the capsid specific cytotoxic T lymphocyte (CTL) response that eradicates AAV transduced hepatocytes and, thus, results in therapeutic failure. This phenomenon has not been demonstrated in animal models following AAV delivery, which points out another variation between preclinical and clinical studies. When a much higher dose of AAV/FIX vector was used, FIX expression was detected in both clinical trials using either AAV2 or AAV8; however the blood FIX level decreased at week 4 or 9 post injection, respectively. Further studies suggested that AAV vector infection elicited a capsid specific CTL response, which appeared to eliminate AAV transduced hepatocytes. Therefore, the results from these clinical trials highlight the necessity to explore effective approaches for enhancement of AAV transduction without increasing vector capsid burden. Any vector improvement that reduces AAV capsid antigen will also impact the daunting vector production concerns and be a welcome addition to viable gene therapy drug development.

Many strategies have been explored to increase AAV vector transduction. One strategy is to optimize the AAV vector cassette by utilization of a strong promoter and/or enhancer, codon-optimization of the transgenic cDNA, effective poly-adenylation sequence, and the use of a self-complementary vector genome if possible. At the level of the AAV capsid, much attention has been focused on employing natural serotypes that display differential tropisms, rationally designed capsids, or capsids selected or screened from a mutant capsid library. However, a drawback of this approach is that the relevant experiments cannot be performed in humans and interspecies variation in AAV capsid tropism continues to be observed given the continued collection of human data. A third method to enhance AAV vector transduction relies on altered cellular physiology via pharmacological agents. Many pharmacological agents have been used to enhance AAV transduction at various levels of infection; however, most of these drugs are used as cancer therapies and have severe side effects.

In our previous neutralizing antibody studies, it was found that human serum had an enhanced effect on AAV transduction. In this study, we have identified several proteins from human serum which directly interact with AAV virions and have the potential to impact AAV transduction. Among these proteins, the most interesting one is human serum albumin (HSA), a therapeutic agent that is the most abundant protein in the blood and has been widely used in clinical practice. If the interaction of HSA with AAV virions enhances AAV transduction, this approach can be immediately applied in AAV clinical trials. Herein, we demonstrated that the interaction of HSA with AAV vector enhances AAV transduction, and that this enhancement is not restricted to specific cells *in vitro* or tissues *in vivo.* Comparable enhancement was achieved regardless of incubation of HSA with AAV vectors before vector freezing or after thawing. Addition of HSA into vector preparations before dialysis did not impact HSA enhancement effect on AAV transduction. Mechanism studies suggest that HSA increased AAV binding to the target cell surface *in vitro* and resulted in the rapid clearance in blood after systemic administration. Neutralizing antibody (Nab) analysis demonstrated that the interaction of albumin with AAV still enhanced AAV transduction in the presence of Nab and didn't impact Nab activity. We applied this approach for treating hemophilia in FIX deficient mice. After systemic administration of AAV/FIX incubated with human albumin, increased transgene FIX expression and improved phenotypic correction were achieved.

***Human serum enhances AAV transduction.*** Our previous results demonstrated enhanced AAV transduction solely by the presence of human serum. We extended this finding to examine AAV transduction enhancement using 10 human serum samples and found that the interaction of human serum with AAV induced an approximately 4-fold increase in transgene activity in an AAV capsid-independent manner *in vitro* **(Fig. 1a**). Since AAV8 has been used in several clinical trials in patients with hemophilia, the AAV8 capsid was chosen for following experiments. Although enhanced transgene activity was observed at shorter durations, the greatest effect was achieved following serum incubation with AAV virions for ≥2h **(Fig. 1b**). To determine whether the enhancement effect of human serum on AAV transduction in Huh7 cells held true *in vivo,* AAV8/luc vectors were incubated with serially diluted serum and then administrated via retro-orbital or muscular injections (contralateral muscle received vector with no serum). As shown in **Figs. 1c** and **1d**, even a > 3000-fold serum dilution still enhanced AAV transduction in the liver and muscles by 2-5 fold or 4-16 fold, respectively. The enhancement of AAV transduction was also demonstrated following incubation of AAV vectors with serum from other species including mouse, dog, primate and fetal bovines **(Figs. 9** and **10).**

***Human serum albumin exerts enhancement effect on AAV transduction.*** The data from the above experiments strongly suggest that some component(s) of serum enhances AAV transduction *in vitro* and *in vivo.* To examine whether the enhancement on transduction requires direct interaction of the AAV virion with a serum protein(s), we designed 5 cohorts: 1. Huh7 cells in complete medium and AAV incubated with PBS, 2. Huh7 cells in complete medium and AAV incubated with human serum, 3. Huh7 cells in serum free medium and AAV incubated with PBS, 4. Huh7 cells in serum free medium and AAV incubated with PBS, and then the same amount of serum was added to culture medium just before application of virus on cells, 5. Huh7 cells in serum free medium and AAV incubated with human serum. Similar to the methodology described above, enhanced transduction was achieved in cohort 2 when compared to cohort 1. Interestingly, no increase of AAV transduction was observed in cohort 4 with a high dilution of serum when compared to group 3, while increased transduction was obtained in cohort 5 **(Fig. 2a).** These results suggest that the human serum mediated enhancement of AAV transduction requires the direct interaction of the human serum protein(s) with AAV virions. It is interesting to note that the fold increase was apparently much larger in the "serum free" group than in the "complete medium" group at 4 to 16 fold dilutions of human serum. This is because complete medium contains fetal bovine serum (FBS) which enhances AAV transduction. When AAV vectors incubated with PBS are added to cells maintained in complete medium, AAV vectors will interact with FBS proteins which induce higher transduction than that AAV vectors are applied to cells in the serum free medium (data not shown). To identify which serum proteins augment AAV transduction, human serum was incubated with AAV8 vectors and then an antibody that recognizes intact AAV8 virions was used to pull down AAV8 binding proteins for mass spectrometry analysis. Among the proteins identified, the most interesting one is human serum albumin **(Table 5).** Serum albumin is the most abundant protein in the circulation and has been widely used in many clinical settings, and therefore, the primary objective of this study is to investigate the effect of HSA on AAV transduction.

To further confirm the mass spectrometry data of HSA binding to AAV8, we incubated AAV8 particles with HSA and then used the human albumin antibody to pull down albumin bound AAV particles. The AAV genome copy number was then quantitated by Q-PCR. As shown in **Fig. 2b,** the immunoprecipitation with the albumin specific antibody (A80-129A, Bethyl Lab, INC) resulted in twice as many genomes pulled down compared to the controls (isotype IgG or PBS). To examine whether the interaction of human albumin with AAV virion impacted AAV transduction, we incubated AAV8 particles with HSA depleted serum (> 99% depletion, **Fig**. **11)** or recombinant HSA. It was demonstrated that the transduction from human albumin depleted serum was lower than the complete serum treated vector **(Fig. 2c).** AAV vectors incubated with recombinant HSA (rHSA) also resulted in higher transduction, but to a lower extent compared to human whole serum **(Fig. 2d).** To explore whether HSA enhances AAV transduction *in vivo,* AAV8/luc vectors were incubated with different concentrations of rHSA and then administered into mice via retro-orbital or muscular injection. Following systemic administration, vectors pre-treated with HSA demonstrated increased liver transduction (1.5- to 8-fold **(Fig. 12a)).** Consistent with the stimulation of AAV transduction by human serum in muscle, higher transduction in muscle was observed (2.1- to 11.5-fold) following incubation of AAV8 vectors with rHSA **(Fig. 12b).** These results implicate that human serum albumin increases AAV transduction *in vitro* and *in vivo.*

***Enhancement effect of clinical grade HSA on AAV transduction.*** Since HSA has been widely applied in clinic, we then tested whether clinical grade HSA also has the ability to enhance AAV transduction. When 5% clinical grade HSA, which is identical to the serum albumin concentration in the blood of normal subjects, was incubated with AAV vectors at different dilutions, increased AAV transduction was observed *in vitro* even at a dilution of 20,000-fold **(Fig. 3a).** Next, we incubated AAV8/luc with 25% HSA at different fold dilutions prior to retro-orbital or muscular injection. A one-fold dilution is defined as $1x10^{12}$ AAV particles incubated with 10 ul of 25% HSA in 1ml solution. As shown in **Figs. 3b** and 3c, clinical grade HSA significantly increased AAV8 transduction by about 3- or 5-fold in the liver and muscle, respectively. Next, the long-term effect of HSA on AAV transduction was documented at weeks 1, 2, 4, and 7 after muscular injection **(Fig. 13).** These results indicate that clinical grade HSA enhances AAV transduction in the muscle for sustained transgene expression. Also, we observed that incubation of HSA with AAV2 or AAV9 induced much higher transduction *in vitro* and *in vivo* **(Fig. 14).**

***The enhancement of AAV transduction by HSA is not altered by freeze/thaw.*** In the above experiments, AAV preparations were thawed and incubated with HSA before being administered to cells or mice. In the clinical setting, it may not be practical for the medical staff to perform this incubation immediately prior to injection. Therefore, incubation of HSA with AAV vectors prior to storage at -80°C would simplify the translation of HSA-enhanced AAV vector transduction. To investigate this, we first incubated AAV vectors with clinical grade HSA for 2 hours at 4°C. Half of the solution was stored in -80°C for three days, while the other aliquot of AAV virus was immediately used to infect Huh7 cells at a dose of $1x10^3$ particles/cell. After thawing the frozen HSA-AAV preparation, vector transduction was analyzed in Huh7 in the same manner. As shown in **Fig**. **4a,** a similar increase in luciferase activity was observed regardless of HSA-vector cryopreservation. HSA's enhancement of AAV transduction following incubation and cryopreservation was also observed after muscular injection **(Figs. 4b** and **4c).**

***AAV transduction following HSA addition to AAV preparations before dialysis.*** During vector production, it is necessary to perform vector dialysis to remove high concentrations of salt regardless of methods used for purification (CsCl or column chromatography). To determine if the incubation of AAV vectors in HSA during dialysis impacts AAV transduction, AAV8/luc vectors purified by CsCl gradient ultra-centrifugation or by anion exchange column were mixed with 10ul of 25% HSA or PBS in 1 ml of $10^{12}$ particles just before dialysis. Then, these formulations were dialyzed against PBS and AAV transduction was analyzed in mice via retro-orbital or direct muscular injection. As shown in **Fig. 5,** HSA incubation during dialysis still increased vector transduction in the liver and muscle by greater than 2-fold or 4-fold, respectively, as compared to the PBS incubationcontrol. The enhancement effect was similar for different approaches of purification. This observation implicates that human albumin could be added to AAV preparations before dialysis of vectors purified in different manners in order to enhance gene delivery.

***Albumin increases AAV binding capacity to target cells.*** The first step for effective AAV transduction is AAV virion binding on the target cells via primary and secondary receptors. To examine whether incubation of albumin with AAV vectors increases cellular binding, AAV8/luc vectors were incubated with HSA or PBS. Then, Huh7 cells were added at 4°C to prevent vector internalization, as shown in our previous study. After extensive washes, total DNA was recovered and AAV genome copy number was determined by Q-PCR. As shown in **Fig. 6a,** incubation with HSA significantly increased AAV vector binding to Huh7 cells by 3-fold. To determine if HSA increases vector binding and uptake by the liver, $1x10^{11}$ particles of AAV8/luc, pre-incubated in HSA or PBS, was administered via retro-orbital injection and luciferase activity was determined 24 hours later. Consistent to the result observed in Huh7 cells, higher transduction was achieved in the liver **(Figs. 6b** and **6c).** Forty eight hours post-injection, mice were sacrificed and the liver was harvested for quantitation of luciferase activity and AAV genome copy number. Similar to live imaging analysis, higher luciferase activity and AAV genome copy number were found in the livers of mice administered with AAV vectors pre-treated with HSA compared to those given AAV vectors incubated in PBS alone **(Figs. 6d** and **6e).** The result of more AAV vector uptake by the liver with HSA pre-incubation was correlated to vector clearance from the blood. After administration of AAV vector, there was a marginal decrease in AAV genome copy number per microliter of plasma in mice receiving HSA treated AAV vector, as compared to control mice at 15min and 24hr post injection ($p>0.05$). However, a significant reduction of AAV genome copy number was observed in the HSA cohort at 2hr after AAV administration ($p<0.05$). These results suggest that enhanced AAV vector transduction by HSA results from increased particle binding to target cells.

***Interaction of albumin with AAV does not interfere with neutralizing antibody activity.*** To investigate whether the interaction of human albumin with AAV virions blocks AAV neutralizing antibody (Nab) activity, we performed the Nab assay *in vitro.* IVIG is the pooled sera from over 1000 subjects and contains AAV Nab against different serotypes. We first incubated AAV8/Luc virions with 100-fold dilution of HSA or PBS, and then added IVIG at different concentrations. After transduction in Huh7 cells, the Nab titer was calculated. As shown in **Fig. 7a,** the same Nab titer (1:200 of IVIG) was obtained regardless of AAV vector pre-incubated with HSA. We also studied whether HSA is still able to enhance AAV transduction in the presence of AAV Nab, and found that the incubation of HSA with AAV increased AAV transduction with similar efficiency in the presence of different amount of IVIG **(Fig. 7b).** These results suggest that interaction of HSA with AAV does not impact AAV virus infection mechanism.

***Improved phenotypic correction of hemophilia B using human albumin to enhance AAV vector transduction.*** To study the phenotypic correction using AAV vectors incubated with HSA, we used hemophilia B mice as a disease model and AAV8/FIX-OPT, which has been used in Phase I clinical trials in patients with hemophilia B. After injection, FIX concentration and function were determined at different time points and phenotypic correction was assessed at week 6. As shown in **Fig**. **8a,** over 5-fold higher FIX levels were detected in mice receiving has incubated AAV8/FIX-OPT than those with the same vectors treated with PBS. Similarly, plasma FIX activity was much higher in mice receiving vector treated with HSA **(Fig. 8b).** At 6 weeks post AAV injection, all mice underwent a tail vein transection bleeding challenge to assess *in vivo* function of the vector-expressed human factor IX. Untreated hemophilia B mice had profound bleeding (30mg of blood/g of mice body weight) following the challenge compared to WT controls. Hemophilia B mice receiving AAV vectors incubated in HSA demonstrated a significant decrease in blood loss compared to AAV vectors incubated in PBS (*$p<0.05$,* **Fig. 8c).** In fact, hemophilic mice treated with vectors incubated in HSA demonstrated blood loss similar to that of WT controls. These results demonstrate improved correction of hemophilia B using AAV vectors pre-incubated with HSA, and also suggest possible utilization of this formulation to increase efficacy at lower vector doses for the treatment of hemophilia and other diseases.

Observations of lower FIX expression and capsid-specific CTL responses to the AAV capsid at high doses in human AAV FIX trials have emphasized the need for more efficient strategies that maintain efficient gene delivery at lower doses. Our earlier report noted that AAV transduction was enhanced by human serum; however, the precise component(s) was not identified. Therefore, for the search of more efficient AAV vectors, the objective of this study was to identify specific protein(s) from human serum that interact with AAV virions to induce higher transduction. Of AAV8 capsid interacting proteins identified from mass spectroscopy analysis **(Table** 5), further experimentation was pursued with HSA. These investigations demonstrated that incubation of AAV8 with recombinant or clinical grade HSA increased AAV transduction while human albumin depleted serum decreased transduction. Clinical grade HSA significantly enhanced AAV transduction in the liver and skeletal muscles of mice. To facilitate the application of HSA in AAV vector production and clinical trials, our studies demonstrated that freezing AAV vectors after incubation with human albumin or addition of HSA into AAV preparations before dialysis still resulted in enhanced transduction. Mechanism studies suggested that human albumin increased AAV vector binding to the target cell surface and resulted in faster blood clearance after systemic administration but did not impact AAV infection pathway. Finally, in a preclinical mouse model of hemophilia B, AAV vectors incubated with albumin increased human FIX expression and improved the bleeding phenotype to WT levels.

Serum proteins are able to interact with viruses and impact virus infection. For example, adenovirus has been widely studied for its interaction with serum proteins including coagulation factors and complements for liver targeting. Our previous Nab study demonstrated that serum at the dilution without Nab activity actually enhanced AAV transduction regardless of serotype. Other studies have found that several serum proteins have an effect on AAV transduction via interaction with AAV virions. Denard et al. have identified galectin 3 binding protein (G3BP) and C-reactive protein (CRP) which interact with AAV. They showed that the interaction of G3BP with AAV virions led to the formation of AAV aggregates which block AAV transduction, and that interaction of CRP with AAV resulted in higher transduction. The CRP enhancement of AAV transduction is species specific and AAV serotype specific. In another study, Sais et al. demonstrated that the AAV2 capsid binds to C3 complement proteins to enhance macrophage uptake of AAV and induce macrophage activation. In this study, incubation of AAV and clinical grade HSA enhances transduction *in vitro* as well as *in vivo.* Generally, the enhancement of human albumin on AAV transduction is lower than whole serum. This finding implicates that other proteins in the serum may also play a role to enhance AAV transduction. It will be worthwhile to study how the interaction of these proteins impacts AAV transduction.

It is noted that the enhancement of AAV transduction with human albumin treated virus in muscle is generally higher than that in liver. An explanation of this phenomenon could be that blood contains a very high concentration of albumin, which enhances transduction to some extent following systemic injections. In contrast, less albumin resides in the muscle tissue, so after muscular injection, the magnitude of increased transduction by albumin is greater than that observed following IV injections. After systemic administration of AAV vector, the AAV virions will immediately interact with albumin. However, our studies demonstrated that the longer incubation of HSA with AAV virions induced higher enhancement of transduction. This result indicates that further enhancement of transduction should be achieved by pre-incubation of HSA with AAV virions following systemic administration. In this study, the transduction enhancement from AAV virions incubated with human serum albumin was achieved in the liver and muscles after systemic administration and direct injection, respectively. Since the liver takes up more AAV virions from circulation after systemic administration, it is possible that less AAV vector will be escaped from blood to transduce other tissues like heart and skeletal muscle. On another hand, AAV vector treated with albumin increases muscular transduction. It is unknown whether the enhanced transduction in heart or skeletal muscle can be achieved after systemic administration of AAV vectors incubated with albumin. To increase AAV transduction in heart and skeletal muscle after systemic administration, several liver detargeted AAV mutants (AAV2i8 and AAV9.45) have been developed, it is under way to test whether transduction enhancement in muscle will be achieved after systemic administration of these liver detargeted AAV vectors incubated with albumin.

Albumin is emerging as a versatile protein carrier for drug targeting and for improvement of the pharmacokinetic profile of peptide- or protein-based drugs. Several albumin receptors have been described that induce endocytosis. The pathway for albumin endocytosis is cell type dependent and includes either clathrin- or caveolin-mediated endocytosis. Although it is unknown how AAV vector interacts with albumin, this study demonstrated that interaction of AAV with albumin increases AAV binding ability of target cells. This can be explained by the fact that albumin, after interaction with AAV, provides another layer for AAV binding on the cell surface via albumin receptors.

Albumin has a prolonged half-life in the blood. It has now become apparent that homeostatic regulation of albumin is controlled by the neonatal Fc receptor (FcRn). FcRn rescues albumin from degradation in cells by binding albumin within intracellular endosomal compartments, which then results in transport of the ternary complex to the cell membrane for release of ligands back into the circulation. Due to these properties of albumin, there are some questions about the effect of interaction of albumin with AAV vector on transduction. Since albumin uptake is via either clathrin- or caveolin-mediated endocytosis, and AAV cellular entry is by clathrin-mediated endocytosis, it is unknown whether albumin and AAV compete in the endocytosis pathway. Another question is whether albumin disassociates from AAV in the endosome or trafficking into the nucleus. The third question is whether albumin exocytosis or transcytosis impacts AAV transduction. The fourth one is the effect of enhanced transduction from interaction with albumin and AAV on AAV capsid specific CTL response. Although the interaction of albumin with AAV virions increases AAV binding to target cell surface, it is possible that the interaction may influence AAV trafficking intracellularly. Further elucidation of these issues will help design more effective approaches to the use of albumin in AAV gene therapy.

Taken together, our study demonstrated that AAV capsids interact with human serum albumin, which increases transduction *in vitro* and *in vivo.* The transduction efficiency enhancement from clinical grade human albumin also allowed phenotypic correction in a hemophilia B mouse model after systemic administration of an otherwise suboptimal dose AAV vector. For clinical purposes, addition of human albumin into AAV virus preparations before dialysis or freezing AAV virus after incubation with albumin still results in transduction enhancement. Although the exact mechanism of AAV virion interaction with human albumin is unknown, our findings are important for immediate inclusion of HSA into diverse clinical applications suffering from subpar transduction at tolerated vector doses. Therefore, our results from these studies strongly indicate that incubation of clinical grade human albumin with AAV vectors during dialysis should be performed to enhance AAV transduction efficiency in future clinical trials.

***Cell lines.*** HEK293 and Huh7 cells (from ATCC) were maintained at 37°C in 5% CO2 in Dulbecco's modified Eagle's medium supplemented with 10% fetal bovine serum and penicillin-streptomycin.

***AAV virus production.*** AAV vector was produced using a standard approach with three-plasmid transfection in HEK293 cells. Briefly, AAV transgene plasmid pTR/CBA-luc or pTTR/FIX-opt was co-transfected with AAV helper plasmid and adenovirus helper plasmid pXX6-80 into HEK293 cells. Sixty hr later, cells were harvested and lysed, and cell lysate was applied for ultra-centrifugation against CsCl gradient or purification with column. AAV virions were collected and tittered by dot-blot.

Individual human serum was purchased from Valley Biomedical (Minchester, VA), and aliquoted and stored at -80°C for future use.

***AAV transduction assay in vitro.*** 1x$10^5$ Huh7 cells were seeded on a 48-well plate in 300 uL DMEM containing 10% FBS or serum-free medium. AAV/luc was incubated with serum or rHSA (Sigma-Aldrich, St. Louis, MO) or clinical grade HSA (Albuminar, CSL Behring LLC, Kankakee, IL). The mixture was then added to the indicated cells. Forty-eight hr later, cells were lysed with passive lysis buffer (Promega) and luciferase activity was measured with a Wallac1420 Victor 2 automated plate reader. The fold increase of transgene expression was calculated as the transgene expression from serum or albumin treated groups compared to that from PBS.

***Animal experiments.*** All mice were maintained in specific pathogen-free facilities according guidelines instituted by the animal committees of the University of North Carolina at Chapel Hill. All animal experiments were reviewed and approved by the University of North Carolina Institutional Animal Care and Usage Committee. The animal experiments were performed in hemophilia B (FIX-/-) mice or normal C57BL/6 mice (purchased from Jackson Laboratories, Bar Harbor, ME). For systemic administration, 1x$10^{10}$ particles of AAV/luc vector were incubated with serum or human albumin for 2 hr at 4°C followed by retro-orbital administration into adult female C57BL mice. At the indicated time points, imaging was performed using a Xenogen IVIS Lumina imaging system (Caliper Lifesciences, Hopkinton, MA) following intraperitoneal injection of D-luciferin substrate at 120 mg/kg (Nanolight, Pinetop, AZ). Bioluminescent images were analyzed using Living Image software. For muscular injection, 1x$10^9$ particles of AAV/luc vector were incubated with serum or human albumin for 2 hr at 4°C. Then the mixture was directly injected into the hind leg muscles of 6-8 week old C57BL mice. At the indicated time points, imaging was performed and bioluminescent images were analyzed.

For hemophilia B studies, adult male hemophilia B mice were injected with 2x$10^9$ particles of AAV8/FIX vectors via the tail vein. At indicated time points, blood was collected from the retro-orbital venous plexus under anesthesia using isoflurane. At week 6 after AAV8/FIX injection, *in vivo* bleeding analysis was performed.

***Human albumin depletion.*** A Pierce™ albumin depletion kit (Cat # 85160, Pierce Biotechnology, Rockford, IL, USA) was used following the company instruction with slight modification. Briefly, after transferring the resin into the column and centrifugation at 12,000rpm for 1 min, the column was washed and loaded with 50 uL of pre-tested AAV Nab negative serum. After centrifugation, the flow through was applied to new resin treated column and above steps were repeated. To achieve maximum depletion of albumin, the flow through was applied for two more times and a total of 4 columns were used for one sample. 50 uL of binding/washing buffer was added to the column to release unbound proteins and centrifuged. The final flow through was applied for detection of albumin using ELISA kit.

***Co-immunoprecipitation.*** Coimmunoprecipitation of serum proteins was performed with Pierce Co-Immunoprecipitation (Co-IP) Kit (Cat# 26149, Pierce Biotechnology, Rockford, IL, USA). First, antibody immobilization was performed. After addition of the resin slurry into a Spin Column and centrifugation, the column was washed and inserted with the bottom plug. Then, diluted antibodies and the Sodium Cyanoborohydride Solution were directly added to the resin in the spin column sequentially, and incubated for 2hrs at RT. After centrifugation and washing, Quenching buffer was added to the column and centrifuged. Quenching buffer was applied to the resin, followed by addition of Sodium Cyanoborohydride Solution for 15 minutes. After centrifugation and washing, the mixture of AAV virus with human serum or PBS was transferred to the resin and incubated for 2hrs at 4°C. After centrifugation and washing, elution buffer was added and incubated for 5 minutes and centrifuged; the flow through solution was collected for mass spectrometry analysis or AAV genome number quantitation by Q-PCR.

***Mass spectrometry.*** The proteins were reduced, alkylated, and digested with trypsin using the FASP protocol. The peptides were resuspended in 2% acetonitrile/98% (0.1% formic acid) prior to analysis by LC-MS/MS. Briefly, the peptides were loaded onto a 2 cm long X 360 $\mu$m o.d. $\times$ 100 $\mu$m i.d. microcapillary fused silica pre-column packed with Magic 5 $\mu$m C18AQ resin (Michrom Biosciences, Inc.). After sample loading, the pre-column was washed with 95% Solvent A (0.1% formic acid in water)/5% Solvent B (0.1% formic acid in Acetonitrile) for 20 min at a flow rate of 2 uL/min. The pre-column was then connected to a 360 $\mu$m o.d. $\times$ 75 $\mu$m i.d. analytical column packed with 22 cm of 5 $\mu$m C18 resin. The peptides were eluted at a flow rate of 250 nL/min by increasing the percentage of solvent B to 40% with a Nano-Acquity HPLC solvent delivery system (Waters Corp.). The LC system was directly connected through an electrospray ionization source interfaced to an LTQ Orbitrap Velos ion trap mass spectrometer (Thermo Fisher Scientific). The mass spectrometer was controlled by Xcalibur software and operated in the data-dependent mode, in which the initial MS scan recorded the mass to charge (m/z) ratios of ions over the range 400-2000. The 10 most abundant ions were automatically selected for subsequent collision-activated dissociation. All files were searched using MASCOT (Matrix Science, Ver. 2.3.02) via Proteome Discoverer (Thermo., Ver. 1.3.0.339) against the database containing human proteins downloaded from Uniprot. The search parameters included peptide mass tolerance of 10 ppm and a fragment ion tolerance of 0.6 mass units. The search allowed for variable modifications of oxidation of Met and carbamidomethyl of Cys. Each sample was run 2 times (R1 and R2). Two fold difference between AAV sample and PBS was considered positive.

***Quantitation of luciferase expression in the tissues.*** Animals utilized for imaging studies were sacrificed two weeks after AAV injection and the following organs were collected: liver, spleen, kidney, heart, lung, skeletal muscle (gastrocnemius), and brain. Tissue was minced and homogenized in passive lysis buffer (Promega, Madison, WI). Tissue lysates were centrifuged at 10,000 rpm for 5 minutes to remove cellular debris. Supernatant was transferred to 96-well plates for luciferase activity analysis as described above. Total protein concentration in tissue lysates were measured using the Bradford assay (BioRad, Hercules, CA).

***AAV genome copy number analysis.*** For determining blood clearance rates of various rAAV vectors, plasma was obtained from mice at 2, 6, 24, and 48 hour after intravenous administration of rAAV vectors. Viral DNA isolation from plasma was performed using the DNeasy Blood & Tissue kit (QIAGEN, CA) following the manufacture's instruction. Viral genomes were quantified by real-time PCR with forward primer: 5'-AAAAGCACTCTGATTGACAAATAC-3' (SEQ ID NO:127) and reverse primer: 5'-CCTTCGCTTCAAAAAATGGAAC-3' (SEQ ID NO:128). Real-time PCR was performed on a LightCycler 480 (Roche Diagnostics Cooperation, Indianapolis, IN) instrument. A 10 uL final volume of absolute quantitation reaction was performed using SYBR green (Roche Diagnostics Cooperation, Indianapolis, IN) mix supplemented with 0.2uM primers. No template control was included in each run to rule out the possibility of contamination for each primer-probe set. The reaction was amplified at 95°C for 10 min followed by 45 cycles of 10 s at 95°C, 10 s at 60°C, and 10 s at 72°C, followed by a melting cycle. Each gene was accessed in duplicates. Absolute quantification was performed based on second-derivative maximum comparisons to standard curves of plasmid DNA (luciferase).

To detect AAV genome copy number in different tissues, the animals were sacrificed and the selected organs were harvested at week 2 after iv injection of rAAV. After DNA isolation using the DNeasy Blood & Tissue kit (QIAGEN, CA), real-time PCR was performed on cach sample for both the luciferase gene and the mouse *Mus musculus* Lamin B2 gene. The primers used for mouse *Mus musculus* Lamin B2 gene were: 5'-GGACCCAAGGACTACCTCAAGGG-3' (SEQ ID NO:129) (forward) and 5'-AGGGCACCTCCATCTCGGAAAC -3' (SEQ ID NO:130) (reverse). The copy of genome was analyzed by Lightcycler software v.4.5 (Roche Diagnostics Cooperation, Indianapolis, IN) based on those of pTR-CBA-Luciferase plasmid used in initial transduction and the endogenous gene.

***AAV binding assay.*** $5x10^{10}$ particles of AAV/luc vector were incubated with clinical grade human serum albumin at 4°C for different durations. Then pre-chilled $5x10^5$ Huh cells were added to AAV vector for 30 min at 4°C. Cells were washed four times with cold PBS and transferred to a new tube. DNA from cells was extracted and applied for Q-PCR to determine AAV genome copy number per cell using luc specific primers.

***Neutralizing antibody analysis.*** Nab assay was carried out as described in our previous study with slight modifications. Briefly, $1x10^8$ particles of AAV8/Luc vector were incubated with 100 fold dilution of 25% HSA at 4°C for 2 hours, then IVIG at different dilution was added for another 2 hours. The mixture was applied to infect Huh7 cells. 48 hours later, luciferase activity was analyzed from cell lysate and neutralizing antibody titer was calculated.

***Human factor IX antigen and activity assays.*** The human factor IX antigen one-stage human factor IX activity assay was performed as previously described. The specific activity of factor IX, expressed as units of factor IX activity per milligram of protein (U/mg), was calculated by dividing the factor IX activity (U/ml) by the concentration of the factor IX protein (factor IX antigen) (mg/ml).

***In vivo bleeding model.*** *In vivo* bleeding was analyzed as previously described by Meeks, et al with slightly modification. After anesthesia, 3mm of the distal tail was transected and the proximal tail was placed into the pre-warmed and pre-weighed tube. Forty minutes after the tail clip or before death due to bleeding, blood loss per gram body weight was calculated.

***Statistical analysis.*** Quantitative data were presented as means ± SD. The Student t test was used to perform all statistical analyses. P values less than 0.05 were considered a statistically significant difference.

<u>Example 2</u>

Therapeutic transgene expression has been successfully achieved in patients with hemophilia after systemic administration of adeno-associated virus (AAV) vector. Numerous preclinical studies have demonstrated that long term transgene expression is induced by AAV mediated delivery transgene. Specifically for hemophilia treatment with AAV vector, although encouraging data has been generated in hemophilic animal models with intra-muscular injection of AAV vector and long-term transgene expression was found in muscles in patients with hemophilia B after IM, the therapeutic transgene FIX was not detected in the blood. The liver is the natural organ to synthsize hemophilic factors (FIX and FVIII). In the clinical trials, both AAV2 and AAV8 have been used to deliver FIX for liver targeting in patients with hemophilia B. After adminsitration of AAV vectors into blood, the virus first will encounter the serum proteins. The interaction of virus with serum proteins may impact AAV transduction in the liver. Our previous study has identified several proteins by mass spectrometry analysis and demonstrated that human serum albumin enhances AAV transduction by direct interaction with AAV virions. However there are several questions needed to be addressed. Do all other AAV binding serum proteins also impact AAV transduction? Does the combination of the serum proteins further enhance AAV transduction? Among AAV8 binding proteins idenified by mass spectrometry, aside from albumin, transferrin and apolipoprotein B (ApoB) are the more interesting ones since they have receptors on the liver. Transferrins are iron-binding blood plasma glycoproteins that control the level of free iron in the blood and other tissue fluids. Transferrin protein loaded with iron binds to a transferrin receptor and is transported into the cell by receptor-mediated endocytosis. ApoB is the primary apolipoprotein of chylomicrons, VLDL, IDL, and LDL particles, and ApoB are essential for the formation of LDL particles. ApoB on the LDL particle acts as a ligand for LDL receptors to deliver fats into the cells. In the following study, we studied the effect of LDL and transferrion on AAV transduction in the liver, and the effect of the combination of serum proteins on AAV liver transduction. Our results have shown that inertaction of LDL or transferrin with AAV8 virions enhanced AAV liver transduction. However, there was no effect of the combination of three proteins (HSA, LDL, transferrin) on further enhancement of AAV transduction.

***Incubation of AAV8 with LDL or transferrin increases transduction in Huh cells.*** Since transferrin and apoB can specifically bind to transferrin receptor and LDL receptor on hepatocytes, we presumed that interaction of transferrin or apoB with AAV virions was capable of enhancing transduction. AAV8/luc vectors which encode firefly luciferase gene were incubated with different dilutions of normal blood concentration of LDL or transferrin for 1hr at 4°C, then transduced onto Huh7 or 293 cells. Forty eight hr later, the cell lysate was collected for luciferase analysis. As we reported before, interaction of AAV8 with human albumin increased AAV8 transduction in Huh7 cells even at the dilution of 1:10000 **(Fig. 15).** We also found that transferrin and LDL exerted the enhancement of AAV8 transduction in Huh7 cells at the dilution of 1:1000 and 1:100, respectively. No marked increase of transgene expressoion was shown in 293 cells regardless of different serum proteins or the dilituions **(Fig. 15).** This result implicates that interaction of LDL or transferrion with AAV8 augments transduction in hepatocytes.

***AAV8 does not utilize LDL or transferrion receptors for liver transduction.*** It has been shown that LDL or transferrion receptors are used for effective liver infection of some viruses. Blocking of these receptors with injection high dose of LDL or lactoferrin decreases these viruses infectivity in the liver of mice. The primary receptors for other serotypes have been identified, but it is unknown which primary receptors are used by AAV8 for effective transduction. AAV8 has been shown to be the best serotype to transduce mouse liver. To study whether AAV8 binds to LDL or transferrin receptors for target cell transduction, we administered 0.5 mg of human LDL (which saturate the LDL receptors) or 1mg of lactoferrin (which saturates the LRP as well as HSPG) into mice, and 5 min later, $1x10^{10}$ particles of AAV8/luc were injected. After three days post AAV injection, the imaging was taken. It was surprising to note that pre-injection of LDL or lactoferrin actually increased AAV8 transduction in the liver **(Fig. 16).** This result suggests that AAV8 may not employ the LDL and transferrin receptors for effective mouse liver transduction.

***Interaction of AAV8 with LDL or transferrin enhances mouse liver transduction.*** Incubation of AAV8 with LDL or transferrin increased transduction in human hepatocyte cell line Huh7 but not non-hepatocyte cell line 293T. As described above, to study the effect of LDL and transferrin on AAV liver transduction in mice, $1x10^{10}$ particles of AAV8/luc were incubated with different dilutions of LDL or transferrin, then injected into mice via retro-orbital vein. At day 3, 10 and 14 post AAV injection, the mouse imaging was carried out. As shown in Fig. 17, even at the dilution of 10000 fold for LDL and 1000 fold for transferrin, the enhancement of liver transduction was observed throughout the experiments.

***Incubation of AAV8 with LDL or transferrin increases virus virions binding to target cells.*** It has been shown that enhanced transduction from HSA is due to an increase of AAV virions binding to target cells. To examine whether the same mechanism applies to the effect of LDL and transferrin on AAV8 transduction in hepatocytes, we first performed the virus-cells binding analysis in Huh7 cells. AAV8 virus was incubated with LDL or TRF at different dilutions of normal blood concentration for 1 hr at 4°C, then Huh7 cell were added and incubated for another 2 hr at 4 °C. After thorough washing with PBS, DNA from Huh7 cells was extracted and applied to measure AAV genome copy number using quantative PCR. Consistent with the result from transgene expression, incubation of LDL or ApoB or transferrin with AAV8 virions increased AAV8 binding to Huh7 cells **(Fig. 18).** The dilution of 10- to 1000-fold of HSA had similar virus binding to Huh7 cells. In contrast, the binding capacity of AAV8 to Huh7 cells for LDL and transferrin was dose-dependent. Therefore, more proteins incubated with AAV vector induced higher virus binding to Huh7 cells.

To study the effect of LDL and transferrin on AAV binding ability on mouse liver, we first investigated the kinetics of virus clearance in blood after AAV administration. $1x10^{11}$ particles of AAV8/luc vectors pre-incubated with LDL or transferrin at a dilution of 100-fold were injected into mice via retro-orbital vein. Mouse imaging was performed at 48hr post AAV8 injection **(Figs. 19A** and 19B). Consistent with the results as decribed above, AAV8 pre-treated with LDL or transferrin increased mouse liver transduction. Also, at 5 min, 2h, 24h and 48h post AAV injection, blood was drawn and plasma was collected after brief centrifugation of blood samples. AAV genome copy number in plasma was measured by quantative PCR **(Fig. 19C).** In contrast to the kinetics of virus clearance for AAV8 incubated with IISA, higher blood virus titer was found in mice receiving AAV8 pre-treated with LDL and transferrin at 5 min post AAV injection. There was no difference after 2hr post AAV administration.

To study the effect of serum proteins LDL and transferrin on bio-distribution of AAV8, at day 7 after AAV administration, mice were sacrificed and different tissues were harvested for transgene expression analysis and AAV genome copy number detection. As shown in **Fig. 20,** compared to control mice receiving AAV8 vector incubated with PBS, the increased transgene expression was only shown in the liver of mice treated with AAV8 vectors pre-incubated with LDL or transferrin. In alignment to transgene expression, higher AAV genome copy number was observed in the liver of mice receiving AAV8 with LDL or transferrin. These results suggest that incubation of AAV8 with LDL or transferrin does not change AAV8 tissue tropism, but rather increases AAV uptake in the liver.

***No further increase of transgene expression from AAV8 incubated with the combination of serum proteins.*** Our previus study and above results demonstrated that individual serum protein (HSA, LDL and transferrin) enhanced AAV8 transduction in liver cells. Next, we wonder whether the combination of these proteins has more potential to increase AAV8 transduction. AAV8 vector was incubated with the combination of two or three proteins and transduced into Huh7 cells. Compared to AAV8 treated with albumin, no further increase of transgene expression was achieved regardless of any combinations or any dilutions of individual protein **(Fig. 21).**

We also incubated $1x10^{10}$ particles of AAV8/luc with three proteins, either individual or in combination, at the dilution of 100-fold and injected into mice via retro-orbital vein. At day 3 and 7 post AAV injection, mice were imaged. Similar to the results in Huh7 cells, compared to individual protein, the combination of HSA, LDL and transferrin did not increase AAV8 liver transgene expression **(Fig. 22).**

***Serum proteins competitively bind to the same location of AAV8 virions.*** The results described above from experiments *in vitro* and *in vivo* demonstrated that the combination of serum proteins does not have superior liver transduction in comparison to individual proteins. Similar enhancement of liver transduction was observed from AAV8 incubated with HSA, or LDL or transferrin. We presume that these proteins may bind to the same location of the AAV virion surface. To support this hypothesis, we performed the competition assay. First, we incubated AAV8 virus with the mixture of albumni at different dilutions and LDL or transferrin at dilution of 1:100. Then AAV virions were pulled down by antibodies specific for ApoB or transferrion and titered by quantative PCR **(Fig. 23A).** Incubation of AAV8 with combinations containing high concentration of HSA completely blocked virus binding to LDL or transferrin. Decreased HSA concentration increased AAV8 virions binding to other proteins. When 10000-fold of HSA was used, no inhibiton of AAV8 binding to LDL or transferrin was shown. Next, we carried out the AAV8-protein binding block analysis. AAV8/luc was incubated with HSA at different dilutions for 30 min, then LDL or transferrin at 100-fold dilution was added for one hr. After pull down with LDL or transferrin specific antibodies, similar to competition analysis, high concentration of HSA blocked later AAV virion binding to LDL or transferrion **(Fig. 23B).**

In summary, serum proteins (LDL-ApoB, transferrin, albumin) are able to enhance AAV8 liver transduction via the mechanism of increasing AAV virion binding to target cells. These proteins interact with the same location on the AAV8 virion surface.

Example 3

Among 12 AAV serotypes, it has been well known that systemic administration of AAV9 induces global transduction in animal models. Thus, it has been proposed in clinical trials to target the brain and the muscles by peripheral infusion of AAV9 vectors to deliver therapeutic transgenes. The results from studies *in vitro* and *in vivo* have suggested that AAV9 vectors are able to cross the blood vessel endothelial barriers via efficient transcytosis, which contribute to its superior transduction in the muscle, the heart and other tissues or organs after systemic gene delivery. AAV9 vectors will first interact with serum proteins before binding to target cells after systemic administration. To elucidate whether some serum proteins are capable of interaction with AAV9 and enhancing its transduction, we performed the mass spectrometry analysis for AAV9 binding serum proteins and studied the potential effect of these binding serum proteins on AAV9 global transduction.

***Serum proteins with modulation of vascular permeability bind to AA V9.*** To identify which serum proteins are able to bind to AAV9 virion surface, we did immunoprecipitation for mass spectrometry assay. AAV9 virions were incubated with human sera for 2 hr at 4°C. Monoclonal antibody ADK9, which only recognizes intact AAV9 capsid, was added. Then, AAV9 binding serum proteins were pulled down and analyzed by mass spectrometry. Among the identified proteins **(Table 6),** several proteins may influence vascular permeability, including: Fibrinogen (Fib), fibronectin (FN), plasminogen (PMG), von Willebrand factor (vWF), Alpha-1-acid glycoprotein (AGP) and platelet factor 4 (PF4) **(Table 7).**

***Direct interaction of fibrinogen with AAV9 enhances the whole body transduction.*** Fib is a glycoprotein that helps with blood clot formation. Fib is a hexamer containing two sets of three different chains ($\alpha$, $\beta$, and $\gamma$), that are linked to one another by disulfide bonds. Fibrinogen is synthesized by the hepatocytes, and the concentration in the blood plasma is 2 - 4mg/ml. The fibrinogen is a soluble with a molecular weight of 340 kDa. Mass spectrometry analysis has shown that all three chains of Fib were identified to bind to AAV9 **(Table 7).** To study the effect of Fib on AAV9 transduction in mice, we incubated $1x10^{10}$ particles of AAV9 with 3 mg of Fib at 4°C for 2 hr, and then injected into mice (Fib-PBS cohort). Three days later, the imaging was performed. When compared to mice receiving AAV9 only (PBS cohort), or mice treated with Fib just prior to injection of AAV9 (PBS-Fib cohort), about 3-fold higher liver transduction was achieved in mice within the Fib-PBS cohort **(Fig. 24).** Also, based on the imaging profile, strong transduction was also observed in the head, the heart and other locations beside the liver in mice receiving AAV9 pre-incubated with Fib. There was no difference in transgene expression, in the liver or the whole body, between PBS cohort and PBS-Fib cohort. This result suggests that incubation of Fib with AAV9 vector is able to increase AAV9 vascular permeability and enhance AAV9 whole body transduction, and the enhancement of transduction requires the direct interaction of Fib with AAV virions. To examine the high transduction in other tissues besides the liver, we performed systemic administration of AAV9 vectors pre-incubated with Fib, and at week one following AAV injection, mice were sacrificed and tissues were harvested for luciferase analysis and genome copy number detection **(Fig. 25).** Consistent to the imaging, the mice in Fib-PBS cohort had higher transgene expression in the liver, heart, lung, muscle and brain than the mice in PBS and PBS-Fib cohorts **(Fig. 25A).** Also, higher AAV genome copy number was found in the tissues of mice from the Fib-PBS cohort than those from the other two cohorts **(Fig. 25B).** When AAV9 was incubated with decreased doses of Fib, the enhanced transduction was only seen at the concentration of 1mg and 100ug of Fib. Fib with lower doses had no effect on AAV9 transduction **(Fig. 26).**

***Higher AAV virions persist after systemic administration of AA V9 incubated with Fibrinogen.*** It has been demonstrated that the blood clearance of AAV9 vector is slower than that of other serotypes, which may contribute to increased vascular permeability for high whole body transduction. To study whether incubation of Fib impacted the kinetics of AAV9 clearance in blood, we injected $2x10^{11}$ particles of AAV9 into mice, and at day 2, mouse imaging was taken **(Fig. 27A).** Similar to the above observation, higher transgene expression in the liver was shown in mice treated with AAV9 pre-incubated with Fib than that in mice of PBS cohort or PBS-Fib cohort **(Fig. 27B).** At different time points, blood was drawn and AAV genome copy number in plasma was detected via quantitative PCR. Significantly higher genome copy number in circulation was found in mice receiving AAV9 incubated with Fib than that of mice within the other two cohorts at 20 min, 2hr and 24 hr post AAV injection. There was no difference at 48hr after AAV administration among the three cohorts **(Fig. 27C).** The result may explain that enhanced whole body transduction may result from higher AAV virions in circulation after systemic administration of AAV9 vector pre-incubated with fibrinogen.

***Interaction of other serum proteins with AA V9 enhances transduction.*** The main focus of this study was to examine which serum proteins enhanced AAV9 whole body transduction. For this purpose, in combination of the results from mass spectrometry analysis, several other proteins which also bind to AAV9 may modulate vascular permeability to impact AAV9 whole body transduction, including: Alpha-1-acid glycoprotein 2 (AGP), fibronectin (FN), von Willebrand factor (vWF), platelet factor 4 (PF4) and plasminogen (PMG). We incubated AAV9 vector with these proteins at the physiological blood concentration and injected into mice. At day 3 after AAV administration, mouse imaging was carried out. All of these proteins induced higher liver transduction in mice treated with AAV9 **(Fig. 28).** In some groups, at week1 post AAV injection, we also found high transgene expression in the brain in mice receiving AAV9 pre-incubated with AGP or FN or PF4 or vWF **(Figs. 29A** and **29B).** Mice were sacrificed at week 1 post AAV administration, and the AAV genome copy number was detected in the liver and the brain. Consistent with the image profile, 3- to 4-fold higher genome copy number was obtained in the liver of mice receiving AAV9 pre-incubated with Fib, or PF4 or vWF. In contrast to the liver, only slightly higher genome copy number observed in the brain of mice receiving AAV9 pre-incubated with serum proteins, except for PF4 **(Fig. 29C).**

Next, we examined the effect of serum protein, at different doses, on enhanced AAV9 transduction. AAV9 was incubated with different dilutions of serum proteins and then administered into mice via retro-orbital vein. As shown in **Fig. 30,** enhanced transduction was still seen for AGP at 1000-fold dilution and fibronectin at 100-fold dilution, but there was no transduction increase for PF4 and vWF, even though 10-fold dilution of these proteins was used **(Fig. 30).**

***Incubation of cryoprecipitate with AA V9 enhances AAV9 transduction.*** Based on the results from the above studies, several serum proteins enhanced AAV9 whole body transduction. The next question is whether we can use these proteins in clinical trials immediately. There is no individual protein available in the clinical practice; however, cryoprecipitate has been used in clinics for a long-time. Cryoprecipitate is a frozen blood product prepared from plasma by centrifugation of fresh frozen plasma and precipitation. Cryoprecipitate mainly contains fibrinogen, factor VIII, vWF, factor XIII and fibronectin. Cryoprecipitate has been used to treat patients with hemophilia, vWF disease, hypofibrinogenemia, afibrinogenemia, et al. Since fibrinogen, vWF and fibronectin have been demonstrated to enhance AAV9 transduction as described above, next we tested whether cryoprecipitate had effect on AAV9 transduction. AAV9 was incubated with cryoprecipitate at different doses and systemically injected into mice. The enhancement of AAV9 transduction in the liver was dose dependent with 100- to 10000-fold dilution of cryoprecipitate. There was no enhancement with the dose of 100000-fold dilution of cryoprecipitate (Fig. 31). This result indicates that cryoprecipitate could be immediately used in future clinical trials when AAV9 vectors are required for systemic administration.

The cryoprecipitate, which is composed of fibrinogen, vWF and fibronectin, could be immediately applied in the clinical trials to increase blood vessel permeability and to target the brain and muscles after systemic administration of AAV9.

Example 4

We have performed one more experiment about the effect of albumin interaction with AAV virions on neutralizing antibody A20 inhibition activity. As shown in **Table 8** and **Figure 32,** regardless of dilution of albumin (no dilution, 5 fold, 50 fold, 500 fold), the incubation of human albumin with AAV2 did not block A20 inhibition function. The A20 neutralizing antibody titer (the dilution at first time inhibits AAV transduction over 50%) was consistently the same (1:640 dilution). This result indicates that interaction of albumin with AAV virion is not able to interfere with neutralizing antibody A20 binding to AAV capsid and blocking AAV transduction.

Based on these studies, 5% HSA at dilution of 20000 fold in 12.5 ul has the effect to enhance AAV transduction (1x10e8 particles of AAV) *in vitro.* In one embodiment, around 3000 molecules (2830) of human albumin can be incubated with one AAV virion for enhancing transduction. See calculation below:

Human serum albumin molecular weight (kDa): 66.5 kDa
1 ng = 15.04 fmol = 905570676 molecules
12.5 ul of 5% HSA (50ug/ul) = 625 ug = 625000 ng
5% HSA at dilution of 20000 in 12.5 ul still has an effect to enhance AAV transduction (1x$10^8$ particles of AAV).

$$\text{So total molecules of HSA is } 625000/20000 \times 905570676 = 282990836466$$

HSA molecules/AAV virion = 282990836466/100000000 =2830 molecules/AAV virion

Example 5: Stability of albumin fusion proteins/AAV complex

***Method.*** 1x$10^{10}$ particles of AAV8/luc vector were loaded on the nitrocellulose membrane in a manifold apparatus, then the membrane was blocked by 1% gelatin followed by incubation with 25% human serum albumin (HSA) at a dilution of 1:1000 for 30 min to allow direct interaction of AAV with albumin. After washing with PBS, the buffer with different concentration of salt or pH was added to individual well of the manifold apparatus. After removal of the different buffers, the membrane was washed and hybridized with HRP conjugated goat anti human albumin followed by color development using Immun-Star™ Chemiluminescence Kits (BioRad).

***Result.*** As shown in **Fig. 33,** human albumin was dissociated from the AAV/HSA complex when different concentration of NaCl was added, the dissociation of the AAV/HAS complex was dependent on the concentration of NaCl **(Fig. 33A).** Higher concentration of NaCl completely disrupted the interaction of AAV8 virions with HSA. We also found that the HSA/AAV complex was stable at pH>6 but dissociated at pH≤5 **(Fig. 33B).**

Example 6

Adeno-associated virus (AAV) vector has been successfully applied in clinical trials in patients with blood diseases and vision disorders. Two concerns restrict broader AAV vector application: AAV capsid specific cytotoxic T cell (CTL) response-mediated elimination of AAV transduced target cells and neutralizing antibody (Nab)-mediated blocking of AAV transduction. It has been demonstrated that capsid antigen presentation is dose-dependent, which indicates that enhancing AAV transduction with low dose of AAV vector will potentially decrease capsid antigen load and hopefully ablate capsid CTL mediated clearance of AAV transduced target cells without compromise of transgene expression. Several approaches have been explored for this purpose including: optimization of transgene cassette, modification of the AAV capsid and interference of AAV trafficking with pharmacological agents. Modification of the AAV capsid may change AAV tropism, especially because AAV transduction efficiency is unknown in human tissues.

Pharmacological reagents for enhancing AAV transduction usually have unwanted side effects. It is imperative to develop ideal strategies to enhance AAV transduction, but without a change in tropism from modification of capsids or negative side effects from pharmacological treatment. We have performed a pioneer study and found that human serum albumin (HSA) has enhanced effect on AAV transduction by direct interaction of the AAV virion with albumin. These observations have a critically important significance for clinical trials since albumin naturally exists and is the most rebounded protein in the circulation.

Effective AAV transduction involves the following steps: binding on the target cell surface via receptors and co-receptors, endocytosis into endosomes, escape from endosomes, nuclear entrance, and AAV virion uncoating followed by transgene expression. Several steps can be modified to enhance AAV transduction, including cell binding, endosomal escape and nuclear entrance. Our preliminary studies have shown that HSA is able to directly interact with the AAV virion and enhance its transduction due to increased AAV virus binding on the cell surface perhaps via HSA receptors.

For effective drug or bio-cargo delivery, a number of studies have demonstrated that numerous peptides (including cell penetrating peptides-CPPs) have been identified to specifically target hepatocytes, help endosome escape (endosomolysis) and increase nuclear entry. The described studies examine whether fusion of these peptides with HSA will further enhance AAV liver transduction. Interference with AAV virion trafficking for enhanced transduction may also influence capsid antigen presentation; previous studies have demonstrated that capsid antigen presentation relies on proteasome mediated degradation of AAV capsid. Many viruses (for example, CMV, herpes) utilize VIPR peptides (viral proteins interfering with antigen presentation, e.g., US6, ICP47) to block antigen presentation. In these experiments, we will study the effect of enhanced AAV transduction with HSA on capsid antigen presentation and explore whether fusion of VIPRs with HSA will interfere with AAV capsid antigen presentation. The enhanced AAV transduction by HSA is due to more virions binding on the target surface after interaction with albumin. This result suggests that albumin fusion peptide may be used as an alternative receptor ligand for AAV transduction to avoid AAV neutralizing antibody (Nab) activity. It has been suggested that the epitopes for neutralizing antibody recognition are located at 9 variable regions (VR) of the virion surface. Prior studies have shown that peptides from AAV VRs block Nab function. The combination of different peptides derived from AAV VRs may have stronger ability to block Nab activity. We will explore whether albumin fusion protein with peptides derived from the surface variable regions of AAV virion interferes with neutralizing antibody activity.

Adeno-associated virus (AAV) vectors have been successfully used to transduce hepatocytes in Phase I clinical trials in patients with hemophilia B. However, clinical results have suggested that capsid specific cytotoxic T lymphocytes (CTLs) eliminate AAV transduced hepatocytes thus resulting in therapeutic failure. Capsid antigen presentation in AAV transduced target cells is dose-dependent. To avoid capsid specific CTL-mediated clearance of AAV transduced liver cells, a lower dose of AAV vectors has been proposed to reduce the capsid antigen load in AAV transduced cells. To obtain similar transduction efficiencies with low vector doses, several approaches have been explored including transgene optimization, capsid alterations, and drug treatments to enhance transduction. A number of pharmacological agents have been used for this purpose, including proteasome inhibitors, DNA synthesis inhibitors and topoisomerase inhibitors. However, these drugs have severe side effects. It has also been demonstrated that modification of the AAV capsid can enhance liver transduction. Our recent study demonstrated that engraftment of the AAV9 galactose receptor binding residues into the AAV2 virion (dual receptors) induced stronger liver transgene expression. However, other mutants such as AAV2i8 (AAV2 with heparin binding site swap from AAV8) change their parents' liver tropism to muscle tropic. Albumin is the most abundant plasma protein and is synthesized in the liver. Albumin is a highly soluble and stable protein. X-ray crystallographic structures of human albumin has revealed that it is a heart-shaped molecule consisting of 67% $\alpha$-helices and no $\beta$-sheets, and folds into three homologous domains where each is divided into A and B subdomains. The domains are connected via long flexible loops. Each of the three domains has hydrophobic binding pockets that allow substances to be carried. Thus, albumin acts as a molecular taxi that transports essential substances and waste products in the bloodstream for optimal distribution to their target sites. Several albumin receptors have been described: the cell-surface glycoprotein (gp)18, gp30, gp60 (albondin), the magalin/cubilin complex, the secreted protein acidic and rich in cysteine (SPARC; also named osteonectin), and FcRn. After albumin binds to the receptors, it is up-taken via endocytosis. The pathway for albumin endocytosis is cell type dependent and includes either clathrin- or caveolin-mediated endocytosis. Human serum albumin (HSA) attracts great interest in the pharmaceutical industry since it can bind a remarkable variety of drugs, impacting their delivery and efficacy and ultimately altering the drug's pharmacokinetic and pharmacodynamic properties.

AAV infection is a multi-step process beginning with the virus binding to the cell surface, followed by viral uptake, intracellular trafficking, nuclear localization, uncoating, and second-strand DNA synthesis. AAV2 initiates infection by binding to its primary receptor (heparan sulfate proteoglycans-HSPG) and co-receptors (integrin and fibroblast growth factor receptor 1). In order for AAV to continue its life cycle, it must be released from the endosome after endocytosis. Following escape from the endosome, AAV rapidly travels to the cell nucleus and accumulates in the perinuclear space, beginning within 30 minutes after the onset of endocytosis. Within two hours, viral particles can be detected in the cell nucleus, suggesting that the AAV particle enters the nucleus prior to uncoating. Interestingly, the majority of the intracellular virus remains in a stable perinuclear compartment. After receptor binding, internalization, and nuclear entry, the AAV virion uncoats and releases a single stranded DNA template, which must be converted to a duplex intermediate before transcription can ensue. Some steps are rate-limiting factors for effective AAV transduction, including virus binding ability on target cells, efficiency of endosomal escape and nuclear entry.

Cell penetrating peptides (CPPs), also known as protein transduction domains (PTDs), are small peptides able to carry peptides, proteins, nucleic acid, and nanoparticles across the cellular membranes into cells, resulting in internalization of the intact cargo. CPPs can serve different functions such as enhancing cargo binding ability and increasing cargo escape from endosome and nuclear entry. Several peptides have been identified that specifically bind to hepatocytes. One peptide is derived from the circumsporozoite protein containing the conserved region I amino acids (KLKQP, SEQ ID NO:131) plus the basic amino acid domain upstream from region I (DNEKLRKPKHKKLKQPADG, SEQ ID NO:132). Another peptide preS1 domain is from hepatitis B virus surface antigen (PLGFFPDHQLDPAFGANSNNPDWDFNP, SEQ ID NO:133). A third one is from the T7 phage tail fiber protein (KNESSTNATNTKQWRDETKGFRDEAKRFKNTAG, SEQ ID NO:134). Some peptides have the property to promote endosomal membrane disruption via different mechanisms: pore formation in the endosome membrane, pH-buffering effect (the proton sponge effect), fusion in the endosomal membrane and photochemical disruption of the endosomal membrane. These peptides are derived from viruses, bacteria and human/animal proteins as well as synthetic. In higher order biological systems, a nuclear localization signal (NLS) is essential for targeting macromolecular cargoes to the nucleus. To improve nuclear import efficiency, the direct or indirect attachment of CPPs with nuclear localization sequences (NLSs) to DNA or gene carriers has attracted much research interest. NLSs are short peptides based on lysine-, arginine- or proline-rich motifs that can be recognized by members of the Importin super family of nuclear transport proteins. Importin-α directly binds the NLS signal, and the complex is translocated into the nucleus through the nuclear pore complex (NPC) by successive docking of Importin β and nucleoporins (Nups). Thus, the NLS overcomes the nuclear membrane barrier and promotes nuclear translocation. The most well-known and extensively studied NLS in the field of gene therapy is from the large tumor antigen of the simian virus 40 (SV40).

For albumin mediated drug delivery, endocytosed cargoes often become trapped in endosomes, where they may be degraded by hydrolytic enzymes. For AAV infection, after uptake, AAV stays in the endosome for a much longer period compared to adenovirus. After escape from the endosome/lysosome, only a small portion of vector enters the nucleus for uncoating. Thus, endosomal escape and nuclear entry become limiting factors in albumin mediated AAV vector delivery. Fusion of CPPs with albumin will increase albumin uptake and travel to the nucleus in hepatocytes. Enhanced AAV transduction in hepatocytes will result when AAV vectors are pre-incubated with these albumin fusion proteins, due to more virus binding to hepatocytes and an increase in AAV escape from the endosome as well as nuclear entrance. Thus high dose related antigen presentation in target cells will be avoided and less labor force is needed to make AAV.

Although albumin is able to directly interact with AAV virions and thus enhances AAV transduction, it is unclear whether the interaction of albumin with AAV affects capsid antigen presentation. Albumin may change the AAV trafficking pathway and then impact capsid antigen presentation efficiency and kinetics, especially when albumin is fused with CPPs. Investigating the effects of albumin and its fusion proteins on capsid-based antigen presentation will allow the community to understand the effective parameters for designing safer AAV vectors with enhanced liver transduction and long-term clinical efficacy. AAV capsid cross-presentation is mediated by proteasome mediated degradation of the capsid using the classic MHC-class I antigen presentation mechanism. Some viruses can persist within a host for the lifetime of the organism by encoding a group of proteins named VIPRs to affect the MHC class I presentation pathway. Potential use of these VIPRs by fusion with albumin for AAV vectors delivery is a focus of these studies.

In the general human population, over 95% of individuals have been infected by AAV serotype 2 (AAV2) and on average 50% of those infected have NAbs. To overcome AAV NAbs, several approaches have been exploited in the laboratory. One approach involves using a polymer coat to mask the AAV surface and block NAb recognition (e.g., polyethylene glycol). While promising, this approach may change the AAV transduction profile. A second approach uses error-prone PCR to generate a library of AAV capsid variants and select for NAb escape mutants in the presence of NAbs *in vitro.* This approach has yielded novel capsids; however, it bears the potential limitation of generating capsids with unknown transduction efficiency *in vivo.* A third approach has been to use alternative serotypes of AAV that show low or absent NAb cross-reactivity - an approach demonstrated in several animal models. A final laboratory approach is to rationally mutate the NAb binding domain on the AAV capsid surface to eliminate the NAb binding site. This strategy requires information about monoclonal antibody epitopes and the structure of AAV virion, and is inherently limited due to the fact that the NAbs from human sera are poly-clonal and it is impossible to obtain mAbs from humans that represent all generated NAbs. Several clinical setting approaches also have been employed: One example is to perform plasma-apheresis prior to vector delivery. However, due to the relative inefficiency of each round of apheresis and the fact that even low titers of NAbs (<1:5) can abrogate AAV transduction, this strategy is only suitable for patients with lower starting titers of AAV NAbs and requires multiple sessions of apheresis. Similarly, the use of anti-CD20 antibody (Rituximab) can achieve B cell depletion for 6-9 months, but is not directed at (antibody-producing) plasma cells and is effective in reducing AAV NAb in a minority of subjects having less than a 1:1000 titer.

Peptides derived from AAV VRs are able to block NAB activity on AAV transduction. The combination of hepatocyte-specific peptides and CPPs as well as peptides from AAV VRs linked to HSA has two functions: as a decoy for blocking AAV neutralizing antibody (VRs from AAV) and as another layer for effective AAV binding/intracellular trafficking (CPPs). The strategy has global applications to any condition requiring systemic administration, or any repeat administration, of AAV vectors.

Numerous studies demonstrate the following: (1) human serum albumin directly interacts with AAV virion and enhances AAV transduction; (2) AAV capsid antigen presentation is dependent on proteasome-mediated capsid degradation *in vitro;* (3) AAV capsid antigen cross-presentation is dose-dependent *in vivo; (4)* modification of AAV virions increases liver transduction; (5) pharmacological agents enhance AAV liver transduction *in vivo;* (6) VIPRs interfere with antigen presentation; and (7) peptides from AAV VRs block neutralizing antibody activity on AAV transduction. All of these preliminary results lay the groundwork to exploit the role of HSA in AAV clinical application, including enhancement of AAV transduction, interference of capsid antigen presentation and evasion of Nabs

Two critical issues faced in the clinical trial for AAV systemic administration are capsid-specific CTL response and AAV neutralizing antibodies. Enhanced AAV transduction may lower the dose of AAV vector necessary for achieving therapeutic effect while decreasing capsid antigen load to avoid capsid-specific CTL recognition. Although capsid modification and application of pharmacological agents have been proposed to enhance AAV transduction, mutation of AAV capsids may change its tropism, and the drugs with enhanced AAV transduction always have side effects.

Utilization of serum protein albumin fused with CPPs to enhance AAV transduction in specific tissues is novel and does not have unwanted side effects.

***Enhanced liver transduction with AAV mutants.*** AAV viruses exploit heparan sulfate (HS), galactose (Gal), or sialic acid (Sia) as primary receptors for cell surface binding. Different AAV strains also require subsequent interaction with co-receptors for cellular uptake. Key amino acid residues involved in Gal recognition by AAV9 capsids have been identified. Modification of receptor binding sites on the AAV capsid can either change transgene profile or transduction efficiency. We have pioneered rational design studies of AAV capsids; for instance, we have mutated the critical residues for primary receptor binding site between different serotypes and demonstrated that the AAV2/AAV8 chimera AAV2i8 displayed an altered transduction profile. AAV2i8 selectively transduces cardiac and whole-body skeletal muscles with high efficiency and loses liver tropism. Further studies integrating AAV9 primary receptor Gal binding residues into the AAV2 capsid (AAV2G9) showed that AAV2G9 has dual receptor function and exploits Gal and heparan sulfate receptors for infection. Of particular interest, AAV2G9 retains a similar tropism to AAV2 but confers more rapid onset and higher liver transgene expression in mice. Similarly, engraftment of the Gal footprint onto the AAV2i8 (AAV2i8G9) also induced higher transduction in muscle and liver, comparable with AAV9.

In addition, we have demonstrated that modifications at residue 265 of the AAV2 capsid change AAV2 tissue tropism and the immune profile. Insertion of an aspartic acid at residue 265 of the AAV2 capsid (AAV2D) induced much higher muscle transduction than AAV2. Similarly, systemic administration of AAV2D also induced higher liver transduction than AAV2. Residue 585 Arg contributes to the AAV2 heparin binding capacity and mutation of the AAV2 heparin binding site (AAV2/585E) ablates AAV2 liver tropism. However, insertion of Asp at residue 265 of AAV2/585E capsid restored the liver tropism to similar transduction efficiency as observed using AAV8. Although these studies demonstrated that higher liver transduction with mutations was achieved, the tissue tropism was also changed.

***Enhanced liver transduction with chemotherapy agents.*** A number of chemical agents have been used to enhance AAV transduction, including proteasome inhibitors such as MG-132 and bortezomib, DNA synthesis inhibitors such as hydroxyurea (HU) and aphidicolin, and topoisomerase inhibitors such as etoposide and camptothecin. Thus far, the leading candidate for enhancing rAAV transduction *in vivo* is the proteasome inhibitor bortezomib, which has been demonstrated to increase transgene expression 3- to 6-fold in a large-animal study. We have further identified a novel agent, arsenic trioxide, as well as an alternative proteasome inhibitor, carfilzomib, which also augment AAV liver transduction **(Figure 34).**

***Enhanced liver transduction with human serum albumin.*** When we performed AAV neutralizing antibody analyses on human sera, enhanced AAV transduction was observed even though a very high dilution of sera was used. To isolate which proteins are able to bind AAV and enhance transduction, we incubated AAV2 with human serum and pulled down AAV2 binding proteins using A20 antibody for immunoprecipitation. After analysis with mass spectrometry, human serum albumin (HSA) was identified **(Figure 35A).** We further confirmed the direct interaction of serum albumin with AAV virions by studying the co-localization with Cy5 labeled AAV2 and Cy3 labeled albumin **(Figure 35B).** To elucidate the effect of HSA on AAV transduction, we performed a transduction assay with HSA depleted serum and recombinant human albumin in Huh7 cells. Lower enhancement of AAV2 transduction was found with HSA depleted serum than complete serum **(Figure 35C).** The enhanced AAV transduction from rHSA was similar to that from complete serum **(Figure 35D).** Also we demonstrated that rHSA enhanced AAV8 transduction **(Figure 35E).** To examine whether the effect of HSA on enhanced AAV transduction is limited to specific tissues, we injected AAV8 vector pre-incubated with rHSA into different tissues and found enhanced transduction in the liver and muscle **(Figures 35F** and **3G).**

***AAV trafficking.*** Using fluorescence labeled AAV virions to track the virus after infection, we observed AAV internalization through clathrin-coated pits and escape from early endosomes ($t_{1/2}$<10 min) allowing penetration into the cytosol. AAV then rapidly trafficked to the cell nucleus and accumulated in the perinuclear space within 30 min after the onset of endocytosis. Within 2 hr, viral particles could be detected within the cell nucleus. To support the florescence results, we carried out immuno-analysis using the monoclonal antibody A20 to detect intact virions after receptor entry. A similar result was observed. To further determine the cellular location of intact virions during infection, we used an $\gamma$-tubulin monoclonal antibody to locate the microtubule-organizing center (MTOC), and found that the AAV virus travels to the nucleus through the MTOC. Using single-particle tracking to monitor the viral movement in real time, we found that AAV2 displayed only unidirectional movement on microtubules (MTs) toward the nuclei. Furthermore, electron microscopy analysis demonstrated that AAV2 particles were transported on MTs within membranous compartments and the acidification of AAV2-containing endosomes was delayed by the disruption of MTs.

***AAV capsid antigen presentation is dose-dependent and occurs early.*** To investigate the dose response of capsid antigen presentation *in vivo,* varying particle numbers of the AAV2-OVA vector were injected into mice to examine OT-1 T cell stimulation. When compared to the untreated control, a dose of $5x10^{10}$ AAV2-OVA particles induced the proliferation of OT-1 T cells **(Figures 36A-C).** Importantly, there was no proliferation of spleen cells in groups with doses of AAV2-OVA less than or equal to $1x10^{10}$ particles.

It has been shown that transgene expression gradually increases following AAV2 administration in mice, peaks at week 6, then remains stable for long-term. To address whether the kinetics of antigen presentation of capsid epitopes *in*

*vivo* corresponds with the dynamics of transgene expression, we injected 1x10$^{11}$ particles of AAV2-OVA/AAT intravenously. At various time points post-injection, splenic OT-1 T cells labeled with CFSE dye were administered to the treated mice. Ten days post-transfer, OT-1 T cell division was measured by flow cytometry. As shown in **Figure 37,** over days 3-12 and days 21-30, OT-1 T cell division was significantly increased in AAV2-OVA/AAT vector-treated animals versus control recipients. However, no difference was observed for OT-1 cell proliferation between AAV2-OVA and the control groups over days 41-50 and days 61-70 ($p>0.05$). This result indicates that antigen cross-presentation from the AAV2 capsid occurs early after AAV2 systemic administration. Similar kinetics and efficiency of antigen presentation were also observed from an AAV8-OVA vector after systemic application. In this study, we will use AAV2/OVA and AAV8/OVA vectors to investigate the effect of human albumin fusion proteins on kinetics and dose-response of AAV (2 and 8) capsid antigen cross-presentation following systemic application in a mouse model.

***The dual function of proteasome inhibitor on AAV capsid antigen presentation.*** As bortezomib enhances AAV transduction, we also studied the effect of this proteasome inhibitor on AAV capsid antigen presentation, and found that a high concentration of bortezomib inhibits capsid antigen presentation with enhanced transgene expression. In contrast, a lower concentration of bortezomib (10 nM) increased antigen presentation from AAV2-OVA transduced HepG2/kb cells without increasing transgene expression, and an intermediate dose (100 nM) enhanced both transduction and antigen presentation.

***VIPRs inhibit antigen presentation.*** To determine whether VIPRs from virus peptides can exert an ability to inhibit antigen presentation, we cotransfected CMV VIPR US6 or HSV VIPR ICP47 into 293 cells with OVA and H-2kb expression constructs, then 293 cells were co-incubated with OT-1 spleen cells for the antigen presentation assay. By analyzing the percentage of CD8/CD69 (for early activation of T cells) or CD8/IFN-$\gamma$ double positive cells in whole CD8 subset, after subtracting the background, the double positive cells of CD8/CD69 or CD8/IFN-$\gamma$ expression in ICP47 or US6 treated group was much lower than the group without VIPRs treatment, over 95% inhibition of OVA epitope presentation was demonstrated with US6 and ICP47 **(Figure 38).** To explore the inhibition of VIPR on antigen presentation *in vivo,* 293 cells were transfected with OVA, H-2kb and ICP47 or GFP constructs, then 1x10$^{7}$ 293 cells in 0.2ml matrigel were injected into mice left flank subcutaneously. Simultaneously, *in vitro* activated OT-1 spleen cells were transfused via tail vein and tumor size was measured every 2-3 days. After 20 days, no tumor was found in mice transplanted with 293 cells transfected with GFP plus OT-1 spleen cell infusion. All other mice developed tumors of similar size after xenograft of 293 cells transfected with GFP without infusion of OT-1 spleen cells or ICP-47 regardless of application of OT-1 spleen cells (Table 9).

***Evasion of A20 activity with insertions at residue 265 of AAV2 capsid.*** Our studies have demonstrated that A20 antibody (which only recognizes intact AAV2 virion) cannot block AAV2.5 transduction (AAV2 mutant with 5 aa substitution from AAV1). To evaluate whether insertions at residue 265 also ablate the A20 binding site and enhance muscle transduction, we first evaluated antibody binding. Analysis of A20 binding affinity by Western dot blotting showed that no mutants were recognized by A20, similar to the result for AAV2.5, which highlights the importance of the 265 site in an antibody-capsid recognition footprint. To study the neutralizing antibody profiles of AAV2 mutants with 20 different single amino acid insertions at residue 265, we analyzed the NAb activity from sera immunized with different mutants in mice. No obvious relationship was found between muscle transduction from mutants and inserted amino acid properties or NAb titer and cross-reactivity.

***Development of IVIG Nab escape mutants from mouse liver using directed evolution.*** We have successfully isolated several mutants with the ability of Nab escape and muscle tropism in the presence of human serum by direct injection of an AAV shuffling library into mouse muscle. To extend this study to isolate liver target AAV mutants with Nab escape, we first injected IVIG into the mice and three hrs later, the AAV library was administered via retro-orbital vein. At day 3 after AAV injection, mice received Ad dl309 for 2 days. Mouse liver was harvested and Hirt DNA was extracted. The PCR product from Hirt DNA was cloned into pSSV9 to make the second library. After repeating the above steps for two more cycles, the PCR product was cloned into pXR and sequenced. Four AAV mutants have been recovered from mouse liver **(Figure 39).** These mutants were used to package luciferase and after administration of these mutants and AAV8 into mice via retro-orbital injection, the imaging was taken. Compared to AAV8 (with superior mouse liver transduction), all mutants induced lower liver transduction (Figure 39). This finding is similar to mutants isolated from muscle that have lower muscle transduction than AAV6.

***Inhibition of AAV peptides on Nab activity.*** We conducted epitope scanning on sera from AAV2 immunized C57BL/6 and Balb/C mice as well as monoclonal antibody A20. After incubation of sera or A20 Ab with a peptide library, AAV2 vector was added to the mixture of sera and peptides, and then AAV2 transduction was compared to the control peptide. Using this approach, several peptides have been identified that increase AAV2 transduction with AAV2/GFP vectors **(Figure 40).** Structural analysis has demonstrated that entire peptides or portions of them are exposed on the AAV2 virion surface. Peptide p28 is partially buried under a beta sheet containing the aa 265 region and is situated outside but between the projections that make up the three fold axis. Exposed portions of p29, p32, and p68 are situated surrounding the 5-fold pore. The peptide p67 localizes to the depression outside of the 5-fold pore. P28 is also situated just outside the 2-fold symmetry depression **(Figure 10).** A20 antibody was originally generated from C57BL/6 mice. Two peptides (p28 and p67) screened for A20 interference were identified in the pool of peptides (p28, p32 and p67) from C57BL/6 mice, which supports the validity of this approach. These preliminary results demonstrate the ability of peptides from AAV VRs to inhibit Nab activity as decoys.

***Exploring albumin fusion proteins to further enhance AAV liver transduction.*** Our preliminary data and clinical results indicate that AAV capsid antigen presentation is dose-dependent, and that a lower dose of AAV vectors reduces the overall antigen presentation. Therefore, it is reasonable to explore effective strategies to achieve the necessary therapeutic levels while using lower vector doses. To achieve this goal, several approaches have been exploited in order to enhance AAV liver transduction, including utilization of different serotypes, genetic modification of AAV capsids, and optimization of AAV vector cassettes, as well as the application of chemical agents. However, these approaches either change AAV tropism (AAV capsid modification) or have unwanted side-effects (pharmacological agents). Our preliminary study has demonstrated that human serum albumin (HSA) is capable of enhancing AAV transduction via direct interaction with AAV virions.

Albumin has been emerging as an important protein carrier for drug targeting. Albumin is the most abundant plasma protein (35-50 g/L human serum) in the circulation. HSA-fusion technology is well-established to improve efficacy, bioavailability and safety of therapeutically relevant polypeptides. The technology has generated albumin fusion proteins in different species, and has been applied to cytokines and bioactive peptides. For hepatocyte-specific targeting, several peptides have been identified. Additionally, cell-penetrating peptides (CPPs) have actively been studied for efficient drug delivery. CPPs are a class of diverse peptides, typically with 5-30 amino acids, and unlike most peptides, they can cross the cellular membrane. CPPs can successfully transport cargoes such as siRNA, nucleic acids, small molecule therapeutic agents, proteins, quantum dots, and MRI contrast agents, both *in vitro* and *in vivo.* This system has lower cytotoxicity compared with other delivery methods. CPPs are attractive for medical applications, not only because of their high internalization ability but also due to their potential for variable modification design. In general, CPPs can be broadly classified into three types: (1) cationic peptides of 6-12 amino acids in length comprised predominantly of arginine, lysine and/or ornithine residues; (2) hydrophobic peptides such as leader sequences of secreted growth factors or cytokines; and (3) amphipathic peptides obtained by linking hydrophobic peptides to NLS.

Endosomal sequestration and insufficient nuclear entry are two limiting steps for effective AAV transduction, based on evidence that direct interaction between albumin and AAV virions is required to enhance transduction, we presume that albumin fusion proteins with hepatocyte specific peptide and CPPs will enhance AAV hepatocyte transduction by increasing liver specific binding, endosomolysis and nuclear entry.

***The effect of albumin fusion proteins on AAV liver transduction.*** Several peptides have been identified to specifically bind to hepatocytes, including a 33 amino acid sequence (KNESSTNATNTKQWRDETKGFRDEAKRFKNTAG, SEQ ID NO:134) within the T7 phage tail fiber protein (p17) coiled-coil rod domain, the peptide CSPI-plus (DNEKLRKPKHKKLKQPADG, SEQ ID NO:132) from the circumsporozoite protein (CSP) and the pre S peptide (PLGFFPDHQLDPAFGANSNN PDWDFNP, SEQ ID NO:133) from the hepatitis B virus. The endocytic pathway is the major uptake mechanism for AAV and albumin. Albumin is entrapped in endosomes after uptake and is degraded by specific enzymes in the lysosome. The acidic pH in endosomes helps AAV exposure of phospholipidase for escape. Thus, one of the rate limiting steps in achieving effective AAV transduction is to facilitate endosomal escape and ensure cytosolic delivery of AAV vector. Numerous peptides derived from virus/bacteria/plant or synthetic surfactants have been identified for this purpose. The peptide (GLFGAIAGFIENGWEGMIDGWYG, SEQ ID NO:135) from HA2 of HA protein has been broadly studied for endosomal escape (endosomolysis). In biological systems, a nuclear localization signal (NLS) is required for delivering macromolecular cargoes to the nucleus. The popular NLS (CGCGPKKKRKVG, SEQ ID NO:136) from SV40 will be used in this study. The trafficking of bioactive cargo from binding on the cell surface to the nucleus involves many steps including receptor-mediated endocytosis, escape from the endosome, and nuclear entry.

The peptides (which have roles in hepatocyte binding, endosomolysis and nuclear entry, singular or in combination) are conjugated with HSA and the effect of HSA fusion proteins on AAV transduction in hepatocytes is determined. The HSA fusion protein cassettes are driven by the CMV promoter and express the fusion protein in 293 cells. Purified fusion protein is used for *in vitro* and *in vivo* transduction analysis. *In vitro* analyses include transduction after incubation with fusion protein in human hepatocyte cell lines (Huh7 and HepG2) and non-hepatocyte cell lines (293, Hela, CHO, C2C12, MG87, etc.). *In vivo* experiments are composed of transduction analysis in liver and other tissues via systemic administration as well as muscle transduction by direct muscular injection. AAV serotypes 2 and 8 will be tested since they have been used in clinical trials to target hepatocytes.

***Cloning of albumin fusion protein.*** A PCR approach will be used to make a HSA fusion construct (with a His 6 tag) with the linker GGGGSGGGGSGGGAS (SEQ ID NO:137). Sequencing of clones will be performed to warrant the correct cassettes.

***Purification of HSA-preS fusion protein.*** Expression constructs pCMV-HSA or pCMV-HSA fusion proteins are transfected into HEK293 cells. After transfection, cells are cultured in Opti-MEMO I (Invitrogen) replacing media every 3 days for 3-4 times total. Supernatants are pooled and proteins are concentrated by ammonium sulfate precipitation (60% saturation), and loaded onto a nickel-nitrilotriacetic acid column (Qiagen, Hilden, Germany) equilibrated with 50 mM sodium phosphate buffer, pH 7.5, 500 mM NaCl, 20 mM imidazole. After a washing step (50 mM sodium phosphate buffer, pH 7.5, 500 mM NaCl, 35 mM imidazole) the His-tagged HSA proteins are eluted with 50 mM sodium phosphate buffer, pH 7.5, 500 mM NaCl, 100 mM imidazole. Protein fractions are pooled and dialyzed against PBS. Protein concentration is determined spectrophotometrically.

***Production of AAV vectors.*** All recombinant AAV (rAAV) viruses are generated using the standard triple transfection method using the XX6-80 adenoviral helper plasmid with a packaging plasmid (AAV serotypes and mutants) and an ITR plasmid (luciferase). rAAV vector is purified using a Cesium gradient and the physical titers of the vectors are evaluated using dot blot hybridization and quantitative PCR.

***AAV transduction in different cell lines.*** $1x10^8$ particles of AAV/luc vector will be incubated with HSA or HSA fusion proteins for 2 hr at 4°C. Then the mixture is added to $1x10^5$ cells in serum free culture medium for two days, and luciferase expression in cell lysate will be detected.

***AAV transduction after systemic administration in mice.*** AAV vectors encoding luciferase transgene will be incubated with HSA or HSA fusion proteins (single or in combination) or PBS for 2hr at 4°C, then the mixture will be administered into C57BL/6 mice via retro-orbital injection at the dose of $1x10^{10}$ particles ($5x10^{12}$/kg). At indicated time points after AAV injection, mouse imaging is performed and bioluminescence images in the liver area are analyzed. At week 2 post injection, mice will be sacrificed for transgene expression and AAV genome copy detection in different tissues.

***AAV transduction after muscular injection in mice.*** $1x10^9$ particles of AAV/luc incubated with HSA will be injected into muscles in the hind legs of mice. At different time points post injection, the imaging is carried out. For each serotype, six groups are designed: PBS, HSA and HSA fusion proteins (HSA-perS, HSA-HA, HSA-NLS and HSA-comb).

***Quantitation of luciferase expression in tissues in vitro.*** Several organs are collected for luciferase expression including: liver, spleen, kidney, heart, lung, skeletal muscle (gastrocnemius) and brain. Tissue is minced and homogenized in passive lysis buffer. Tissue lysates are briefly centrifuged at 10,000 rpm for 5 minutes to remove cellular debris. Supernatant is transferred to 96-well plates for luciferase activity analysis with a Wallac1420 Victor 2 automated plate reader. Total protein concentration in tissue lysates are measured using the Bradford assay.

***Tissue distribution of AAV vector.*** To detect AAV genome copy number in different tissues, DNA from tissues is isolated using the Qiagen DNeasy Blood & Tissue kit. Real-time PCR is performed on each sample for both the luciferase gene and the mouse Mus musculus Lamin B2 gene. The primers used for Lamin B2 gene are: 5'-GGACCCAAGGAC TACCTCAAGGG-3' (SEQ ID NO:129) (forward) and 5'- AGGGCACCTCCATCTCGGAAAC -3' (SEQ ID NO:130) (reverse); for luciferase: forward primer: 5'-AAAAGCACTCTGATTGACAAATAC-3' (SEQ ID NO:127) and reverse primer: 5'-CCTTCGCTTCAAAAAATGGAAC-3' (SEQ ID NO: 128). Viral genomes are quantified by real-time PCR on a Light-Cycler 480 instrument. The copy of genome is analyzed by Lightcycler software v.4.5 based on those of pTR-CBA-Luciferase plasmid used in initial transduction and the endogenous gene.

***Statistical analyses.*** All data are presented as means ± SEM. We will compare mean values from different experimental groups by a two-tailed Student's t test or one-way ANOVA. A P value of <0.05 is considered to be significant.

***The mechanism of AAV liver transduction using HSA fusion proteins.*** To study the mechanism of HSA effect on AAV transduction, we will perform the following experiments: AAV binding analysis, intracellular trafficking analysis using florescent labeled virus and confocal microscopy, and AAV clearance in blood after systemic administration.

***AAV binding analysis.*** $1x10^6$ cells will be incubated with AAV vector pre-incubated with HSA or HSA fusion proteins at $10^3$-$10^5$ particles/cell at 4°C for 1hr and washed with cold medium to remove unbound virus. After washing, the AAV genomic DNA is isolated and copies of AAV vector genome/cell are quantified by qPCR with specific primers for firefly luciferase.

***Fluorescence dye labeling of viral particles and albumin fusion proteins.*** As previously described, purified AAV virions or HSA fusion proteins will be incubated for 1 hour at 4°C in PBS with a tenfold molar excess of Cy5 or Cy3 mono N-Hydroxysuccinimide (NHS) esters (GE Healthcare, Piscataway, NJ) over capsid protein or albumin units. Labeled viruses or proteins are dialyzed to remove free dyes against PBS containing 5% sorbitol.

***Immunocytochemistry.*** Huh7 cells are incubated with fluorescence labeled AAV2 vectors pre-incubated with HSA fusion proteins at a dose of 10000 particles/ cell for different time (30 min, 2hr, 4hr, 8hr, 16hr and 24 hr). These cells are fixed with 2% paraformaldehyde for 15 min at RT. After washing, cells will be plated in LabTek 4-chamber slides pre-coated with L-lysine. Two hours later, the cells are permeabilized with 0.1% Triton X-100 in PBS at room temp for 5 min. Cells are washed and incubated with primary antibodies against LAMP (for endosome) or γ-tubulin (for MTOC) for 1hr at 37°C. After washing, cells are incubated with Cy3 conjugated secondary antibodies. After a final wash, cells are mounted in Vectashield (Vector Laboratories; Burlingame, CA) and analyzed with a confocal laser scanning microscope (Leica SP2).

***Blood clearance of AAV.*** After tail vein injection of $1x10^{11}$ particles of AAV/luc in C57BL/6 mice, the plasma is obtained from mice at 2, 6, 24, and 48 hours. Viral DNA isolation from plasma is performed using the DNeasy Blood & Tissue kit. Viral genomes are quantified by real-time PCR.

CPP has been demonstrated to enhance AAV transduction when directly mixed with AAV vector in one study. Enhanced AAV transduction in hepatocytes may be achieved using HSA fusion proteins from a single peptide or combination of three peptides for incubation with AAV *in vitro* and *in vivo.* HSA fusion protein conjugated with the combination of three peptides has much higher enhancement effect on AAV transduction than any single peptide. HSA fusion proteins conjugated with endosomolytic or NLS peptide or combined peptides also exert enhancement effect on AAV transduction in non-hepatocyte cell lines or tissues. HSA fusion proteins conjugated with endosomolytic peptide (single or in combination) is able to increase AAV vector escape from endosome and decrease AAV perinuclear accumulation, which is visualized using florescent dye labeled virus and confocal microscopy. More virus will be visualized in the nucleus when AAV vector is incubated with HSA fusion proteins conjugated with NLS. More copies of AAV genome will be detected in the liver when HSA fusion proteins are conjugated with HBV preS peptide; also this fusion protein will induce faster blood clearance of AAV vector after systemic administration.

*Investigation the effect of albumin fusion proteins on AAV capsid antigen presentation.* The data indicate that AAV capsid antigen presentation is dose-dependent, and that a lower dose of AAV vectors may reduce the overall antigen presentation. Further studies explore different strategies to achieve the necessary therapeutic levels while using lower vector doses. These include approaches to exploit the enhancement of AAV liver transduction, including utilization of different serotypes, genetic modification of AAV capsids, and optimization of AAV vector cassettes, as well as the application of chemical reagents..

Many viruses evade the human immune response by producing specific peptides (VIPRs) to interfere with antigen-MHC I presentation using various mechanisms. Two VIPRs (US6 and ICP47) have been well characterized and can be used for this specific purpose. The US6 gene product of human cytomegalovirus (HCMV), a 23 kDa endoplasmic reticulum (ER)-resident type I integral membrane protein that binds to TAP, inhibits peptide translocation and prevents MHC class-I assembly. The C-terminal 20 residues of the luminal domain are demonstrated to be essential for the inhibition of TAP. The herpes simplex virus (HSV)-1 protein ICP47 binds specifically to TAP, thereby blocking peptide-binding, translocation by TAP and subsequent loading of peptides onto MHC class I molecules in the ER. A short fragment of 32 amino acid residues, ICP47 (aa3-34), was found to be the minimal region harboring the ability to inhibit peptide-binding to TAP. The results demonstrate that antigen presentation was inhibited *in vitro* with supplementation of ICP47 and US6.

***The effect of albumin treatment on AAV capsid antigen presentation in*** *vivo.* To study the capsid antigen presentation, we will use our engineered viruses with integration of OVA peptide SIINFEKL into HI loop (AAV2/OVA and AAV8/OVA) to perform these studies in hepatocyte HepG2/K2b cell lines and after systemic administration in mice including dose-response and kinetics.

***Antigen presentation analysis in HepG2/H2kb cells.*** $1x10^9$ particles of AAV/OVA vector are incubated with HSA for 2hr at 4°C and then used to infect $1x10^5$ HepG2/H2kb cells. 24 hr later, $1x10^6$ spleen cells from OT-1 mice will be added to HepG2 cells overnight. The cells will be harvested and stained with CD8 and CD69 for detection of early T cell activation.

***Animal experiments for kinetics of antigen presentation from AAV empty capsids.*** Seven groups of experiments will be designed: HSA, HSA/preS, HSA/HA2, IISA/NSL, HSA/comb, vehicle treatment -PBS and no AAV as negative control. AAV vectors ($1x10^{11}$ particles) are incubated with HSA or fusion proteins, then administered to C57BL mice via tail vein injection. At different time points (days 3, 21, 41 and 61 post AAV administration), $5x10^6$ CFSE-labeled OT-1 spleen cells will be transfused into these treated mice via IV administration. Ten days after the OT-1 cell infusion, mice will be sacrificed and the proliferation of OT-1 CD8 cells will be analyzed.

***Animal experiments for dose-response of AAV capsid antigen presentation from AAV empty capsids.*** Escalating doses of AAV particles ($10^8$, $10^9$, $10^{10}$, $10^{11}$ and $10^{12}$) incubated with HSA or fusion proteins will be injected into C57BL mice via tail vein. Three days later, CFSE-labeled OT-1 cells will be infused for an OT-1 CD8 proliferation assay. Six groups of mice (0, $10^8$, $10^9$, $10^{10}$, $10^{11}$ and $10^{12}$ of AAV particles) will be assigned.

***Albumin fused with VIPR on capsid antigen presentation.*** We will use US6 to make fusion proteins with HSA (HSA-US6) and HSA-comb (HSA-comb-US6). The effect of US6 fusion proteins on transgene expression and capsid antigen presentation will be studied *in vitro* and *in vivo* as described herein.

***Mouse experiments.*** For transgene expression, four groups (HSA, HSA-US6, HSA-comb, HSA-comb-US6) will be designed. For kinetics of antigen presentation, 6 groups (control, PBS, HSA, HSA-US6, HSA-comb, HSA-comb-US6) will be assigned. For dose-response of antigen presentation, 6 doses (0, $10^8$, $10^9$, $10^{10}$, $10^{11}$, $10^{12}$ particles/mouse) will be studied.

The proteasome inhibitor bortezomib at a high dose has dual functions: increased AAV transduction and decreased antigen presentation. However, when a relatively low dose is used, AAV transduction is increased or not changed, but antigen presentation is increased. Our prior study has demonstrated that effective AAV capsid antigen presentation requires virus escape from endosomes into cytosol for ubiquitination and degradation by proteasome. This enables more virus to escape from endosomes and travel to the nucleus quickly when incubated with HSA or fusion proteins with the result that the HSA or its fusion proteins will increase capsid antigen presentation *in vitro* and at an early time period after AAV systemic administration in mice. Viruses bound to HSA fusion proteins with endosomolytic and NLS peptides may move into the nucleus faster, so the efficiency of capsid antigen presentation should be lower or undetectable from HSA-bound virus transduction at later time points *in vivo.*

***Study of the effect of albumin fusion proteins on AAV Nab escape.*** In the general human population, over 95% of subjects are naturally infected by AAV viruses and half of them generate neutralizing antibodies, which may exclude these subjects from beneficial AAV gene therapy. This particularly impacts patients with liver disorders, who need systemic administration of AAV vectors to target the liver. What are principally sought after are AAV vectors with the ability to evade human Nabs and confer human liver tropism. Several approaches have been exploited to overcome AAV Nabs, including masking the AAV surface with coated polymers, developing AAV mutants with an AAV shuffling library in the presence of Nab, as well as using alternative serotypes of AAV, AAV mutants generated from rational mutation of the Nab binding domain on the AAV capsid surface, plasma-apheresis, anti-CD20 antibody (Rituximab) to deplete B cells, and excessive empty AAV capsids as decoys for Nabs. These approaches either change AAV tropism, take a long time, or have low efficiency or other negative side-effects (Ig deficiency, increased capsid antigen load). The ideal approach would be to have the ability to escape Nab without liver tropism change and severe side effects. Although direct interaction of HSA with AAV enhances AAV transduction, the locations of the binding sites of HSA on AAV virions remain unknown. It is possible that HSA binding sites on the AAV virion surface are the ones that AAV uses for effective binding and effective transduction. In other words, HSA binding sites are also targeted by Nabs. To explore the role of albumin and its fusion proteins in blocking Nab function, we will study whether HSA rescues AAV transduction in the presence of Nabs. AAV surface has 9 variable regions (VRs) which play a role for AAV transduction and Nab binding. It is assumed that an AAV vector is able to escape Nab binding if peptides from 9 VRs are used as decoys. This is similar to an empty virion application. Experiments were conducted to determine whether HAS or its fusion proteins impact AAV transduction in hepatocytes in the presence of Nabs. The effect of HSA and fusion proteins is tested on A20 monoclonal antibody neutralizing activity. Next the effect of HSA and its fusion proteins on AAV transduction is examined in the presence of sera from mouse immunized with AAV and human with positive Nab including IVIG.

***The effect of albumin and its fusion proteins on Nab escape.*** To study whether albumin or albumin fusion proteins interfere with Nab activity, AAV2/luc viruses are incubated with HSA or HSA fusion proteins, and then Nab (A20, AAV immunized mouse sera and human sera or IVIG) is added for neutralizing activity assay.

***Neutralizing antibody assay in Huh7 cells.*** $1x10^8$ particles of AAV2/luc vector in 10ul are incubated with 10ul of HSA or HSA-preS or PBS for 2hr at 4°C, then 20 ul of A20 or sera from mouse or human at serial dilutions are added for another 2hr at 4°C. The mixture of AAV, HSA and Nab is co-cultured with $1x10^5$ Huh cells in serum-free medium for 48 hr. Cell lysate will be used to measure luciferase activity. Nab titers are defined as the highest dilution for which luciferase activity is 50% lower than controls.

***Immunization in mice.*** $1x10^{10}$ particles of AAV2/luciferase (AAV/luc) are intraperitoneally injected into C57BL/C mice, and mice are boosted with the same virus at day 14. Blood sera are collected via the retro-orbital plexus at the indicated time points for NAb assays.

***Human IVIG and plasma.*** 10% human IVIG (Gamunex-c) is purchased from Grifols Therapeutics Inc. (Research Triangle Park, NC). Individual human serum is purchased from Valley Biomedical (Minchester, VA). Human IVIG and serum are aliquoted and stored at -80°C for future use.

***The effect of albumin fused with AAV VR on Nab escape.*** There are 9 VRs on AAV virion surface. HSA fusion proteins are constructed with each single VR peptide and the combination of peptides from all 9 VRs. After purification of HSA fusion proteins, the effect on AAV transduction in Huh7 cells and in mouse liver is tested to verify whether incubation of HSA fusion proteins with AAV VR peptide impacts AAV transduction. Next, the effect of these HSA fusion proteins is studied to determine their effect on AAV transduction in the presence of Nab from mouse and human in Huh 7 cells.

***Mouse experiments.*** To study the effect of HSA fusion protein with AAV2 VRs on AAV transduction, $1x10^{10}$ particles of AAV2/luc are incubated with HSA or HSA-VR fusion proteins for 2 hr at 4°C and then administered into mice via retro-orbital injection. At different time points, the imaging will be taken and analyzed. 12 groups will be designed: PBS, HSA, 9 HSA-VR (from single) and HSA-VRs (from the combination of 9 VRs).

***The effect of albumin fusion protein with CPPs and VRs on Nab escape.*** Based on the studies described herein, it is shown that albumin fusion protein HSA-comb with hepatocyte specific ligand and amphipathic CPP induces higher AAV hepatocyte transduction than any fusion proteins with single CPP, and HSA-VRs (containing 9 VRs) possess stronger inhibition on Nab activity than any single HSA-VR. The effect of albumin fusion protein HSA-Comb-VRs (which is composed of HAS and peptides from CPPs and 9 AAV2 VRs) is then tested on hepatocyte transduction as described herein and Nab evasion capacity.

***Mouse experiments.*** For transgene expression, 4 groups (PBS, HSA-comb, HSA-VRs and HSA-comb-VRs) will be assigned. AAV2/luc vectors are incubated with HSA-comb-VRs for 2hr at 4°C and then administered into mice. The mouse imaging is taken and analyzed.

It has been demonstrated that AAV Nabs bind to the common regions on the AAV virion surface and different Nabs recognize different epitopes. Based on this testing is conducted on the understanding that HSA may share some binding sites with Nabs from some serum samples, which suggests that incubation of HSA with AAV2 will block Nab activity. Since VRs play a major role for effective AAV transduction involving AAV binding, intracellular trafficking, etc., and a peptide scanning study has shown that peptides derived from AAV VRs are able to block Nab activity, we anticipate that BSA fusion proteins with peptides from AAV VRs as a decoy will inhibit Nab activity on AAV transduction.

The overall objective of these studies is to explore the application of human albumin in AAV gene delivery including enhanced AAV transduction, suppression of AAV capsid antigen presentation and blockage of AAV neutralizing antibody recognition, which is critical for wider applications in defects of liver protein synthesis. These preliminary studies and continued contributions towards AAV vector development support the overall experimental design.

Although the present invention that has been described with reference to specific details of certain embodiments thereof, it is not intended that such details should be regarded as limitations upon the scope of the invention except as and to the extent that they are included in the accompanying claims.

Example 7: The effect of IVIG on AAV transduction

It is well known that immunoglobin from AAV immunized sera has neutralizing activity on AAV transduction. In our studies, when human IVIG was used as the source for AAV neutralizing antibody analysis, actually we demonstrated that neutralizing effect was detected when high concentration of IVIG was used, however, when the lower dose of IVIG was applied, the enhanced transgene expression was observed in the liver after systemic administration **(Figure 41).** The result indicates that non neutralizing immunoglobin increases AAV transduction.

Example 8: Application of Polyploid Adeno-Associated Virus Vector for Transduction Enhancement and Neutralizing Antibody Evasion

Adeno-associated virus (AAV) vectors have been successfully used in clinical trials in patients with hemophilia and blindness. Exploration of effective strategies to enhance AAV transduction and escape neutralizing antibody activity is still imperative. Previous studies have shown the compatibility of capsids from AAV serotypes and recognition sites of AAV Nab located on different capsid subunits of one virion. In this study, we co-transfected AAV2 and AAV8 helper plasmids at different ratios (3:1, 1:1 and 1:3) to assemble haploid capsids and study their transduction and Nab escape activity. The haploid virus yield was similar to the parental ones and the heparin sulfate binding ability was positively correlated with AAV2 capsid input. After muscular injection, all of the haploid viruses induced higher transduction than parental AAV vectors (2- to 9-fold over AAV2) with the highest of these being the haploid vector AAV2/8 1:3. After systemic administration, 4-fold higher transduction in the liver was observed with haploid AAV2/8 1:3 than that with AAV8 alone. Importantly, we packaged the therapeutic factor IX cassette into haploid AAV2/8 1:3 capsids and injected them into FIX knockout mice via tail vein. Higher FIX expression and improved phenotypic correction were achieved with haploid AAV2/8 1:3 virus vector compared to that of AAV8. Additionally, haploid virus AAV2/8 1:3 was able to escape AAV2 neutralization and had very low Nab cross-reactivity with AAV2.

To improve Nab evasion ability of polyploid virus, we produced triploid vector AAV2/8/9 vector by co-transfecting AAV2, AAV8 and AAV9 helper plasmids at the ratio of 1:1:1. After systemic administration, 2-fold higher transduction in the liver was observed with triploid vector AAV2/8/9 than that with AAV8. Neutralizing antibody analysis demonstrated that AAV2/8/9 vector was able to escape neutralizing antibody activity from mouse sera immunized with parental serotypes. These results indicate that polyploid virus might potentially acquire advantage from parental serotypes for enhancement of transduction and evasion of Nab recognition. This strategy should be explored in future clinical trials in patients with positive neutralizing antibodies.

The number of helper plasmids with different cap genes is not limited and can be mixed and matched based on the specific requirements of a particular treatment regimen.

***Cell lines.*** HEK293 cells, Huh7 cells and C2C12 cells were maintained at 37 °C in 5% CO2 in Dulbecco's Modified Eagle's Medium with 10% fetal bovine serum and 10% penicillin-streptomycin.

***Recombinant AAV virus production.*** Recombinant AAV was produced by a triple-plasmid transfection system. A 15-cm dish of HEK293 cells was transfected with 9 $\mu$g of AAV transgene plasmid pTR/CBA-Luc, 12 $\mu$g of AAV helper plasmid, and 15$\mu$g of Ad helper plasmid XX680. To generate triploid AAV2/8 virions, the amount of each helper plasmid for AAV2 or AAV8 transfected was co-transfected at three different ratios of 1:1, 1:3 and 3:1. To make haploid AAV2/8/9 vectors, the ratio of helper plasmid for each serotype was 1:1:1. Sixty hours post-transfection, HEK293 cells were collected and lysed. Supernatant was subjected to CsCl gradient ultra-centrifugation. Virus titer was determined by quantitative PCR.

***Western and Immune-blot.*** According to the virus titer, the same amount of virions were loaded in each lane, followed by electrophoresis on a NuPage 4-10% polyacrylamide Bis-Tris gel (Invitrogen, Carlsbad, CA) and then transferred to PVDF membrane *via* iBlot® 2 Dry Blotting System (Invitrogen, Carlsbad, CA). The membrane was incubated with the B1 antibody specific to AAV capsid proteins.

A native immunoblot assay was carried out as previously described. Briefly, purified capsids were transferred to a Hybond-ECL membrane (Amersham, Piscataway, NJ) by using vacuum dot-blotter. The membranes were blocked for 1 h in 10% milk PBS and then incubated with monoclonal antibody A20 or ADK8. The membranes were incubated with a peroxidase-coupled goat anti-mouse antibody for 1 hr. The proteins were visualized by Amersham Imager 600 (GE Healthcare Biosciences, Pittsburg, PA).

***In vitro transduction assay.*** Huh7 and C2C12 cells were transduced by recombinant viruses with 1 x $10^4$ vg/cell in a flat-bottom, 24-well plate. Forty-eight hours later, cells were harvested and evaluated by a luciferase assay system (Promega, Madison, WI).

***Heparin inhibition assays.*** The ability of soluble heparin to inhibit the binding of recombinant viruses to Huh7 or C2Cl2 cells was assayed. Briefly, AAV2, AAV8, haploid viruses AAV2/8 1:1, AAV2/8 1:3 and AAV2/8 3:1 were incubated in DMEM in the presence, or absence, of soluble HS for 1 hat 37°C. After the pre-incubation, the mixture of recombinant viruses and soluble HS were added into Huh7 or C2C12 cells. At 48 h post-transduction, cells were harvested and evaluated by luciferase assay.

***Animal study.*** Animal experiments performed in this study were conducted with C57BL/6 mice and FIX-/- mice. The mice were maintained in accordance to NIH guidelines, as approved by the UNC Institutional Animal Care and Use Committee (IACUC). Six- week-old female C57BL/6 mice were injected with 3 x $10^{10}$ vg of recombinant viruses *via* retro-orbital injection. Luciferase expression was imaged one week post-injection using a Xenogen IVIS Lumina (Caliper Lifesciences, Waltham, MA) following i.p. injection of D-luciferin substrate (Nanolight Pinetop, AZ). Bioluminescent images were analyzed using Living Image (PerkinElmer, Waltham, MA). For muscle transduction, 1 x $10^{10}$ particles of AAV/Luc were injected into the gastrocnemius of 6-week-old C57BL/6 females. Mice were imaged at the indicated time points.

FIX knockout male mice (FIX KO mice) received 1 x $10^{10}$ vg *via* tail vein injection. At various time points after injection, blood was collected from the retro-orbital plexus. At week 6, mouse bleeding analysis was performed.

Quantitation of luciferase expression in the liver Animals utilized for imaging studies were sacrificed at week 4 after recombinant virus injection, and the livers were collected. Livers were minced and homogenized in passive lysis buffer. After the liver lysates were centrifuged, luciferase activity in supernatant was detected. Total protein concentration in tissue lysates were measured using the Bradford assay (BioRad, Hercules, CA).

***Detection of AAV genome copy number in the liver.*** The minced livers were treated by Protease K. The total genome DNA was isolated by PureLink Genomic DNA mini Kit (Invitrogen, Carlsbad, CA). The luciferase gene was detected by qPCR assay. The mouse lamin gene served as an internal control.

***Human FIX expression, function and tail-bleeding time assays.*** The human FIX expression, one-stage hFIX activity assay and tail-bleeding time assay were performed as previously described. Neutralization assay Huh7 cells were seeded in a 48-well plate at a density of $10^5$ cells for each well. Two-fold dilutions of the mouse antibody were incubated with AAV-Luc (1x$10^8$ vg) for 1 hr 37 °C. The mixture was added into cells and incubated for 48 hers at 37 °C. Cells were lysed with passive lysis buffer (Promega, Madison, WI) and luciferase activity was measured. Nab titers were defined as the highest dilution for which luciferase activity was 50% lower than serum-free controls.

***Statistical analysis.*** The data were presented as mean ± SD. The Student *t* test was used to carry out all statistical analyses. P values< 0.05 were considered a statistically significant difference.

***Characterization of haploid viruses in vitro.*** Our previous study has demonstrated the capsid compatibility among AAV1, 2, 3 and 5 capsids. The haploid viruses were produced by transfection of AAV helper plasmids from two serotypes at the different ratios with AAV transgene and adenovirus helper pXX6-80. The enhanced transduction from haploid virus was observed in some cell lines compared to the parental vectors. AAV2 is well characterized for its biology and as a gene delivery vehicle and AAV8 has attracted a lot of attention due to high transduction in mouse liver. Both serotypes have been utilized in several clinical trials in patients with hemophilia. To investigate the possibility of AAV serotype 2 and 8 capsid to form haploid virus and their transduction profile, we transfected the helper plasmids of AAV2 and AAV8 at the ratios of 3:1, 1:1 and 1:3 to make haploid vectors. All of the haploid viruses were purified using cesium gradient and tittered by Q-PCR. There was no significant difference in virus yield between the haploid viruses and the parental AAV2 or AAV8. To determine whether the capsid proteins of haploid viruses were expressed, Western blot analysis was performed on equivalent virus genomes from purified haploid viruses using monoclonal antibody B1 which recognizes the capsid proteins of AAV2 and AAV8. In all haploid viruses, the mixture of VP2 capsids from AAV2 and AAV8 was observed, the intensity of VP2 capsid from AAV2 or AAV8 in haploid viruses was related to the ratio of two helper plasmids. These results suggested that the capsids from AAV 2 and AAV8 were compatible and able to be ensemble into AAV virions.

To determine whether the tropism of haploid virus was changed by mixing the capsid proteins, the transduction efficacy of haploid viruses was analyzed by transducing human Huh7 and mouse C2Cl2 cell lines. The transduction efficiency of AAV8 was much lower than AAV2 in both of the cell lines. The transduction from all haploid vectors was higher than that from AAV8, and the efficiency was positively correlated with addition of AAV2 capsid in both cell lines. Although haploid vector transduction was lower than AAV2 in Huh7 cells, haploid vector AAV2/8 3:1 induced 3-fold higher transduction than AAV2 in C2Cl2 cells.

This *in vitro* transduction data supports that the virus preparation is composed of haploid vectors but not the mixture of individual serotype vector and indicate that haploid vector may enhance AAV transduction. Heparin sulfate proteoglycan has been identified as the primary receptor of AAV2. Next, we investigated whether inhibition of heparin binding ability changed transduction of haploid viruses. Pre-incubation of AAV vectors with soluble heparin blocked AAV2 transduction by nearly 100% in both Huh7 and C2C12 cells, and blocked AAV8 transduction by 37% and 56% in Huh7 and C2C12 cells, respectively. The inhibition of haploid vector transduction by soluble heparin was dependent on the input of AAV2 capsid in both cell lines. Higher inhibition of transduction was observed with more AAV2 capsid input. This result suggests that haploid viruses may use both primary receptors from parental vectors for effective transduction.

***Increased muscular transduction of haploid viruses.*** As described above, the transduction efficiency of haploid virus AAV2/8 3:1 is higher than that of AAV2 and AAV8 in the muscle cell line C2C12. Next we studied whether the high transduction *in vitro* was translated into mouse muscle tissues. AAV2/8 haploid and parental vectors were directly injected into muscle of hind legs in C57BL/6 mouse. As controls, the mixtures of AAV2 and AAV8 viruses at the ratios of 3:1, 1:1 and 1:3 were also investigated. For convenient comparison, one leg was injected with AAV2 and the other one with tested vector. A total vector of 1 x $10^{10}$ vg for each virus was administered. Compared to AAV2, similar muscular transduction was achieved for AAV8. Contrary to the result in C2C12 cells, enhanced muscular transduction was observed from all of the haploid viruses.

Haploid vectors AAV2/8 1:1 and AAV2/8 1:3 achieved 4- and 2-fold higher transduction than AAV2, respectively. Notably, the muscular transduction of haploid vector AAV2/8 3:1 was over 6-fold higher than that of AAV2. However, all of the mixture viruses had similar transduction efficiencies to AAV2. These results suggest that haploid virus is able to increase muscular transduction and further supports that viruses produced from co-transfection of two capsid plasmids are haploid.

***Enhanced liver transduction of haploid viruses.*** AAV2 and AAV8 have been used for liver targeting in several clinical trials in patients with hemophilia B. We also evaluated the transduction efficiency of haploid viruses in mouse liver. The viruses mixed with AAV2 and AAV8 were also injected as controls. A dose of 3 x $10^{10}$ vg of AAV/luc vector was administered in C57BL mice via retro-orbital vein; the imaging was carried out at day 3 post-AAV injection. The haploid virus AAV2/8 1:3 induced the highest transduction efficiency than other haploid, mixture viruses and even parental AAV8 in mouse livers. The transduction efficiency of haploid vector AAV2/8 1:3 was about 4-fold higher than that of AAV8. The liver transduction from other haploid viruses was lower than that from the parental vector AAV8 but higher than AAV2. At day 7 post-injection, the mice were sacrificed, the livers were harvested and the genomic DNA was isolated. The luciferase gene copy number in the liver was determined by qPCR. Different from the result for liver transduction efficiency, similar AAV vector genome copy number was found in the liver regardless of haploid viruses or AAV serotypes 2 and 8. When transgene expression was normalized to gene copy number, consistent to transgene expression in the liver, haploid vector AAV2/8 1:3 induced the highest relative transgene expression than any other haploid vectors and serotypes. The transduction profile of haploid viruses in the liver was different from that in muscle transduction, in which all haploid viruses induced higher transgene expression than that from parental serotypes, with the best from AAV2/8 3:1.

***Augmented therapeutic FIX expression and improved bleeding phenotypic correction with haploid vector in a hemophilia B mouse model.*** Based on the above results, haploid vector AAV2/8 1:3 induced much higher liver transduction than AAV8. Next, we further tested whether the haploid vector AAV2/8 1:3 could increase the therapeutic transgene expression in an animal disease model. We used human FIX (hFIX) as a therapeutic gene and injected haploid vector AAV2/8 1:3/hFIX, which encoded human-optimized FIX transgene, and driven by the liver-specific promoter, TTR, into FIX knockout (KO) mice via tail vein at a dose of 1 x $10^{10}$ vg/mouse. At week 1, 2 and 4 post-injection, the hFIX expression and activity in circulation were analyzed by ELISA and one-stage factor activity, respectively. At week 6, the blood loss for *in vivo* hFIX function was evaluated using a tail clipping assay. Consistent to the observation of high liver transduction with haploid AAV vector in wide-type C57BL/6 mice, haploid vector AAV2/8 1:3 liver targeting produced much more hFIX than AAV8 vector after 2 weeks post-injection. The higher hFIX protein expression of AAV2/8 1:3 was closely related to high FIX activity. The blood loss for the mice with AAV2/8 1:3/hFIX injection was similar to that of wild-type C57BL/6 mice and less than that of KO mice. However, AAV8-treated mice had more blood loss than that in wild type mice. These data show that haploid vector AAV2/8 1:3 increases therapeutic transgene expression from the liver and improves disease phenotypic correction.

***The ability of haploid viruses AAV2/8 to escape neutralizing antibody.*** Each individual haploid virus virion is composed of 60 subunits from different AAV serotype capsids. Insertion of some capsid subunits from one serotype into other capsid subunits from a different serotype may change the virion surface structure. It is well known that most AAV monoclonal antibodies recognize residues on the different subunits of one single virion. To study whether haploid virus is able to escape Nabs generated from parental vector, first we performed Nab binding assay using monoclonal antibodies by an immune-blot assay. Three dilutions of virus-genome-containing particles were adsorbed to a nitrocellulose membrane and probed with Nab A20 or ADK8, which recognizes intact AAV2 or AAV8, respectively. All of the haploid viruses and virus with mixture of AAV2 and AAV8 were recognized by monoclonal antibody ADK8 or A20. The reactivity of haploid viruses with A20 was increased by incorporation of more AAV2 capsids into haploid virus virion. However, there was no obvious change for the recognition of anti-AAV8 Nab ADK8 among the haploid viruses, regardless of capsid ratios. Notably, the binding of haploid AAV2/8 1:3 to A20 was much weaker than those of parental AAV2 and the virus with mixture of AAV2 and 8 at the ratio 1:3, which indicated that A20 binding sites are depleted on the haploid AAV2/8 1:3 virion surface.

Next we analyzed the immunological profile of haploid viruses against sera from AAV-immunized mice. Nab titers were used to evaluate the ability of scrum to inhibit vector transduction. Sera were collected from mice treated with parental viruses at week 4 post-injection. As shown in Table 10, the neutralization profiles of the haploid viruses against A20 or ADK8 were similar to the data from native immune-blot. There was no Nab cross-reactivity between AAV8 and AAV2. It is interesting to note that AAV8-immunized mouse sera had similar neutralizing activity against AAV8 virus and all of the haploid viruses, regardless of the amount of AAV8 capsid incorporation, but not the viruses mixed with AAV2 and AAV8. No inhibition of AAV8 serum on mixture viruses may be explained by the superior transduction from AAV2 to AAV8 in tested cell line. However, haploid viruses partially escaped the neutralization from AAV2 serum. The transduction of haploid AAV2/8 1:1 got a 16-fold decrease than parental AAV2 after incubation of virus and anti-AAV2 serum. The ability to escape AAV2 serum Nab for haploid viruses was much higher than that for viruses mixed with AAV2 and AAV8. Strikingly, the haploid AAV2/8 1:3 almost completely escaped the AAV2 serum and A20 neutralization, suggesting that the haploid virus has the potential to be used for the individuals who have the anti-AAV2 Nab (Table 10).

***Improved neutralizing antibody evasion ability with triploid vector made from three serotypes.*** Our data described above demonstrated that haploid AAV2/8 viruses were not able to escape AAV8 neutralizing antibody activity, but had the capacity to evade AAV2 neutralizing antibody, which depended on the amount of capsid integration from AAV8. To study whether the polyploid virus made from more serotypes capsids improved the Nab escaping ability, we made the triploid virus AAV2/8/9 with the ratio of 1:1:1. After injection of the triploid vector AAV2/8/9 into mice, compared to AAV2, triploid virus AAV2/8/9 induced 2 fold higher transduction in the liver than AAV8. No difference in liver transduction was observed among AAV8 and haploid vectors AAV2/9 and AAV8/9 in which the triploid vector was made from two AAV helper plasmids at ratio of 1:1. It was noted that AAV9 systemic administration induced higher liver transduction than AAV8. When neutralizing antibody assay was performed, haploid AAV2/8/9 vector improved its Nab escape ability by about 20 fold, 32 fold and 8 fold, respectively when compared to AAV2, 8 and 9 **(Table 11).**

In this study, polyploid AAV virions were assembled from capsids of 2 serotypes or 3 serotypes. The binding ability of haploid viruses to AAV2 primary receptor heparin was dependent on the amount of AAV2 capsid input. All of the haploid viruses achieved higher transduction efficacy than parental AAV2 vector in mouse muscle and liver, while haploid virus AAV2/8 1:3 had a significant enhancement of liver transduction than parental AAV8 vector. Compared to AAV8, systemic administration of the haploid virus AAV2/8 1:3 to deliver human FIX induced much higher FIX expression and improved hemophilia phenotypic correction in FIX-/- mice. Importantly, the haploid virus AAV2/8 1:3 was able to escape the neutralization of anti-AAV2 serum. Integration of AAV9 capsid into haploid AAV2/8 virions further improved neutralizing antibody escape capacity.

The primary receptor of AAV2 is HSPG, while the primary receptor of AAV8 is still unclear. To study whether haploid viruses could use receptors from both AAV2 and AAV8, we performed heparin inhibition assay to test the ability of haploid viruses to binding heparin receptor motif. The heparin inhibition results, in Huh7 and C2C12 cell lines, support that haploid viruses use the heparin receptor motif of AAV2 capsids for effective transduction. To some extent, AAV8 also showed decreased transduction efficiency in the presence of heparin, but the transduction efficiency is still higher than that of AAV2.

One of the most challenging aspects of efficient transduction in clinical trials is broad prevalence of neutralizing antibodies to AAV vector. Nab-mediated clearance of AAV vectors has become a limited factor for repeating administration of AAV gene transfer. Several studies have explored genetically modifying AAV capsids for Nab evasion by rational mutation of neutralizing antibody recognizing sites or directed evolution approaches. Capsid mutation may change AAV tropism and transduction efficiency. Additionally, the identification of Nab binding sites on AAV virions is far behind vector application in clinical trials, and it is impossible to figure out all Nab binding sites from poly sera. Previous studies have demonstrated that the recognition sites of several AAV monoclonal antibodies are spun on the different subunits of one virion. When AAV8 capsid is introduced into AAV2 virion, the A20 binding ability and neutralizing activity from AAV2-immunized sera were dramatically decreased for haploid viruses. Integration of AAV2 capsids into AAV8 virions did not reduce the capacity to bind intact AAV8 monoclonal antibody ADK8 and did not escape the neutralizing activity of anti-AAV8 sera **(Table 10).** This suggests that all Nab recognition sites from poly-sera may be located on the same subunit of AAV8 virion. Also, the result suggests that the AAV8 capsids integrated into AAV2 virions may play a major role in virus intracellular trafficking.

When triploid virus was made from capsids of three serotypes AAV2, 8 and 9, different from triploid vectors AAV2/8, haploid AAV2/8/9 virus has an ability to escape neutralizing antibody activity sera from AAV2, 8 or 9 immunized mice, which suggests that AAV8 and AAV9 share the similar transduction pathway.

Several lines of evidences from this study support the polyploid virion assembly from transfection of two or three AAV helper plasmids. (1) Two VP2 bands of different sizes were displayed from haploid viruses using western blot analysis. These VP2s match the size from different serotypes. (2) The transduction profiles were different in C2C12 versus Huh7 cells. Haploid AAV2/8 3:1 vector, in particular, demonstrated lower transduction than that with AAV2 in Huh7 cells, but higher in C2C12 cells. (3) Higher muscle transduction was demonstrated with all haploid AAV2/8 viruses as compared with parental vectors AAV2 and AAV8, as well as the viruses with a mixture of AAV2 and AAV8. (4) Triploid virus AAV2/8 1:3 had enhanced liver tropism when compared to AAV8. (5) The binding pattern of haploid viruses to A20 and ADK8 is different from the viruses with a mixture of AAV2 and AAV8. (6) The profile of AAV2 serum neutralizing activity is different between haploid viruses and mixture viruses. (7) Triploid AAV2/8/9 virus evades neutralizing antibody activity of sera from mice immunized with any parental serotypes.

These polyploid viruses enhance the transduction efficiency *in vitro* and *in vivo,* and even escape neutralization from parental vector immunized sera. Application of the polyploid virus to deliver a therapeutic transgene FIX was able to increase FIX expression and improve hemophilia phenotypic correction in mice with FIX deficiency. These results indicate that haploid AAV vectors have the ability to enhance transduction and evade Nabs.

## Example 9: Enhanced AAV Transduction from Haploid AAV vectors by Assembly of ***AAV*** **Virions with VP1/VP2 from One AAV Vector and VP3 from an Alternative One**

In above studies, we have demonstrated that increased AAV transduction has been achieved using polyploid vectors which are produced by transfection of two AAV helper plasmids (AAV2 and AAV8 or AAV9) or three plasmids (AAV2, AAV8 and AAV9). These individual polyploid vector virions may be composed of different capsid subunits from different serotypes. For example, haploid AAV2/8, which is generated by transfection of AAV2 helper and AAV8 helper plasmids, may have capsid subunits with different combinations in one virion for effective transduction: VP1 from AAV8 and VP2/VP3 from AAV2, or VP1/VP2 from AAV8 and VP3 from AAV2, or VP1 from AAV2 and VP2/VP3 from AAV8, or VP1/VP2 from AAV2 and VP3 from AAV8, or VP1 from AAV8 and VP3 from AAV2, or VP1 from AAV2 and VP3 from AAV8, or VP1/VP2/VP3 from AAV2, or VP1/VP2/VP3 from AAV8. In the following studies, we found that enhanced transduction could be achieved from haploid vectors with VP1/VP2 from one AAV vector capsid and VP3 from an alternative one.

The generation of VP1, VP2 and VP3 by different AAV serotypes offers two different strategies for producing these different proteins. Interestingly, the VP proteins are translated from a single CAP nucleotide sequence with overlapping sequences for VP1, VP2 and VP3.

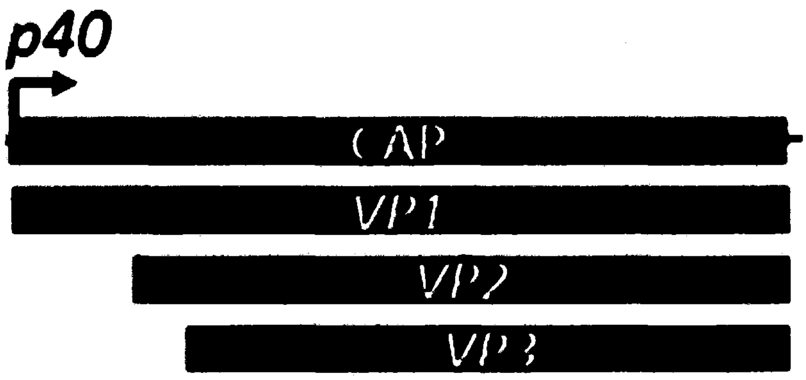


The Cap gene encodes for 3 proteins - VP1, VP2 and VP3. As shown in the above figure, VP1 contains the VP2 and VP3 proteins, and VP2 contains the VP3 protein. Therefore, the Cap gene has 3 segments, start of VP1 - start of VP2 - start of VP3 - end of all 3 VP proteins.

The overlap of the nucleotide sequences for the VP proteins presents an opportunity to generate the different VP proteins from different AAV serotypes. For example, if the nucleotide sequence contained the nucleotides from "A" in the first segment, wherein A is a first serotype, "B" in the second segment, wherein B is a second serotype that is distinct and different from A and "B" in the third segment; the resulting VP1 protein would contain ABB segments, VP2 protein would contain BB segments and VP3 would contain a B segment. Therefore, depending on which segments contained which nucleotides sequences, the result capsid will have differing amounts of each Cap protein.

In the case of sourcing the Cap genes from two different AAV serotypes (designated as A and B), there are 6 possible combinations of the three Cap proteins.

| VP1 | VP2 | VP3 |
|---|---|---|
| A | B | B |
| A | B | A |
| A | A | B |
| B | B | A |

(continued)

| VP1 | VP2 | VP3 |
|---|---|---|
| B | A | B |
| B | A | A |

In the case of sourcing the Cap genes from three different AAV serotypes (designated as A, B and C), there are 6 possible combination of the three Cap proteins.

| VP1 | VP2 | VP3 |
|---|---|---|
| A | B | C |
| A | C | B |
| B | A | C |
| B | C | A |
| C | A | B |
| C | B | A |

Additionally, the Cap gene in the helper plasmid can be generated with a full copy of the nucleotide sequence for the particular VP protein from the two AAV serotypes. The individual Cap genes will generate the VP proteins associated with that particular AAV serotype (designated as A and B).

| VP1 | VP2 | VP3 |
|---|---|---|
| A | B | B |
| A | B | A |
| A | A | B |
| B | B | A |
| B | A | B |
| B | A | A |

In the case of 3 different Cap genes, the helper plasmid can be generated with a full copy of the nucleotide sequence for the particular VP protein from the three AAV serotypes. The individual Cap genes will generate the VP proteins associated with that particular AAV serotype (designated as A, B and C).

| VP1 | VP2 | VP3 |
|---|---|---|
| A | B | C |
| A | C | B |
| B | A | C |
| B | C | A |
| C | A | B |
| C | B | A |

***Haploid vector with C-terminal of VP1/VP2 from AAV8 and VP3 from AAV2 enhances AAV transduction.*** It has been demonstrated that haploid vectors AAV2/8 at any ratio of AAV2 capsid to AAV8 capsid induced higher liver transduction than AAV2 or the viruses with mixture of AAV2 vectors and AAV8 vectors at the same ratio. To elucidate which AAV subunits in individual haploid AAV2/8 vector contributes to higher transduction than AAV2, we made different constructs which expressed AAV8 VP1/VP2 only, AAV2 VP3 only, chimeric VP1/VP2 (28m-2VP3) with N-terminal from AAV2 and C-terminal from AAV8, or chimeric AAV8/2 with N-terminal from AAV8 and C-terminal from AAV2 without mutation of VP3 start codon. These plasmids were used to produce haploid AAV vector with different combination. After injection of 1x$10^{10}$ particles of these haploid vectors in mice via retro-orbital vein, the liver transduction efficiency was evaluated. Chimeric AAV82 vector (AAV82) induced a little higher liver transduction than AAV2. However, haploid AAV82 (H-AAV82) had much higher liver transduction than AAV2. A further increase in liver transduction with haploid vector 28m-2vp3 was observed. We also administered these haploid vectors into the muscles of mice. For easy comparison, the right leg was injected with AAV2 vector and the left leg was injected with haploid vector when the mouse was face up. At week 3 after AAV injection, the images were taken. Consistent to observation in the liver, all haploid vectors and chimeric vectors had higher muscular transduction with the best from haploid vector 28m-2vp3. This result indicates that the chimeric VP1/VP2 with N-terminal from AAV2 and C-terminal from AAV8 attributes to high liver transduction of haploid AAV82 vectors.

***Enhanced AAV liver transduction from haploid vector with*** **VP1/VP2** ***from other serotypes and VP3 from AAV2.*** We have shown that haploid vector AAV82 with VP1/VP2 from AAV8 and VP3 from AAV2 increases the liver transduction as described above. Next, we would like to examine whether other haploid virions, in which VP1/VP2 is derived from different serotypes, also increases transduction. In preclinical studies, AAV9 has been shown to efficiently transduce different tissues. We have made a haploid AAV92 vector (H-AAV92) in which VP1/VP2 was from AAV9 and VP3 from AAV2. After systemic administration, the imaging was performed at week 1. About 4- fold higher liver transduction was achieved with H-AAV92 than that with AAV2. This data indicates that VP1/VP2 from other serotypes is also capable of increasing AAV2 transduction.

***Enhanced AAV liver transduction from haploid vector with VP3 from AAV2 mutant or other serotypes.*** AAV9 uses glycan as primary receptor for effective transduction. In our previous studies, we have engrafted AAV9 glycan receptor binding site into AAV2 to make AAV2G9 and found that AAV2G9 has higher liver tropism than AAV2. Herein we made haploid vector (H-AAV82G9) in which **VP1/VP2** from AAV8 and VP3 from AAV2G9. After systemic injection into mice, compared to AAV2G9, more than 10 fold higher liver transduction was observed at both week 1 and week 2 post H-AAV82G9 application. To study haploid vectors in which VP3 from other serotypes and VP1/VP2 from different serotypes or variants, we cloned other constructs: AAV3 VP3 only, AAV th10 VP1/VP2 only, and made different haploid vectors with various combination (H-AAV83, H-AAV93 and H-AAVrh10-3). After systemic injection into mice, the imaging was carried out at week 1. Consistent to the results obtained from other haploid vectors, higher liver transduction was achieved with haploid vectors (H-AAV83, H-AAV93 and H-AAVrh10-3) than that with AAV3. It is interesting to note that these haploid vectors also induced a whole body transduction based on imaging profile, which is different from the results from haploid vectors 5 with VP3 from AAV2, which only transduced the liver efficiently. Collectively, haploid vectors with VP1/VP2 from one serotype and VP3 from an alternative one are able to enhance transduction and perhaps change tropism.

### *Example 10: Polyploid Adeno-Associated Virus Vectors Enhance Transduction and Escape Neutralizing Antibody*

Adeno-associated virus (AAV) vectors have been successfully used in clinical trials in patients with hemophilia and blindness. Although the application of AAV vectors has proven safe and shown therapeutic effect in these clinical trials, one of the major challenges is its low infectivity that requires relatively large amount of virus genomes. Additionally, a large portion of the population has neutralizing antibodies (Nabs) against AAVs in the blood and other bodily fluids. The presence of Nabs poses another major challenge for broader AAV applications in future clinical trials. Effective strategies to enhance AAV transduction and escape neutralizing antibody activity are highly demanded. Previous studies have shown the compatibility of capsids from AAV serotypes and recognition sites of AAV Nab located on different capsid subunits of one virion. In this study, we propose to study whether polyploid AAV viruses produced from co-transfection of different AAV helper plasmids have the ability for enhanced AAV transduction and escape of Nabs. We co-transfected AAV2 and AAV8 helper plasmids at different ratios (3:1, 1:1 and 1:3) to assemble haploid capsids. The haploid virus yield was similar to the parental ones, suggesting that these two AAV capsids were compatible. In Huh7 and C2Cl2 cell lines, the transduction efficiency of AAV8 was much lower than those from AAV2; however, the transduction from all haploid vectors was higher than that from AAV8. The transduction efficiency and the heparin sulfate binding ability for haploid vectors were positively correlated with amount of integrated AAV2 capsid. These results indicate that the haploid virus vectors retain their parental virus properties and take advantage of the parental vectors for enhanced transduction. After muscular injection, all of the haploid viruses induced higher transduction than parental AAV vectors (2- to 9-fold over AAV2) with the highest of these being the haploid vector AAV2/8 3:1.

After systemic administration, 4-fold higher transduction in the liver was observed with haploid vector AAV2/8 1:3 than that with AAVS alone. Importantly, we packaged the therapeutic factor IX cassette into haploid vector AAV2/8 1:3 capsids and injected them into FIX knockout mice via tail vein. Higher FIX expression and improved phenotypic correction were achieved with haploid vector AAV2/8 1:3 virus vector compared to that of AAVS. Strikingly, haploid virus AAV2/8 1:3 was able to escape AAV2 neutralization and had very low Nab cross-reactivity with AAV2. But AAVS neutralizing antibody can inhibit haploid vector AAV2/8 transduction the same efficiency as AAV8. Next, we produced triploid vector AAV2/8/9 vector by co-transfecting AAV2, AAV8 and AAV9 helper plasmids at the ratio of 1:1:1. After systemic administration, 2-fold higher transduction in the liver was observed with triploid vector AAV2/8/9 than that with AAV8. Neutralizing antibody analysis demonstrated that AAV2/8/9 vector was able to escape neutralizing antibody activity from mouse sera immunized with parental serotype, different from AAV2/8 triploid vector. The results indicate that polyploid virus might potentially acquire advantage from parental serotypes for enhancement of transduction and has ability for evasion of Nab recognition. This strategy should be explored in future clinical trials in patients with positive neutralizing antibodies.

Example 11: Substitution of AAV capsid subunits enhances transduction and escapes neutralizing antibody

Therapeutic effect has been achieved in clinical trials in patients with blood diseases and blind disorders using adeno-associated virus (AAV) vector. However, two concerns restrict broadening AAV vector application: AAV capsid specific cytotoxic Tcell (CTL) and neutralizing antibodies (Nabs). Enhancing AAV transduction with low dose of AAV vector will potentially decrease capsid antigen load and hopefully ablate capsid CTL mediated clearance of AAV transduced target cells without compromise of transgene expression. Currently, 12 serotypes and over 100 variants or mutants have been explored for gene delivery due to their different tissue tropism and transduction efficiency. It has been demonstrated that there is compatibility of capsid among AAV serotypes, and integration of specific amino acids from one serotype into another AAV capsid enhances AAV transduction. By taking advantage of different mechanisms for effective AAV transduction from different serotypes, enhanced AAV transduction was achieved using mosaic virus in which AAV capsid subunits are derived from different serotypes *in vitro* and *in vivo.* The recent structural studies on interaction of AAV vectors with monoclonal neutralizing antibodies demonstrated that Nab binds to residues on several different subunits of one virion surface, which suggests that change of subunit assembly of AAV virion may ablate the AAV Nab binding site and then escape Nab activity. We have demonstrated that the mosaic AAV vector is able to evade Nab activity. These results indicate that substitution of AAV capsid subunits has the potential to enhance AAV transduction and the ability of neutralizing antibody evasion.

Adeno-associated virus (AAV) vector has been successfully applied in clinical trials in patients with blood diseases and blind disorders. Two concerns restrict broad AAV vector application: AAV capsid specific cytotoxic T cell (CTL) response mediated elimination of AAV transduced target cells and neutralizing antibodies (Nabs) mediated blocking of AAV transduction. It has been demonstrated that capsid antigen presentation is dose-dependent, which indicates that enhancing AAV transduction with low dose of AAV vector will potentially decrease capsid antigen load and hopefully ablate capsid CTL mediated clearance of AAV transduced target cells without compromise of transgene expression. Several approaches have been explored for this purpose including: optimization of transgene cassette, modification of AAV capsid and interference of AAV trafficking with pharmacological agents. Modification of AAV capsid may change AAV tropism; especially AAV transduction efficiency is unknown in human tissues. Though several clinical trials have been ongoing, the AAV vector was empirically chosen based on observation from animal models. Pharmacological reagents for enhancing AAV transduction usually have unwanted side effects. It is imperative to develop ideal strategies to enhance AAV transduction but without changing its tropism from modification of capsids and no side effects from pharmacological treatment. Currently, there are 12 serotypes and over 100 variants or mutants which have been explored for gene delivery. Effective AAV transduction involves following steps including: binding on the target cell surface via receptors and co-receptors, endocytosis into endosomes, escape from endosomes, nuclear entrance, AAV virion uncoating followed by transgene expression. To rationally design novel AAV vectors for enhanced transduction, we have developed chimeric viruses: AAV2.5 (in which AAV2 mutant with 5 aa substitution from AAV1) and AAV2G9 (in which galactose receptor from AAV9 is engrafted into AAV2 capsid). Both chimeric mutants induce a much higher transduction than AAV2 in mouse muscle and liver, respectively. These observations indicate that these chimeric viruses may use properties from both AAV serotypes for enhanced transduction (for example, AAV2G9 uses two primary receptors-heparin and galactose for effective cell surface binding). Based on the compatibility among capsid subunits from different AAV serotypes for virus assembly and our preliminary results, which demonstrated that integration of specific amino acids from other serotypes (1 or 9) into AAV serotype 2 enhanced AAV2 transduction in muscle and the liver, we reason that substitution of some capsid subunits from other serotypes is able to enhance AAV transduction by taking advantage of different mechanisms for effective AAV transduction from different serotypes. In addition, pre-existing antibodies to naturally occurring AAV have impacted success for hemophilia B and other AAV gene transfer studies. In the general human population, around 50% carry neutralizing antibodies. Several approaches have been considered to design NAb-evading AAV vectors, including chemical modification, different serotype of AAV vector, rational design and combinatorial mutagenesis of the capsid *in situ* as well as biological depletion of NAb titer (empty capsid utilization, B cell depletion and plasma-apheresis). These approaches have low efficiency or side-effect or change of AAV tropism. The recent structural studies on interaction of AAV vectors with monoclonal neutralizing antibodies demonstrated that Nab binds to residues on several different subunits of one virion surface, which suggests that change of subunit assembly of AAV virion may ablate the AAV Nab binding site and then escape Nab activity. We have results strongly supporting the notion that novel mosaic AAV vectors have potential to enhance transduction in various tissues and are able to escape neutralizing antibody activity.

***Example 12: Treatment of Diseases of the Central Nervous System with VP1/VP2/VP3 from Two or More Different AAV Serotypes***

In a first experiment, two helper plasmids are used. The first helper plasmid has the Rep and Cap genes from AAV2 and the second helper plasmid has the Rep gene from AAV2 and the Cap gene from AAV4. A third plasmid encodes for the nucleotide sequence for Glutamic Acid Decarboxylase 65 (GAD65) and/or Glutamic Acid Decarboxylase 67 (GAD67), which nucleotide sequence is inserted between two ITRs. The haploid AAV generated from the three plasmids contains the nucleotide sequence for GAD65 and/or GAD67protein to treat Parkinson's disease, in part by increasing the specificity for central nervous system tissues associated with Parkinson's disease through the use of multiple AAV serotypes to source the proteins that code for VP1, VP2 and VP3 according to the methods of the present invention. In fact, the haploid virus created by this method to treat Parkinson's disease has a higher specificity for the relevant tissue than a virus vector comprised of only AAV2 or AAV4.

In a further experiment, two helper plasmids are again used with different AAV serotypes as the source for the Rep and Cap genes. The first helper plasmid has the Rep and Cap genes from AAV3 and the second helper plasmid has the Rep gene from AAV3 and the Cap gene from AAV5. A third plasmid encodes the nucleotide sequence for CLN2 to treat Batten's disease is contained in a third plasmid and has been inserted between two ITRs. The haploid AAV generated from the three plasmids contains the nucleotide sequence to treat Batten's disease, in part by increasing the specificity for central nervous system tissues associated with Parkinson's disease through the use of multiple AAV serotypes to source the proteins that code for VP1, VP2 and VP3 according to the methods of the present invention. In fact, the haploid virus created by this method to treat Batten's disease has a higher specificity for the relevant central nervous system tissue than a virus vector comprised of only AAV3 or AAV5.

In another experiment, three helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV4. A third helper plasmid has the Rep gene from AAV3 and the Cap gene from AAV5. A fourth plasmid encodes the nucleotide sequence for Nerve Growth Factor (NGF) to treat Alzheimer's disease is contained in a third plasmid and has been inserted between two ITRs. The triploid AAV generated from the four plasmids contains the nucleotide sequence to treat Alzheimer's disease, in part by increasing the specificity for central nervous system tissues associated with Alzheimer's disease through the use of multiple AAV serotypes (e.g., AAV3, AAV4 and AAV5) to source the proteins that code for VP1, VP2 and VP3 according to the methods of the present invention. In fact, the triploid virus created by this method to treat Alzheimer's disease has a higher specificity for the relevant central nervous system tissue than a virus vector comprised of only AAV3, AAV4 or AAV5.

In another experiment, one helper plasmid is used with different AAV serotypes as the source for the Rep and Cap genes. The helper plasmid has the Rep from AAV2 and VP1 from AAV2, VP2 from AAV4 and VP3 from AAV5. A second plasmid encodes the nucleotide sequence for AAC inserted between two ITRs to treat Canavan's disease. The triploid AAV generated from the two plasmids contains the nucleotide sequence to treat Canavan's disease, in part by increasing the specificity for central nervous system tissues associated with Canavan's disease through the use of multiple AAV serotypes (e.g., AAV2, AAV4 and AAV5) to source the proteins that code for VP1, VP2 and VP3 according to the methods of the present invention. In fact, the triploid virus created by this method to treat Canavan's disease has a higher specificity for the relevant central nervous system tissue than a virus vector comprised of only AAV2, AAV4 or AAV5.

***Treatment of Diseases of Heart with VP1/VP2/VP3 from Two or More Different AAV Serotypes.*** In an experiment, two helper plasmids are used with different AAV serotypes as the source for the Rep and Cap genes. The first helper plasmid has the Rep and Cap genes from AAV2 and the second helper plasmid has the Rep gene from AAV2 and the Cap gene from AAV6. A third plasmid encodes the nucleotide sequence for the protein to treat heart disease is contained in a third plasmid and has been inserted between two ITRs. The haploid AAV generated from the three plasmids contains the nucleotide sequence to treat heart disease, in part by increasing the specificity heart tissue associated with heart's disease through the use of multiple AAV serotypes to source the proteins that code for VP1, VP2 and VP3 according to the methods of the present invention. In fact, the haploid virus created by this method to treat heart disease has a higher specificity for the relevant heart tissue than a virus vector comprised of only AAV2 or AAV6.

In a further experiment, two helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV9. A third plasmid encodes the nucleotide sequence for the protein to treat heart disease is contained in a third plasmid and has been inserted between two ITRs. The haploid AAV generated from the three plasmids contains a nucleotide sequence encoding a protein to treat heart disease, in part by increasing the specificity heart tissue associated with heart's disease through the use of multiple AAV serotypes to source the proteins that code for VP1, VP2 and VP3 according to the methods of the present invention. In fact, the haploid virus created by this method to treat heart disease has a higher specificity for the relevant heart tissue than a virus vector comprised of only AAV3 or AAV9.

In an experiment, three helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV6. A third helper plasmid has the Rep gene from AAV3 and the Cap gene from AAV9. A fourth plasmid contains a nucleotide sequence that encodes a protein to treat heart disease is contained in a third plasmid and has been inserted between two ITRs. The triploid AAV generated from the four plasmids contains the nucleotide sequence to treat heart disease, in part by increasing the specificity for heart tissue associated with heart disease through the use of multiple AAV serotypes (e.g., AAV3, AAV6 and AAV9) to source the proteins that code for VP1, VP2 and VP3 according to the methods of the present invention. In fact, the triploid virus created by this method to treat heart disease has a higher specificity for the relevant heart tissue than a virus vector comprised of only AAV3, AAV6 or AAV9.

In another experiment, one helper plasmid is used with different AAV serotypes as the source for the Rep and Cap genes. The helper plasmid has the Rep from AAV2 and VP1 from AAV2, VP2 from AAV3 and VP3 from AAV9. A second plasmid contains a nucleotide sequence encoding a protein to treat heart disease inserted between two ITRs. The triploid AAV generated *from* the two plasmids encodes the nucleotide sequence to treat heart disease, in part by increasing the specificity for heart tissues associated with heart disease through the use of multiple AAV serotypes (e.g., AAV2, AAV3 and AAV9) to source the proteins that code for VP1, VP2 and VP3 according to the methods of the present invention. In fact, the triploid virus created by this method to treat heart disease has a higher specificity for the relevant heart tissue than a virus vector comprised of only AAV2, AAV3 or AAV9.

In another experiment, one helper plasmid is used with different AAV serotypes as the source for the Rep and Cap genes. The helper plasmid has the Rep from AAV3 and VP1 from AAV3, VP2 from AAV6 and VP3 from AAV6. A second plasmid contains a nucleotide sequence encoding a protein to treat heart disease inserted between two ITRs. The haploid AAV generated from the two plasmids encodes the nucleotide sequence to treat heart disease, in part by increasing the specificity for heart tissues associated with heart disease through the use of multiple AAV serotypes (e.g. AAV3 and AAV6) to source the proteins that code for VP1, VP2 and VP3 according to the methods of the present invention. In fact, the haploid virus created by this method to treat heart disease has a higher specificity for the relevant heart tissue than a virus vector comprised of only AAV2 or AAV6.

In another experiment, one helper plasmid is used with different AAV serotypes as the source for the Rep and Cap genes. The helper plasmid has the Rep from AAV3 and VP1 from AAV3, VP2 from AAV6 and VP3 from AAV9. A second plasmid contains a nucleotide sequence encoding a protein to treat heart disease inserted between two ITRs. The triploid AAV generated from the two plasmids encodes the nucleotide sequence to treat heart disease, in part by increasing the specificity for heart tissues associated with heart disease through the use of multiple AAV serotypes (*e.g.,* AAV3, AAV6 and AAV9) to source the proteins that code for VP1, VP2 and VP3 according to the methods of the present invention. In fact, the triploid virus created by this method to treat heart disease has a higher specificity for the relevant heart tissue than a virus vector comprised of only AAV3, AAV6 or AAV9.

***Treatment of Diseases of the Lung with VP1/VP2/VP3 from Two or More Different AAV V Serotypes.*** In an experiment, two helper plasmids are again used with different AAV serotypes as the source for the Rep and Cap genes. The first helper plasmid has the Rep and Cap genes from AAV2 and the second helper plasmid has the Cap gene from AAV9. A third plasmid encodes for the nucleotide sequence for CFTR to treat Cystic Fibrosis is inserted between two ITRs. The haploid AAV generated from the three plasmids contains the nucleotide sequence for CFTR to treat Cystic Fibrosis, in part by increasing the specificity for lung tissue associated with Cystic Fibrosis through the use of multiple AAV serotypes to source the proteins that code for VP1, VP2 and VP3 according to the methods of the present invention. In fact, the haploid virus created by this method to treat Cystic Fibrosis has a higher specificity for the relevant tissue than a virus vector comprised of only AAV2 or AAV9.

In an experiment, two helper plasmids are again used with different AAV serotypes as the source for the Rep and Cap genes. The first helper plasmid has the Rep and Cap genes from AAV3 and the second helper plasmid has the Rep from AAV3 and the Cap gene from AAV10. A third plasmid encodes for the nucleotide sequence for CFTR to treat Cystic Fibrosis is inserted between two ITRs. The haploid AAV generated from the three plasmids contains the nucleotide sequence for CFTR to treat Cystic Fibrosis, in part by increasing the specificity for lung tissue associated with Cystic Fibrosis through the use of multiple AAV serotypes to source the proteins that code for VP1, VP2 and VP3 according to the methods of the present invention. In fact, the haploid virus created by this method to treat Cystic Fibrosis has a higher specificity for the relevant tissue than a virus vector comprised of only AAV3 or AAV10.

In an experiment, three helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV9. A third helper plasmid has the Rep gene from AAV3 and the Cap gene from AAV10. A fourth plasmid encodes a nucleotide sequence for CFTR to treat Cystic Fibrosis is contained in a third plasmid and has been inserted between two ITRs. The triploid AAV generated from the four plasmids contains the nucleotide sequence for CFTR to treat Cystic Fibrosis, in part by increasing the specificity for lung tissue associated with Cystic Fibrosis through the use of multiple AAV serotypes (e.g. AAV3, AAV9 and AAV10) to source the proteins that code for VP1, VP2 and VP3 according to the methods of the present invention. In fact, the triploid virus created by this method to treat Cystic Fibrosis has a higher specificity for the relevant tissue than a virus vector comprised of only AAV3, AAV9 or AAV10.

In another experiment, one helper plasmid is used with different AAV serotypes as the source for the Rep and Cap genes. The helper plasmid has the Rep from AAV2 and VP1 from AAV2, VP2 from AAV9 and VP3 from AAV9. A second plasmid encodes the nucleotide sequence for CFTR inserted between two ITRs to treat Cystic Fibrosis. The haploid AAV generated from the two plasmids contains the nucleotide sequence to treat Cystic Fibrosis, in part by increasing the specificity for central nervous system tissues associated with Cystic Fibrosis through the use of multiple AAV serotypes (e.g., AAV2 and AAV9) to source the proteins that code for VP1, VP2 and VP3 according to the methods of the present invention. In fact, the haploid virus created by this method to treat Cystic Fibrosis has a higher specificity for the relevant tissue than a virus vector comprised of only AAV2 or AAV9.

In a further experiment, one helper plasmid is used with different AAV serotypes as the source for the Rep and Cap genes. The helper plasmid has the Rep from AAV3 and VP1 from AAV2, VP2 from AAV10 and VP3 from AAV10. A second plasmid encodes the nucleotide sequence for CFTR inserted between two ITRs to treat Cystic Fibrosis. The haploid AAV generated from the two plasmids contains the nucleotide sequence to treat Cystic Fibrosis, in part by increasing the specificity for central nervous system tissues associated with Cystic Fibrosis through the use of multiple AAV serotypes (e.g. AAV3 and AAV10) to source the proteins that code for VP1, VP2 and VP3 according to the methods of the present invention. In fact, the haploid virus created by this method to treat Cystic Fibrosis has a higher specificity for the relevant tissue than a virus vector comprised of only AAV3 or AAV10.

In another experiment, one helper plasmid is used with different AAV serotypes as the source for the Rep and Cap genes. The helper plasmid has the Rep from AAV2 and VP1 from AAV2, VP2 from AAV9 and VP3 from AAV10. A second plasmid encodes the nucleotide sequence for CFTR inserted between two ITRs to treat Cystic Fibrosis. The triploid AAV generated from the two plasmids contains the nucleotide sequence to treat Cystic Fibrosis, in part by increasing the specificity for central nervous system tissues associated with Canavan's disease through the use of multiple AAV serotypes (e.g., AAV2, AAV9 and AAV10) to source the proteins that code for VP1, VP2 and VP3 according to the methods of the present invention. In fact, the triploid virus created by this method to treat Cystic Fibrosis has a higher specificity for the relevant tissue than a virus vector comprised of only AAV2, AAV9 or AAV10.

***Treatment of Diseases of the Skeletal Muscle with VP1/VP2/VP3 from Two or More Different AAV Serotypes.*** For the following experiments, the skeletal muscle disease can be, but is not limited to, Duchene Muscular Dystrophy, Limb Girdle Muscular Dystrophy, Cerebral Palsy, Myasthenia Gravis and Amyotrophic Lateral Sclerosis (ALS).

In an experiment, two helper plasmids are again used with different AAV serotypes as the source for the Rep and Cap genes. The first helper plasmid has the Rep and Cap genes from AAV2 and the second helper plasmid has the Rep from AAV2 and the Cap gene from AAV8. A third plasmid encodes for the nucleotide sequence for a protein to treat a disease of the skeletal muscle that is inserted between two ITRs. The haploid AAV generated from the three plasmids contains the nucleotide sequence for a protein to treat a disease of the skeletal muscle, in part by increasing the specificity for skeletal muscle associated with a disease of the skeletal muscle through the use of multiple AAV serotypes to source the proteins that code for VP1, VP2 and VP3 according to the methods of the present invention. In fact, the haploid virus created by this method to treat a skeletal muscle disease has a higher specificity for the relevant skeletal muscle tissue than a virus vector comprised of only AAV2 or AAV8.

In an experiment, two helper plasmids are again used with different AAV serotypes as the source for the Rep and Cap genes. The first helper plasmid has the Rep and Cap genes from AAV3 and the second helper plasmid has the Rep from AAV3 and the Cap gene from AAV9. A third plasmid encodes for the nucleotide sequence for a protein to treat a disease of the skeletal muscle that is inserted between two ITRs. The haploid AAV generated from the three plasmids contains the nucleotide sequence for a protein to treat a disease of the skeletal muscle, in part by increasing the specificity for skeletal muscle associated with a disease of the skeletal muscle through the use of multiple AAV serotypes to source the proteins that code for VP1, VP2 and VP3 according to the methods of the present invention. In fact, the haploid virus created by this method to treat a skeletal muscle disease has a higher specificity for the relevant skeletal muscle tissue than a virus vector comprised of only AAV3 or AAV9.

In an experiment, three helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV8. A third helper plasmid has the Rep gene from AAV3 and the Cap gene from AAV9. A fourth plasmid encodes for the nucleotide sequence for a protein to treat a disease of the skeletal muscle that is inserted between two ITRs. The triploid AAV generated from the four plasmids contains the nucleotide sequence for a protein to treat a skeletal muscle disease, in part by increasing the specificity for skeletal muscle associated with a disease of the skeletal muscle through the use of multiple AAV serotypes (*e.g.,* AAV3, AAV8 and AAV9) to source the proteins that code for VP1, VP2 and VP3 according to the methods of the present invention. In fact, the triploid virus created by this method to treat a skeletal muscle disease has a higher specificity for the relevant tissue than a virus vector comprised of only AAV3, AAV8 or AAV9.

In another experiment, one helper plasmid is used with different AAV serotypes as the source for the Rep and Cap genes. The helper plasmid has the Rep from AAV3 and VP1 from AAV3, VP2 from AAV9 and VP3 from AAV9. A second plasmid encodes for the nucleotide sequence for a protein to treat a disease of the skeletal muscle that is inserted between two ITRs. The haploid AAV generated from the two plasmids contains the nucleotide sequence to treat a disease of the skeletal muscle that, in part by increasing the specificity for skeletal muscle tissues associated with a skeletal muscle disease through the use of multiple AAV serotypes (e.g. AAV3 and AAV9) to source the proteins that code for VP1, VP2 and VP3 according to the methods of the present invention. In fact, the haploid virus created by this method to treat a skeletal muscle disease has a higher specificity for the relevant skeletal muscle tissue than a virus vector comprised of only AAV3 or AAV9.

In another experiment, one helper plasmid is used with different AAV serotypes as the source for the Rep and Cap genes. The helper plasmid has the Rep from AAV3 and VP1 from AAV3, VP2 from AAV8 and VP3 from AAV8. A second plasmid encodes for the nucleotide sequence for a protein to treat a disease of the skeletal muscle that is inserted between two ITRs. The haploid AAV generated from the two plasmids contains the nucleotide sequence to treat a disease of the skeletal muscle that, in part by increasing the specificity for skeletal muscle tissues associated with a skeletal muscle disease through the use of multiple AAV serotypes (e.g. AAV3 and AAV8) to source the proteins that code for VP1, VP2 and VP3 according to the methods of the present invention. In fact, the haploid virus created by this method to treat a skeletal muscle disease has a higher specificity for the relevant skeletal muscle tissue than a virus vector comprised of only AAV3 or AAV8.

In another experiment, one helper plasmid is used with different AAV serotypes as the source for the Rep and Cap genes. The helper plasmid has the Rep from AAV3 and VP1 from AAV3, VP2 from AAV8 and VP3 from AAV9. A second plasmid encodes for the nucleotide sequence for a protein to treat a disease of the skeletal muscle that is inserted between two ITRs. The triploid AAV generated from the two plasmids contains the nucleotide sequence to treat a disease of the skeletal muscle that, in part by increasing the specificity for skeletal muscle tissues associated with a skeletal muscle disease through the use of multiple AAV serotypes (e.g. AAV3, AAV8 and AAV9) to source the proteins that code for VP1, VP2 and VP3 according to the methods of the present invention. In fact, the triploid virus created by this method to treat a skeletal muscle disease has a higher specificity for the relevant skeletal muscle tissue than a virus vector comprised of only AAV3, AAV8 or AAV9.

***Treatment of Diseases of the Liver with VP1/VP2/VP3 from Two or More Different AAV Serotypes.*** In an experiment, two helper plasmids are again used with different AAV serotypes as the source for the Rep and Cap genes. The first helper plasmid has the Rep and Cap genes from AAV2 and the second helper plasmid has the Rep from AAV2 and the Cap gene from AAV6. A third plasmid encodes for the nucleotide sequence for a Factor IX (FIX) to treat Hemophilia B that is inserted between two ITRs. The haploid AAV generated from the three plasmids contains the nucleotide sequence for a protein to treat a disease of the skeletal muscle, in part by increasing the specificity for FIX associated with Hemophilia B through the use of multiple AAV serotypes to source the proteins that code for VP1, VP2 and VP3 according to the methods of the present invention. In fact, the haploid virus created by this method to treat liver tissue in a patient suffering from Hemophilia B has a higher specificity for the relevant tissue than a virus vector comprised of only AAV2 or AAV6.

In an experiment, two helper plasmids are again used with different AAV serotypes as the source for the Rep and Cap genes. The first helper plasmid has the Rep and Cap genes from AAV2 and the second helper plasmid has the Rep from AAV3 and the Cap gene from AAV7. A third plasmid encodes for the nucleotide sequence for a Factor IX (FIX) to treat Hemophilia B that is inserted between two ITRs. The haploid AAV generated from the three plasmids contains the nucleotide sequence for a protein to treat a disease of the skeletal muscle, in part by increasing the specificity for FIX associated with Hemophilia B through the use of multiple AAV serotypes to source the proteins that code for VP1, VP2 and VP3 according to the methods of the present invention. In fact, the haploid virus created by this method to treat liver tissue in a patient suffering from Hemophilia B has a higher specificity for the relevant tissue than a virus vector comprised of only AAV3 or AAV7.

In an experiment, three helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV6. A third helper plasmid has the Rep gene from AAV3 and the Cap gene from AAV7. A fourth plasmid encodes for the nucleotide sequence for a Factor IX (FIX) to treat Hemophilia B that is inserted between two ITRs. The triploid AAV generated from the four plasmids contains the nucleotide sequence for a protein to treat Hemophilia B, in part by increasing the specificity for liver tissue associated with Hemophilia B through the use of multiple AAV serotypes (*e.g.*, AAV3, AAV6 and AAV7) to source the proteins that code for VP1, VP2 and VP3 according to the methods of the present invention. In fact, the triploid virus created by this method to treat liver tissue in a patient suffering from Hemophilia B has a higher specificity for the relevant tissue than a virus vector comprised of only AAV3, AAV6 or AAV7.

In another experiment, one helper plasmid is used with different AAV serotypes as the source for the Rep and Cap genes. The helper plasmid has the Rep from AAV2 and VP1 from AAV2, VP2 from AAV6 and VP3 from AAV6. A second plasmid encodes for the nucleotide sequence for FIX to treat Hemophilia B that is inserted between two ITRs. The haploid AAV generated from the two plasmids contains the nucleotide sequence to treat Hemophilia B that, in part by increasing the specificity for liver tissues associated with Hemophilia B through the use of multiple AAV serotypes (*e.g.,* AAV2 and AAV6) to source the proteins that code for VP1, VP2 and VP3 according to the methods of the present invention. In fact, the haploid virus created by this method to treat liver tissue in a patient suffering from Hemophilia B has a higher specificity for the relevant tissue than a virus vector comprised of only AAV2 or AAV6.

In another experiment, one helper plasmid is used with different AAV serotypes as the source for the Rep and Cap genes. The helper plasmid has the Rep from AAV2 and VP1 from AAV3, VP2 from AAV7 and VP3 from AAV7. A second plasmid encodes for the nucleotide sequence for FIX to treat Hemophilia B that is inserted between two ITRs. The haploid AAV generated from the two plasmids contains the nucleotide sequence to treat Hemophilia B that, in part by increasing the specificity for liver tissues associated with Hemophilia B through the use of multiple AAV serotypes (*e.g.,* AAV3 and AAV7) to source the proteins that code for VP1, VP2 and VP3 according to the methods of the present invention. In fact, the haploid virus created by this method to treat liver tissue in a patient suffering from Hemophilia B has a higher specificity for the relevant tissue than a virus vector comprised of only AAV3 or AAV7.

In another experiment, one helper plasmid is used with different AAV serotypes as the source for the Rep and Cap genes. The helper plasmid has the Rep from AAV2 and VP1 from AAV3, VP2 from AAV6 and VP3 from AAV7. A second plasmid encodes for the nucleotide sequence for FIX to treat Hemophilia B that is inserted between two ITRs. The triploid AAV generated from the two plasmids contains the nucleotide sequence to treat Hemophilia B that, in part by increasing the specificity for liver tissues associated with Hemophilia B through the use of multiple AAV serotypes (*e.g.,* AAV3, AAV6 and AAV7) to source the proteins that code for VP1, VP2 and VP3 according to the methods of the present invention. In fact, the triploid virus created by this method to treat liver tissue in a patient suffering from Hemophilia B has a higher specificity for the relevant tissue than a virus vector comprised of only AAV3, AAV6 or AAV7.

In an experiment, two helper plasmids are again used with different AAV serotypes as the source for the Rep and Cap genes. The first helper plasmid has the Rep and Cap genes from AAV2 and the second helper plasmid has the Rep from AAV2 and the Cap gene from AAV6. A third plasmid encodes for the nucleotide sequence for a Factor VIII (FVIII) to treat Hemophilia A that is inserted between two ITRs. The haploid AAV generated from the three plasmids contains the nucleotide sequence for a protein to treat a disease of the skeletal muscle, in part by increasing the specificity for FVIII associated with Hemophilia A through the use of multiple AAV serotypes to source the proteins that code for VP1, VP2 and VP3 according to the methods of the present invention. In fact, the haploid virus created by this method to treat liver tissue in a patient suffering from Hemophilia A has a higher specificity for the relevant tissue than a virus vector comprised of only AAV2 or AAV6.

In an experiment, two helper plasmids are again used with different AAV serotypes as the source for the Rep and Cap genes. The first helper plasmid has the Rep and Cap genes from AAV2 and the second helper plasmid has the Rep from AAV3 and the Cap gene from AAV7. A third plasmid encodes for the nucleotide sequence for a FVIII to treat Hemophilia A that is inserted between two ITRs. The haploid AAV generated from the three plasmids contains the nucleotide sequence for a protein to treat a disease of the skeletal muscle, in part by increasing the specificity for FVIII associated with Hemophilia A through the use of multiple AAV serotypes to source the proteins that code for VP1, VP2 and VP3 according to the methods of the present invention. In fact, the haploid virus created by this method to treat liver tissue in a patient suffering from Hemophilia A has a higher specificity for the relevant tissue than a virus vector comprised of only AAV3 or AAV7.

In an experiment, three helper plasmids are used with different AAV serotypes as the source for the Rep and Cap genes. The first helper plasmid has the Rep and Cap genes from AAV3 and the second helper plasmid has the Rep gene from AAV3 and the Cap gene from AAV6. A third helper plasmid has the Rep gene from AAV3 and the Cap gene from AAV7. A fourth plasmid encodes for the nucleotide sequence for a FVIII to treat Hemophilia A that is inserted between two ITRs. The triploid AAV generated from the four plasmids contains the nucleotide sequence for a FVIII protein to treat Hemophilia A, in part by increasing the specificity for liver tissue associated with Hemophilia B through the use of multiple AAV serotypes (*e.g.,* AAV3, AAV6 and AAV7) to source the proteins that code for VP1, VP2 and VP3 according to the methods of the present invention. In fact, the triploid virus created by this method to treat liver tissue in a patient suffering from Hemophilia A has a higher specificity for the relevant tissue than a virus vector comprised of only AAV3, AAV6 or AAV7.

In another experiment, one helper plasmid is used with different AAV serotypes as the source for the Rep and Cap genes. The helper plasmid has the Rep from AAV2 and VP1 from AAV2, VP2 from AAV6 and VP3 from AAV6. A second plasmid encodes for the nucleotide sequence for FVIII to treat Hemophilia B that is inserted between two ITRs. The haploid AAV generated from the two plasmids contains the nucleotide sequence for FVIII to treat Hemophilia A that, in part by increasing the specificity for liver tissues associated with Hemophilia A through the use of multiple AAV serotypes (e.g. AAV2 and AAV6) to source the proteins that code for VP1, VP2 and VP3 according to the methods of the present invention. In fact, the haploid virus created by this method to treat liver tissue in a patient suffering from Hemophilia A has a higher specificity for the relevant tissue than a virus vector comprised of only AAV2 or AAV6.

In another experiment, one helper plasmid is used with different AAV serotypes as the source for the Rep and Cap genes. The helper plasmid has the Rep from AAV2 and VP1 from AAV3, VP2 from AAV7 and VP3 from AAV7. A second plasmid encodes for the nucleotide sequence for FVIII to treat Hemophilia A that is inserted between two ITRs. The haploid AAV generated from the two plasmids contains the nucleotide sequence for FVIII to treat Hemophilia A that, in part by increasing the specificity for liver tissues associated with Hemophilia B through the use of multiple AAV serotypes (*e.g.,* AAV3 and AAV7) to source the proteins that code for VP1, VP2 and VP3 according to the methods of the present invention. In fact, the haploid virus created by this method to treat liver tissue in a patient suffering from Hemophilia A has a higher specificity for the relevant tissue than a virus vector comprised of only AAV3 or AAV7.

In another experiment, one helper plasmid is used with different AAV serotypes as the source for the Rep and Cap genes. The helper plasmid has the Rep from AAV2 and VP1 from AAV3, VP2 from AAV6 and VP3 from AAV7. A second plasmid encodes for the nucleotide sequence for FVIII to treat Hemophilia A that is inserted between two ITRs. The triploid AAV generated from the two plasmids contains the nucleotide sequence for FVIII to treat Hemophilia B that, in part by increasing the specificity for liver tissues associated with Hemophilia A through the use of multiple AAV serotypes (*e.g.,* AAV3, AAV6 and AAV7) to source the proteins that code for VP1, VP2 and VP3 according to the methods of the present invention. In fact, the triploid virus created by this method to treat liver tissue in a patient suffering from Hemophilia A has a higher specificity for the relevant tissue than a virus vector comprised of only AAV3, AAV6 or AAV7.

***Example 13: Use of AAV's of the Instant Invention to Treat a Disease Treatment of Parkinson's Disease***

A male patient of 45 years of age suffering from Parkinson's disease is treated with an AAV generated from a cell line, such as the isolated HEK293 cell line with ATCC No. PTA 13274 (see e.g., U.S. Patent No. 9,441,206), which contains a first helper plasmid that has the Rep and Cap genes from AAV2 and a second helper plasmid that has the Rep gene from AAV2 and the Cap gene from AAV4 and a third plasmid that encodes for the nucleotide sequence for Glutamic Acid Decarboxylase 65 (GAD65) and/or Glutamic Acid Decarboxylase 67 (GAD67), which nucleotide sequence is inserted between two ITRs. The haploid AAV generated from the three plasmids contains the nucleotide sequence for GAD65 and/or GAD67 protein to treat Parkinson's disease. The AAV is administered to the patient, who shortly after administration shows a reduction in the frequency of tremors and an improvement in the patient's balance. Over time the patient also sees a reduction in the number and severity of hallucinations and delusions that the patient suffered from prior to administration of the AAV.

***Treatment of Batten's Disease.*** A male patient of 8 years of age suffering from Batten disease is treated with an AAV generated from a cell line, such as the isolated HEK293 cell line with ATCC No. PTA 13274 (see e.g., U.S. Patent No. 9,441,206), which contains a first helper plasmid that has the Rep and Cap genes from AAV3 and a second helper plasmid that has the Rep gene from AAV3 and the Cap gene from AAV5. A third plasmid encodes the nucleotide sequence for CLN2 to treat Batten's disease, wherein the CLN 2 gene has been inserted between two ITRs. The haploid AAV generated from the three plasmids contains the nucleotide sequence to treat Batten's disease. The AAV is administered to the patient, who shortly after administration shows an increase in mental acuity. Additionally, the patient sees a reduction in seizures and improvement in sign and motor skills that the patient suffered from prior to administration of the AAV.

***Treatment of Alzheimer's Disease.*** A female patient of 73 years suffering from Alzheimer's disease is treated with an AAV generated from a cell line, such as the isolated HEK293 cell line with ATCC No. PTA 13274 (see e.g., U.S. Patent No. 9,441,206), which contains a first helper plasmid that has the Rep and Cap genes from AAV3; a second helper plasmid that has the Rep gene from AAV3 and the Cap gene from AAV4; and, a third helper plasmid that has the Rep gene from AAV3 and the Cap gene from AAV5. A fourth plasmid encodes the nucleotide sequence for Nerve Growth Factor (NGF) to treat Alzheimer's disease, wherein NGF has been inserted between two ITRs. The triploid AAV is administered to the patient, who shortly after administration shows an increase in mental acuity and short-term memory. The patient also is able to better communicate with others and begins to function more independently than prior to administration of the AAV.

***Treatment of Heart Disease.*** A male patient of 63 years suffering from heart disease is treated with an AAV generated from a cell line, such as the isolated HEK293 cell line with ATCC No. PTA 13274 (*see, e.g.,* U.S. Patent No. 9,441,206), which contains either:

(1) a first helper plasmid that has the Rep and Cap genes from AAV2; a second helper plasmid that has the Rep gene from AAV2 and the Cap gene from AAV6; and, a third plasmid encodes the nucleotide sequence for the protein to treat heart disease that is contained in a third plasmid and has been inserted between two ITRs;

(2) a first helper plasmid that has the Rep and Cap genes from AAV3; a second helper plasmid that has the Rep gene from AAV3 and the Cap gene from AAV9; and, a third plasmid encodes the nucleotide sequence for the protein to treat heart disease that is contained in a third plasmid and has been inserted between two ITRs;

(3) a first helper plasmid that has the Rep and Cap genes from AAV3; a second helper plasmid that has the Rep gene from AAV3 and the Cap gene from AAV6; a third helper plasmid that has the Rep gene from AAV3 and the Cap gene from AAV9; and, a fourth plasmid contains a nucleotide sequence that encodes a protein to treat heart disease is contained in a third plasmid and has been inserted between two ITRs;

(4) a helper plasmid that has the Rep from AAV2 and VP1 from AAV2, VP2 from AAV3 and VP3 from AAV9; and, a second plasmid that contains a nucleotide sequence encoding a protein to treat heart disease inserted between two ITRs;

(5) a helper plasmid that has the Rep from AAV3 and VP1 from AAV3, VP2 from AAV6 and VP3 from AAV6; and, a second plasmid contains a nucleotide sequence encoding a protein to treat heart disease inserted between two ITRs; or,

(6) a helper plasmid that has the Rep from AAV3 and VP1 from AAV3, VP2 from AAV6 and VP3 from AAV9; and, a second plasmid contains a nucleotide sequence encoding a protein to treat heart disease inserted between two ITRs, wherein the polyploid AAV is administered to the patient, who shortly after administration shows a reduction in the symptoms associated with heart disease and shows a commensurate improvement in the patient's heart health.

***Treatment of Cystic Fibrosis.*** A 19 year old female suffering from Cystic Fibrosis is treated with an AAV generated from a cell line, such as the isolated HEK293 cell line with ATCC No. PTA 13274 (see e.g., U.S. Patent No. 9,441,206), which contains either:

(1) a first helper plasmid that has the Rep and Cap genes from AAV3; a second helper plasmid that has the Rep from AAV3 and the Cap gene from AAV10; and, a third plasmid that encodes for the nucleotide sequence for CFTR that is inserted between two ITRs;

(2) a first helper plasmid that has the Rep and Cap genes from AAV3; a second helper plasmid that has the Rep gene from AAV3 and the Cap gene from AAV9; a third helper plasmid that has the Rep gene from AAV3 and the Cap gene from AAV10; and a fourth plasmid that encodes a nucleotide sequence for CFTR that has been inserted between two ITRs;

(3) a helper plasmid that has the Rep from AAV2 and VP1 from AAV2, VP2 from AAV9 and VP3 from AAV9; and a second plasmid that encodes the nucleotide sequence for CFTR inserted between two ITRs;

(4) a helper plasmid that has the Rep from AAV3 and VP1 from AAV2, VP2 from AAV10 and VP3 from AAV10; and, a second plasmid that encodes the nucleotide sequence for CFTR inserted between two ITRs; or,

(7) a helper plasmid that has the Rep from AAV2 and VP1 from AAV2, VP2 from AAV9 and VP3 from AAV10; and, a second plasmid encodes the nucleotide sequence for CFTR inserted between two ITRs, wherein

the AAV is administered to the patient, who shortly after administration shows a slowing in the increase of damage to the patient's lung; a reduction in the increase in the loss of lung function and a reduction in the speed by which the liver is damaged and a slowdown in the increase in the severity of liver cirrhosis. The same patient also sees a reduction in the severity of the Cystic Fibrosis-related diabetes that the patient had begun to suffer.

***Treatment of Skeletal Muscle Disease - Amyotrophic Lateral Sclerosis (ALS).*** A male of 33 years of age who is suffering from Amyotrophic Lateral Sclerosis (ALS) is treated with an AAV generated from a cell line, such as the isolated HEK293 cell line with ATCC No. PTA 13274 (see e.g., U.S. Patent No. 9,441,206), which contains either:

(1) a first helper plasmid that has the Rep and Cap genes from AAV2; a second helper plasmid that has the Rep from AAV2 and the Cap gene from AAV8; and, a third plasmid that encodes for the nucleotide sequence for superoxide dismutase 1 (SOD1) that is inserted between two ITRs;

(2) a first helper plasmid that has the Rep and Cap genes from AAV3; a second helper plasmid that has the Rep from AAV3 and the Cap gene from AAV9; and, a third plasmid that encodes for the nucleotide sequence for SOD1 that is inserted between two TTRs;

(3) a first helper plasmid that has the Rep and Cap genes from AAV3; a second helper plasmid that has the Rep gene from AAV3 and the Cap gene from AAV8; a third helper plasmid that has the Rep gene from AAV3 and the Cap gene from AAV9; and, a fourth plasmid that encodes for the nucleotide sequence for SOD1 that is inserted between two ITRs;

(4) a helper plasmid that has the Rep from AAV3 and VP1 from AAV3, VP2 from AAV9 and VP3 from AAV9; and, a second plasmid that encodes for the nucleotide sequence for SOD1 that is inserted between two ITRs;

(5) a helper plasmid that has the Rep from AAV3 and VP1 from AAV3, VP2 from AAV8 and VP3 from AAV8; and, a second plasmid encodes for the nucleotide sequence for SOD1 that is inserted between two ITRs; or,

(6) a helper plasmid that has the Rep from AAV3 and VP1 from AAV3, VP2 from AAV8 and VP3 from AAV; and, a second plasmid encodes for the nucleotide sequence for SOD1 that is inserted between two ITRs, wherein

the AAV is administered to the patient, who shortly after administration shows a reduction in the symptoms associated with ALS, including a slow down or stop in the progression of damage to motor neurons in the brain and the spinal cord and the maintenance of communication between the brain and the muscles of the patient.

***Duchenne Muscular Dystrophy.*** A male of 5 years of age who is suffering from Duchenne Muscular Dystrophy (DMD) is treated with an AAV generated from a cell line, such as the isolated HEK293 cell line with ATCC No. PTA 13274, which contains either:

(1) a first helper plasmid that has the Rep and Cap genes from AAV2; a second helper plasmid that has the Rep from AAV2 and the Cap gene from AAV8; and, a third plasmid that encodes for the nucleotide sequence for dystrophin that is inserted between two ITRs;

(2) a first helper plasmid that has the Rep and Cap genes from AAV3; a second helper plasmid that has the Rep from AAV3 and the Cap gene from AAV9; and, a third plasmid that encodes for the nucleotide sequence for dystrophin that is inserted between two ITRs;

(3) a first helper plasmid that has the Rep and Cap genes from AAV3; a second helper plasmid that has the Rep gene from AAV3 and the Cap gene from AAV8; a third helper plasmid that has the Rep gene from AAV3 and the Cap gene from AAV9; and, a fourth plasmid that encodes for the nucleotide sequence for dystrophin that is inserted between two ITRs;

(4) a helper plasmid that has the Rep from AAV3 and VP1 from AAV3, VP2 from AAV9 and VP3 from AAV9; and, a second plasmid that encodes for the nucleotide sequence for dystrophin that is inserted between two ITRs;

(5) a helper plasmid that has the Rep from AAV3 and VP1 from AAV3, VP2 from AAV8 and VP3 from AAV8; and, a second plasmid encodes for the nucleotide sequence for dystrophin that is inserted between two ITRs; or,

(6) a helper plasmid that has the Rep from AAV3 and VP1 from AAV3, VP2 from AAV8 and VP3 from AAV; and, a second plasmid encodes for the nucleotide sequence for dystrophin that is inserted between two ITRs, wherein

the AAV is administered to the patient, who shortly after administration shows a slowing in the increase of damage and wasting to the patient's skeletal muscles, as well a slowing or stoppage to the damage suffered by heart and lung as a result of Duchene Muscular Dystrophy.

***Myasthenia Gravis.*** A female of 33 years of age who is suffering from Myasthenia Gravis (MG) is treated with an AAV generated from a cell line, such as the isolated HEK293 cell line with ATCC No. PTA 13274, which contains either:

(1) a first helper plasmid that has the Rep and Cap genes from AAV2; a second helper plasmid that has the Rep from AAV2 and the Cap gene from AAV8; and, a third plasmid that encodes the nucleotide sequence for the gene such that the patient will no longer suffer from MG that is inserted between two ITRs;

(2) a first helper plasmid that has the Rep and Cap genes from AAV3; a second helper plasmid that has the Rep from AAV3 and the Cap gene from AAV9; and, a third plasmid that encodes for the gene such that the patient will no longer suffer from MG that is inserted between two ITRs;

(3) a first helper plasmid that has the Rep and Cap genes from AAV3; a second helper plasmid that has the Rep gene from AAV3 and the Cap gene from AAV8; a third helper plasmid that has the Rep gene from AAV3 and the Cap gene from AAV9; and, a fourth plasmid that encodes for the gene such that the patient will no longer suffer from MG that is inserted between two ITRs;

(4) a helper plasmid that has the Rep from AAV3 and VP1 from AAV3, VP2 from AAV9 and VP3 from AAV9; and, a second plasmid that encodes for the gene such that the patient will no longer suffer from MG that is inserted between two ITRs;

(5) a helper plasmid that has the Rep from AAV3 and VP1 from AAV3, VP2 from AAV8 and VP3 from AAV8; and, a second plasmid encodes for the gene such that the patient will no longer suffer from MG that is inserted between two ITRs; or,

(6) a helper plasmid that has the Rep from AAV3 and VP1 from AAV3, VP2 from AAV8 and VP3 from AAV; and, a second plasmid encodes for the gene such that the patient will no longer suffer from MG that is inserted between two ITRs, wherein

the AAV is administered to the patient, who shortly after administration shows a slowing in the increase breakdown in the communication between muscles and the nerves of the patient's body, resulting in a slow down or stoppage in the severity in the loss of muscle control. The patient's mobility stabilizes and no longer worsens after administration of the AAV and the patient's breathing also does not worsen after administration of the AAV.

***Limb Girdle Muscular Dystrophy.*** A male of 13 years of age who is suffering from Limb Girdle Muscular Dystrophy (LGMD) is treated with an AAV generated from a cell line, such as the isolated HEK293 cell line with ATCC No. PTA 13274, which contains either:

(1) a first helper plasmid that has the Rep and Cap genes from AAV2; a second helper plasmid that has the Rep from AAV2 and the Cap gene from AAV8; and, a third plasmid that encodes for the nucleotide sequence for one of the fifteen genes with a mutation associated with LGMD, including, but not limited to myotilin, telethonin, calpain-3, alpha-sarcoglycan and beta-sarcoglycan that is inserted between two ITRs;

(2) a first helper plasmid that has the Rep and Cap genes from AAV3; a second helper plasmid that has the Rep from AAV3 and the Cap gene from AAV9; and, a third plasmid that encodes for the nucleotide sequence for one of the fifteen genes with a mutation associated with LGMD, including, but not limited to myotilin, telethonin, calpain-3, alpha-sarcoglycan and beta-sarcoglycan that is inserted between two ITRs;

(3) a first helper plasmid that has the Rep and Cap genes from AAV3; a second helper plasmid that has the Rep gene from AAV3 and the Cap gene from AAV8; a third helper plasmid that has the Rep gene from AAV3 and the Cap gene from AAV9; and, a fourth plasmid that encodes for the nucleotide sequence for one of the fifteen genes with a mutation associated with LGMD, including, but not limited to myotilin, telethonin, calpain-3, alpha-sarcoglycan and beta-sarcoglycan that is inserted between two ITRs;

(4) a helper plasmid that has the Rep from AAV3 and VP1 from AAV3, VP2 from AAV9 and VP3 from AAV9; and, a second plasmid that encodes for the nucleotide sequence for one of the fifteen genes with a mutation associated with LGMD, including, but not limited to myotilin, telethonin, calpain-3, alpha-sarcoglycan and beta-sarcoglycan that is inserted between two ITRs;

(5) a helper plasmid that has the Rep from AAV3 and VP1 from AAV3, VP2 from AAV8 and VP3 from AAV8; and, a second plasmid encodes for the nucleotide sequence for one of the fifteen genes with a mutation associated with LGMD, including, but not limited to myotilin, telethonin, calpain-3, alpha-sarcoglycan and beta-sarcoglycan that is inserted between two ITRs; or,

(6) a helper plasmid that has the Rep from AAV3 and VP1 from AAV3, VP2 from AAV8 and VP3 from AAV; and, a second plasmid encodes for the nucleotide sequence for one of the fifteen genes with a mutation associated with LGMD, including, but not limited to myotilin, telethonin, calpain-3, alpha-sarcoglycan and beta-sarcoglycan that is inserted between two ITRs, wherein

one or more of the AAV's, each encoding one of the 15 different genes associated with LGMD is administered to the patient, who shortly after administration shows a slowing or stoppage in additional muscle wasting and atrophy.

***Treatment of Diseases of the Liver - Hemophilia B.*** A male of 9 years of age who is suffering from a Hemophilia B resulting from a deficiency of Factor IX (FIX) is treated with an AAV generated from a cell line, such as the isolated HEK293 cell line with ATCC No. PTA 13274, which contains either:

(1) a first helper plasmid that has the Rep and Cap genes from AAV2; a second helper plasmid that has the Rep from AAV2 and the Cap gene from AAV6; and, a third plasmid that encodes for the nucleotide sequence for FIX to treat Hemophilia B that is inserted between two ITRs;

(2) a first helper plasmid that has the Rep and Cap genes from AAV2; a second helper plasmid that has the Rep from AAV3 and the Cap gene from AAV7; and a third plasmid that encodes for the nucleotide sequence for FIX to treat Hemophilia B that is inserted between two ITRs;

(3) a first helper plasmid that has the Rep and Cap genes from AAV3; a second helper plasmid that has the Rep gene from AAV3 and the Cap gene from AAV6; a third helper plasmid that has the Rep gene from AAV3 and the Cap gene from AAV7; and a fourth plasmid that encodes for the nucleotide sequence for FIX that is inserted between two ITRs;

(4) a helper plasmid that has the Rep from AAV2 and VP1 from AAV2, VP2 from AAV6 and VP3 from AAV6; and a second plasmid that encodes for the nucleotide sequence for FIX that is inserted between two ITRs;

(5) a helper plasmid that has the Rep from AAV2 and VP1 from AAV3, VP2 from AAV7 and VP3 from AAV7; and a second plasmid that encodes for the nucleotide sequence for FIX that is inserted between two ITRs; or,

(6) a helper plasmid that has the Rep from AAV2 and VP1 from AAV3, VP2 from AAV6 and VP3 from AAV7' and a second plasmid encodes for the nucleotide sequence for FIX that is inserted between two ITRs, wherein

the AAV is administered to the patient, who shortly after administration shows a reduction in the severity of the Hemophilia B, including a reduction in bleeding episodes.

***Hemophilia A.*** A male of 8 years of age who is suffering from a Hemophilia A resulting from a deficiency of Factor VIII (FVIII) is treated with an AAV generated from a cell line, such as the isolated HEK293 cell line with ATCC No. PTA 13274, which contains either:

(1) a first helper plasmid that has the Rep and Cap genes from AAV2; a second helper plasmid that has the Rep from AAV2 and the Cap gene from AAV6; and, a third plasmid that encodes for the nucleotide sequence for FVIII that is inserted between two ITRs;

(2) a first helper plasmid that has the Rep and Cap genes from AAV2; a second helper plasmid that has the Rep from AAV3 and the Cap gene from AAV7; and a third plasmid that encodes for the nucleotide sequence for FVIII that is inserted between two ITRs;

(3) a first helper plasmid that has the Rep and Cap genes from AAV3; a second helper plasmid that has the Rep gene from AAV3 and the Cap gene from AAV6; a third helper plasmid that has the Rep gene from AAV3 and the Cap gene from AAV7; and a fourth plasmid that encodes for the nucleotide sequence for FVIII that is inserted between two ITRs;

(4) a helper plasmid that has the Rep from AAV2 and VP1 from AAV2, VP2 from AAV6 and VP3 from AAV6; and a second plasmid that encodes for the nucleotide sequence for FVIII that is inserted between two ITRs;

(5) a helper plasmid that has the Rep from AAV2 and VP1 from AAV3, VP2 from AAV7 and VP3 from AAV7; and a second plasmid that encodes for the nucleotide sequence for FVIII that is inserted between two ITRs; or,

(6) a helper plasmid that has the Rep from AAV2 and VP1 from AAV3, VP2 from AAV6 and VP3 from AAV7' and a second plasmid encodes for the nucleotide sequence for FVIII that is inserted between two TTRs, wherein

the AAV is administered to the patient, who shortly after administration shows a reduction in the severity of the Hemophilia A, including a reduction in bleeding episodes.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein without departing from the spirit of the present specification. Lastly, the terminology used herein is for the purpose of describing particular embodiments only, and is not intended to limit the scope of the present invention, which is defined solely by the claims. Accordingly, the present invention is not limited to that precisely as shown and described.

Certain embodiments of the present invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the present invention to be practiced otherwise than specifically described herein. Accordingly, this invention includes all modifications and equivalents of the subject matter recited in the claims appended hereto as permitted by applicable law. Moreover, any combination of the above-described embodiments in all possible variations thereof is encompassed by the invention unless otherwise indicated herein or otherwise clearly contradicted by context.

Groupings of alternative embodiments, elements, or steps of the present invention are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein.

All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the present invention so claimed are inherently or expressly described and enabled herein.

All patents, patent publications, and other publications referenced and identified in the present specification are individually and expressly incorporated herein by reference in their entirety for the purpose of describing and disclosing, for example, the compositions and methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

**Table 1:**

| | GenBank Accession Number | | | GenBank Accession Number | | | GenBank Accession Number |
|---|---|---|---|---|---|---|---|
| Complete Genomes | | | HuS17 | AY695376 | | Hu66 | AY530626 |
| Adeno-associated virus 1 | NC_002077, AF063497 | | HuT88 | AY695375 | | Hu42 | AY530605 |
| Adeno-associated virus 2 | NC_001401 | | HuT71 | AY695374 | | Hu67 | AY530627 |
| Adeno-associated virus 3 | NC_001729 | | HuT70 | AY695373 | | Hu40 | AY530603 |
| Adeno-associated virus 3B | NC_001863 | | HuT40 | AY695372 | | Hu41 | AY530604 |
| Adeno-associated virus 4 | NC_OO1829 | | Hu T32 | AY695371 | | Hu37 | AY530600 |
| Adeno-associated virus 5 | Y18065, AF085716 | | Hu T17 | AY695370 | | Rh40 | AY530559 |
| Adeno-associated virus 6 | NC_001862 | | Hu LG15 | AY695377 | | Rh2 | AY243007 |
| Avian AAVA TCC VR-865 | AY186198, AY629583 , NC 004828 | | Clade C | | | Bb1 | AY243023 |
| Avian AAV strain DA-I | NC_006263, AY629583 | | Hu9 | AY530629 | | Bb2 | AY243022 |
| Bovine AAV | NC_005889, AY388617, AAR26465 | | Hu JO | AY530576 | | | |
| AAVIJ | AAT46339, AY631966 | | Hull | AY530577 | | RhlO | AY243015 |
| AAV12 | AB116639, DQ813647 | | | | | Hui? | AY530582 |
| Clade A | | | Hu53 | AY530615 | | Hu6 | AY530621 |
| AAVI | NC_002077, AF063497 | | Hu55 | AY530617 | | Rh25 | AY530557 |
| AAV6 | NC_001862 | | Hu54 | AY530616 | | Pi2 | AY530554 |
| Hu.48 | AY530611 | | Hu7 | AY530628 | | Pi1 | AY530553 |
| Hu43 | AY530606 | | Hu18 | AY530583 | | Pi3 | AY530555 |
| Hu 44 | AY530607 | | Hu IS | AY530580 | | Rh57 | AY530569 |
| Hu 46 | AY530609 | | Hu16 | AY530581 | | Rh50 | AY530563 |
| Clade B | | | Hu25 | AY530591 | | RM9 | AY530562 |
| Hu19 | AY530584 | | Hu60 | AY530622 | | Hu39 | AY530601 |

(continued)

| | GenBank Accession Number | | | GenBank Accession Number | | | GenBank Accession Number |
|---|---|---|---|---|---|---|---|
| Hu20 | AY530586 | | Ch5 | AY243021 | | Rh58 | AY530570 |
| Hu23 | AY530589 | | Hu3 | AY530595 | | Rh61 | AY530572 |
| Hu22 | AY530588 | | Hui | AY530575 | | Rh52 | AY530565 |
| Hu24 | AY530590 | | Hu4 | AY530602 | | Rh53 | AY530566 |
| Hu21 | AY530587 | | Hu2 | AY530585 | | RhSI | AY530564 |
| Hu27 | AY530592 | | Hu61 | AY530623 | | Rh64 | AY530574 |
| Hu28 | AY530593 | | Clade D | | | Rh43 | AY530560 |
| Hu 29 | AY530594 | | Rh62 | AY530573 | | AAV8 | AF513852 |
| Hu63 | AY530624 | | RMB | AY530561 | | Rh8 | AY242997 |
| Hu64 | AY530625 | | Rh54 | AY5 30567 | | Rh1 | AY530556 |
| Hul3 | AY530578 | | Rh55 | AY530568 | | Clade F | |
| Hu56 | AY530618 | | Cy2 | AY243020 | | Hul4 (AAV9) | AY530579 |
| Hu57 | AY530619 | | AAV7 | AF5J3851 | | Hu31 | AY530596 |
| I-lu49 | AY530612 | | Rh35 | AY243000 | | Hu32 | AY530597 |
| Hu58 | AY530620 | | Rh37 | AY242998 | | Clonal Isolate | |
| Hu34 | AY530598 | | Rh36 | AY242999 | | AAVS | Y18065 , AF085716 |
| Hu35 | AY530599 | | Cy6 | AY243016 | | AAV3 | NC_001729 |
| AAV2 | NC 001401 | | Cy4 | AY243018 | | AAV3B | NC_001863 |
| Hu45 | AY530608 | | Cy3 | AY243019 | | AAV4 | NC_001829 |
| Hu47 | AY5306JO | | Cy5 | AY243017 | | Rh34 | AY243001 |
| Hu51 | AY530613 | | RJ1(3 | AY243013 | | Rh33 | AY243002 |
| Hu52 | AY530614 | | Clade E | | | Rh32 | AY243003 |
| HuT41 | AY695378 | | Rh38 | AY530558 | | | |

**Table 2: Amino acid residues and abbreviations**

| Amino Acid Residue | Abbreviation | |
|---|---|---|
| | Three-Letter Code | One-Letter Code |
| Alanine | Ala | A |
| Arginine | Arg | R |
| Asparagine | Asn | N |
| Aspartic acid (Aspartate) | Asp | D |
| Cysteine | Cys | C |
| Glutamine | Gln | Q |
| Glutamic acid (Glutamate) | Glu | E |
| Glycine | Gly | G |
| Histidine | His | H |
| Isoleucine | Ile | I |

(continued)

| Amino Acid Residue | Abbreviation | |
|---|---|---|
| | Three-Letter Code | One-Letter Code |
| Leucine | Leu | L |
| Lysine | Lys | K |
| Methionine | Met | M |
| Phenylalanine | Phe | F |
| Praline | Pro | P |
| Serine | Ser | S |
| Threonine | Thr | T |
| Tryptophan | Trp | W |
| Tyrosine | Tyr | Y |
| Valine | Val | V |

**Table 3:**

| Serotype | Position 1 | Position 2 |
|---|---|---|
| AAV1 | A263X | T265X |
| AAV2 | Q263X | -265X |
| AAV3a | Q263X | -265X |
| AAV3b | Q263X | -265X |
| AAV4 | S257X | -259X |
| AAV5 | G253X | V255X |
| AAV6 | A263X | T265X |
| AAV7 | E264X | A266X |
| AAV8 | G264X | S266X |
| AAV9 | S263X | S265X |
| Where, (X)→ mutation to any amino acid<br>(-) → insertion of any amino acid<br>Note: Position 2 inserts are indicated by the site of insertion | | |

**Table 4:**

| Modified Amino Acid Residue<br>Amino Acid Residue Derivatives | Abbreviation |
|---|---|
| 2-Aminoadipic acid | Aad |
| 3-Aminoadipic acid | bAad |
| beta-Alanine, beta-Aminoproprionic acid | bAla |
| 2-Aminobutyric acid | Abu |
| 4-Aminobutyric acid, Piperidinic acid | 4Abu |
| 6-Aminocaproic acid | Acp |
| 2-Aminoheptanoic acid | Ahe |
| 2-Aminoisobutyric acid | Aib |
| 3-Aminoisobutyric acid | bAib |

(continued)

| Modified Amino Acid Residue Amino Acid Residue Derivatives | Abbreviation |
|---|---|
| 2-Aminopimelic acid | Apm |
| t-butylalanine | t-BuA |
| Citrulline | Cit |
| Cyclohexylalanine | Cha |
| 2,4-Diaminobutyric acid | Dbu |
| Desmosine | Des |
| 2,2'-Diaminopimelic acid | Dpm |
| 2,3-Diaminoproprionic acid | Dpr |
| N-Ethylglycine | EtGly |
| N-Ethylasparagine | EtAsn |
| Homoarginine | hArg |
| Homocysteine | hCys |
| Homoserine | hSer |
| Hydroxylysine | Hyl |
| Allo-Hydroxylysine | aHyl |
| 3-Hydroxyproline | 3Hyp |
| 4-Hydroxyproline | 4Hyp |
| Isodesmosine | Ide |
| allo-Isoleucine | alle |
| Methionine sulfoxide | MSO |
| N-Methylglycine, sarcosine | MeGly |
| N-Methylisoleucine | Melle |
| 6-N-Methyllysine | MeLys |
| N-Methylvaline | MeVal |
| 2-Naphthylalanine | 2-Nal |
| Norvaline | Nva |
| Norleucine | Nle |
| Ornithine | Orn |
| 4-Chlorophenylalanine | Phe(4-Cl) |
| 2-Fluorophenylalanine | Phe(2-F) |
| 3-Fluorophenylalanine | Phe(3-F) |
| 4-Fluorophenylalanine | Phe(4-F) |
| Phenylglycine | Phg |
| Beta-2-thienylalanine | Thi |

**Table 5: MS result for AAV8 binding serum proteins**

| Accession | Description | fold change |
|---|---|---|
| P02671 | Fibrinogen alpha chain OS=Homo sapiens GN=FGA PE=1 SV=2 | 1.75 |
| P02675 | Fibrinogen beta chain OS=Homo sapiens GN=FGB PE=1 SV=2 | 1.42857143 |

(continued)

| Accession | Description | fold change |
|---|---|---|
| P02679 | Fibrinogen gamma chain OS=Homo sapiens GN=FGG PE=1 SV=3 | 1.6 |
| C9JU00 | Fibrinogen gamma chain (Fragment) OS=Homo sapiens GN=FGG PE=2 SV=1 | 0.4173913 |
| P02768 | Serum albumin OS=Homo sapiens GN=ALB PE=1 SV=2 | 3.73913043 |
| P02751 | Fibronectin OS=Homo sapiens GN=FN1 PE=1 SV=4 | 2.5 |
| P01857 | Ig gamma-1 chain C region OS=Homo sapiens GN=IGHG1 PE=1 SV=1 | 3.38461538 |
| P01871-2 | Isoform 2 of Ig mu chain C region OS=Homo sapiens GN=IGHM | 3 |
| P01834 | Ig kappa chain C region OS=Homo sapiens GN=IGKC PE=1 SV=1 | 2.76923077 |
| P01876 | Ig alpha-1 chain C region OS=Homo sapiens GN=IGHA1 PE=1 SV=2 | 2.24719101 |
| P01009 | Alpha-1-antitrypsin OS=Homo sapiens GN=SERPINA1 PE=1 SV=3 | 3.05555556 |
| P0CG05 | Ig lambda-2 chain C regions OS=Homo sapiens GN=IGLC2 PE=1 SV=1 | 1.5 |
| P10909-2 | Isoform 2 of Clusterin OS=Homo sapiens GN=CLU | 2.85714286 |
| P01860 | Ig gamma-3 chain C region OS=Homo sapiens GN=IGHG3 PE=1SV=2 | 6.33333333 |
| P01877 | Ig alpha-2 chain C region OS=Homo sapiens GN=IGHA2 PE=1 SV=3 | ND |
| P01861 | Ig gamma-4 chain C region OS=Homo sapiens GN=IGHG4 PE=1 SV=1 | ND |
| P02647 | Apolipoprotein A-I OS=Homo sapiens GN=APOA1 PE=1 SV=1 | 3.73684211 |
| P01859 | Ig gamma-2 chain C region OS=Homo sapiens GN=IGHG2 PE=1SV=2 | 5.06024096 |
| B9A064 | Immunoglobulin lambda-like polypeptide 5 OS=Homo sapiens GN=IGLL5 PE=2 SV=2 | 3.3 |
| P00739-2 | Isoform 2 of Haptoglobin-related protein OS=Homo sapiens GN=HPR | 2.81690141 |
| P02766 | Transthyretin OS=Homo sapiens GN=TTR PE=1 SV=1 | 5.54545455 |
| P01024 | Complement C3 OS=Homo sapiens GN=C3 PE=1 SV=2 | 5.3125 |
| P00747 | Plasminogen OS=Homo sapiens GN=PLG PE=1 SV=2 | 1.47692308 |
| C9JV77 | Alpha-2-HS-glycoprotein OS=Homo sapiens GN=AHSG PE=2 SV=1 | 3.16666667 |
| H0Y300 | Haptoglobin OS=Homo sapiens GN=HP PE=2 SV=4 | ND |
| P04275 | von Willebrand factor OS-Homo sapiens GN-VWF PE=1 SV-4 | ND |
| P01620 | Ig kappa chain V-III region SIE OS=Homo sapiens PE=1 SV=1 | 2.78571429 |
| P0C0L4 | Complement C4-A OS=Homo sapiens GN=C4A PE=1 SV=2 | 2.42424242 |
| P06310 | Ig kappa chain V-II region RPMI 6410 OS=Homo sapiens PE=4 SV=1 | 2.64705882 |
| O43866 | CD5 antigen-like OS=Homo sapiens GN=CD5L PE=1 SV=1 | 1.2345679 |
| P01766 | Ig heavy chain V-III region BRO OS=Homo sapiens PE=1 SV=1 | 2.46875 |
| P01598 | Ig kappa chain V-I region EU OS=Homo sapiens PE=1 SV=1 | 3.829787234 |
| P02787 | Serotransferrin OS=Homo sapiens GN=TF PE=1 SV=3 | ND |
| P04003 | C4b-binding protein alpha chain OS=Homo sapiens GN=C4BPA PE=1 SV=2 | 1.527777778 |
| P01591 | Immunoglobulin J chain OS=Homo sapiens GN=IGJ PE=1 SV=4 | 2.716049383 |
| P01619 | Ig kappa chain V-III region B6 OS=Homo sapiens PE=1 SV=1 | 2.611111111 |
| P02747 | Complement C1q subcomponent subunit C OS=Homo sapiens GN=C1QC PE=1 SV=3 | 2.529411765 |
| P02790 | Hemopexin OS=Homo sapiens GN=HPX PE=1 SV=2 | 30.71428571 |
| P05452 | Tetranectin OS=Homo sapiens GN=CLEC3B PE=1 SV=3 | ND |
| P80748 | Ig lambda chain V-III region LOI OS=Homo sapiens PE=1 SV=1 | ND |
| P01717 | Ig lambda chain V-IV region HII OS=Homo sapiens PE=1 SV=1 | 2.142857143 |
| P49908 | Selenoprotein P OS=Homo sapiens GN=SEPP1 PE=1SV=3 | ND |
| P01714 | Ig lambda chain V-III region SH OS=Homo sapiens PE=1 SV=1 | ND |
| P01625 | Ig kappa chain V-IV region Len OS=Homo sapiens PE=1 SV=2 | 5 |
| Q9BSK4 | Protein fem-1 homolog A OS=Homo sapiens GN=FEM1A PE=1 SV=1 | 0.264285714 |
| K7ER74 | Protein APOC4-APOC2 OS=Homo sapiens GN=APOC4-APOC2 PE=2 SV=1 | ND |
| P02743 | Serum amyloid P-component OS=Homo sapiens GN-APCS PE=1 SV=2 | ND |
| P08603 | Complement factor H OS=Homo sapiens GN=CFH PE=1 SV=4 | ND |
| P02746 | Complement C1q subcomponent subunit B OS=Homo sapiens GN=C1QB PE=1 SV=3 | ND |
| B4E1Z4 | Complement factor B OS=Homo sapiens GN=CFB PE=2 SV=1 | ND |
| P01597 | Ig kappa chain V-I region DEE OS=Homo sapiens PE=1 SV=1 | 2.989690722 |
| V9GYM3 | Apolipoproteln A-II OS=Homo sapiens GN=APOA2 PE=4 SV=1 | ND |

(continued)

| Accession | Description | fold change |
|---|---|---|
| P01774 | Ig heavy chain V-III region POM OS=Homo sapiens PE=1 SV=1 | 2.636363636 |
| P01019 | Angiotensinogen OS=Homo sapiens GN=AGT PE=1 SV=1 | ND |
| S4R460 | Uncharacterized protein OS=Homo sapiens PE=4 SV=1 | ND |
| P19652 | Alpha-1-acid glycoprotein 2 OS=Homo sapiens GN=ORM2 PE=1 SV=2 | ND |
| P22352 | Glutathione peroxidase 3 OS=Homo sapiens GN=GPX3 PE=1 SV=2 | 2.741935484 |
| J3KPZ1 | Grainyhead-like protein 1 homolog OS=Homo sapiens GN=GRHL1 PE=2 SV=1 | ND |
| P02749 | Beta-2-glycoprotein 1 OS=Homo sapiens GN=APOH PE=1 SV=3 | 3.428571429 |
| P01008 | Antithrombin-III OS=Homo sapiens GN=SERPINC1 PE=1 SV=1 | ND |
| Accession | Description | fold change |
| P10720 | Platelet factor 4 variant OS=Homo sapiens GN=PF4V1 PE=1 SV=1 | 0.9375 |
| Q8IWC1 | MAP7 domain-containing protein 3 OS=Homo sapiens GN=MAP7D3 PE=1 SV=2 | ND |
| P02774-3 | Isoform 3 of Vitamin D-binding protein OS=Homo sapiens GN=GC | ND |
| P02649 | Apolipoprotein E OS=Homo sapiens GN=APOE PE=1 SV=1 | 1.909091 |
| P01042 | Kininogen-1 OS=Homo sapiens GN=KNG1 PE=1 SV=2 | 0.418919 |
| P01621 | Ig kappa chain V-III region NG9 (Fragment) OS=Homo sapiens PE=1 SV=1 | ND |
| P01023 | Alpha-2-macroglobulin OS=Homo sapiens GN=A2M PE=1 SV=3 | ND |
| P04114 | Apolipoprotein B-100 OS=Homo sapiens GN=APOB PE=1 SV=2 | ND |
| P02654 | Apolipoprotein C-I OS=Homo sapiens GN=APOC1 PE=1 SV=1 | 2 |
| Q92945 | Far upstream element-binding protein 2 OS=Homo sapiens GN=KHSRP PE=1 SV=4 | ND |

*ND indicates no detection of protein in control group

**Table 6: MS result for AAV9 binding serum proteins**

| Accession | Description | Fold Change |
|---|---|---|
| P02671 | Fibrinogen alpha chain OS=Homo sapiens GN=FGA PE=1 SV=2 | 1.75 |
| P02675 | Fibrinogen beta chain OS=Homo sapiens GN=FGB PE=1 SV=2 | 1.42857143 |
| P02679 | Fibrinogen gamma chain OS=Homo sapiens GN=FGG PE=1 SV=3 | 1.6 |
| C9JU00 | Fibrinogen gamma chain (Fragment) OS=Homo sapiens GN=FGG PE=2 SV=1 | 0.4173913 |
| P02768 | Serum albumin OS=Homo sapiens GN=ALB PE=1 SV=2 | 3.73913043 |
| P02751 | Fibronectin OS=Homo sapiens GN=FN1 PE=1 SV=4 | 2.5 |
| P01857 | Ig gamma-1 chain C region OS=Homo sapiens GN=IGHG1 PE=1 SV=1 | 3.38461538 |
| P01871-2 | Isoform 2 of Ig mu chain C region OS=Homo sapiens GN=IGHM | 3 |
| P01834 | Ig kappa chain C region OS=Homo sapiens GN=IGKC PE=1 SV=1 | 2.76923077 |
| P01876 | Ig alpha-1 chain C region OS=Homo sapiens GN=IGHA1 PE=1 SV=2 | 2.24719101 |
| P01009 | Alpha-1-antitrypsin OS=Homo sapiens GN=SERPINA1 PE=1 SV=3 | 3.05555556 |
| P0CG05 | Ig lambda-2 chain C regions OS=Homo sapiens GN=IGLC2 PE=1 SV=1 | 1.5 |
| P10909-2 | Isoform 2 of Clusterin OS=Homo sapiens GN=CLU | 2.85714286 |
| P01860 | Ig gamma-3 chain C region OS=Homo sapiens GN=IGHG3 PE=1 SV=2 | 6.33333333 |
| P01877 | Ig alpha-2 chain C region OS=Homo sapiens GN=IGHA2 PE=1 SV=3 | ND* |
| P01861 | Ig gamma-4 chain C region OS=Homo sapiens GN=IGHG4 PE=1 SV=1 | ND |
| P02647 | Apolipoprotein A-I OS=Homo sapiens GN=APOA1 PE=1 SV=1 | 3.73684211 |
| P01859 | Ig gamma-2 chain C region OS=Homo sapiens GN=IGHG2 PE=1 SV=2 | 5.06024096 |
| B9A064 | Immunoglobulin lambda-like polypeptide 5 OS=Homo sapiens GN=IGLLS PE=2 SV=2 | 3.3 |
| P00739-2 | Isoform 2 of Haptoglobin-related protein OS=Homo sapiens GN=HPR | 2.81690141 |
| P02766 | Transthyretin OS=Homo sapiens GN=TTR PE=1 SV=1 | 5.54545455 |
| P01024 | Complement C3 OS=Homo sapiens GN=C3 PE=1 SV=2 | 5.3125 |
| P00747 | Plasminogen OS=Homo sapiens GN=PLG PE=1 SV=2 | 1.47692308 |
| C9JV77 | Alpha-2-HS-glycoprotein OS=Homo sapiens GN=AHSG PE=2 SV=1 | 3.16666667 |
| H0Y300 | Haptoglobin OS=Homo sapiens GN=HP PE=2 SV=4 | ND |

(continued)

| Accession | Description | Fold Change |
|---|---|---|
| P04275 | von Willebrand factor OS=Homo sapiens GN=VWF PE=1 SV=4 | ND |
| P01620 | Ig kappa chain V-III region SIE OS=Homo sapiens PE=1 SV=1 | 2.78571429 |
| P0C0L4 | Complement C4-A OS=Homo sapiens GN=C4A PE=1 SV=2 | 2.42424242 |
| P06310 | Ig kappa chain V-II region RPMI 6410 OS=Homo sapiens PE=4 SV=1 | 2.64705882 |
| 043866 | CD5 antigen-like OS=Homo sapiens GN=CD5L PE=1 SV=1 | 1.2345679 |
| P01766 | Ig heavy chain V-III region BRO OS=Homo sapiens PE=1 SV=1 | 2.46875 |
| P01598 | Ig kappa chain V-I region EU OS=Homo sapiens PE=1 SV=1 | 3.829787234 |
| P02787 | Serotransferrin OS=Homo sapiens GN=TF PE=1 SV=3 | ND |
| P04003 | C4b-binding protein alpha chain OS=Homo sapiens GN=C4BPA PE=1 SV=2 | 1.527777778 |
| P01591 | Immunoglobulin J chain OS=Homo sapiens GN=IGJ PE=1 SV=4 | 2.716049383 |
| P01619 | Ig kappa chain V-III region B6 OS=Homo sapiens PE=1 SV=1 | 2.611111111 |
| P02747 | Complement C1q subcomponent subunit C OS=Homo sapiens GN=C1QC PE=1 SV=3 | 2.529411765 |
| P02790 | Hemopexin OS=Homo sapiens GN=HPX PE=1 SV=2 | 30.71428571 |
| P05452 | Tetranectin OS=Homo sapiens GN=CLEC3B PE=1 SV=3 | ND |
| P80748 | Ig lambda chain V-III region LOI OS=Homo sapiens PE=1 SV=1 | ND |
| P01717 | Ig lambda chain V-IV region Hil OS=Homo sapiens PE=1 SV=1 | 2.142857143 |
| P49908 | Selenoprotein P OS=Homo sapiens GN=SEPP1 PE=1 SV=3 | ND |
| P01714 | Ig lambda chain V-III region SH OS=Homo sapiens PE=1 SV=1 | ND |
| P01625 | Ig kappa chain V-IV region Len OS=Homo sapiens PE=1 SV=2 | 5 |
| Q9BSK4 | Protein fem-1 homolog A OS=Homo sapiens GN=FEM1A PE=1 SV=1 | 0.264285714 |
| K7ER74 | Protein APOC4-APOC2 OS=Homo sapiens GN=APOC4-APOC2 PE=2 SV=1 | ND |
| P02743 | Serum amyloid P-component OS=Homo sapiens GN=APCS PE=1 SV=2 | ND |
| P08603 | Complement factor H OS=Homo sapiens GN=CFH PE=1 SV=4 | ND |
| P02746 | Complement C1q subcomponent subunit B OS=Homo sapiens GN=C1QB PE=1 SV=3 | ND |
| B4E1Z4 | Complement factor B OS=Homo sapiens GN=CFB PE=2 SV=1 | ND |
| P01597 | Ig kappa chain V-I region DEE OS=Homo sapiens PE=1 SV=1 | 2.989690722 |
| V9GYM3 | Apolipoprotein A-II OS=Homo sapiens GN=APOA2 PE=4 SV=1 | ND |
| P01774 | Ig heavy chain V-III region POM OS=Homo sapiens PE=1 SV=1 | 2.636363636 |
| P01019 | Angiotensinogen OS=Homo sapiens GN=AGT PE=1 SV=1 | ND |
| S4R460 | Uncharacterized protein OS=Homo sapiens PE=4 SV=1 | ND |
| P19652 | Alpha-1-acid glycoprotein 2 OS=Homo sapiens GN=ORM2 PE=1 SV=2 | ND |
| P22352 | Glutathione peroxidase 3 OS=Homo sapiens GN=GPX3 PE=1 SV=2 | 2.741935484 |
| J3KPZ1 | Grainyhead-like protein 1 homolog OS=Homo sapiens GN=GRHLI PE=2 SV=1 | ND |
| P02749 | Beta-2-glycoprotein 1 OS=Homo sapiens GN=APOH PE=1 SV=3 | 3.428571429 |
| P01008 | Antithrombin-III OS=Homo sapiens GN=SERPINC1 PE=1 SV=1 | ND |
| P10720 | Platelet factor 4 variant OS=Homo sapiens GN=PF4V1 PE=1 SV=1 | 0.9375 |
| Q8IWC 1 | MAP7 domain-containing protein 3 OS=Homo sapiens GN=MAP7D3 PE=1 SV=2 | ND |
| P02774-3 | Isoform 3 of Vitamin D-binding protein OS=Homo sapiens GN=GC | ND |
| P02649 | Apolipoprotein E OS=Homo sapiens GN=APOE PE=1 SV=1 | 1.909091 |
| P01042 | Kininogen-1 OS=Homo sapiens GN=KNG1 PE=1 SV=2 | 0.418919 |
| P01621 | Ig kappa chain V-III region NG9 (Fragment) OS=Homo sapiens PE=1 SV=1 | ND |
| P01023 | Alpha-2-macroglobulin OS=Homo sapiens GN=A2M PE=1 SV=3 | ND |
| P04114 | Apolipoprotein B-100 OS=Homo sapiens GN=APOB PE=1 SV=2 | ND |
| P02654 | Apolipoprotein C-I OS=Homo sapiens GN=APOC1 PE=1 SV=1 | 2 |
| Q92945 | Far upstream element-binding protein 2 OS=Homo sapiens GN=KHSRP PE=1 SV=4 | ND |
| *ND indicates no detection of protein in control group | | |

**Table 7: Potential serum proteins to bind to AAV9 for increased vascular permeability**

| Accession | Description | Fold increase |
|---|---|---|
| P02671 | Fibrinogen alpha chain OS=Homo sapiens GN=FGA PE=1 SV=2 | 4.945652 |
| P02675 | Fibrinogen beta chain OS=Homo sapiens GN=FGB PE=1 SV=2 | 4.974026 |
| P02679 | Fibrinogen gamma chain OS=Homo sapiens GN=FGG PE=1 SV=3 | 4.853614 |
| C9JU00 | Fibrinogen gamma chain (Fragment) OS=Homo sapiens GN=FGG PE=2 SV=1 | 8.362319 |
| P02751 | Fibronectin OS=Homo sapiens GN=FN1 PE=1 SV=4 | 5.405263 |
| P00747 | Plasminogen OS=Homo sapiens GN=PLG PE=1 SV=2 | 3.232278 |
| P04275 | von Willebrand factor OS=Homo sapiens GN=VWF PE=1 SV=4 | ND* |
| P19652 | Alpha-1-acid glycoprotein 2 OS=Homo sapiens GN=ORM2 PE=1 SV=2 | ND |
| P10720 | Platelet factor 4 variant OS=Homo sapiens GN=PF4V1 PE=1 SV=1 | 1.466667 |

*ND indicates no detection of protein in control group

**Table 8:**

| | | A20 dilution | | | | | |
|---|---|---|---|---|---|---|---|
| | | 1:40 | 1:80 | 1:160 | 1:320 | 1:640 | 1:1280 |
| 25% HSA dilution | 1 | <1% | <1% | <1% | <1% | 3% | 62.83% |
| | 1:5 | <1% | <1% | <1% | <1% | 4.7% | 63.35% |
| | 1:50 | <1% | <1% | <1% | <1% | 4.7% | 56.23% |
| | 1:500 | <1% | <1% | <1% | <1% | 7.5% | 57.92% |
| | PBS | <1% | <1% | <1.7% | <7.3% | 22% | 52.37% |

**Table 9: The effect of VIPR on antigen presentation *in vivo***

| | 293/GFP | 293/GFP + OT-1 | 293/ICP47 | 293/ICP47 + OT-1 |
|---|---|---|---|---|
| Tumor formation | 6/6 | 0/6 | 4/4 | 4/4 |
| Tumor size ($cm^3$) | 0.545± 0.139 | | 0.612±0.198 | 0.483±0.157 |

**Table 10: Neutralization antibody titer and cross-reactivity for triploid virus AAV2/8**

| | | Vector | | | | | | | |
|---|---|---|---|---|---|---|---|---|---|
| | | AAV2 | Haploid virus AAV2/8 | | | Mixture virus AAV2 and AAV8 | | | AAV8 |
| | | | 3:1 | 1:1 | 1:3 | 3:1 | 1:1 | 1:3 | |
| mAb | A20 | 512 | 2048 | 32 | <2 | ND | ND | ND | <2 |
| | ADK8 | <2 | 512 | 512 | 1024 | ND | ND | ND | 1024 |
| serum | AAV2 | 4096 | 1024 | 256 | 8 | 4096 | 2048 | 1024 | <2 |
| | AAV8 | <2 | 256 | 256 | 512 | <2 | <2 | <2 | 512 |

**Table 11: Neutralization antibody titer and cross-reactivity for haploid virus AAV2/8/9**

| | AAV2 | AAV8 | AAV9 | AAV2/9 | AAV8/9 | AAV2/8/9 |
|---|---|---|---|---|---|---|
| SerumAAV2 | >2048 | <2 | | 512 | | 128 |
| SerumAAV8 | <2 | 128 | | | 32 | 4 |
| SerumAAV9 | <2 | 16 | 2048 | 512 | | 256 |
| Serum AAV2/8/9 | 8 | 128 | 128 | 64 | 512 | 128 |

SEQUENCES

AAV1 (SEQ ID NO:138)

1 ctctcccccc tgtcgcgttc gctcgctcgc tggctcgttt ggggggtgg cagctcaaag
61 agctgccaga cgacggccct ctggccgtcg cccccccaaa cgagccagcg agcgagcgaa
121 cgcgacaggg gggagagtgc cacactctca agcaaggagg ttttgtaagt ggtgatgtca
181 tatagttgtc acgcgatagt taatgattaa cagtcaggtg atgtgtgtta tccaatagga
241 tgaaagcgcg cgcatgagtt ctcgcgagac ttccggggta taaaggggtg agtgaacgag
301 cccgccgcca ttctctgctc tgaactgcta gaggaccctc gctgccatgg ctaccttcta
361 cgaagtcatt gttcgcgtcc catttgacgt ggaggaacat ctgcctggaa tttctgacag
421 ctttgtggac tgggtaactg gtcaaatttg ggagctgcct cccgagtcag atttgaattt
481 gactctgatt gagcagcctc agctgacggt tgctgacaga attcgccgcg tgttcctgta
541 cgagtggaac aaattttcca agcaggaatc caaattcttt gtgcagtttg aaaagggatc
601 tgaatatttt catctgcaca cgcttgtgga gacctccggc atctcttcca tggtcctagg
661 ccgctacgtg agtcagattc gcgcccagct ggtgaaagtg gtcttccagg gaatcgagcc
721 acagatcaac gactgggtcg ccatcaccaa ggtaaagaag ggcggagcca ataaggtggt
781 ggattctggg tatattcccg cctacctgct gccgaaggtc caaccggagc ttcagtgggc
841 gtggacaaac ctggacgagt ataaattggc cgccctgaac ctggaggagc gcaaacggct
901 cgtcgcgcag tttctggcag aatcctcgca gcgctcgcag gaggcggctt cgcagcgtga
961 gttctcggct gacccggtca tcaaaagcaa gacttcccag aaatacatgg cgctcgtcaa
1021 ctggctcgtg gagcacggca tcacttccga gaagcagtgg atccaggaga atcaggagag
1081 ctacctctcc ttcaactcca cgggcaactc tcggagccaa atcaaggccg cgctcgacaa
1141 cgcgaccaaa atcatgagtc tgacaaaaag cgcggtggac tacctcgtgg ggagctccgt
1201 tcccgaggac atttcaaaaa acagaatctg gcaaattttt gagatgaacg gctacgaccc
1261 ggcctacgcg ggatccatcc tctacggctg gtgtcagcgc tccttcaaca agaggaacac
1321 cgtctggctc tacggacccg ccacgaccgg caagaccaac atcgcggagg ccatcgccca
1381 cactgtgccc ttttacggct gcgtgaactg gaccaatgaa aactttccct ttaatgactg
1441 tgtggacaaa atgctcattt ggtgggagga gggaaagatg accaacaagg tggttgaatc
1501 cgccaaggcc atcctggggg gctccaaggt gcgggtcgat cagaaatgta aatcctctgt
1561 tcaaattgat tctacccccg tcattgtaac ttccaataca aacatgtgtg tggtggtgga
1621 tgggaattcc acgacctttg aacaccagca gccgctggag gaccgcatgt tcaaatttga
1681 actgactaag cggctcccgc cagattttgg caagattact aagcaggaag tcaaagactt
1741 ttttgcttgg gcaaaggtca atcaggtgcc ggtgactcac gagtttaaag ttcccaggga
1801 attggcggga actaaagggg cggagaaatc tctaaaacgc ccactgggtg acgtcaccaa
1861 tactagctat aaaagtccag agaagcgggc ccggctctca tttgttcccg agacgcctcg
1921 cagttcagac gtgactgtcg atcccgctcc tctgcgaccg ctcaattgga attcaaggta
1981 tgattgcaaa tgtgaccatc atgctcaatt tgacaacatt tctgacaaat gtgatgaatg
2041 tgaatatttg aatcggggca aaaatggatg tatctgtcac aatgtaactc actgtcaaat
2101 ttgtcacggg attcccccct gggagaagga aaacttgtca gattttgggg attttgacga
2161 tgccaataaa gaacagtaaa taaagcgagt agtcatgtct tttgttgatc accctccaga
2221 ttggttggaa gaagttggtg aaggtcttcg cgagtttttg ggccttgaag cgggcccacc
2281 gaaaccgaaa cccaatcagc agcatcaaga tcaagcccgt ggtcttgtgc tgcctggtta
2341 taactatctc ggacccggaa acggtctcga tcgaggagag cctgtcaaca gggcagacga
2401 ggtcgcgcga gagcacgaca tctcgtacaa cgagcagctt gaggcgggag acaacccct
2461 cctcaagtac aaccacgcgg acgccgagtt tcaggagaag ctcgccgacg acacatcctt
2521 cgggggaaac ctcggaaagg cagtctttca ggccaagaaa agggttctcg aaccttttgg
2581 cctggttgaa gagggtgcta agacggcccc taccggaaag cggatagacg accactttcc
2641 aaaaagaaag aaggctcgga ccgaagagga ctccaagcct tccacctcgt cagacgccga
2701 agctggaccc agcggatccc agcagctgca aatcccagca caaccagcct caagtttggg

2761 agctgataca atgtctgcgg gaggtggcgg cccattgggc gacaataacc aaggtgccga
2821 tggagtgggc aatgcctcgg gagattggca ttgcgattcc acgtggatgg gggacagagt
2881 cgtcaccaag tccacccgca cctgggtgct gcccagctac aacaaccacc agtaccgaga
2941 gatcaaaagc ggctccgtcg acggaagcaa cgccaacgcc tactttggat acagcacccc
3001 ctgggggtac tttgacttta accgcttcca cagccactgg agcccccgag actggcaaag
3061 actcatcaac aactattggg gcttcagacc ccggtctctc agagtcaaaa tcttcaacat
3121 ccaagtcaaa gaggtcacgg tgcaggactc caccaccacc atcgccaaca acctcacctc
3181 caccgtccaa gtgtttacgg acgacgacta ccaactcccg tacgtcgtcg gcaacgggac
3241 cgagggatgc ctgccggcct tcccccccgca ggtctttacg ctgccgcagt acggctacgc
3301 gacgctgaac cgagacaacg gagacaaccc gacagagcgg agcagcttct tttgcctaga
3361 gtactttccc agcaagatgc tgaggacggg caacaacttt gagtttacct acagctttga
3421 agaggtgccc ttccactgca gcttcgcccc gagccagaac ctctttaagc tggccaaccc
3481 gctggtggac cagtacctgt accgcttcgt gagcacctcg gccacgggcg ccatccagtt
3541 ccaaaagaac ctggcgggca gatacgccaa cacctacaaa aactggttcc cggggcccat
3601 gggccgaacc cagggctgga acacgagctc tggcagcagc accaacagag tcagcgtcaa
3661 caacttttcc gtctcaaacc ggatgaacct ggagggggcc agctaccaag tgaaccccca
3721 gcccaacggg atgacaaaca cgctccaagg cagcaaccgc tacgcgctgg aaaacaccat
3781 gatcttcaac gctcaaaacg ccacgccggg aactacctcg gtgtacccag aggacaatct
3841 actgctgacc agcgagagcg agactcagcc cgtcaaccgg gtggcttaca acacgggcgg
3901 tcagatggcc accaacgccc agaacgccac cacggctccc acggtcggga cctacaacct
3961 ccaggaagtg cttcctggca gcgtatggat ggagagggac gtgtacctcc aaggacccat
4021 ctgggccaag atcccagaga cgggggcgca ctttcacccc tctccggcca tgggcggatt
4081 cggactcaaa cacccgccgc ccatgatgct catcaaaaac acgccggtgc ccggcaacat
4141 caccagcttc tcggacgtgc ccgtcagcag cttcatcacc cagtacagca ccgggcaggt
4201 caccgtggag atggaatggg agctcaaaaa ggaaaactcc aagaggtgga acccagagat
4261 ccagtacacc aacaactaca acgacccccca gtttgtggac tttgctccag acggctccgg
4321 cgaatacaga accaccagag ccatcggaac ccgatacctc acccgacccc tttaacccat
4381 tcatgtcgca taccctcaat aaaccgtgta ttcgtgtcag tgaaatactg cctcttgtgg
4441 tcattcaatg aacatcagct tacaacatct acaaaacccc cttgcttgag agtgtggcac
4501 tctcccccct gtcgcgttcg ctcgctcgct ggctcgtttg ggggggtggc agctcaaaga
4561 gctgccagac gacggccctc tggccgtcgc ccccccaaac gagccagcga gcgagcgaac
4621 gcgacagggg ggagag

AAV2 (SEQ ID NO:139)

1 ttggccactc cctctctgcg cgctcgctcg ctcactgagg ccgggcgacc aaaggtcgcc
61 cgacgcccgg gctttgcccg ggcggcctca gtgagcgagc gagcgcgcag agagggagtg
121 gccaactcca tcactagggg ttcctggagg ggtggagtcg tgacgtgaat tacgtcatag
181 ggttagggag gtcctgtatt agaggtcacg tgagtgtttt gcgacatttt gcgacaccat
241 gtggtcacgc tgggtattta agcccgagtg agcacgcagg gtctccattt tgaagcggga
301 ggtttgaacg cgcagccgcc atgccggggt tttacgagat tgtgattaag gtccccagcg
361 accttgacga gcatctgccc ggcatttctg acagctttgt gaactgggtg gccgagaagg
421 aatgggagtt gccgccagat tctgacatgg atctgaatct gattgagcag gcacccctga
481 ccgtggccga gaagctgcag cgcgactttc tgacggaatg gcgccgtgtg agtaaggccc
541 cggaggccct tttctttgtg caatttgaga agggagagag ctacttccac atgcacgtgc
601 tcgtggaaac caccggggtg aaatccatgg ttttgggacg tttcctgagt cagattcgcg
661 aaaaactgat tcagagaatt taccgcggga tcgagccgac tttgccaaac tggttcgcgg
721 tcacaaagac cagaaatggc gccggaggcg ggaacaaggt ggtggatgag tgctacatcc
781 ccaattactt gctccccaaa acccagcctg agctccagtg ggcgtggact aatatggaac
841 agtatttaag cgcctgtttg aatctcacgg agcgtaaacg gttggtggcg cagcatctga
901 cgcacgtgtc gcagacgcag gagcagaaca aagagaatca gaatcccaat tctgatgcgc
961 cggtgatcag atcaaaaact tcagccaggt acatggagct ggtcgggtgg ctcgtggaca
1021 aggggattac ctcggagaag cagtggatcc aggaggacca ggcctcatac atctccttca
1081 atgcggcctc caactcgcgg tcccaaatca aggctgcctt ggacaatgcg ggaaagatta
1141 tgagcctgac taaaaccgcc cccgactacc tggtgggcca gcagcccgtg gaggacattt
1201 ccagcaatcg gatttataaa attttggaac taaacgggta cgatccccaa tatgcggctt
1261 ccgtctttct gggatgggcc acgaaaaagt tcggcaagag gaacaccatc tggctgtttg
1321 ggcctgcaac taccgggaag accaacatcg cggaggccat agcccacact gtgcccttct
1381 acgggtgcgt aaactggacc aatgagaact ttcccttcaa cgactgtgtc gacaagatgg
1441 tgatctggtg ggaggagggg aagatgaccg ccaaggtcgt ggagtcggcc aaagccattc
1501 tcggaggaag caaggtgcgc gtggaccaga aatgcaagtc ctcggcccag atagacccga
1561 ctcccgtgat cgtcacctcc aacaccaaca tgtgcgccgt gattgacggg aactcaacga
1621 ccttcgaaca ccagcagccg ttgcaagacc ggatgttcaa atttgaactc acccgccgtc
1681 tggatcatga ctttgggaag gtcaccaagc aggaagtcaa agactttttc cggtgggcaa
1741 aggatcacgt ggttgaggtg gagcatgaat tctacgtcaa aaagggtgga gccaagaaaa
1801 gacccgcccc cagtgacgca gatataagtg agcccaaacg ggtgcgcgag tcagttgcgc
1861 agccatcgac gtcagacgcg gaagcttcga tcaactacgc agacaggtac caaaacaaat
1921 gttctcgtca cgtgggcatg aatctgatgc tgtttccctg cagacaatgc gagagaatga
1981 atcagaattc aaatatctgc ttcactcacg gacagaaaga ctgtttagag tgctttcccg
2041 tgtcagaatc tcaacccgtt tctgtcgtca aaaaggcgta tcagaaactg tgctacattc
2101 atcatatcat gggaaaggtg ccagacgctt gcactgcctg cgatctggtc aatgtggatt
2161 tggatgactg catctttgaa caataaatga tttaaatcag gtatggctgc cgatggttat
2221 cttccagatt ggctcgagga cactctctct gaaggaataa gacagtggtg gaagctcaaa
2281 cctggcccac caccaccaaa gcccgcagag cggcataagg acgacagcag gggtcttgtg
2341 cttcctgggt acaagtacct cggacccttc aacggactcg acaagggaga gccggtcaac
2401 gaggcagacg ccgcggccct cgagcacgac aaagcctacg accggcagct cgacagcgga
2461 gacaacccgt acctcaagta caaccacgcc gacgcggagt ttcaggagcg ccttaaagaa
2521 gatacgtctt ttgggggcaa cctcggacga gcagtcttcc aggcgaaaaa gagggttctt
2581 gaacctctgg gcctggttga ggaacctgtt aagacggctc cgggaaaaaa gaggccggta
2641 gagcactctc ctgtggagcc agactcctcc tcgggaaccg gaaaggcggg ccagcagcct
2701 gcaagaaaaa gattgaattt tggtcagact ggagacgcag actcagtacc tgacccccag
2761 cctctcggac agccaccagc agccccctct ggtctgggaa ctaatacgat ggctacaggc
2821 agtggcgcac caatggcaga caataacgag ggcgccgacg gagtgggtaa ttcctcggga

2881 aattggcatt gcgattccac atggatgggc gacagagtca tcaccaccag caccegaacc
2941 tgggccctgc ccacctacaa caaccacctc tacaaacaaa tttccagcca atcaggagcc
3001 tcgaacgaca atcactactt tggctacagc accccttggg ggtattttga cttcaacaga
3061 ttccactgcc acttttcacc acgtgactgg caaagactca tcaacaacaa ctggggattc
3121 cgacccaaga gactcaactt caagctcttt aacattcaag tcaaagaggt cacgcagaat
3181 gacggtacga cgacgattgc caataacctt accagcacgg ttcaggtgtt tactgactcg
3241 gagtaccagc tcccgtacgt cctcggctcg gcgcatcaag gatgcctccc gccgttccca
3301 gcagacgtct tcatggtgcc acagtatgga tacctcaccc tgaacaacgg gagtcaggca
3361 gtaggacgct cttcatttta ctgcctggag tactttcctt ctcagatgct gcgtaccgga
3421 aacaacttta ccttcagcta cacttttgag gacgttcctt tccacagcag ctacgctcac
3481 agccagagtc tggaccgtct catgaatcct ctcatcgacc agtacctgta ttacttgagc
3541 agaacaaaca ctccaagtgg aaccaccacg cagtcaaggc ttcagttttc tcaggccgga
3601 gcgagtgaca ttcgggacca gtctaggaac tggcttcctg gaccctgtta ccgccagcag
3661 cgagtatcaa agacatctgc ggataacaac aacagtgaat actcgtggac tggagctacc
3721 aagtaccacc tcaatggcag agactctctg gtgaatccgg gcccggccat ggcaagccac
3781 aaggacgatg aagaaaagtt ttttcctcag agcggggttc tcatctttgg gaagcaaggc
3841 tcagagaaaa caaatgtgga cattgaaaag gtcatgatta cagacgaaga ggaaatcagg
3901 acaaccaatc ccgtggctac ggagcagtat ggttctgtat ctaccaacct ccagagaggc
3961 aacagacaag cagctaccgc agatgtcaac acacaaggcg ttcttccagg catggtctgg
4021 caggacagag atgtgtacct tcaggggccc atctgggcaa agattccaca cacggacgga
4081 cattttcacc cctctcccct catgggtgga ttcggactta aacaccctcc tccacagatt
4141 ctcatcaaga acaccccggt acctgcgaat ccttcgacca ccttcagtgc ggcaaagttt
4201 gcttccttca tcacacagta ctccacggga caggtcagcg tggagatcga gtgggagctg
4261 cagaaggaaa acagcaaacg ctggaatccc gaaattcagt acacttccaa ctacaacaag
4321 tctgttaatg tggactttac tgtggacact aatggcgtgt attcagagcc tcgccccatt
4381 ggcaccagat acctgactcg taatctgtaa ttgcttgtta atcaataaac cgtttaattc
4441 gtttcagttg aactttggtc tctgcgtatt tctttcttat ctagtttcca tggctacgta
4501 gataagtagc atggcgggtt aatcattaac tacaaggaac ccctagtgat ggagttggcc
4561 actccctctc tgcgcgctcg ctcgctcact gaggccgggc gaccaaaggt cgcccgacgc
4621 ccgggctttg cccgggcggc ctcagtgagc gagcgagcgc gcagagaggg agtggccaa

AAV3 (SEQ ID NO:140)

1 ttggccactc cctctatgcg cactcgctcg ctcggtgggg cctggcgacc aaaggtcgcc
61 agacggacgt gctttgcacg tccggcccca ccgagcgagc gagtgcgcat agagggagtg
121 gccaactcca tcactagagg tatggcagtg acgtaacgcg aagcgcgcga agcgagacca
181 cgcctaccag ctgcgtcagc agtcaggtga cccttttgcg acagtttgcg acaccacgtg
241 gccgctgagg gtatatattc tcgagtgagc gaaccaggag ctccattttg accgcgaaat
301 ttgaacgagc agcagccatg ccggggttct acgagattgt cctgaaggtc ccgagtgacc
361 tggacgagcg cctgccgggc atttctaact cgtttgttaa ctgggtggcc gagaaggaat
421 gggacgtgcc gccggattct gacatggatc cgaatctgat tgagcaggca cccctgaccg
481 tggccgaaaa gcttcagcgc gagttcctgg tggagtggcg ccgcgtgagt aaggccccgg
541 aggccctctt ttttgtccag ttcgaaaagg gggagaccta cttccacctg cacgtgctga
601 ttgagaccat cggggtcaaa tccatggtgg tcggccgcta cgtgagccag attaaagaga
661 agctggtgac ccgcatctac cgcggggtcg agccgcagct tccgaactgg ttcgcggtga
721 ccaaaacgcg aaatggcgcc gggggcggga acaaggtggt ggacgactgc tacatcccca
781 actacctgct ccccaagacc cagcccgagc tccagtgggc gtggactaac atggaccagt
841 atttaagcgc ctgtttgaat ctcgcggagc gtaaacggct ggtggcgcag catctgacgc
901 acgtgtcgca gacgcaggag cagaacaaag agaatcagaa ccccaattct gacgcgccgg
961 tcatcaggtc aaaaacctca gccaggtaca tggagctggt cgggtggctg gtggaccgcg
1021 ggatcacgtc agaaaagcaa tggattcagg aggaccaggc ctcgtacatc tccttcaacg
1081 ccgcctccaa ctcgcggtcc cagatcaagg ccgcgctgga caatgcctcc aagatcatga
1141 gcctgacaaa gacggctccg gactacctgg tgggcagcaa cccgccggag gacattacca
1201 aaaatcggat ctaccaaatc ctggagctga acgggtacga tccgcagtac gcggcctccg
1261 tcttcctggg ctgggcgcaa aagaagttcg ggaagaggaa caccatctgg ctctttgggc
1321 cggccacgac gggtaaaacc aacatcgcgg aagccatcgc ccacgccgtg cccttctacg
1381 gctgcgtaaa ctggaccaat gagaactttc ccttcaacga ttgcgtcgac aagatggtga
1441 tctggtggga ggagggcaag atgacggcca aggtcgtgga gagcgccaag gccattctgg
1501 gcggaagcaa ggtgcgcgtg gaccaaaagt gcaagtcatc ggcccagatc gaacccactc
1561 ccgtgatcgt cacctccaac accaacatgt gcgccgtgat tgacgggaac agcaccacct
1621 tcgagcatca gcagccgctg caggaccgga tgtttgaatt tgaacttacc cgccgtttgg
1681 accatgactt tgggaaggtc accaaacagg aagtaaagga ctttttccgg tgggcttccg
1741 atcacgtgac tgacgtggct catgagttct acgtcagaaa gggtggagct aagaaacgcc
1801 ccgcctccaa tgacgcggat gtaagcgagc caaaacggga gtgcacgtca cttgcgcagc
1861 cgacaacgtc agacgcggaa gcaccggcgg actacgcgga caggtaccaa aacaaatgtt
1921 ctcgtcacgt gggcatgaat ctgatgcttt ttccctgtaa aacatgcgag agaatgaatc
1981 aaatttccaa tgtctgtttt acgcatggtc aaagagactg tggggaatgc ttccctggaa
2041 tgtcagaatc tcaacccgtt tctgtcgtca aaaagaagac ttatcagaaa ctgtgtccaa
2101 ttcatcatat cctgggaagg gcacccgaga ttgcctgttc ggcctgcgat ttggccaatg
2161 tggacttgga tgactgtgtt tctgagcaat aaatgactta aaccaggtat ggctgctgac
2221 ggttatcttc cagattggct cgaggacaac ctttctgaag gcattcgtga gtggtgggct
2281 ctgaaacctg gagtccctca acccaaagcg aaccaacaac accaggacaa ccgtcggggt
2341 cttgtgcttc cgggttacaa atacctcgga cccggtaacg gactcgacaa aggagagccg
2401 gtcaacgagg cggacgcggc agccctcgaa cacgacaaag cttacgacca gcagctcaag
2461 gccggtgaca acccgtacct caagtacaac cacgccgacg ccgagtttca ggagcgtctt
2521 caagaagata cgtcttttgg gggcaacctt ggcagagcag tcttccaggc caaaaagagg
2581 atccttgagc ctcttggtct ggttgaggaa gcagctaaaa cggctcctgg aaagaagggg
2641 gctgtagatc agtctcctca ggaaccggac tcatcatctg gtgttggcaa atcgggcaaa
2701 cagcctgcca gaaaaagact aaatttcggt cagactggag actcagagtc agtcccagac
2761 cctcaacctc tcggagaacc accagcagcc cccacaagtt tgggatctaa tacaatggct
2821 tcaggcggtg gcgcaccaat ggcagacaat aacgagggtg ccgatggagt gggtaattcc

2881 tcaggaaatt ggcattgcga ttcccaatgg ctgggcgaca gagtcatcac caccagcacc
2941 agaacctggg ccctgcccac ttacaacaac catctctaca agcaaatctc cagccaatca
3001 ggagcttcaa acgacaacca ctactttggc tacagcaccc cttgggggta ttttgacttt
3061 aacagattcc actgccactt ctcaccacgt gactggcagc gactcattaa caacaactgg
3121 ggattccggc ccaagaaact cagcttcaag ctcttcaaca tccaagttag aggggtcacg
3181 cagaacgatg gcacgacgac tattgccaat aaccttacca gcacggttca agtgtttacg
3241 gactcggagt atcagctccc gtacgtgctc gggtcggcgc accaaggctg tctcccgccg
3301 tttccagcgg acgtcttcat ggtccctcag tatggatacc tcaccctgaa caacggaagt
3361 caagcggtgg gacgctcatc cttttactgc ctggagtact tcccttcgca gatgctaagg
3421 actggaaata acttccaatt cagctatacc ttcgaggatg taccttttca cagcagctac
3481 gctcacagcc agagtttgga tcgcttgatg aatcctctta ttgatcagta tctgtactac
3541 ctgaacagaa cgcaaggaac aacctctgga acaaccaacc aatcacggct gctttttagc
3601 caggctgggc ctcagtctat gtctttgcag gccagaaatt ggctacctgg gccctgctac
3661 cggcaacaga gactttcaaa gactgctaac gacaacaaca acagtaactt tccttggaca
3721 gcggccagca aatatcatct caatggccgc gactcgctgg tgaatccagg accagctatg
3781 gccagtcaca aggacgatga agaaaaattt ttccctatgc acggcaatct aatatttggc
3841 aaagaaggga caacggcaag taacgcagaa ttagataatg taatgattac ggatgaagaa
3901 gagattcgta ccaccaatcc tgtggcaaca gagcagtatg gaactgtggc aaataacttg
3961 cagagctcaa atacagctcc cacgactgga actgtcaatc atcagggggc cttacctggc
4021 atggtgtggc aagatcgtga cgtgtacctt caaggaccta tctgggcaaa gattcctcac
4081 acggatggac actttcatcc ttctcctctg atgggaggct ttggactgaa acatccgcct
4141 cctcaaatca tgatcaaaaa tactccggta ccggcaaatc ctccgacgac tttcagcccg
4201 gccaagtttg cttcatttat cactcagtac tccactggac aggtcagcgt ggaaattgag
4261 tgggagctac agaaagaaaa cagcaaacgt tggaatccag agattcagta cacttccaac
4321 tacaacaagt ctgttaatgt ggactttact gtagacacta atggtgttta tagtgaacct
4381 cgccctattg gaacccggta tctcacacga aacttgtgaa tcctggttaa tcaataaacc
4441 gtttaattcg tttcagttga actttggctc ttgtgcactt ctttatcttt atcttgtttc
4501 catggctact gcgtagataa gcagcggcct gcggcgcttg cgcttcgcgg tttacaactg
4561 ctggttaata tttaactctc gccatacctc tagtgatgga gttggccact ccctctatgc
4621 gcactcgctc gctcggtggg gcctggcgac caaaggtcgc cagacggacg tgctttgcac
4681 gtccggcccc accgagcgag cgagtgcgca tagagggagt ggccaa

AAV4 (SEQ ID NO:141)

1 ttggccactc cctctatgcg cgctcgctca ctcactcggc cctggagacc aaaggtctcc
61 agactgccgg cctctggccg gcagggccga gtgagtgagc gagcgcgcat agagggagtg
121 gccaactcca tcatctaggt ttgcccactg acgtcaatgt gacgtcctag ggttagggag
181 gtccctgtat tagcagtcac gtgagtgtcg tatttcgcgg agcgtagcgg agcgcatacc
241 aagctgccac gtcacagcca cgtggtccgt ttgcgacagt ttgcgacacc atgtggtcag
301 gagggtatat aaccgcgagt gagccagcga ggagctccat tttgcccgcg aattttgaac
361 gagcagcagc catgccgggg ttctacgaga tcgtgctgaa ggtgcccagc gacctggacg
421 agcacctgcc cggcatttct gactcttttg tgagctgggt ggccgagaag gaatgggagc
481 tgccgccgga ttctgacatg gacttgaatc tgattgagca ggcacccctg accgtggccg
541 aaaagctgca acgcgagttc ctggtcgagt ggcgccgcgt gagtaaggcc ccggaggccc
601 tcttctttgt ccagttcgag aagggggaca gctacttcca cctgcacatc ctggtggaga
661 ccgtgggcgt caaatccatg gtggtgggcc gctacgtgag ccagattaaa gagaagctgg
721 tgacccgcat ctaccgcggg gtcgagccgc agcttccgaa ctggttcgcg gtgaccaaga
781 cgcgtaatgg cgccggaggc gggaacaagg tggtggacga ctgctacatc cccaactacc
841 tgctccccaa gacccagccc gagctccagt gggcgtggac taacatggac cagtatataa
901 gcgcctgttt gaatctcgcg gagcgtaaac ggctggtggc gcagcatctg acgcacgtgt
961 cgcagacgca ggagcagaac aaggaaaacc agaaccccaa ttctgacgcg ccggtcatca
1021 ggtcaaaaac ctccgccagg tacatggagc tggtcgggtg gctggtggac cgcgggatca
1081 cgtcagaaaa gcaatggatc caggaggacc aggcgtccta catctccttc aacgccgcct
1141 ccaactcgcg gtcacaaatc aaggccgcgc tggacaatgc ctccaaaatc atgagcctga
1201 caaagacggc tccggactac ctggtgggcc agaacccgcc ggaggacatt tccagcaacc
1261 gcatctaccg aatcctcgag atgaacgggt acgatccgca gtacgcggcc tccgtcttcc
1321 tgggctgggc gcaaaagaag ttcgggaaga ggaacaccat ctggctcttt gggccggcca
1381 cgacgggtaa aaccaacatc gcggaagcca tcgcccacgc cgtgcccttc tacggctgcg
1441 tgaactggac caatgagaac tttccgttca acgattgcgt cgacaagatg gtgatctggt
1501 gggaggaggg caagatgacg gccaaggtcg tagagagcgc caaggccatc ctgggcggaa
1561 gcaaggtgcg cgtggaccaa aagtgcaagt catcggccca gatcgaccca actcccgtga
1621 tcgtcacctc caacaccaac atgtgcgcgg tcatcgacgg aaactcgacc accttcgagc
1681 accaacaacc actccaggac cggatgttca agttcgagct caccaagcgc ctggagcacg
1741 actttggcaa ggtcaccaag caggaagtca aagacttttt ccggtgggcg tcagatcacg
1801 tgaccgaggt gactcacgag ttttacgtca gaaagggtgg agctagaaag aggcccgccc
1861 ccaatgacgc agatataagt gagcccaagc gggcctgtcc gtcagttgcg cagccatcga
1921 cgtcagacgc ggaagctccg gtggactacg cggacaggta ccaaaacaaa tgttctcgtc
1981 acgtgggtat gaatctgatg ctttttccct gccggcaatg cgagagaatg aatcagaatg
2041 tggacatttg cttcacgcac ggggtcatgg actgtgccga gtgcttcccc gtgtcagaat
2101 ctcaacccgt gtctgtcgtc agaaagcgga cgtatcagaa actgtgtccg attcatcaca
2161 tcatggggag ggcgcccgag gtggcctgct cggcctgcga actggccaat gtggacttgg
2221 atgactgtga catggaacaa taaatgactc aaaccagata tgactgacgg ttaccttcca
2281 gattggctag aggacaacct ctctgaaggc gttcgagagt ggtgggcgct gcaacctgga
2341 gcccctaaac ccaaggcaaa tcaacaacat caggacaacg ctcggggtct tgtgcttccg
2401 ggttacaaat acctcggacc cggcaacgga ctcgacaagg gggaacccgt caacgcagcg
2461 gacgcggcag ccctcgagca cgacaaggcc tacgaccagc agctcaaggc cggtgacaac
2521 ccctacctca agtacaacca cgccgacgcg gagttccagc agcggcttca gggcgacaca
2581 tcgtttgggg gcaacctcgg cagagcagtc ttccaggcca aaaagagggt tcttgaacct
2641 cttggtctgg ttgagcaagc gggtgagacg gctcctggaa agaagagacc gttgattgaa
2701 tccccccagc agcccgactc ctccacgggt atcggcaaaa aaggcaagca gccggctaaa
2761 aagaagctcg ttttcgaaga cgaaactgga gcaggcgacg gacccccctga gggatcaact
2821 tccggagcca tgtctgatga cagtgagatg cgtgcagcag ctggcggagc tgcagtcgag

2881 ggcggacaag gtgccgatgg agtgggtaat gcctcgggtg attggcattg cgattccacc
2941 tggtctgagg gccacgtcac gaccaccagc accagaacct gggtcttgcc cacctacaac
3001 aaccacctct acaagcgact cggagagagc ctgcagtcca acacctacaa cggattctcc
3061 accccctggg gatactttga cttcaaccgc ttccactgcc acttctcacc acgtgactgg
3121 cagcgactca tcaacaacaa ctggggcatg cgacccaaag ccatgcgggt caaaatcttc
3181 aacatccagg tcaaggaggt cacgacgtcg aacggcgaga caacggtggc taataacctt
3241 accagcacgg ttcagatctt tgcggactcg tcgtacgaac tgccgtacgt gatggatgcg
3301 ggtcaagagg gcagcctgcc tcctttcccc aacgacgtct ttatggtgcc ccagtacggc
3361 tactgtggac tggtgaccgg caacacttcg cagcaacaga ctgacagaaa tgccttctac
3421 tgcctggagt actttccttc gcagatgctg cggactggca acaactttga aattacgtac
3481 agttttgaga aggtgccttt ccactcgatg tacgcgcaca gccagagcct ggaccggctg
3541 atgaaccctc tcatcgacca gtacctgtgg ggactgcaat cgaccaccac cggaaccacc
3601 ctgaatgccg ggactgccac caccaacttt accaagctgc ggcctaccaa cttttccaac
3661 tttaaaaaga actggctgcc cgggccttca atcaagcagc agggcttctc aaagactgcc
3721 aatcaaaact acaagatccc tgccaccggg tcagacagtc tcatcaaata cgagacgcac
3781 agcactctgg acggaagatg gagtgccctg acccccggac ctccaatggc cacggctgga
3841 cctgcggaca gcaagttcag caacagccag ctcatctttg cggggcctaa acagaacggc
3901 aacacggcca ccgtacccgg gactctgatc ttcacctctg aggaggagct ggcagccacc
3961 aacgccaccg atacggacat gtggggcaac ctacctggcg gtgaccagag caacagcaac
4021 ctgccgaccg tggacagact gacagccttg ggagccgtgc ctggaatggt ctggcaaaac
4081 agagacattt actaccaggg tcccatttgg gccaagattc ctcataccga tggacacttt
4141 cacccctcac cgctgattgg tgggtttggg ctgaaacacc cgcctcctca aatttttatc
4201 aagaacaccc cggtacctgc gaatcctgca acgaccttca gctctactcc ggtaaactcc
4261 ttcattactc agtacagcac tggccaggtg tcggtgcaga ttgactggga gatccagaag
4321 gagcggtcca aacgctggaa ccccgaggtc cagtttacct ccaactacgg acagcaaaac
4381 tctctgttgt gggctcccga tgcggctggg aaatacactg agcctagggc tatcggtacc
4441 cgctacctca cccaccacct gtaataacct gttaatcaat aaaccggttt attcgtttca
4501 gttgaacttt ggtctccgtg tccttcttat cttatctcgt ttccatggct actgcgtaca
4561 taagcagcgg cctgcggcgc ttgcgcttcg cggtttacaa ctgccggtta atcagtaact
4621 tctggcaaac cagatgatgg agttggccac attagctatg cgcgctcgct cactcactcg
4681 gccctggaga ccaaaggtct ccagactgcc ggcctctggc cggcagggcc gagtgagtga
4741 gcgagcgcgc atagagggag tggccaa

<u>AAV5</u> (SEQ ID NO:142)

1 ctctcccccc tgtcgcgttc gctcgctcgc tggctcgttt gggggggtgg cagctcaaag
61 agctgccaga cgacggccct ctggccgtcg cccccccaaa cgagccagcg agcgagcgaa
121 cgcgacaggg gggagagtgc cacactctca agcaaggggg ttttgtaagc agtgatgtca
181 taatgatgta atgcttattg tcacgcgata gttaatgatt aacagtcatg tgatgtgttt
241 tatccaatag gaagaaagcg cgcgtatgag ttctcgcgag acttccgggg tataaaagac
301 cgagtgaacg agcccgccgc cattctttgc tctggactgc tagaggaccc tcgctgccat
361 ggctaccttc tatgaagtca ttgttcgcgt cccatttgac gtggaggaac atctgcctgg
421 aatttctgac agctttgtgg actgggtaac tggtcaaatt tgggagctgc ctccagagtc
481 agatttaaat ttgactctgg ttgaacagcc tcagttgacg gtggctgata gaattcgccg
541 cgtgttcctg tacgagtgga acaaattttc caagcaggag tccaaattct ttgtgcagtt
601 tgaaaaggga tctgaatatt ttcatctgca cacgcttgtg gagacctccg gcatctcttc
661 catggtcctc ggccgctacg tgagtcagat tcgcgcccag ctggtgaaag tggtcttcca
721 gggaattgaa ccccagatca acgactgggt cgccatcacc aaggtaaaga agggcggagc
781 caataaggtg gtggattctg ggtatattcc cgcctacctg ctgccgaagg tccaaccgga
841 gcttcagtgg gcgtggacaa acctggacga gtataaattg gccgccctga atctggagga
901 gcgcaaacgg ctcgtcgcgc agtttctggc agaatcctcg cagcgctcgc aggaggcggc
961 ttcgcagcgt gagttctcgg ctgacccggt catcaaaagc aagacttccc agaaatacat
1021 ggcgctcgtc aactggctcg tggagcacgg catcacttcc gagaagcagt ggatccagga
1081 aaatcaggag agctacctct ccttcaactc caccggcaac tctcggagcc agatcaaggc
1141 cgcgctcgac aacgcgacca aaattatgag tctgacaaaa agcgcggtgg actacctcgt
1201 ggggagctcc gttcccgagg acatttcaaa aaacagaatc tggcaaattt ttgagatgaa
1261 tggctacgac ccggcctacg cgggatccat cctctacggc tggtgtcagc gctccttcaa
1321 caagaggaac accgtctggc tctacggacc cgccacgacc ggcaagacca acatcgcgga
1381 ggccatcgcc cacactgtgc ccttttacgg ctgcgtgaac tggaccaatg aaaactttcc
1441 ctttaatgac tgtgtggaca aaatgctcat ttggtgggag gagggaaaga tgaccaacaa
1501 ggtggttgaa tccgccaagg ccatcctggg gggctcaaag gtgcgggtcg atcagaaatg
1561 taaatcctct gttcaaattg attctacccc tgtcattgta acttccaata caaacatgtg
1621 tgtggtggtg gatgggaatt ccacgacctt tgaacaccag cagccgctgg aggaccgcat
1681 gttcaaattt gaactgacta agcggctccc gccagatttt ggcaagatta ctaagcagga
1741 agtcaaggac tttttttgctt gggcaaaggt caatcaggtg ccggtgactc acgagtttaa
1801 agttcccagg gaattggcgg gaactaaagg ggcggagaaa tctctaaaac gcccactggg
1861 tgacgtcacc aatactagct ataaaagtct ggagaagcgg gccaggctct catttgttcc
1921 cgagacgcct cgcagttcag acgtgactgt tgatcccgct cctctgcgac cgctcaattg
1981 gaattcaagg tatgattgca aatgtgacta tcatgctcaa tttgacaaca tttctaacaa
2041 atgtgatgaa tgtgaatatt tgaatcgggg caaaaatgga tgtatctgtc acaatgtaac
2101 tcactgtcaa atttgtcatg ggattccccc ctgggaaaag gaaaacttgt cagattttgg
2161 ggattttgac gatgccaata aagaacagta aataaagcga gtagtcatgt cttttgttga
2221 tcaccctcca gattggttgg aagaagttgg tgaaggtctt cgcgagtttt tgggccttga
2281 agcgggccca ccgaaaccaa aacccaatca gcagcatcaa gatcaagccc gtggtcttgt
2341 gctgcctggt tataactatc tcggacccgg aaacggtctc gatcgaggag agcctgtcaa
2401 cagggcagac gaggtcgcgc gagagcacga catctcgtac aacgagcagc ttgaggcggg
2461 agacaacccc tacctcaagt acaaccacgc ggacgccgag tttcaggaga agctcgccga
2521 cgacacatcc ttcgggggaa acctcggaaa ggcagtcttt caggccaaga aaagggttct
2581 cgaacctttt ggcctggttg aagagggtgc taagacggcc cctaccggaa agcggataga
2641 cgaccacttt ccaaaaagaa agaaggctcg gaccgaagag gactccaagc cttccacctc
2701 gtcagacgcc gaagctggac ccagcggatc ccagcagctg caaatcccag cccaaccagc
2761 ctcaagtttg ggagctgata caatgtctgc gggaggtggc ggcccattgg gcgacaataa
2821 ccaaggtgcc gatggagtgg gcaatgcctc gggagattgg cattgcgatt ccacgtggat

2881 gggggacaga gtcgtcacca agtccacccg aacctgggtg ctgcccagct acaacaacca
2941 ccagtaccga gagatcaaaa gcggctccgt cgacggaagc aacgccaacg cctactttgg
3001 atacagcacc ccctgggggt actttgactt taaccgcttc cacagccact ggagcccccg
3061 agactggcaa agactcatca acaactactg gggcttcaga ccccggtccc tcagagtcaa
3121 aatcttcaac attcaagtca aagaggtcac ggtgcaggac tccaccacca ccatcgccaa
3181 caacctcacc tccaccgtcc aagtgtttac ggacgacgac taccagctgc cctacgtcgt
3241 cggcaacggg accgagggat gcctgccggc cttccctccg caggtcttta cgctgccgca
3301 gtacggttac gcgacgctga accgcgacaa cacagaaaat cccaccgaga ggagcagctt
3361 cttctgccta gagtactttc ccagcaagat gctgagaacg ggcaacaact ttgagtttac
3421 ctacaacttt gaggaggtgc ccttccactc cagcttcgct cccagtcaga acctgttcaa
3481 gctggccaac ccgctggtgg accagtactt gtaccgcttc gtgagcacaa ataacactgg
3541 cggagtccag ttcaacaaga acctggccgg gagatacgcc aacacctaca aaaactggtt
3601 cccggggccc atgggccgaa cccagggctg gaacctgggc tccggggtca accgcgccag
3661 tgtcagcgcc ttcgccacga ccaataggat ggagctcgag ggcgcgagtt accaggtgcc
3721 cccgcagccg aacggcatga ccaacaacct ccagggcagc aacacctatg ccctggagaa
3781 cactatgatc ttcaacagcc agccggcgaa cccgggcacc accgccacgt acctcgaggg
3841 caacatgctc atcaccagcg agagcgagac gcagccggtg aaccgcgtgg cgtacaacgt
3901 cggcgggcag atggccacca acaaccagag ctccaccact gcccccgcga ccggcacgta
3961 caacctccag gaaatcgtgc ccggcagcgt gtggatggag agggacgtgt acctccaagg
4021 acccatctgg gccaagatcc cagagacggg ggcgcacttt cacccctctc cggccatggg
4081 cggattcgga ctcaaacacc caccgcccat gatgctcatc aagaacacgc ctgtgcccgg
4141 aaatatcacc agcttctcgg acgtgcccgt cagcagcttc atcacccagt acagcaccgg
4201 gcaggtcacc gtggagatgg agtgggagct caagaaggaa aactccaaga ggtggaaccc
4261 agagatccag tacacaaaca actacaacga cccccagttt gtggactttg ccccggacag
4321 caccggggaa tacagaacca ccagacctat cggaacccga taccttaccc gacccctta
4381 acccattcat gtcgcatacc ctcaataaac cgtgtattcg tgtcagtaaa atactgcctc
4441 ttgtggtcat tcaatgaata acagcttaca acatctacaa aacctccttg cttgagagtg
4501 tggcactctc ccccctgtcg cgttcgctcg ctcgctggct cgtttggggg ggtggcagct
4561 caaagagctg ccagacgacg gccctctggc cgtcgccccc ccaaacgagc cagcgagcga
4621 gcgaacgcga cagggggag ag

AAV6 (SEQ ID NO:143)

1 ttggccactc cctctctgcg cgctcgctcg ctcactgagg ccgggcgacc aaaggtcgcc
61 cgacgcccgg gctttgcccg ggcggcctca gtgagcgagc gagcgcgcag agagggagtg
121 gccaactcca tcactagggg ttcctggagg ggtggagtcg tgacgtgaat tacgtcatag
181 ggttagggag gtcctgtatt agaggtcacg tgagtgtttt gcgacatttt gcgacaccat
241 gtggtcacgc tgggtattta agcccgagtg agcacgcagg gtctccattt tgaagcggga
301 ggtttgaacg cgcagcgcca tgccggggtt ttacgagatt gtgattaagg tccccagcga
361 ccttgacgag catctgcccg gcatttctga cagctttgtg aactgggtgg ccgagaagga
421 atgggagttg ccgccagatt ctgacatgga tctgaatctg attgagcagg caccoctgac
481 cgtggccgag aagctgcagc gcgacttcct ggtccagtgg cgccgcgtga gtaaggcccc
541 ggaggccctc ttctttgttc agttcgagaa gggcgagtcc tacttccacc tccatattct
601 ggtggagacc acgggggtca aatccatggt gctgggccgc ttcctgagtc agattaggga
661 caagctggtg cagaccatct accgcgggat cgagccgacc ctgcccaact ggttcgcggt
721 gaccaagacg cgtaatggcg ccggaggggg gaacaaggtg gtggacgagt gctacatccc
781 caactacctc ctgcccaaga ctcagcccga gctgcagtgg gcgtggacta acatggagga
841 gtatataagc gcgtgtttaa acctggccga gcgcaaacgg ctcgtggcgc acgacctgac
901 ccacgtcagc cagacccagg agcagaacaa ggagaatctg aaccccaatt ctgacgcgcc
961 tgtcatccgg tcaaaaacct ccgcacgcta catggagctg gtcgggtggc tggtggaccg
1021 gggcatcacc tccgagaagc agtggatcca ggaggaccag gcctcgtaca tctccttcaa
1081 cgccgcctcc aactcgcggt cccagatcaa ggccgctctg gacaatgccg gcaagatcat
1141 ggcgctgacc aaatccgcgc ccgactacct ggtaggcccc gctccgcccg ccgacattaa
1201 aaccaaccgc atttaccgca tcctggagct gaacggctac gaccctgcct acgccggctc
1261 cgtctttctc ggctgggccc agaaaaggtt cggaaaacgc aacaccatct ggctgtttgg
1321 gccggccacc acgggcaaga ccaacatcgc ggaagccatc gcccacgccg tgcccttcta
1381 cggctgcgtc aactggacca atgagaactt tcccttcaac gattgcgtcg acaagatggt
1441 gatctggtgg gaggagggca agatgacggc caaggtcgtg gagtccgcca aggccattct
1501 cggcggcagc aaggtgcgcg tggaccaaaa gtgcaagtcg tccgcccaga tcgatcccac
1561 ccccgtgatc gtcacctcca acaccaacat gtgcgccgtg attgacggga acagcaccac
1621 cttcgagcac cagcagccgt tgcaggaccg gatgttcaaa tttgaactca cccgccgtct
1681 ggagcatgac tttggcaagg tgacaaagca ggaagtcaaa gagttcttcc gctgggcgca
1741 ggatcacgtg accgaggtgg cgcatgagtt ctacgtcaga aagggtggag ccaacaagag
1801 acccgccccc gatgacgcgg ataaaagcga gcccaagcgg gcctgcccct cagtcgcgga
1861 tccatcgacg tcagacgcgg aaggagctcc ggtggacttt gccgacaggt accaaaacaa
1921 atgttctcgt cacgcgggca tgcttcagat gctgtttccc tgcaaaacat gcgagagaat
1981 gaatcagaat ttcaacattt gcttcacgca cgggaccaga gactgttcag aatgtttccc
2041 cggcgtgtca gaatctcaac cggtcgtcag aaagaggacg tatcggaaac tctgtgccat
2101 tcatcatctg ctggggcggg ctcccgagat tgcttgctcg gcctgcgatc tggtcaacgt
2161 ggatctggat gactgtgttt ctgagcaata aatgacttaa accaggtatg gctgccgatg
2221 gttatcttcc agattggctc gaggacaacc tctctgaggg cattcgcgag tggtgggact
2281 tgaaacctgg agccccgaaa cccaaagcca accagcaaaa gcaggacgac ggccggggtc
2341 tggtgcttcc tggctacaag tacctcggac ccttcaacgg actcgacaag ggggagcccg
2401 tcaacgcggc ggatgcagcg gccctcgagc acgacaaggc ctacgaccag cagctcaaag
2461 cgggtgacaa tccgtacctg cggtataacc acgccgacgc cgagtttcag gagcgtctgc
2521 aagaagatac gtcttttggg ggcaacctcg ggcgagcagt cttccaggcc aagaagaggg
2581 ttctcgaacc ttttggtctg gttgaggaag gtgctaagac ggctcctgga aagaaacgtc
2641 cggtagagca gtcgccacaa gagccagact cctcctcggg cattggcaag acaggccagc
2701 agcccgctaa aaagagactc aattttggtc agactggcga ctcagagtca gtccccgacc
2761 cacaacctct cggagaacct ccagcaaccc ccgctgctgt gggacctact acaatggctt
2821 caggcggtgg cgcaccaatg gcagacaata acgaaggcgc cgacggagtg ggtaatgcct

2881 caggaaattg gcattgcgat tccacatggc tgggcgacag agtcatcacc accagcaccc
2941 gaacatgggc cttgcccacc tataacaacc acctctacaa gcaaatctcc agtgcttcaa
3001 cgggggccag caacgacaac cactacttcg gctacagcac cccctggggg tattttgatt
3061 tcaacagatt ccactgccat ttctcaccac gtgactggca gcgactcatc aacaacaatt
3121 ggggattccg gcccaagaga ctcaacttca agctcttcaa catccaagtc aaggaggtca
3181 cgacgaatga tggcgtcacg accatcgcta ataaccttac cagcacggtt caagtcttct
3241 cggactcgga gtaccagttg ccgtacgtcc tcggctctgc gcaccagggc tgcctccctc
3301 cgttcccggc ggacgtgttc atgattccgc agtacggcta cctaacgctc aacaatggca
3361 gccaggcagt gggacggtca tccttttact gcctggaata tttcccatcg cagatgctga
3421 gaacgggcaa taactttacc ttcagctaca ccttcgagga cgtgcctttc cacagcagct
3481 acgcgcacag ccagagcctg gaccggctga tgaatcctct catcgaccag tacctgtatt
3541 acctgaacag aactcagaat cagtccggaa gtgcccaaaa caaggacttg ctgtttagcc
3601 gggggtctcc agctggcatg tctgttcagc ccaaaaactg gctacctgga ccctgttacc
3661 ggcagcagcg cgtttctaaa acaaaaacag acaacaacaa cagcaacttt acctggactg
3721 gtgcttcaaa atataacctt aatgggcgtg aatctataat caaccctggc actgctatgg
3781 cctcacacaa agacgacaaa gacaagttct ttcccatgag cggtgtcatg atttttggaa
3841 aggagagcgc cggagcttca aacactgcat tggacaatgt catgatcaca gacgaagagg
3901 aaatcaaagc cactaacccc gtggccaccg aaagatttgg gactgtggca gtcaatctcc
3961 agagcagcag cacagaccct gcgaccggag atgtgcatgt tatgggagcc ttacctggaa
4021 tggtgtggca agacagagac gtatacctgc agggtcctat ttgggccaaa attcctcaca
4081 cggatggaca ctttcacccg tctcctctca tgggcggctt tggacttaag cacccgcctc
4141 ctcagatcct catcaaaaac acgcctgttc ctgcgaatcc tccggcagag ttttcggcta
4201 caaagtttgc ttcattcatc acccagtatt ccacaggaca agtgagcgtg gagattgaat
4261 gggagctgca gaaagaaaac agcaaacgct ggaatcccga agtgcagtat acatctaact
4321 atgcaaaatc tgccaacgtt gatttcactg tggacaacaa tggactttat actgagcctc
4381 gccccattgg cacccgttac ctcacccgtc ccctgtaatt gtgtgttaat caataaaccg
4441 gttaattcgt gtcagttgaa ctttggtctc atgtcgttat tatcttatct ggtcaccata
4501 gcaaccggtt acacattaac tgcttagttg cgcttcgcga atacccctag tgatggagtt
4561 gcccactccc tctatgcgcg ctcgctcgct cggtggggcc ggcagagcag agctctgccg
4621 tctgcggacc tttggtccgc aggccccacc gagcgagcga gcgcgcatag agggagtggg
4681 caa

AAV7 (SEQ ID NO:144)

1 ttggccactc cctctatgcg cgctcgctcg ctcggtgggg cctgcggacc aaaggtccgc
61 agacggcaga gctctgctct gccggcccca ccgagcgagc gagcgcgcat agagggagtg
121 gccaactcca tcactagggg taccgcgaag cgcctcccac gctgccgcgt cagcgctgac
181 gtaaatcacg tcatagggga gtggtcctgt attagctgtc acgtgagtgc ttttgcgaca
241 ttttgcgaca ccacgtggcc atttgaggta tatatggccg agtgagcgag caggatctcc
301 attttgaccg cgaaatttga acgagcagca gccatgccgg gtttctacga gatcgtgatc
361 aaggtgccga gcgacctgga cgagcacctg ccgggcattt ctgactcgtt tgtgaactgg
421 gtggccgaga aggaatggga gctgcccccg gattctgaca tggatctgaa tctgatcgag
481 caggcacccc tgaccgtggc cgagaagctg cagcgcgact tcctggtcca atggcgccgc
541 gtgagtaagg ccccggaggc cctgttcttt gttcagttcg agaagggcga gagctacttc
601 caccttcacg ttctggtgga gaccacgggg gtcaagtcca tggtgctagg ccgcttcctg
661 agtcagattc gggagaagct ggtccagacc atctaccgcg gggtcgagcc cacgctgccc
721 aactggttcg cggtgaccaa gacgcgtaat ggcgccggcg gggggaacaa ggtggtggac
781 gagtgctaca tccccaacta cctcctgccc aagacccagc ccgagctgca gtgggcgtgg
841 actaacatgg aggagtatat aagcgcgtgt ttgaacctgg ccgaacgcaa acggctcgtg
901 gcgcagcacc tgacccacgt cagccagacg caggagcaga acaaggagaa tctgaacccc
961 aattctgacg cgcccgtgat caggtcaaaa acctccgcgc gctacatgga gctggtcggg
1021 tggctggtgg accggggcat cacctccgag aagcagtgga tccaggagga ccaggcctcg
1081 tacatctcct tcaacgccgc ctccaactcg cggtcccaga tcaaggccgc gctggacaat
1141 gccggcaaga tcatggcgct gaccaaatcc gcgcccgact acctggtggg gccctcgctg
1201 cccgcggaca ttaaaaccaa ccgcatctac cgcatcctgg agctgaacgg gtacgatcct
1261 gcctacgccg gctccgtctt tctcggctgg gcccagaaaa agttcgggaa gcgcaacacc
1321 atctggctgt ttgggcccgc caccaccggc aagaccaaca ttgcggaagc catcgcccac
1381 gccgtgccct tctacggctg cgtcaactgg accaatgaga actttccctt caacgattgc
1441 gtcgacaaga tggtgatctg gtgggaggag ggcaagatga cggccaaggt cgtggagtcc
1501 gccaaggcca ttctcggcgg cagcaaggtg cgcgtggacc aaaagtgcaa gtcgtccgcc
1561 cagatcgacc ccaccccgt gatcgtcacc tccaacacca acatgtgcgc cgtgattgac
1621 gggaacagca ccaccttcga gcaccagcag ccgttgcagg accggatgtt caaatttgaa
1681 ctcacccgcc gtctggagca cgactttggc aaggtgacga agcaggaagt caaagagttc
1741 ttccgctggg ccagtgatca cgtgaccgag gtggcgcatg agttctacgt cagaaagggc
1801 ggagccagca aaagacccgc ccccgatgac gcggatataa gcgagcccaa gcgggcctgc
1861 ccctcagtcg cggatccatc gacgtcagac gcggaaggag ctccggtgga ctttgccgac
1921 aggtaccaaa acaaatgttc tcgtcacgcg ggcatgattc agatgctgtt tccctgcaaa
1981 acgtgcgaga gaatgaatca gaatttcaac atttgcttca cacacggggt cagagactgt
2041 ttagagtgtt tccccggcgt gtcagaatct caaccggtcg tcagaaaaaa gacgtatcgg
2101 aaactctgcg cgattcatca tctgctgggg cgggcgcccg agattgcttg ctcggcctgc
2161 gacctggtca acgtggacct ggacgactgc gtttctgagc aataaatgac ttaaaccagg
2221 tatggctgcc gatggttatc ttccagattg gctcgaggac aacctctctg agggcattcg
2281 cgagtggtgg gacctgaaac ctggagcccc gaaacccaaa gccaaccagc aaaagcagga
2341 caacggccgg ggtctggtgc ttcctggcta caagtacctc ggacccttca acggactcga
2401 caaggggggag cccgtcaacg cggcggacgc agcggccctc gagcacgaca aggcctacga
2461 ccagcagctc aaagcgggtg acaatccgta cctgcggtat aaccacgccg acgccgagtt
2521 tcaggagcgt ctgcaagaag atacgtcatt tgggggcaac ctcgggcgag cagtcttcca
2581 ggccaagaag cgggttctcg aacctctcgg tctggttgag gaaggcgcta agacggctcc
2641 tgcaaagaag agaccggtag agccgtcacc tcagcgttcc cccgactcct ccacgggcat
2701 cggcaagaaa ggccagcagc ccgccagaaa gagactcaat ttcggtcaga ctggcgactc
2761 agagtcagtc cccgaccctc aacctctcgg agaacctcca gcagcgccct ctagtgtggg
2821 atctggtaca gtggctgcag gcggtggcgc accaatggca gacaataacg aaggtgccga

2881 cggagtgggt aatgcctcag gaaattggca ttgcgattcc acatggctgg gcgacagagt
2941 cattaccacc agcacccgaa cctgggccct gcccacctac aacaaccacc tctacaagca
3001 aatctccagt gaaactgcag gtagtaccaa cgacaacacc tacttcggct acagcacccc
3061 ctgggggtat tttgacttta acagattcca ctgccacttc tcaccacgtg actggcagcg
3121 actcatcaac aacaactggg gattccggcc caagaagctg cggttcaagc tcttcaacat
3181 ccaggtcaag gaggtcacga cgaatgacgg cgttacgacc atcgctaata accttaccag
3241 cacgattcag gtattctcgg actcggaata ccagctgccg tacgtcctcg gctctgcgca
3301 ccagggctgc ctgcctccgt tcccggcgga cgtcttcatg attcctcagt acggctacct
3361 gactctcaac aatggcagtc agtctgtggg acgttcctcc ttctactgcc tggagtactt
3421 cccctctcag atgctgagaa cgggcaacaa ctttgagttc agctacagct tcgaggacgt
3481 gcctttccac agcagctacg cacacagcca gagcctggac cggctgatga atcccctcat
3541 cgaccagtac ttgtactacc tggccagaac acagagtaac ccaggaggca cagctggcaa
3601 tcgggaactg cagttttacc agggcgggcc ttcaactatg gccgaacaag ccaagaattg
3661 gttacctgga ccttgcttcc ggcaacaaag agtctccaaa acgctggatc aaaacaacaa
3721 cagcaacttt gcttggactg gtgccaccaa atatcacctg aacggcagaa actcgttggt
3781 taatcccggc gtcgccatgg caactcacaa ggacgacgag gaccgctttt tcccatccag
3841 cggagtcctg atttttggaa aaactggagc aactaacaaa actacattgg aaaatgtgtt
3901 aatgacaaat gaagaagaaa ttcgtcctac taatcctgta gccacggaag aatacgggat
3961 agtcagcagc aacttacaag cggctaatac tgcagcccag acacaagttg tcaacaacca
4021 gggagcctta cctggcatgg tctggcagaa ccgggacgtg tacctgcagg gtcccatctg
4081 ggccaagatt cctcacacgg atggcaactt tcacccgtct cctttgatgg gcggctttgg
4141 acttaaacat ccgcctcctc agatcctgat caagaacact cccgttcccg ctaatcctcc
4201 ggaggtgttt actcctgcca agtttgcttc gttcatcaca cagtacagca ccggacaagt
4261 cagcgtggaa atcgagtggg agctgcagaa ggaaaacagc aagcgctgga acccggagat
4321 tcagtacacc tccaactttg aaaagcagac tggtgtggac tttgccgttg acagccaggg
4381 tgtttactct gagcctcgcc ctattggcac tcgttacctc acccgtaatc tgtaattgca
4441 tgttaatcaa taaaccggtt gattcgtttc agttgaactt tggtctcctg tgcttcttat
4501 cttatcggtt tccatagcaa ctggttacac attaactgct tgggtgcgct tcacgataag
4561 aacactgacg tcaccgcggt acccctagtg atggagttgg ccactccctc tatgcgcgct
4621 cgctcgctcg gtggggcctg cggaccaaag gtccgcagac ggcagagctc tgctctgccg
4681 gccccaccga gcgagcgagc gcgcatagag ggagtggcca a

AAV8 (SEQ ID NO:145)

1 cagagaggga gtggccaact ccatcactag gggtagcgcg aagcgcctcc cacgctgccg
61 cgtcagcgct gacgtaaatt acgtcatagg ggagtggtcc tgtattagct gtcacgtgag
121 tgcttttgcg gcattttgcg acaccacgtg gccatttgag gtatatatgg ccgagtgagc
181 gagcaggatc tccattttga ccgcgaaatt tgaacgagca gcagccatgc cgggcttcta
241 cgagatcgtg atcaaggtgc cgagcgacct ggacgagcac ctgccgggca tttctgactc
301 gtttgtgaac tgggtggccg agaaggaatg ggagctgccc ccggattctg acatggatcg
361 gaatctgatc gagcaggcac ccctgaccgt ggccgagaag ctgcagcgcg acttcctggt
421 ccaatggcgc cgcgtgagta aggccccgga ggccctcttc tttgttcagt tcgagaaggg
481 cgagagctac tttcacctgc acgttctggt cgagaccacg gggtcaagt ccatggtgct
541 aggccgcttc ctgagtcaga ttcgggaaaa gcttggtcca gaccatctac ccgcggggtc
601 gagccccacc ttgcccaact ggttcgcggt gaccaaagac gcggtaatgg cgccggcggg
661 ggggaacaag gtggtggacg agtgctacat ccccaactac ctcctgccca agactcagcc
721 cgagctgcag tgggcgtgga ctaacatgga ggagtatata agcgcgtgct tgaacctggc
781 cgagcgcaaa cggctcgtgg cgcagcacct gacccacgtc agccagacgc aggagcagaa
841 caaggagaat ctgaacccca attctgacgc gcccgtgatc aggtcaaaaa cctccgcgcg
901 ctatatggag ctggtcgggt ggctggtgga ccggggcatc acctccgaga agcagtggat
961 ccaggaggac caggcctcgt acatctcctt caacgccgcc tccaactcgc ggtcccagat
1021 caaggccgcg ctggacaatg ccggcaagat catggcgctg accaaatccg cgcccgacta
1081 cctggtgggg ccctcgctgc ccgcggacat tacccagaac cgcatctacc gcatcctcgc
1141 tctcaacggc tacgaccctg cctacgccgg ctccgtcttt ctcggctggg ctcagaaaaa
1201 gttcgggaaa cgcaacacca tctggctgtt tggacccgcc accaccggca agaccaacat
1261 tgcggaagcc atcgcccacg ccgtgccctt ctacggctgc gtcaactgga ccaatgagaa
1321 ctttcccttc aatgattgcg tcgacaagat ggtgatctgg tgggaggagg gcaagatgac
1381 ggccaaggtc gtggagtccg ccaaggccat tctcggcggc agcaaggtgc gcgtggacca
1441 aaagtgcaag tcgtccgccc agatcgaccc cacccccgtg atcgtcacct ccaacaccaa
1501 catgtgcgcc gtgattgacg ggaacagcac caccttcgag caccagcagc ctctccagga
1561 ccggatgttt aagttcgaac tcacccgccg tctggagcac gactttggca aggtgacaaa
1621 gcaggaagtc aaagagttct tccgctgggc cagtgatcac gtgaccgagg tggcgcatga
1681 gttttacgtc agaaagggcg gagccagcaa aagacccgcc cccgatgacg cggataaaag
1741 cgagcccaag cgggcctgcc cctcagtcgc ggatccatcg acgtcagacg cggaaggagc
1801 tccggtggac tttgccgaca ggtaccaaaa caaatgttct cgtcacgcgg gcatgcttca
1861 gatgctgttt ccctgcaaaa cgtgcgagag aatgaatcag aatttcaaca tttgcttcac
1921 acacggggtc agagactgct cagagtgttt ccccggcgtg tcagaatctc aaccggtcgt
1981 cagaaagagg acgtatcgga aactctgtgc gattcatcat ctgctggggc gggctcccga
2041 gattgcttgc tcggcctgcg atctggtcaa cgtggacctg gatgactgtg tttctgagca
2101 ataaatgact taaaccaggt atggctgccg atggttatct tccagattgg ctcgaggaca
2161 acctctctga gggcattcgc gagtggtggg cgctgaaacc tggagccccg aagcccaaag
2221 ccaaccagca aaagcaggac gacggccggg gtctggtgct tcctggctac aagtacctcg
2281 gacccttcaa cggactcgac aagggggagc ccgtcaacgc ggcggacgca gcggccctcg
2341 agcacgacaa ggcctacgac cagcagctgc aggcgggtga caatccgtac ctgcggtata
2401 accacgccga cgccgagttt caggagcgtc tgcaagaaga tacgtctttt gggggcaacc
2461 tcgggcgagc agtcttccag gccaagaagc gggttctcga acctctcggt ctggttgagg
2521 aaggcgctaa gacggctcct ggaaagaaga gaccggtaga gccatcaccc cagcgttctc
2581 cagactcctc tacgggcatc ggcaagaaag gccaacagcc cgccagaaaa agactcaatt
2641 ttggtcagac tggcgactca gagtcagttc cagaccctca acctctcgga gaacctccag
2701 cagcgccctc tggtgtggga cctaatacaa tggctgcagg cggtggcgca ccaatggcag
2761 acaataacga aggcgccgac ggagtgggta gttcctcggg aaattggcat tgcgattcca
2821 catggctggg cgacagagtc atcaccacca gcacccgaac ctgggccctg cccacctaca

2881 acaaccacct ctacaagcaa atctccaacg ggacatcggg aggagccacc aacgacaaca
2941 cctacttcgg ctacagcacc ccctgggggt attttgactt taacagattc cactgccact
3001 tttcaccacg tgactggcag cgactcatca acaacaactg gggattccgg cccaagagac
3061 tcagcttcaa gctcttcaac atccaggtca aggaggtcac gcagaatgaa ggcaccaaga
3121 ccatcgccaa taacctcacc agcaccatcc aggtgtttac ggactcggag taccagctgc
3181 cgtacgttct cggctctgcc caccagggct gcctgcctcc gttcccggcg gacgtgttca
3241 tgattcccca gtacggctac ctaacactca acaacggtag tcaggccgtg ggacgctcct
3301 ccttctactg cctggaatac tttccttcgc agatgctgag aaccggcaac aacttccagt
3361 ttacttacac cttcgaggac gtgcctttcc acagcagcta cgcccacagc cagagcttgg
3421 accggctgat gaatcctctg attgaccagt acctgtacta cttgtctcgg actcaaacaa
3481 caggaggcac ggcaaatacg cagactctgg gcttcagcca aggtgggcct aatacaatgg
3541 ccaatcaggc aaagaactgg ctgccaggac cctgttaccg ccaacaacgc gtctcaacga
3601 caaccgggca aaacaacaat agcaactttg cctggactgc tgggaccaaa taccatctga
3661 atggaagaaa ttcattggct aatcctggca tcgctatggc aacacacaaa gacgacgagg
3721 agcgtttttt tcccagtaac gggatcctga tttttggcaa acaaaatgct gccagagaca
3781 atgcggatta cagcgatgtc atgctcacca gcgaggaaga aatcaaaacc actaaccctg
3841 tggctacaga ggaatacggt atcgtggcag ataacttgca gcagcaaaac acggctcctc
3901 aaattggaac tgtcaacagc caggggggcct tacccggtat ggtctggcag aaccgggacg
3961 tgtacctgca gggtcccatc tgggccaaga ttcctcacac ggacggcaac ttccacccgt
4021 ctccgctgat gggcggcttt ggcctgaaac atcctccgcc tcagatcctg atcaagaaca
4081 cgcctgtacc tgcggatcct ccgaccacct tcaaccagtc aaagctgaac tctttcatca
4141 cgcaatacag caccggacag gtcagcgtgg aaattgaatg ggagctgcag aaggaaaaca
4201 gcaagcgctg gaaccccgag atccagtaca cctccaacta ctacaaatct acaagtgtgg
4261 actttgctgt taatacagaa ggcgtgtact ctgaaccccg ccccattggc acccgttacc
4321 tcacccgtaa tctgtaattg cctgttaatc aataaaccgg ttgattcgtt tcagttgaac
4381 tttggtctct gcg

AAV9 (SEQ ID NO:146)

1 gcccaatacg caaaccgcct ctccccgcgc gttggccgat tcattaatgc agctggcgta
61 atagcgaaga ggcccgcacc gatcgccctt cccaacagtt gcgcagcctg aatggcgaat
121 ggcgattccg ttgcaatggc tggcggtaat attgttctgg atattaccag caaggccgat
181 agtttgagtt cttctactca ggcaagtgat gttattacta atcaaagaag tattgcgaca
241 acggttaatt tgcgtgatgg acagactctt ttactcggtg gcctcactga ttataaaaac
301 acttctcagg attctggcgt accgttcctg tctaaaatcc ctttaatcgg cctcctgttt
361 agctcccgct ctgattctaa cgaggaaagc acgttatacg tgctcgtcaa agcaaccata
421 gtacgcgccc tgtagcggcg cattaagcgc ggcgggtgtg gtggttacgc gcagcgtgac
481 cgctacactt gccagcgccc tagcgcccgc tcctttcgct ttcttccctt cctttctcgc
541 cacgttcgcc ggctttcccc gtcaagctct aaatcggggg ctccctttag ggttccgatt
601 tagtgcttta cggcacctcg accccaaaaa acttgattag ggtgatggtt cacgtagtgg
661 gccatcgccc tgatagacgg tttttcgccc tttgacgttg gagtccacgt tctttaatag
721 tggactcttg ttccaaactg gaacaacact caaccctatc tcggtctatt cttttgattt
781 ataagggatt ttgccgattt cggcctattg gttaaaaaat gagctgattt aacaaaaatt
841 taacgcgaat tttaacaaaa tattaacgct tacaatttaa atatttgctt atacaatctt
901 cctgtttttg gggcttttct gattatcaac cggggtacat atgattgaca tgctagtttt
961 acgattaccg ttcatcgccc tgcgcgctcg ctcgctcact gaggccgccc gggcaaagcc
1021 cgggcgtcgg gcgacctttg gtcgcccggc ctcagtgagc gagcgagcgc gcagagaggg
1081 agtggaattc acgcgtggat ctgaattcaa ttcacgcgtg gtacctctgg tcgttacata
1141 acttacggta aatggcccgc ctggctgacc gcccaacgac ccccgcccat tgacgtcaat
1201 aatgacgtat gttcccatag taacgccaat agggactttc cattgacgtc aatgggtgga
1261 gtatttacgg taaactgccc acttggcagt acatcaagtg tatcatatgc caagtacgcc
1321 ccctattgac gtcaatgacg gtaaatggcc cgcctggcat tatgcccagt acatgacctt
1381 atgggacttt cctacttggc agtacatcta ctcgaggcca cgttctgctt cactctcccc
1441 atctcccccc cctccccacc cccaattttg tatttattta ttttttaatt attttgtgca
1501 gcgatggggg cggggggggg gggggggcgc gcgccaggcg gggcggggcg gggcgagggg
1561 cggggcgggg cgaggcggag aggtgcggcg gcagccaatc agagcggcgc gctccgaaag
1621 tttcctttta tggcgaggcg gcggcggcgg cggccctata aaaagcgaag cgcgcggcgg
1681 gcgggagcgg gatcagccac cgcggtggcg gcctagagtc gacgaggaac tgaaaaacca
1741 gaaagttaac tggtaagttt agtctttttg tcttttattt caggtcccgg atccggtggt
1801 ggtgcaaatc aaagaactgc tcctcagtgg atgttgcctt tacttctagg cctgtacgga
1861 agtgttactt ctgctctaaa agctgcggaa ttgtacccgc ggccgatcca ccggtccgga
1921 attcccggga tatcgtcgac ccacgcgtcc gggcccccacg ctgcgcaccc gcgggtttgc
1981 tatggcgatg agcagcggcg gcagtggtgg cggcgtcccg gagcaggagg attccgtgct
2041 gttccggcgc ggcacaggcc agagcgatga ttctgacatt tgggatgata cagcactgat
2101 aaaagcatat gataaagctg tggcttcatt taagcatgct ctaaagaatg gtgacatttg
2161 tgaaacttcg ggtaaaccaa aaaccacacc taaaagaaaa cctgctaaga agaataaaag
2221 ccaaaagaag aatactgcag cttccttaca acagtggaaa gttggggaca aatgttctgc
2281 catttggtca gaagacggtt gcatttaccc agctaccatt gcttcaattg attttaagag
2341 agaaacctgt gttgtggttt acactggata tggaaataga gaggagcaaa atctgtccga
2401 tctactttcc ccaatctgtg aagtagctaa taatatagaa cagaatgctc aagagaatga
2461 aaatgaaagc caagtttcaa cagatgaaag tgagaactcc aggtctcctg gaaataaatc
2521 agataacatc aagcccaaat ctgctccatg gaactctttt ctccctccac cacccccat
2581 gccagggcca agactgggac caggaaagcc aggtctaaaa ttcaatggcc caccaccgcc
2641 accgccacca ccaccacccc acttactatc atgctggctg cctccatttc cttctggacc
2701 accaataatt cccccaccac ctcccatatg tccagattct cttgatgatg ctgatgcttt
2761 gggaagtatg ttaatttcat ggtacatgag tggctatcat actggctatt atatgggttt
2821 tagacaaaat caaaaagaag gaaggtgctc acattcctta aattaaggag aaatgctggc

2881 atagagcagc actaaatgac accactaaag aaacgatcag acagatctag aaagcttatc
2941 gataccgtcg actagagctc gctgatcagc ctcgactgtg ccttctagtt gccagccatc
3001 tgttgtttgc ccctcccccg tgccttcctt gaccctggaa ggtgccactc ccactgtcct
3061 ttcctaataa aatgaggaaa ttgcatcgca ttgtctgagt aggtgtcatt ctattctggg
3121 gggtggggtg gggcaggaca gcaaggggga ggattgggaa gacaatagca ggcatgctgg
3181 ggagagatcg atctgaggaa cccctagtga tggagttggc cactccctct ctgcgcgctc
3241 gctcgctcac tgaggccggg cgaccaaagg tcgcccgacg cccgggcttt gcccgggcgg
3301 cctcagtgag cgagcgagcg cgcagagagg gagtggcccc cccccccccc cccccggcga
3361 ttctcttgtt tgctccagac tctcaggcaa tgacctgata gcctttgtag agacctctca
3421 aaaatagcta ccctctccgg catgaattta tcagctagaa cggttgaata tcatattgat
3481 ggtgatttga ctgtctccgg cctttctcac ccgtttgaat ctttacctac acattactca
3541 ggcattgcat ttaaaatata tgagggttct aaaaattttt atccttgcgt tgaaataaag
3601 gcttctcccg caaaagtatt acagggtcat aatgtttttg gtacaaccga tttagcttta
3661 tgctctgagg ctttattgct taattttgct aattctttgc cttgcctgta tgatttattg
3721 gatgttggaa tcgcctgatg cggtattttc tccttacgca tctgtgcggt atttcacacc
3781 gcatatggtg cactctcagt acaatctgct ctgatgccgc atagttaagc cagccccgac
3841 acccgccaac actatggtgc actctcagta caatctgctc tgatgccgca tagttaagcc
3901 agccccgaca cccgccaaca cccgctgacg cgccctgacg ggcttgtctg ctcccggcat
3961 ccgcttacag acaagctgtg accgtctccg ggagctgcat gtgtcagagg ttttcaccgt
4021 catcaccgaa acgcgcgaga cgaaagggcc tcgtgatacg cctattttta taggttaatg
4081 tcatgataat aatggtttct tagacgtcag gtggcacttt tcggggaaat gtgcgcggaa
4141 cccctatttg tttatttttc taaatacatt caaatatgta tccgctcatg agacaataac
4201 cctgataaat gcttcaataa tattgaaaaa ggaagagtat gagtattcaa catttccgtg
4261 tcgcccttat tccctttttt gcggcatttt gccttcctgt ttttgctcac ccagaaacgc
4321 tggtgaaagt aaaagatgct gaagatcagt tgggtgcacg agtgggttac atcgaactgg
4381 atctcaacag cggtaagatc cttgagagtt ttcgccccga agaacgtttt ccaatgatga
4441 gcacttttaa agttctgcta tgtggcgcgg tattatcccg tattgacgcc gggcaagagc
4501 aactcggtcg ccgcatacac tattctcaga atgacttggt tgagtactca ccagtcacag
4561 aaaagcatct tacggatggc atgacagtaa gagaattatg cagtgctgcc ataaccatga
4621 gtgataacac tgcggccaac ttacttctga caacgatcgg aggaccgaag gagctaaccg
4681 cttttttgca caacatgggg gatcatgtaa ctcgccttga tcgttgggaa ccggagctga
4741 atgaagccat accaaacgac gagcgtgaca ccacgatgcc tgtagcaatg gcaacaacgt
4801 tgcgcaaact attaactggc gaactactta ctctagcttc ccggcaacaa ttaatagact
4861 ggatggaggc ggataaagtt gcaggaccac ttctgcgctc ggcccttccg gctggctggt
4921 ttattgctga taaatctgga gccggtgagc gtgggtctcg cggtatcatt gcagcactgg
4981 ggccagatgg taagccctcc cgtatcgtag ttatctacac gacggggagt caggcaacta
5041 tggatgaacg aaatagacag atcgctgaga taggtgcctc actgattaag cattggtaac
5101 tgtcagacca agtttactca tatatacttt agattgattt aaaacttcat ttttaattta
5161 aaaggatcta ggtgaagatc ctttttgata atctcatgac caaaatccct taacgtgagt
5221 tttcgttcca ctgagcgtca gaccccgtag aaaagatcaa aggatcttct tgagatcctt
5281 tttttctgcg cgtaatctgc tgcttgcaaa caaaaaaacc accgctacca gcggtggttt
5341 gtttgccgga tcaagagcta ccaactcttt ttccgaaggt aactggcttc agcagagcgc
5401 agataccaaa tactgttctt ctagtgtagc cgtagttagg ccaccacttc aagaactctg
5461 tagcaccgcc tacatacctc gctctgctaa tcctgttacc agtggctgct gccagtggcg
5521 ataagtcgtg tcttaccggg ttggactcaa gacgatagtt accggataag gcgcagcggt
5581 cgggctgaac ggggggttcg tgcacacagc ccagcttgga gcgaacgacc tacaccgaac
5641 tgagatacct acagcgtgag ctatgagaaa gcgccacgct tcccgaaggg agaaaggcgg
5701 acaggtatcc ggtaagcggc agggtcggaa caggagagcg cacgagggag cttccagggg
5761 gaaacgcctg gtatctttat agtcctgtcg ggtttcgcca cctctgactt gagcgtcgat
5821 tttgtgatg ctcgtcaggg gggcggagcc tatggaaaaa cgccagcaac gcggcctttt

5881 tacggttcct ggccttttgc tggccttttg ctcacatgtt ctttcctgcg ttatcccctg
5941 attctgtgga taaccgtatt accgcctttg agtgagctga taccgctcgc cgcagccgaa
6001 cgaccgagcg cagcgagtca gtgagcgagg aagcggaaga gc

AAV10 (SEQ ID NO:147)

1 atgccgggct tctacgagat cgtgatcaag gtgccgagcg acctggacga gcacctgccg
61 ggcatttctg actcgtttgt gaactgggtg gccgagaagg aatgggagct gcccccggat
121 tctgacatgg atcggaatct gatcgagcag gcacccctga ccgtggccga gaagctgcag
181 cgcgacttcc tggtccactg gcgccgcgtg agtaaggccc cggaggccct cttctttgtt
241 cagttcgaga agggcgagtc ctactttcac ctgcacgttc tggtcgagac cacgggggtc
301 aagtccatgg tcctgggccg cttcctgagt cagatcagag acaggctggt gcagaccatc
361 taccgcgggg tagagcccac gctgcccaac tggttcgcgg tgaccaagac gcgaaatggc
421 gccggcgggg ggaacaaggt ggtggacgag tgctacatcc ccaactacct cctgcccaag
481 acgcagcccg agctgcagtg ggcgtggact aacatggagg agtatataag cgcgtgtctg
541 aacctcgcgg agcgtaaacg gctcgtggcg cagcacctga cccacgtcag ccagacgcag
601 gagcagaaca aggagaatct gaacccgaat tctgacgcgc ccgtgatcag gtcaaaaacc
661 tccgcgcgct acatggagct ggtcgggtgg ctggtggacc ggggcatcac ctccgagaag
721 cagtggatcc aggaggacca ggcctcgtac atctccttca acgccgcctc caactcgcgg
781 tcccagatca aggccgcgct ggacaatgcc ggaaagatca tggcgctgac caaatccgcg
841 cccgactacc tggtaggccc gtccttaccc gcggacatta aggccaaccg catctaccgc
901 atcctggagc tcaacggcta cgacccegcc tacgccggct ccgtcttcct gggctgggcg
961 cagaaaaagt tcggtaaaag gaatacaatt tggctgttcg ggcccgccac caccggcaag
1021 accaacatcg cggaagccat cgcccacgcc gtgcccttct acggctgcgt caactggacc
1081 aatgagaact ttcccttcaa cgattgcgtc gacaagatgg tgatctggtg ggaggagggc
1141 aagatgaccg ccaaggtcgt ggagtccgcc aaggccattc tgggcggaag caaggtgcgc
1201 gtcgaccaaa agtgcaagtc ctcggcccag atcgacccca cgcccgtgat cgtcacctcc
1261 aacaccaaca tgtgcgccgt gatcgacggg aacagcacca ccttcgagca ccagcagccc
1321 ctgcaggacc gcatgttcaa gttcgagctc acccgccgtc tggagcacga ctttggcaag
1381 gtgaccaagc aggaagtcaa agagttcttc cgctgggctc aggatcacgt gactgaggtg
1441 acgcatgagt tctacgtcag aaagggcgga gccaccaaaa gacccgcccc cagtgacgcg
1501 gatataagcg agcccaagcg ggcctgcccc tcagttgcgg agccatcgac gtcagacgcg
1561 gaagcaccgg tggactttgc ggacaggtac caaaacaaat gttctcgtca cgcgggcatg
1621 cttcagatgc tgtttccctg caagacatgc gagagaatga atcagaattt caacgtctgc
1681 ttcacgcacg ggtcagaga ctgctcagag tgcttccccg gcgcgtcaga atctcaacct
1741 gtcgtcagaa aaaagacgta tcagaaactg tgcgcgattc atcatctgct ggggcgggca
1801 cccgagattg cgtgttcggc ctgcgatctc gtcaacgtgg acttggatga ctgtgtttct
1861 gagcaataaa tgacttaaac caggtatggc tgctgacggt tatcttccag attggctcga
1921 ggacaacctc tctgagggca ttcgcgagtg gtgggacctg aaacctggag cccccaagcc
1981 caaggccaac cagcagaagc aggacgacgg ccggggtctg gtgcttcctg gctacaagta
2041 cctcggaccc ttcaacggac tcgacaaggg ggagcccgtc aacgcggcgg acgcagcggc
2101 cctcgagcac gacaaggcct acgaccagca gctcaaagcg ggtgacaatc cgtacctgcg
2161 gtataaccac gccgacgccg agtttcagga gcgtctgcaa gaagatacgt cttttggggg
2221 caacctcggg cgagcagtct tccaggccaa gaagcgggtt ctcgaacctc tcggtctggt
2281 tgaggaagct gctaagacgg ctcctggaaa gaagagaccg gtagaaccgt cacctcagcg
2341 ttcccccgac tcctccacgg gcatcggcaa gaaaggccag cagcccgcta aaaagagact
2401 gaactttggg cagactggcg agtcagagtc agtccccgac cctcaaccaa tcggagaacc
2461 accagcaggc ccctctggtc tgggatctgg tacaatggct gcaggcggtg gcgctccaat
2521 ggcagacaat aacgaaggcg ccgacggagt gggtagttcc tcaggaaatt ggcattgcga

2581 ttccacatgg ctgggcgaca gagtcatcac caccagcacc cgaacctggg ccctgcccac
2641 ctacaacaac cacctctaca agcaaatctc caacgggaca tcgggaggaa gcaccaacga
2701 caacacctac ttcggctaca gcacccccctg ggggtatttt gacttcaaca gattccactg
2761 ccacttctca ccacgtgact ggcagcgact catcaacaac aactggggat tccggccaaa
2821 aagactcagc ttcaagctct tcaacatcca ggtcaaggag gtcacgcaga atgaaggcac
2881 caagaccatc gccaataacc ttaccagcac gattcaggta tttacggact cggaatacca
2941 gctgccgtac gtcctcggct ccgcgcacca gggctgcctg cctccgttcc cggcggatgt
3001 cttcatgatt ccccagtacg gctacctgac actgaacaat ggaagtcaag ccgtaggccg
3061 ttcctccttc tactgcctgg aatattttcc atctcaaatg ctgcgaactg gaaacaattt
3121 tgaattcagc tacaccttcg aggacgtgcc tttccacagc agctacgcac acagccagag
3181 cttggaccga ctgatgaatc ctctcattga ccagtacctg tactacttat ccagaactca
3241 gtccacagga ggaactcaag gtacccagca attgttattt tctcaagctg ggcctgcaaa
3301 catgtcggct caggccaaga actggctgcc tggaccttgc taccggcagc agcgagtctc
3361 cacgacactg tcgcaaaaca acaacagcaa ctttgcttgg actggtgcca ccaaatatca
3421 cctgaacgga agagactctc tggtgaatcc cggtgtcgcc atggcaaccc acaaggacga
3481 cgaggaacgc ttcttcccgt cgagcggagt cctgatgttt ggaaaacagg gtgctggaag
3541 agacaatgtg gactacagca gcgttatgct aacaagcgaa gaagaaatta aaaccactaa
3601 ccctgtagcc acagaacaat acggcgtggt ggctgacaac ttgcagcaag ccaatacagg
3661 gcctattgtg ggaaatgtca acagccaagg agccttacct ggcatggtct ggcagaaccg
3721 agacgtgtac ctgcagggtc ccatctgggc caagattcct cacacggacg gcaactttca
3781 cccgtctcct ctgatgggcg gctttggact taaacacccg cctccacaga tcctgatcaa
3841 gaacacgccg gtacctgcgg atcctccaac aacgttcagc caggcgaaat tggcttcctt
3901 catcacgcag tacagcaccg gacaggtcag cgtggaaatc gagtgggagc tgcagaagga
3961 gaacagcaaa cgctggaacc cagagattca gtacacttca aactactaca aatctacaaa
4021 tgtggacttt gctgtcaata cagagggaac ttattctgag cctcgcccca ttggtactcg
4081 ttatctgaca cgtaatctgt aa

AAV11 (SEQ ID NO:148)

1 atgccgggct tctacgagat cgtgatcaag gtgccgagcg acctggacga gcacctgccg
61 ggcatttctg actcgtttgt gaactgggtg gccgagaagg aatgggagct gcccccggat
121 tctgacatgg atcggaatct gatcgagcag gcacccctga ccgtggccga gaagctgcag
181 cgcgacttcc tggtccactg gcgccgcgtg agtaaggccc cggaggccct cttctttgtt
241 cagttcgaga agggcgagtc ctacttccac ctccacgttc tcgtcgagac cacgggggtc
301 aagtccatgg tcctgggccg cttcctgagt cagatcagag acaggctggt gcagaccatc
361 taccgcgggg tcgagcccac gctgcccaac tggttcgcgg tgaccaagac gcgaaatggc
421 gccggcgggg ggaacaaggt ggtggacgag tgctacatcc ccaactacct cctgcccaag
481 acccagcccg agctgcagtg ggcgtggact aacatggagg agtatataag cgcgtgtcta
541 aacctcgcgg agcgtaaacg gctcgtggcg cagcacctga cccacgtcag ccagacgcag
601 gagcagaaca aggagaatct gaacccgaat tctgacgcgc ccgtgatcag gtcaaaaacc
661 tccgcgcgct acatggagct ggtcgggtgg ctggtggacc ggggcatcac ctccgagaag
721 cagtggatcc aggaggacca ggcctcgtac atctccttca acgccgcctc caactcgcgg
781 tcccagatca aggccgcgct ggacaatgcc ggaaagatca tggcgctgac caaatccgcg
841 cccgactacc tggtaggccc gtccttaccc gcggacatta aggccaaccg catctaccgc
901 atcctggagc tcaacggcta cgacccccgcc tacgccggct ccgtcttcct gggctgggcg
961 cagaaaaagt tcggtaaacg caacaccatc tggctgtttg ggcccgccac caccggcaag
1021 accaacatcg cggaagccat agcccacgcc gtgcccttct acggctgcgt gaactggacc
1081 aatgagaact ttcccttcaa cgattgcgtc gacaagatgg tgatctggtg ggaggagggc
1141 aagatgaccg ccaaggtcgt ggagtccgcc aaggccattc tgggcggaag caaggtgcgc
1201 gtggaccaaa agtgcaagtc ctcggcccag atcgacccca cgcccgtgat cgtcacctcc
1261 aacaccaaca tgtgcgccgt gatcgacggg aacagcacca ccttcgagca ccagcagccg
1321 ctgcaggacc gcatgttcaa gttcgagctc acccgccgtc tggagcacga ctttggcaag
1381 gtgaccaagc aggaagtcaa agagttcttc cgctgggctc aggatcacgt gactgaggtg
1441 gcgcatgagt tctacgtcag aaagggcgga gccaccaaaa gacccgcccc cagtgacgcg
1501 gatataagcg agcccaagcg ggcctgcccc tcagttccgg agccatcgac gtcagacgcg
1561 gaagcaccgg tggactttgc ggacaggtac caaaacaaat gttctcgtca cgcgggcatg
1621 cttcagatgc tgtttccctg caagacatgc gagagaatga atcagaattt caacgtctgc
1681 ttcacgcacg ggtcagaga ctgctcagag tgcttccccg gcgcgtcaga atctcaaccc
1741 gtcgtcagaa aaaagacgta tcagaaactg tgcgcgattc atcatctgct ggggcgggca
1801 cccgagattg cgtgttcggc ctgcgatctc gtcaacgtgg acttggatga ctgtgtttct
1861 gagcaataaa tgacttaaac caggtatggc tgctgacggt tatcttccag attggctcga
1921 ggacaacctc tctgagggca ttcgcgagtg gtgggacctg aaacctggag ccccgaagcc
1981 caaggccaac cagcagaagc aggacgacgg ccggggtctg gtgcttcctg gctacaagta
2041 cctcggaccc ttcaacggac tcgacaaggg ggagcccgtc aacgcggcgg acgcagcggc
2101 cctcgagcac gacaaggcct acgaccagca gctcaaagcg ggtgacaatc cgtacctgcg
2161 gtataaccac gccgacgccg agtttcagga gcgtctgcaa gaagatacgt cttttggggg
2221 caacctcggg cgagcagtct tccaggccaa gaagagggta ctcgaacctc tgggcctggt
2281 tgaagaaggt gctaaaacgg ctcctggaaa gaagagaccg ttagagtcac cacaagagcc
2341 cgactcctcc tcgggcatcg gcaaaaaagg caaacaacca gccagaaaga ggctcaactt
2401 tgaagaggac actggagccg gagacggacc ccctgaagga tcagatacca gcgccatgtc
2461 ttcagacatt gaaatgcgtg cagcaccggg cggaaatgct gtcgatgcgg gacaaggttc
2521 cgatggagtg ggtaatgcct cgggtgattg gcattgcgat tccacctggt ctgagggcaa
2581 ggtcacaaca acctcgacca gaacctgggt cttgcccacc tacaacaacc acttgtacct
2641 gcgtctcgga acaacatcaa gcagcaacac ctacaacgga ttctccaccc cctggggata
2701 ttttgacttc aacagattcc actgtcactt ctcaccacgt gactggcaaa gactcatcaa
2761 caacaactgg ggactacgac caaaagccat gcgcgttaaa atcttcaata tccaagttaa
2821 ggaggtcaca acgtcgaacg gcgagactac ggtcgctaat aaccttacca gcacggttca

2881 gatatttgcg gactcgtcgt atgagctccc gtacgtgatg gacgctggac aagaggggag
2941 cctgcctcct ttccccaatg acgtgttcat ggtgcctcaa tatggctact gtggcatcgt
3001 gactggcgag aatcagaacc aaacggacag aaacgctttc tactgcctgg agtattttcc
3061 ttcgcaaatg ttgagaactg gcaacaactt tgaaatggct tacaactttg agaaggtgcc
3121 gttccactca atgtatgctc acagccagag cctggacaga ctgatgaatc ccctcctgga
3181 ccagtacctg tggcacttac agtcgactac ctctggagag actctgaatc aaggcaatgc
3241 agcaaccaca tttggaaaaa tcaggagtgg agactttgcc ttttacagaa agaactggct
3301 gcctgggcct tgtgttaaac agcagagatt ctcaaaaact gccagtcaaa attacaagat
3361 tcctgccagc gggggcaacg ctctgttaaa gtatgacacc cactatacct taaacaaccg
3421 ctggagcaac atcgcgcccg gacctccaat ggccacagcc ggaccttcgg atggggactt
3481 cagtaacgcc cagcttatat tccctggacc atctgttacc ggaaatacaa caacttcagc
3541 caacaatctg ttgtttacat cagaagaaga aattgctgcc accaacccaa gagacacgga
3601 catgtttggc cagattgctg acaataatca gaatgctaca actgctccca taaccggcaa
3661 cgtgactgct atgggagtgc tgcctggcat ggtgtggcaa aacagagaca tttactacca
3721 agggccaatt tgggccaaga tcccacacgc ggacggacat tttcatcctt caccgctgat
3781 tggtgggttt ggactgaaac acccgcctcc ccagatattc atcaagaaca ctcccgtacc
3841 tgccaatcct gcgacaacct tcactgcagc cagagtggac tctttcatca cacaatacag
3901 caccggccag gtcgctgttc agattgaatg ggaaattgaa aaggaacgct ccaaacgctg
3961 gaatcctgaa gtgcagttta cttcaaacta tgggaaccag tcttctatgt tgtgggctcc
4021 tgatacaact gggaagtata cagagccgcg ggttattggc tctcgttatt tgactaatca
4081 tttgtaa

AAV12 (SEQ ID NO:149)

1 ttgcgacagt ttgcgacacc atgtggtcac aagaggtata taaccgcgag tgagccagcg
61 aggagctcca ttttgcccgc gaagtttgaa cgagcagcag ccatgccggg gttctacgag
121 gtggtgatca aggtgcccag cgacctggac gagcacctgc ccggcatttc tgactccttt
181 gtgaactggg tggccgagaa ggaatgggag ttgccccccgg attctgacat ggatcagaat
241 ctgattgagc aggcacccct gaccgtggcc gagaagctgc agcgcgagtt cctggtggaa
301 tggcgccgag tgagtaaatt tctggaggcc aagtttttttg tgcagtttga aaagggggac
361 tcgtactttc atttgcatat tctgattgaa attaccggcg tgaaatccat ggtggtgggc
421 cgctacgtga gtcagattag ggataaactg atccagcgca tctaccgcgg ggtcgagccc
481 cagctgccca actggttcgc ggtcacaaag acccgaaatg gcgccggagg cgggaacaag
541 gtggtggacg agtgctacat ccccaactac ctgctcccca aggtccagcc cgagcttcag
601 tgggcgtgga ctaacatgga ggagtatata agcgcctgtt tgaacctcgc ggagcgtaaa
661 cggctcgtgg cgcagcacct gacgcacgtc tcccagaccc aggagggcga caaggagaat
721 ctgaacccga attctgacgc gccggtgatc cggtcaaaaa cctccgccag gtacatggag
781 ctggtcgggt ggctggtgga caagggcatc acgtccgaga agcagtggat ccaggaggac
841 caggcctcgt acatctcctt caacgcggcc tccaactccc ggtcgcagat caaggcggcc
901 ctggacaatg cctccaaaat catgagcctc accaaaacgg ctccggacta tctcatcggg
961 cagcagcccg tgggggacat taccaccaac cggatctaca aaatcctgga actgaacggg
1021 tacgaccccc agtacgccgc ctccgtcttt ctcggctggg cccagaaaaa gtttggaaag
1081 cgcaacacca tctggctgtt tgggcccgcc accaccggca agaccaacat cgcggaagcc
1141 atcgcccacg cggtcccctt ctacggctgc gtcaactgga ccaatgagaa ctttcccttc
1201 aacgactgcg tcgacaaaat ggtgatttgg tgggaggagg gcaagatgac cgccaaggtc
1261 gtagagtccg ccaaggccat tctgggcggc agcaaggtgc gcgtggacca aaaatgcaag
1321 gcctctgcgc agatcgaccc cacccccgtg atcgtcacct ccaacaccaa catgtgcgcc
1381 gtgattgacg ggaacagcac caccttcgag caccagcagc ccctgcagga ccggatgttc
1441 aagtttgaac tcacccgccg cctcgaccac gactttggca aggtcaccaa gcaggaagtc
1501 aaggactttt tccggtgggc ggctgatcac gtgactgacg tggctcatga gttttacgtc
1561 acaaagggtg gagctaagaa aaggcccgcc ccctctgacg aggatataag cgagcccaag
1621 cggccgcgcg tgtcatttgc gcagccggag acgtcagacg cggaagctcc cggagacttc
1681 gccgacaggt accaaaacaa atgttctcgt cacgcgggta tgctgcagat gctctttccc
1741 tgcaagacgt gcgagagaat gaatcagaat tccaacgtct gcttcacgca cggtcagaaa
1801 gattgcgggg agtgctttcc cgggtcagaa tctcaaccgg tttctgtcgt cagaaaaacg
1861 tatcagaaac tgtgcatcct tcatcagctc cgggggggcac ccgagatcgc ctgctctgct
1921 tgcgaccaac tcaaccccga tttggacgat tgccaatttg agcaataaat gactgaaatc
1981 aggtatggct gctgacggtt atcttccaga ttggctcgag gacaacctct ctgaaggcat
2041 tcgcgagtgg tgggcgctga aacctggagc tccacaaccc aaggccaacc aacagcatca
2101 ggacaacggc aggggtcttg tgcttcctgg gtacaagtac ctcggaccct tcaacggact
2161 cgacaaggga gagccggtca acgaggcaga cgccgcggcc ctcgagcacg acaaggccta
2221 cgacaagcag ctcgagcagg gggacaaccc gtatctcaag tacaaccacg ccgacgccga
2281 gttccagcag cgcttggcga ccgacacctc ttttgggggc aacctcgggc gagcagtctt
2341 ccaggccaaa aagaggattc tcgagcctct gggtctggtt gaagagggcg ttaaaacggc
2401 tcctggaaag aaacgcccat tagaaaagac tccaaatcgg ccgaccaacc cggactctgg
2461 gaaggccccg gccaagaaaa agcaaaaaga cggcgaacca gccgactctg ctagaaggac
2521 actcgacttt gaagactctg gagcaggaga cggacccccct gagggatcat cttccggaga
2581 aatgtctcat gatgctgaga tgcgtgcggc gccaggcgga aatgctgtcg aggcgggaca
2641 aggtgccgat ggagtgggta atgcctccgg tgattggcat tgcgattcca cctggtcaga
2701 gggccgagtc accaccacca gcacccgaac ctgggtccta cccacgtaca acaaccacct
2761 gtacctgcga atcggaacaa cggccaacag caacacctac aacggattct ccacccccctg
2821 gggatacttt gactttaacc gcttccactg ccacttttcc ccacgcgact ggcagcgact

2881 catcaacaac aactggggac tcaggccgaa atcgatgcgt gttaaaatct tcaacataca
2941 ggtcaaggag gtcacgacgt caaacggcga gactacggtc gctaataacc ttaccagcac
3001 ggttcagatc tttgcggatt cgacgtatga actcccatac gtgatggacg ccggtcagga
3061 ggggagcttt cctccgtttc ccaacgacgt ctttatggtt ccccaatacg gatactgcgg
3121 agttgtcact ggaaaaaacc agaaccagac agacagaaat gccttttact gcctggaata
3181 ctttccatcc caaatgctaa gaactggcaa caattttgaa gtcagttacc aatttgaaaa
3241 agttcctttc cattcaatgt acgcgcacag ccagagcctg gacagaatga tgaatccttt
3301 actggatcag tacctgtggc atctgcaatc gaccactacc ggaaattccc ttaatcaagg
3361 aacagctacc accacgtacg ggaaaattac cactggagac tttgcctact acaggaaaaa
3421 ctggttgcct ggagcctgca ttaaacaaca aaaattttca aagaatgcca atcaaaacta
3481 caagattccc gccagcgggg gagacgccct tttaaagtat gacacgcata ccactctaaa
3541 tgggcgatgg agtaacatgg ctcctggacc tccaatggca accgcaggtg ccggggactc
3601 ggattttagc aacagccagc tgatctttgc cggacccaat ccgagcggta acacgaccac
3661 atcttcaaac aatttgttgt ttacctcaga agaggagatt gccacaacaa acccacgaga
3721 cacggacatg tttggacaga ttgcagataa taatcaaaat gccaccaccg cccctcacat
3781 cgctaacctg gacgctatgg gaattgttcc cggaatggtc tggcaaaaca gagacatcta
3841 ctaccagggc cctatttggg ccaaggtccc tcacacggac ggacactttc acccttcgcc
3901 gctgatggga ggatttggac tgaaacaccc gcctccacag attttcatca aaaacacccc
3961 cgtacccgcc aatcccaata ctacctttag cgctgcaagg attaattctt ttctgacgca
4021 gtacagcacc ggacaagttg ccgttcagat cgactgggaa attcagaagg agcattccaa
4081 acgctggaat cccgaagttc aatttacttc aaactacggc actcaaaatt ctatgctgtg
4141 ggctcccgac aatgctggca actaccacga actccgggct attgggtccc gtttcctcac

## Embodiments of the invention

1. An adeno-associated virus (AAV) capsid, wherein the capsid comprises capsid protein VP1, wherein said capsid protein VP1 is from one or more than one first AAV serotype, and capsid protein VP3, wherein said capsid protein VP3 is from one or more than one second AAV serotype, and wherein at least one of said first AAV serotype is different from at least one of said second AAV serotype, in any combination.

2. The AAV capsid of embodiment 1, wherein the capsid comprises capsid protein VP2, wherein said capsid protein VP2 is from one or more than one third AAV serotype, wherein at least one of said one or more than one third AAV serotype is different from said first AAV serotype and/or said second AAV serotype, in any combination.

3. The AAV capsid of embodiment 2, wherein the capsid comprises capsid protein VP1.5.

4. The AAV capsid of embodiment 1, wherein the capsid comprises capsid protein VP1 .5, wherein said capsid protein VP1 .5 is from one or more than one fourth AAV serotype, wherein at least one of said one or more than one fourth AAV serotype is different from said first AAV serotype and/or said second AAV serotype, in any combination.

5. The AAV capsid of embodiment 4, wherein the capsid comprises capsid protein VP2.

6. An adeno-associated virus (AAV) capsid, wherein the capsid comprises capsid protein VP1, wherein said capsid protein VP1 is from one or more than one first AAV serotype, and capsid protein VP2, wherein said capsid protein VP2 is from one or more than one second AAV serotype and wherein at least one of said first AAV serotype is different from at least one of said second AAV serotype, in any combination.

7. The AAV capsid of embodiment 6, wherein the capsid comprises capsid protein VP3, wherein said capsid protein VP3 is from one or more than one third AAV serotype, wherein at least one of said one or more than one third AAV serotype is different from said first AAV serotype and/or said second AAV serotype, in any combination.

8. The AAV capsid of embodiment 7, wherein the capsid comprises capsid protein VP1.5.

9. An adeno-associated virus (AAV) capsid, wherein the capsid comprises capsid protein VP1, wherein said capsid protein VP1 is from one or more than one first AAV serotype, and capsid protein VP1.5, wherein said capsid protein VP1.5 is from one or more than one second AAV serotype, and wherein at least one of said first AAV serotype is different from at least one of said second AAV serotype, in any combination.

10. The AAV capsid of embodiment 9, wherein the capsid comprises capsid protein VP3, wherein said capsid protein VP3 is from one or more than one third AAV serotype, wherein at least one of said one or more than one third AAV serotype is different from said first AAV serotype and/or said second AAV serotype, in any combination.

11. The AAV capsid of embodiment 10, wherein the capsid comprises capsid protein VP2.

12. The AAV capsid of any preceding embodiment, wherein said one or more than one first AAV serotype, said one or more than one second AAV serotype, said one or more than one third AAV serotype and said one or more than one fourth AAV serotype are selected from the group consisting of the AAV serotypes listed in **Table 3,** in any combination.

13. The AAV capsid of any preceding embodiment, comprising a chimeric capsid VP1 protein, a chimeric capsid VP2 protein, a chimeric capsid VP3 protein and/or a chimeric capsid VP1.5 protein.

14. The AAV capsid of any of embodiments 1, 3, 4, 9 or 10, wherein said AAV capsid lacks capsid protein VP2.

15. The AAV capsid of embodiment 1, wherein the AAV capsid is AAV2/8/9.

16. The AAV capsid of embodiment 1, wherein the AAV capsid is H-AAV82.

17. The AAV capsid of embodiment 1, wherein the AAV capsid is H-AAV92.

18. The AAV capsid of embodiment 1, wherein the AAV capsid is H-AAV82G9.

19. The AAV capsid of embodiment 1, wherein the AAV capsid is AAV2/8 3:1.

20. The AAV capsid of embodiment 1, wherein the AAV capsid is AAV2/8 1:1.

21. The AAV capsid of embodiment 1, wherein the AAV capsid is AAV2/8 1:3.

22. The AAV capsid of embodiment 1, wherein the AAV capsid is AAV8/9.

23. The AAV capsid of embodiment 1, comprising an AAV capsid protein selected from the group consisting of LK3, LK0I-19, AAV-DJ, Olig001, rAAV2- retro, AAV-LiC, AAV0Keral, AAV-Kera2, AAV-Kera3, AAV 7m8, AAVI,9, AAVr3.45, AAV clone 32, AAV clone 83, AAV-U87R7-C5, AAV ShH13, AAV ShH19, AAV LI-12, AAV HAE-1, AAV HAE-2, AAV variant ShH10, AAV2.5T, AAV LSI-4, AAV Lsm, AAV1289, AAVHSC 1-17, AAV2 Rec 1-4, AAV8BP2, AAV-BI, AAV-PHP.B, AAV9.45, AAV9.61, AAV9.47, AAVM41, AAV2 displayed peptides, AAV2-GMN, AAV9-peptide displayed, AAV8 and AAV9 peptide displayed, AAVpo2.1, AAVpo4, AAVpo5, AAVpo6, AAV rh, AAV Hu, AAV-Go.1, AAV-1110.I, DAAV, AAAV, AAV8 K137R, AAV Anc80L65, AAV2G9, AAV2 265 insertion-AAV2/265D, AAV2.5, AAV3 SASTG, AAV2i8, AAV8G9, AAV2tyrosine mutants AAV2 Y-F, AAV8 Y-F, AAV9 Y-F, AAV6 Y-F, AAV6.2 and any combination thereof.

24. A virus vector comprising:

(a) the AAV capsid of any preceding embodiment; and
(b) a nucleic acid comprising at least one terminal repeat sequence, wherein the nucleic acid is encapsidated by the AAV capsid.

25. A method of making an AAV particle comprising the AAV capsid of any preceding embodiment, comprising:

(a) transfecting a host cell with one or more plasmids that provide, in combination all functions and genes needed to assemble AAV particles;
(b) introducing one or more nucleic acid constructs into a packaging cell line or producer cell line to provide, in combination all functions and genes needed to assemble AAV particles;

(c) introducing into a host cell one or more recombinant baculovirus vectors that provide in combination all functions and genes needed to assemble AAV particles; and/or
(d) introducing into a host cell one or more recombinant herpesvirus vectors that provide in combination all functions and genes needed to assemble AAV particles.

26. An adeno-associated virus (AAV) capsid, wherein the capsid comprises capsid protein VP1, wherein said capsid protein VP1 is from one or more than one first AAV serotype, and capsid protein VP2, wherein said capsid protein VP2 is from one or more than one second AAV serotype, wherein said capsid protein VP3 is from one or more than one first AAV serotype, and wherein at least one of said first AAV serotype is different from at least one of said second AAV serotype and from one of said third AAV serotype, in any combination.

27. An adeno-associated virus (AAV), wherein the Rep protein is from a first AAV serotype and the capsid proteins VP1, VP2 and VP3 is from a second AAV serotype that is different from the Rep protein.

28. An adeno-associated virus (AAV), wherein the Rep protein is from a first AAV serotype and the capsid proteins VP1, VP2 and VP3 are from two or more different AAV serotypes wherein the capsid proteins are from two or more different AAV serotypes which differ from the AAV serotype of the Rep protein.

29. An adeno-associated virus (AAV), wherein the Rep protein is from a first AAV serotype and the capsid proteins VP1, VP2 and VP3 are from two or more different AAV serotypes, wherein the AAV serotype of the Rep protein is the same as one of the AAV serotypes of a capsid protein.

30. The AAV of embodiment 28, wherein the Rep protein is selected from one of AAV1. AAV2, AAV3, AAV4, AAV5, AAV6, AAV7, AAV8, AAV9, AAV10 or any chimeric of each AAV.

31. The AAV of embodiment 29, wherein the Rep protein is selected from one of AAV1. AAV2, AAV3, AAV4, AAV5, AAV6, AAV7, AAV8, AAV9, AAV10 or any chimeric of each AAV.

32. The AAV of embodiment 28, wherein the VP1, VP2 and VP3 capsid proteins are selected from two or more of AAV1. AAV2, AAV3, AAV4, AAV5, AAV6, AAV7, AAV8, AAV9, AAV10 or any chimeric of each AAV.

33. The AAV of embodiment 29, wherein the VP1, VP2 and VP3 capsid proteins are selected from two or more of AAV1. AAV2, AAV3, AAV4, AAV5, AAV6, AAV7, AAV8, AAV9, AAV10 or any chimeric of each AAV.

34. An adeno-associated virus (AAV), wherein the Rep protein is from a first AAV serotype and the capsid protein VP1 in the AAV is from two or more different AAV serotypes wherein the capsid protein is from two or more different AAV serotypes which differ from the AAV serotype of the Rep protein.

35. The AAV of embodiment 34, wherein the ratio of one AAV serotype VP1 capsid to the second AAV serotype VP1 capsid in the AAV is 1:1.

36. The AAV of embodiment 34, wherein the ratio of one AAV serotype VP1 capsid to the second AAV serotype VP1 capsid in the AAV is not 1:1.

37. The AAV of embodiment 34, wherein the ratio of one AAV serotype VP1 capsid to the second AAV serotype VP1 capsid in the AAV is predetermined.

38. The AAV capsid of embodiment 34, wherein three or more AAV serotypes of VP1 capsid are present in the AAV in a predetermined ratio.

39. An adeno-associated virus (AAV), wherein the Rep protein is from a first AAV serotype and the capsid protein VP2 in the AAV is from two or more different AAV serotypes wherein the capsid protein is from two or more different AAV serotypes which differ from the AAV serotype of the Rep protein.

40. The AAV of embodiment 34, wherein the ratio of one AAV serotype VP2 capsid to the second AAV serotype VP2 capsid in the AAV is 1:1.

41. The AAV of embodiment 34, wherein the ratio of one AAV serotype VP2 capsid to the second AAV serotype VP2 capsid in the AAV is not 1:1.

42. The AAV of embodiment 34, wherein the ratio of one AAV serotype VP2 capsid to the second AAV serotype VP2 capsid in the AAV is predetermined.

43. The AAV capsid of embodiment 34, wherein three or more AAV serotypes of VP2 capsid are present in the AAV in a predetermined ratio.

44. An adeno-associated virus (AAV), wherein the Rep protein is from a first AAV serotype and the capsid protein VP3 in the AAV is from two or more different AAV serotypes wherein the capsid protein is from two or more different AAV serotypes which differ from the AAV serotype of the Rep protein.

45. The AAV of embodiment 34, wherein the ratio of one AAV serotype VP3 capsid to the second AAV serotype VP3 capsid in the AAV is 1:1.

46. The AAV of embodiment 34, wherein the ratio of one AAV serotype VP3 capsid to the second AAV serotype VP3 capsid in the AAV is not 1:1.

47. The AAV of embodiment 34, wherein the ratio of one AAV serotype VP3 capsid to the second AAV serotype VP3 capsid in the AAV is predetermined.

48. The AAV capsid of embodiment 34, wherein three or more AAV serotypes of VP3 capsid are present in the AAV in a predetermined ratio.

49. A method of manufacture of an AAV, wherein an AAV is manufactured using a first helper plasmid containing a Rep protein from a first AAV serotype and the capsid proteins VP1, VP2 and VP3 from a second AAV serotype that is different from the Rep protein, and a second helper plasmid that contains a coding region of a gene for the treatment of a disease.

50. The method of embodiment 49, wherein the disease is selected from a lysosomal storage disorder such as a mucopolysaccharidosis disorder (e.g., Sly syndrome[ - glucuronidase], Hurler Syndrome [a-L-iduronidase], Scheie Syndrome [a-L- iduronidase], Hurler-Scheie Syndrome [a-L-iduronidase], Hunter's Syndrome [iduronate sulfatase], Sanfilippo Syndrome A [heparan sulfamidase], B [N- acetylglucosaminidase], C [acetyl-CoA:a-glucosaminide acetyltransferase], D [N- acetylglucosamine 6-sulfatase], Morquio Syndrome A [galactose-6-sulfate sulfatase], B [ -galactosidase], Maroteaux-Lamy Syndrome [N-acetylgalactosamine-4-sulfatase], *etc.),* Fabry disease (a-galactosidase), Gaucher's disease (glucocerebrosidase), or a glycogen storage disorder (e.g., Pompe disease; lysosomal acid a-glucosidase).

51. A method of manufacture of an AAV, wherein an AAV is manufactured using a first helper plasmid containing a Rep protein from a first AAV serotype and a second helper plasmid that contains the capsid proteins VP1, VP2 and VP3 from a second AAV serotype that is different from the Rep protein, and a third helper plasmid that contains a coding region of a gene for the treatment of a disease.

52. The method of embodiment 52, wherein the disease is selected from a lysosomal storage disorder such as a mucopolysaccharidosis disorder (e.g., Sly syndrome[ - glucuronidase], Hurler Syndrome [a-L-iduronidase], Scheie Syndrome [a-L- iduronidase], Hurler-Scheie Syndrome [a-L-iduronidase], Hunter's Syndrome [iduronate sulfatase], Sanfilippo Syndrome A [heparan sulfamidase], B [N- acetylglucosaminidase], C [acetyl-CoA:a-glucosaminide acetyltransferase], D [N- acetylglucosamine 6-sulfatase], Morquio Syndrome A [galactose-6-sulfate sulfatase], B [ -galactosidase], Maroteaux-Lamy Syndrome [N-acetylgalactosamine-4-sulfatase], *etc.),* Fabry disease (a-galactosidase), Gaucher's disease (glucocerebrosidase), or a glycogen storage disorder (e.g., Pompe disease; lysosomal acid a-glucosidase).

53. A method of manufacture of an AAV, wherein an AAV is manufactured using a first helper plasmid containing a Rep protein from a first AAV serotype and a second helper plasmid that contains the capsid proteins VP1, VP2 and VP3 from two or more different AAV serotypes, wherein the capsid protein is from two or more different AAV serotypes which differ from the AAV serotype of the Rep protein, and a third helper plasmid that contains a coding region of a gene for the treatment of disease.

54. The method of embodiment 51, wherein the disease is selected from a lysosomal storage disorder such as a mucopolysaccharidosis disorder (e.g., Sly syndrome[ - glucuronidase], Hurler Syndrome [a-L-iduronidase], Scheie Syndrome [a-L- iduronidase], Hurler-Scheie Syndrome [a-L-iduronidase], Hunter's Syndrome [iduronate sulfatase], Sanfilippo Syndrome A [heparan sulfamidase], B [N- acetylglucosaminidase], C [acetyl-CoA:a-glucosaminide acetyltransferase], D [N- acetylglucosamine 6-sulfatase], Morquio Syndrome A [galactose-6-sulfate sulfatase], B [ -galactosidase], Maroteaux-Lamy Syndrome [N-acetylgalactosamine-4-sulfatase], *etc.),* Fabry disease (a-galactosidase), Gaucher's disease (glucocerebrosidase), or a glycogen storage disorder (e.g., Pompe disease; lysosomal acid a-glucosidase).

55. A method of manufacture of an AAV, wherein an AAV is manufactured using a first helper plasmid containing a Rep protein from a first AAV serotype and capsid proteins VP1, VP2 and VP3, wherein the capsid proteins are from two or more different AAV serotypes, and further wherein the AAV serotype of the Rep protein is the same as one of the AAV serotypes of a capsid protein and a second plasmid that contains a coding region of a gene for the treatment of a disease.

56. The method of embodiment 51, wherein the disease is selected from a lysosomal storage disorder such as a mucopolysaccharidosis disorder (e.g., Sly syndrome[ - glucuronidase], Hurler Syndrome [a-L-iduronidase], Scheie Syndrome [a-L- iduronidase], Hurler-Scheie Syndrome [a-L-iduronidase], Hunter's Syndrome [iduronate sulfatase], Sanfilippo Syndrome A [heparan sulfamidase], B [N- acetylglucosaminidase], C [acetyl-CoA:a-glucosaminide acetyltransferase], D [N- acetylglucosamine 6-sulfatase], Morquio Syndrome A [galactose-6-sulfate sulfatase], B [ -galactosidase], Maroteaux-Lamy Syndrome [N-acetylgalactosamine-4-sulfatase], *etc.),* Fabry disease (a-galactosidase), Gaucher's disease (glucocerebrosidase), or a glycogen storage disorder (e.g., Pompe disease; lysosomal acid a-glucosidase).

## Claims

**1.** A method of creating an adeno-associated virus (AAV) virion comprising contacting cells, under conditions for formation of AAV virions, with a first nucleic acid sequence and a second nucleic acid sequence, wherein the AAV virion is formed from at least AAV8 viral protein 1 (VP1) and AAV3 viral protein 3 (VP3) viral structural proteins, wherein the first nucleic acid encodes VP1 from a AAV8 serotype only but is not capable of expressing VP3, and the second nucleic acid sequence encodes VP3 from a AAV3 serotype only and further is not capable of expressing VP1, and wherein, the AAV virion comprises VP1 from the AAV8 serotype only and VP3 from the AAV3 serotype only, and wherein if viral protein 2 (VP2) is expressed, it is only from the AAV8 serotype.

**2.** A plurality of AAV vector particles generated by the method of claim 1.

**3.** An adeno-associated virus (AAV) capsid comprising an AAV8 viral protein 1 (VP1), AAV8 viral protein 2 (VP2), and AAV3 viral protein 3 (VP3), wherein the VP1 and VP2 in the capsid are only from AAV8, and the VP3 in the capsid is only from AAV3.

**4.** An adeno-associated virus (AAV) vector particle comprising:

(a) the AAV capsid of claim 3; and
(b) a nucleic acid molecule comprising at least one terminal repeat sequence, and optionally comprising a heterologous nucleic acid molecule, wherein the nucleic acid molecule is encapsidated by the AAV capsid.

**5.** The AAV vector particle of claim 4 for use in a method of treatment.

**6.** A composition comprising the AAV capsid of claim 3, and/or the AAV vector particle of claim 4 in a pharmaceutically acceptable carrier.

**7.** A method of making an adeno-associated virus (AAV) vector, comprising:

a) transfecting a host cell with one or more plasmids that provide, in combination, all functions and genes needed to assemble AAV particles, wherein capsid proteins are encoded exclusively by one or more nucleic acid constructs that express AAV8 viral protein 1 and viral protein 2 (VP1/VP2) only and AAV3 viral protein 3 (VP3) only;

b) introducing one or more nucleic acid constructs into a packaging cell line or producer cell line to provide, in combination, all functions and genes needed, including helper-virus sequences to assemble AAV particles, wherein capsid proteins are encoded exclusively by one or more nucleic acid constructs that express AAV8 VP1/VP2 only and AAV3 VP3 only;
c) introducing into a host cell one or more recombinant baculovirus vectors that provide, in combination, all functions and genes needed to assemble AAV particles, wherein capsid proteins are encoded exclusively by one or more nucleic acid constructs that express AAV8 VP1/VP2 only and AAV3 VP3 only; and/or
d) introducing into a host cell one or more recombinant herpesvirus vectors that provide, in combination, all functions and genes needed to assemble AAV particles, wherein capsid proteins are encoded exclusively by one or more nucleic acid constructs that express AAV8 VP1/VP2 only and AAV3 VP3 only.

**8.** The method of claim 7, wherein the helper-virus sequences are adenovirus helper-virus sequences or herpesvirus helper-virus sequences.

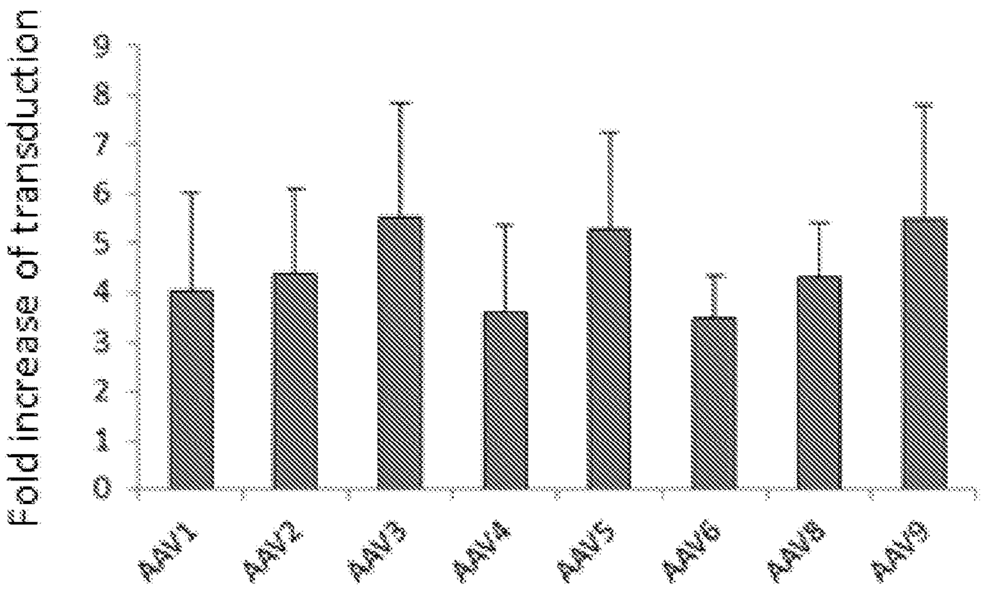
Fold increase of transduction
0
1
2
3
4
5
6
7
8
9
AAV1
AAV2
AAV3
AAV4
AAV5
AAV6
AAV8
AAV9

Fig. 1a

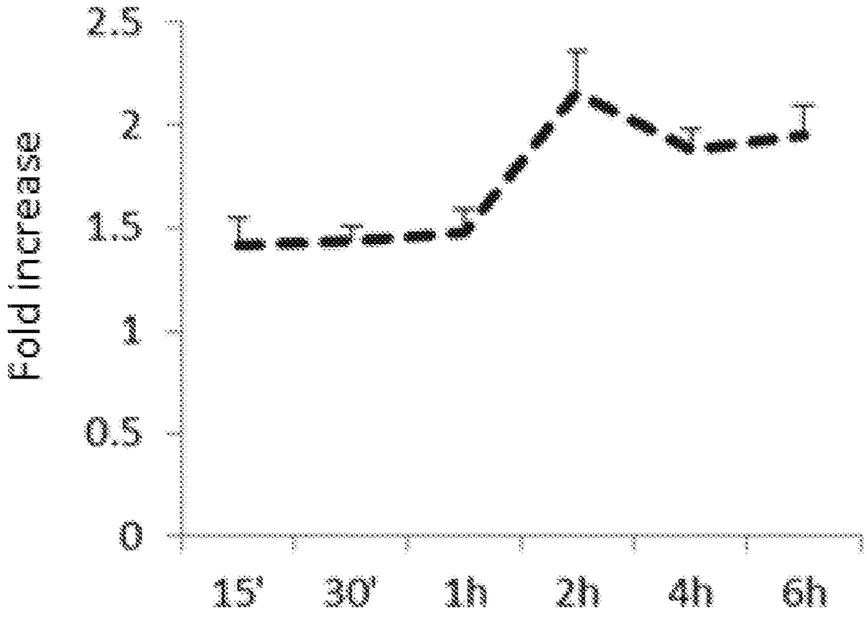
Fold increase
0
0.5
1
1.5
2
2.5
15'
30'
1h
2h
4h
6h

Fig. 1b

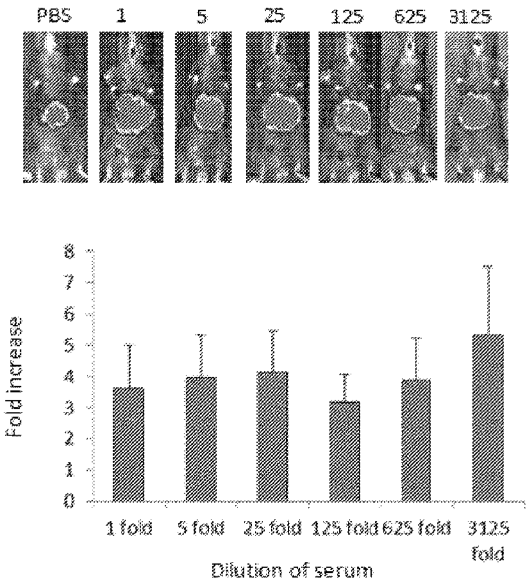
PBS
1
5
25
125
625
3125
Fold increase
1 fold
5 fold
25 fold
125 fold
625 fold
3125 fold
Dilution of serum

Fig. 1c

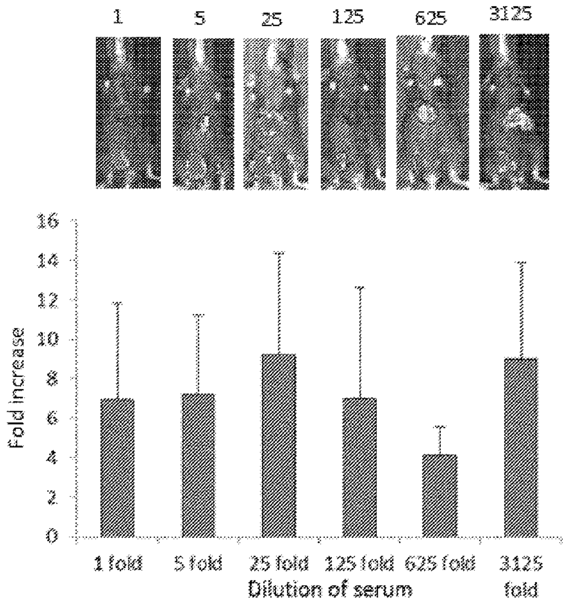
1
5
25
125
625
3125
Fold increase
1 fold
5 fold
25 fold
125 fold
625 fold
3125 fold
Dilution of serum

Fig. 1d

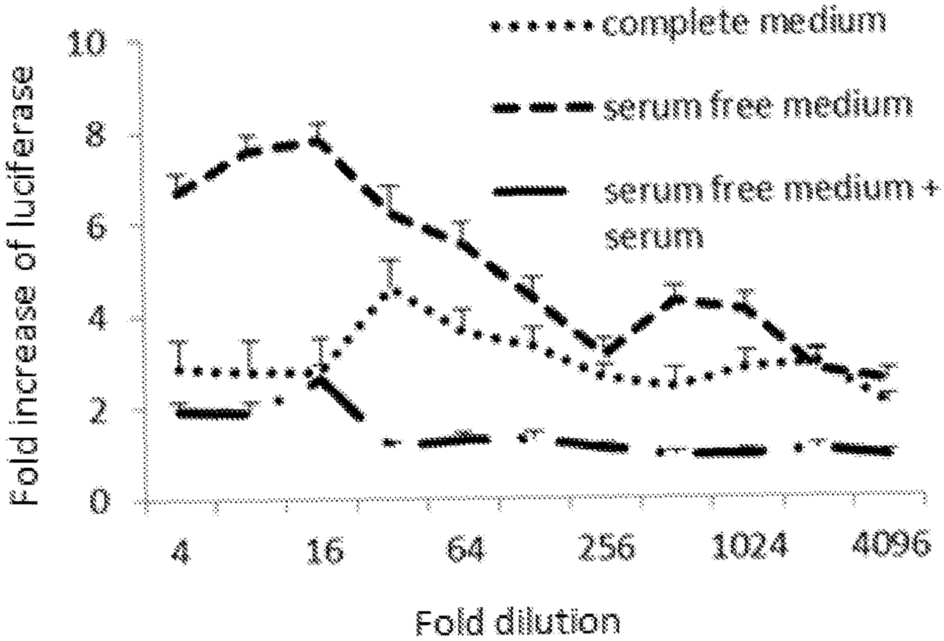
complete medium
serum free medium
serum free medium + serum
Fold increase of luciferase
0
2
4
6
8
10
4
16
64
256
1024
4096
Fold dilution

**Fig. 2a**

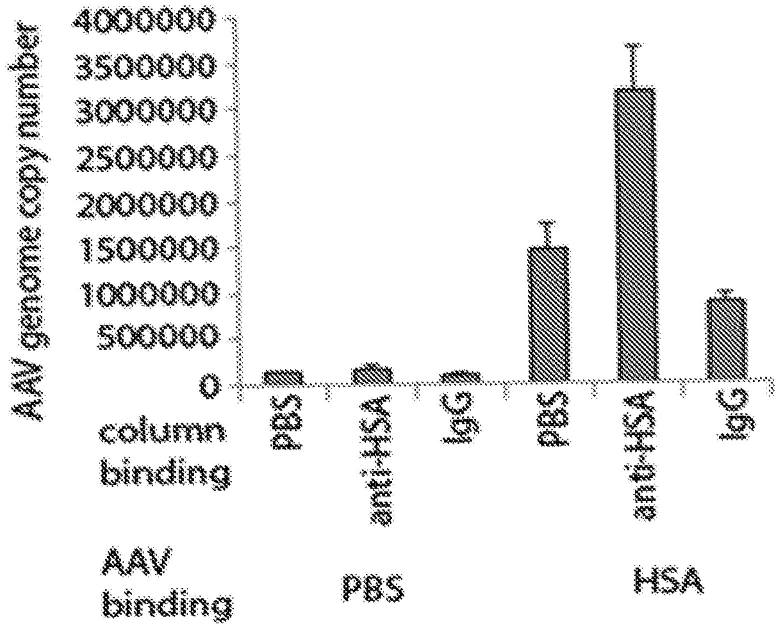
AAV genome copy number
4000000
3500000
3000000
2500000
2000000
1500000
1000000
500000
0
column binding
PBS
anti-HSA
IgG
PBS
anti-HSA
IgG
AAV binding
PBS
HSA

**Fig. 2b**

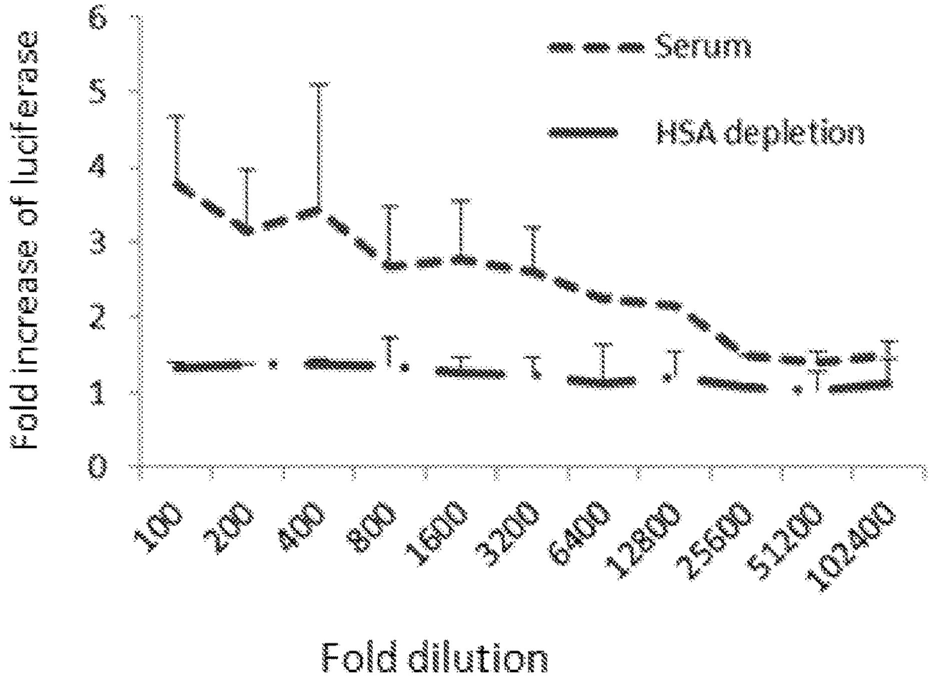
6
5
4
3
2
1
0
Serum
HSA depletion
Fold increase of luciferase
100
200
400
800
1600
3200
6400
12800
25600
51200
102400
Fold dilution

**Fig. 2c**

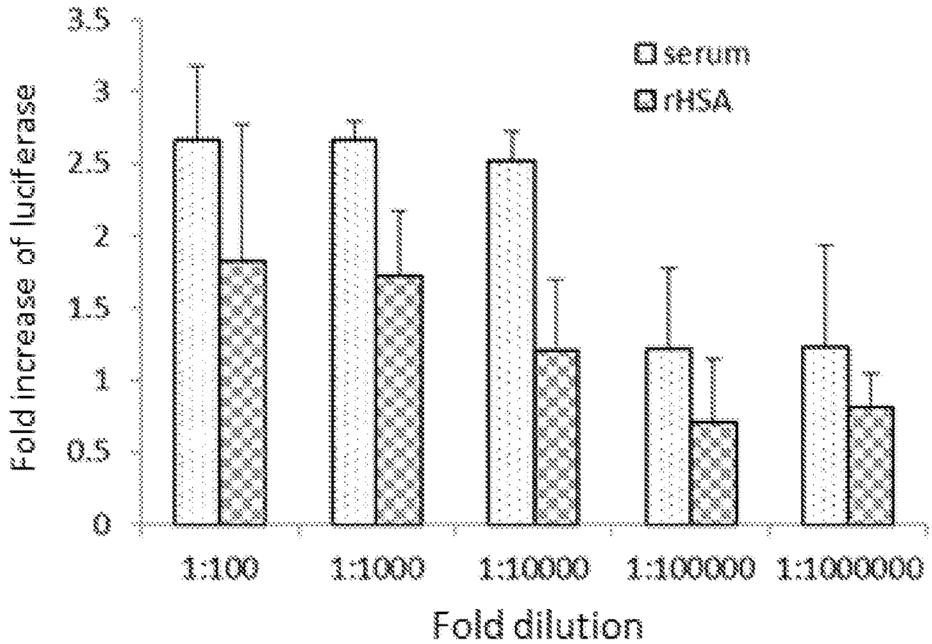
3.5
3
2.5
2
1.5
1
0.5
0
serum
rHSA
Fold increase of luciferase
1:100
1:1000
1:10000
1:100000
1:1000000
Fold dilution

**Fig. 2d**

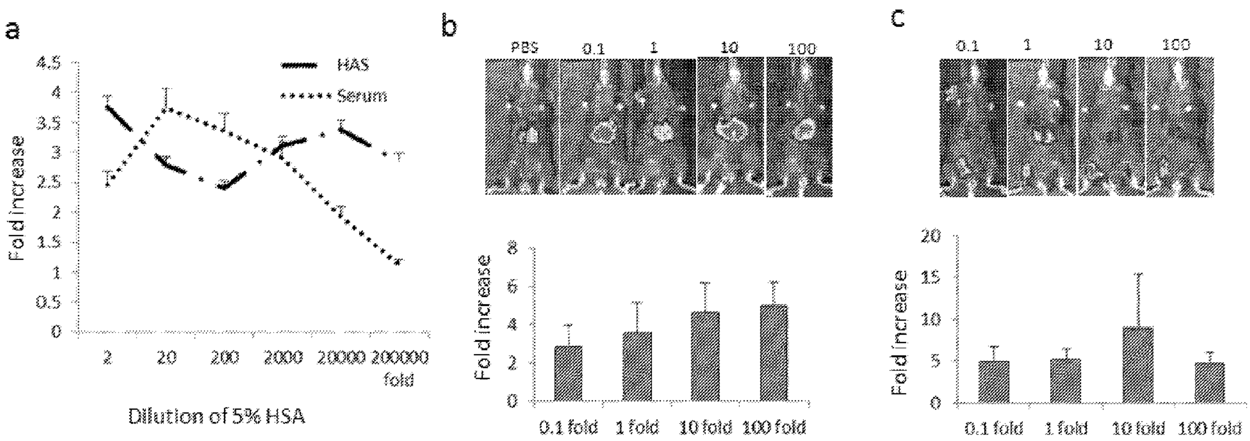
a
4.5
4
3.5
3
2.5
2
1.5
1
0.5
0
Fold increase
HAS
Serum
2
20
200
2000
20000
200000 fold
Dilution of 5% HSA
b
PBS
0.1
1
10
100
8
6
4
2
0
Fold increase
0.1 fold
1 fold
10 fold
100 fold
c
0.1
1
10
100
20
15
10
5
0
Fold increase
0.1 fold
1 fold
10 fold
100 fold

Fig. 3

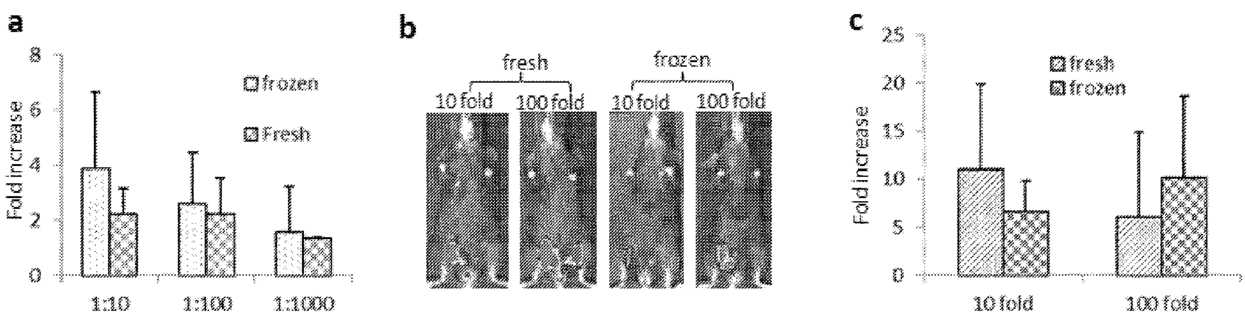
a
Fold increase
8
6
4
2
0
frozen
Fresh
1:10
1:100
1:1000
b
fresh
frozen
10 fold
100 fold
10 fold
100 fold
c
Fold increase
25
20
15
10
5
0
fresh
frozen
10 fold
100 fold

Fig. 4

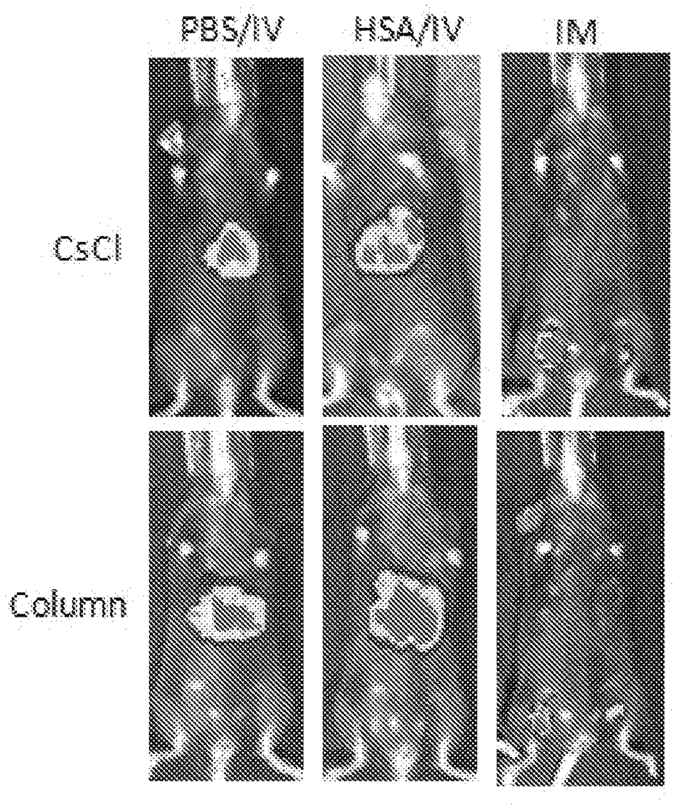
PBS/IV
HSA/IV
IM
CsCl
Column

Fig. 5a

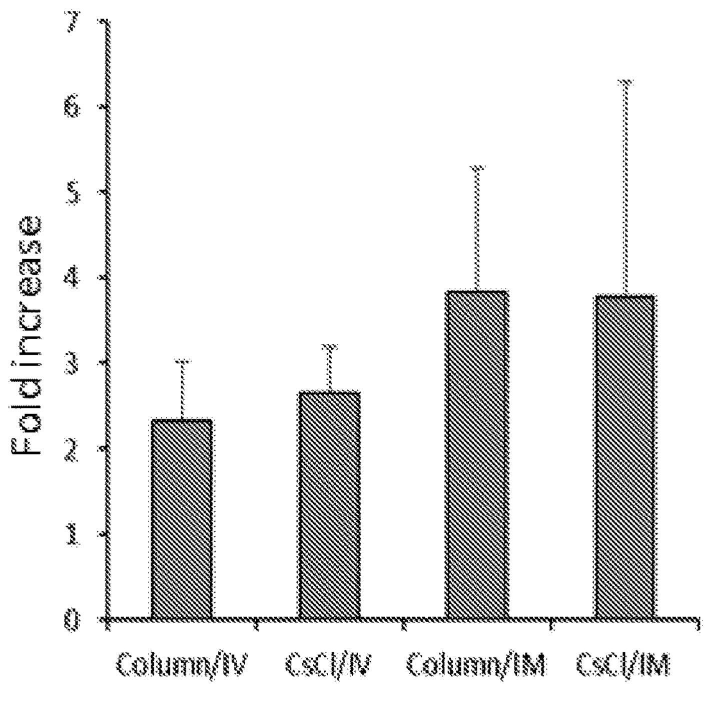
Fold increase
0
1
2
3
4
5
6
7
Column/IV
CsCl/IV
Column/IM
CsCl/IM

Fig. 5b

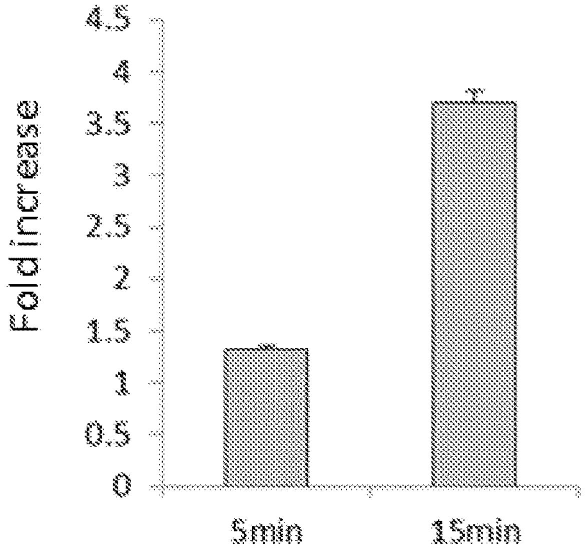
Fold increase
4.5
4
3.5
3
2.5
2
1.5
1
0.5
0
5min
15min

Fig. 6a

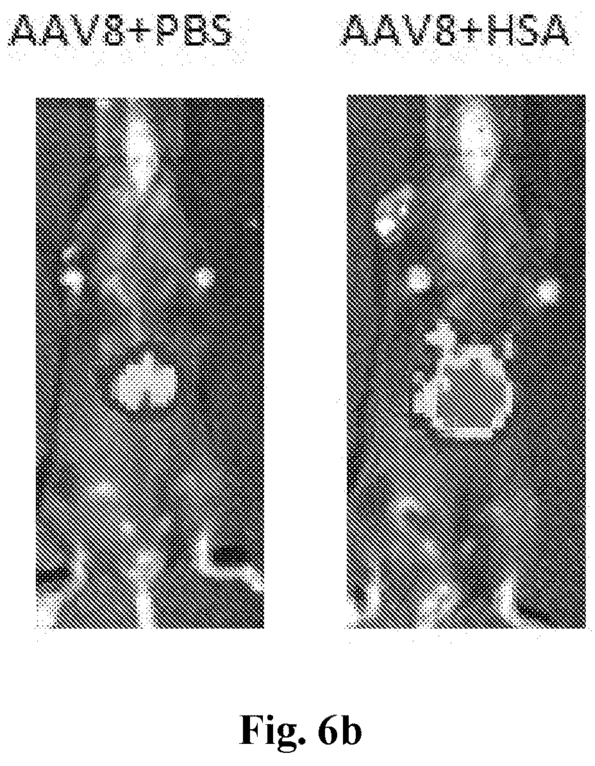
AAV8+PBS
AAV8+HSA

Fig. 6b

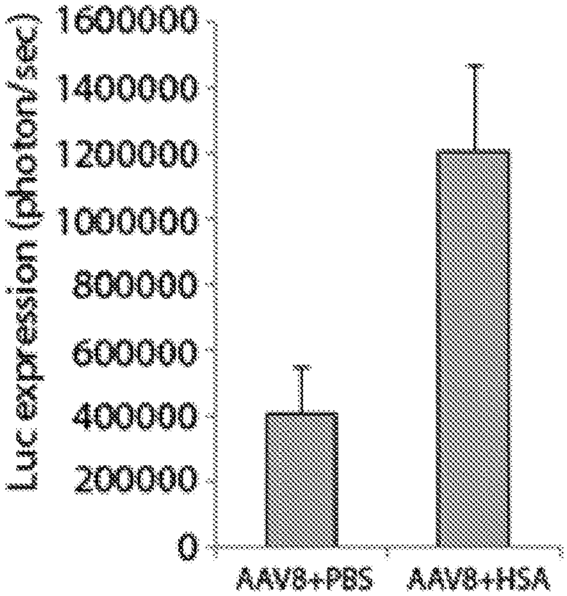
Luc expression (photon/sec)
1600000
1400000
1200000
1000000
800000
600000
400000
200000
0
AAV8+PBS
AAV8+HSA

Fig. 6c

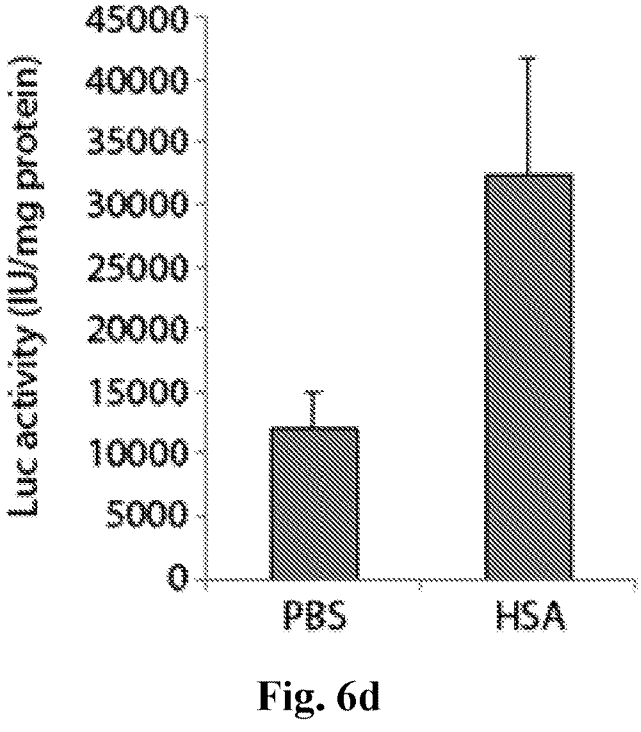
Luc activity (IU/mg protein)
45000
40000
35000
30000
25000
20000
15000
10000
5000
0
PBS
HSA

Fig. 6d

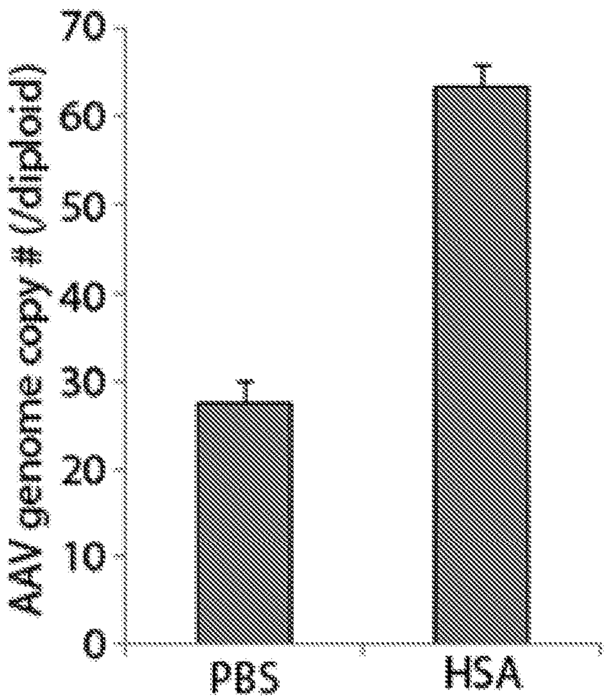
AAV genome copy # (/diploid)
70
60
50
40
30
20
10
0
PBS
HSA

Fig. 6e

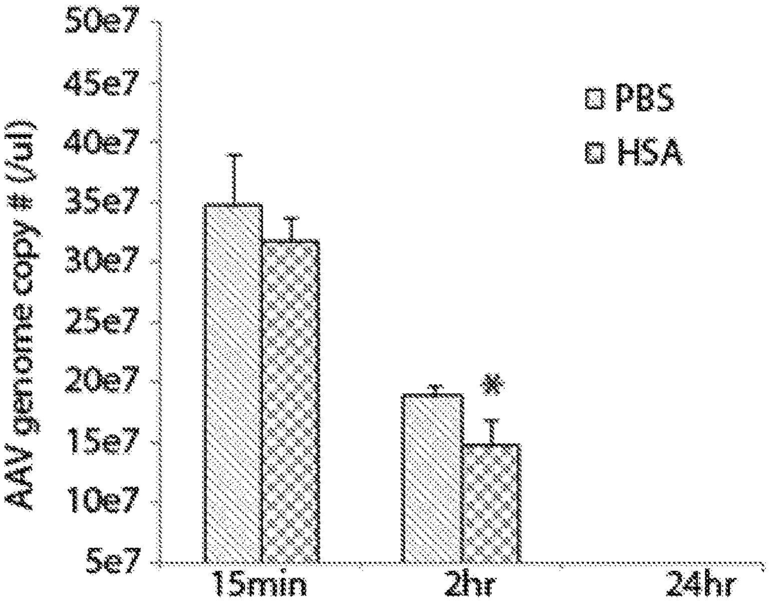
AAV genome copy # (/ul)
50e7
45e7
40e7
35e7
30e7
25e7
20e7
15e7
10e7
5e7
PBS
HSA
*
15min
2hr
24hr

Fig. 6f

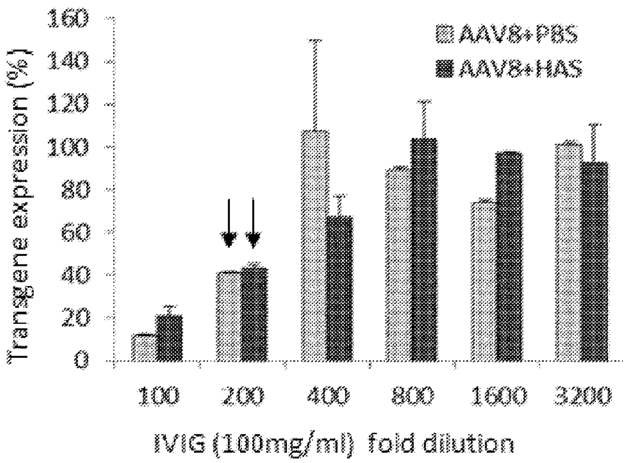
AAV8+PBS
AAV8+HAS
Transgene expression (%)
160
140
120
100
80
60
40
20
0
100
200
400
800
1600
3200
IVIG (100mg/ml) fold dilution

Fig. 7a

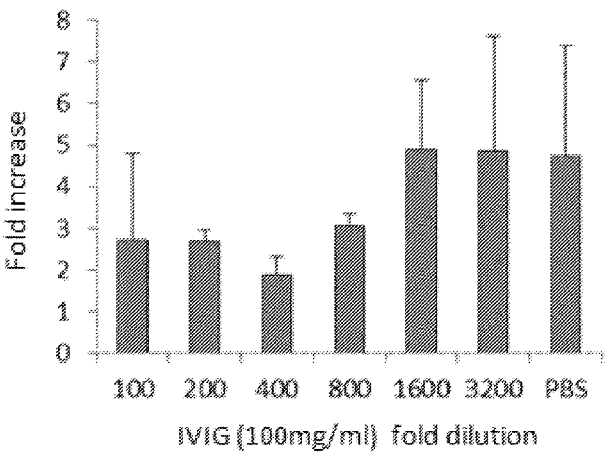
Fold increase
8
7
6
5
4
3
2
1
0
100
200
400
800
1600
3200
PBS
IVIG (100mg/ml) fold dilution

Fig. 7b

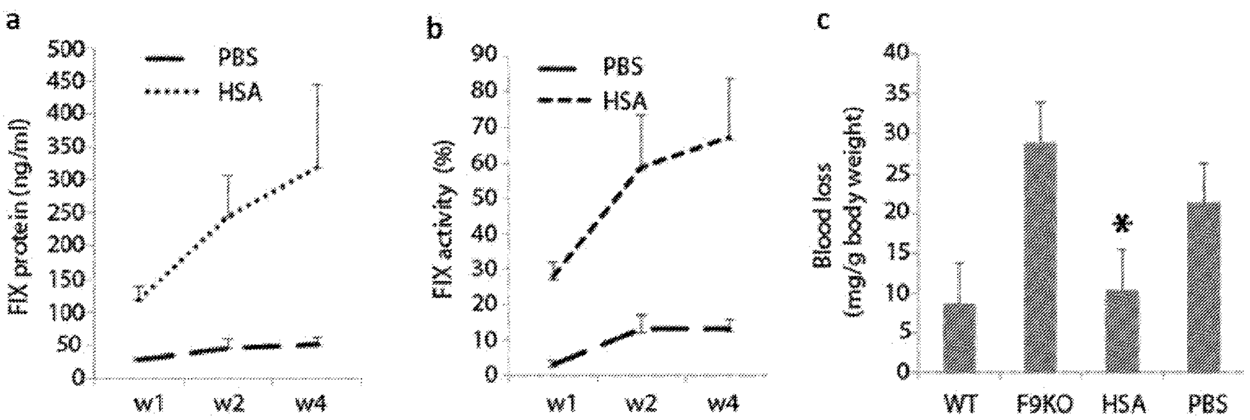
a
500
450
400
350
300
250
200
150
100
50
0
FIX protein (ng/ml)
PBS
HSA
w1
w2
w4
b
90
80
70
60
50
40
30
20
10
0
FIX activity (%)
PBS
HSA
w1
w2
w4
c
40
35
30
25
20
15
10
5
0
Blood loss
(mg/g body weight)
*
WT
F9KO
HSA
PBS

Fig. 8

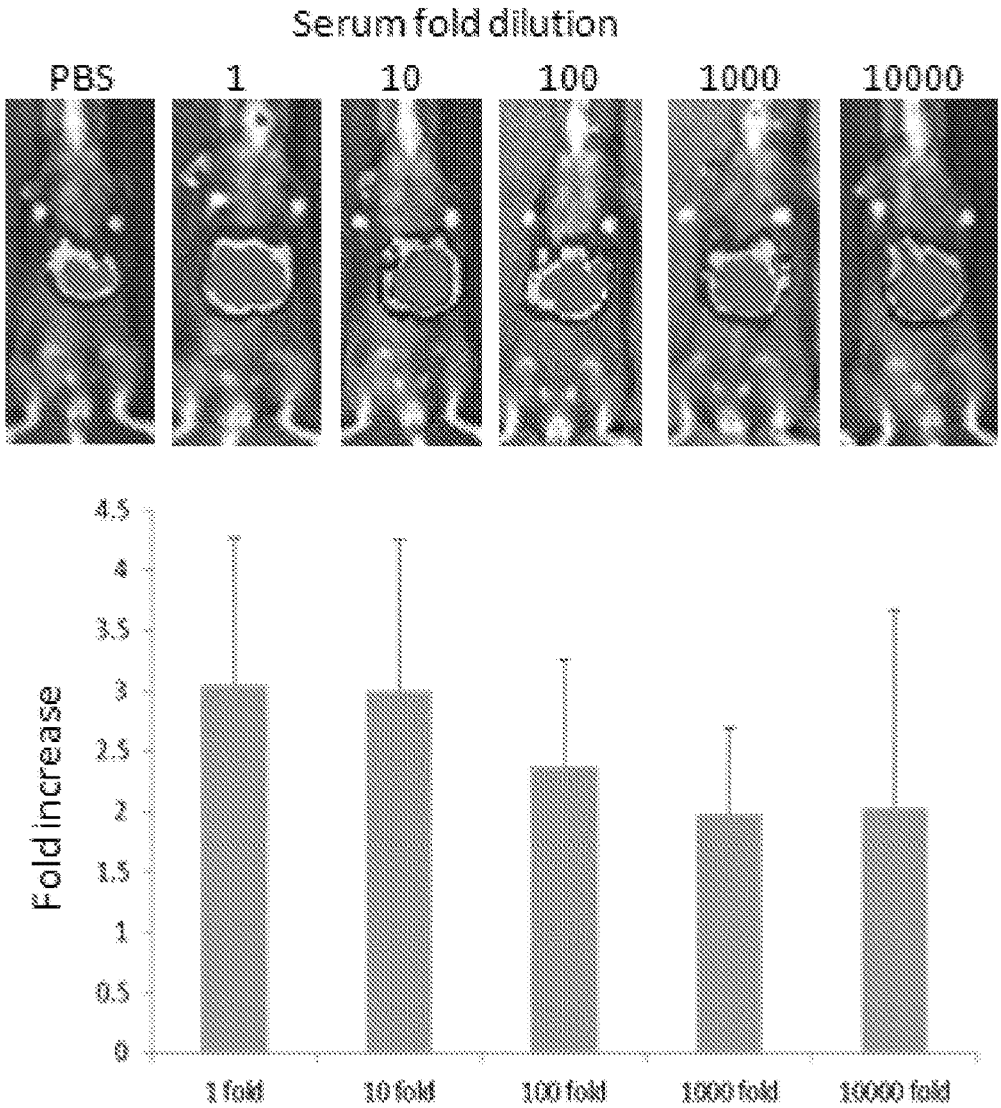
Serum fold dilution
PBS
1
10
100
1000
10000
Fold increase
4.5
4
3.5
3
2.5
2
1.5
1
0.5
0
1 fold
10 fold
100 fold
1000 fold
10000 fold

**Fig. 9a**

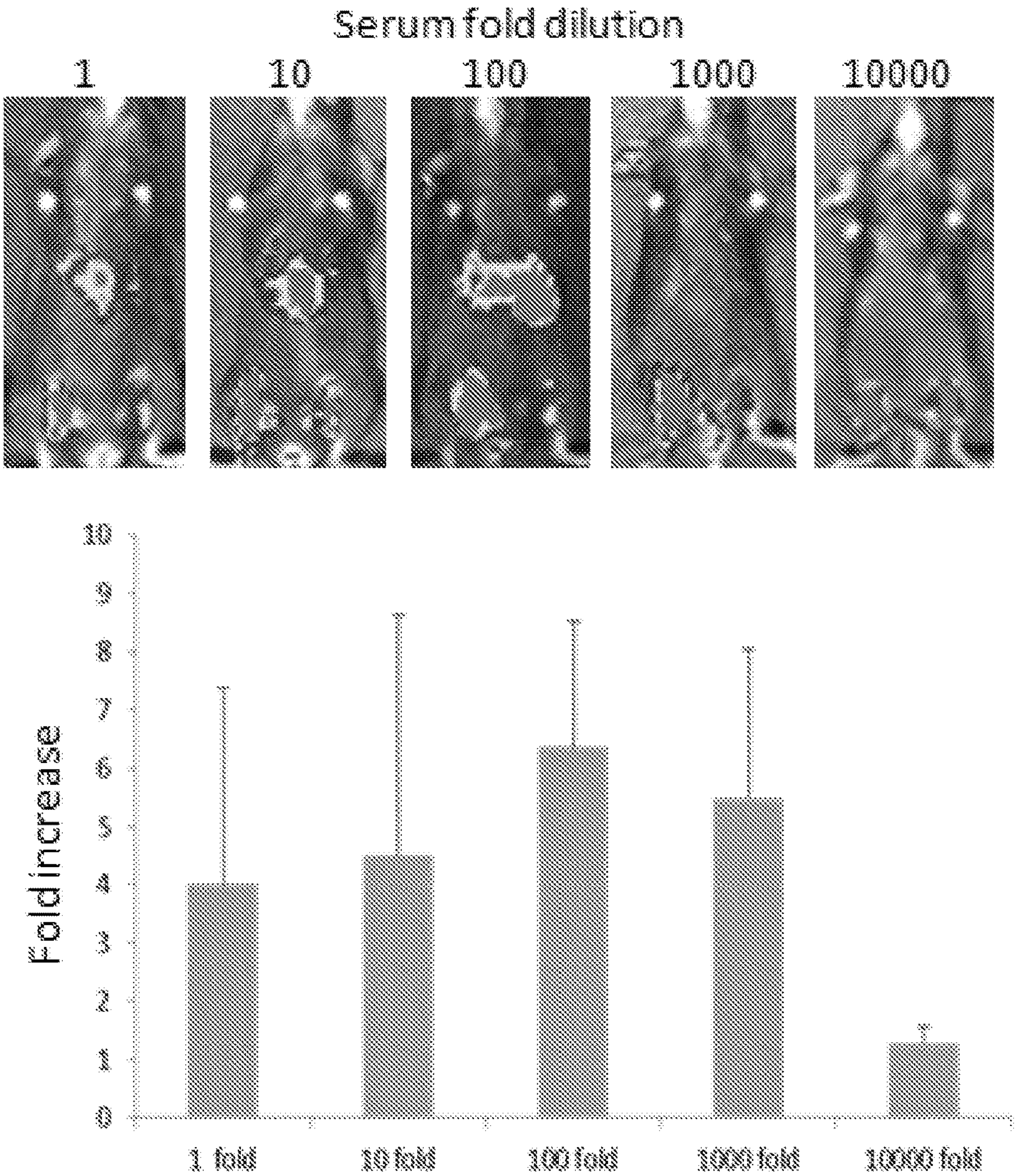
Serum fold dilution
1
10
100
1000
10000
Fold increase
0
1
2
3
4
5
6
7
8
9
10
1 fold
10 fold
100 fold
1000 fold
10000 fold

**Fig. 9b**

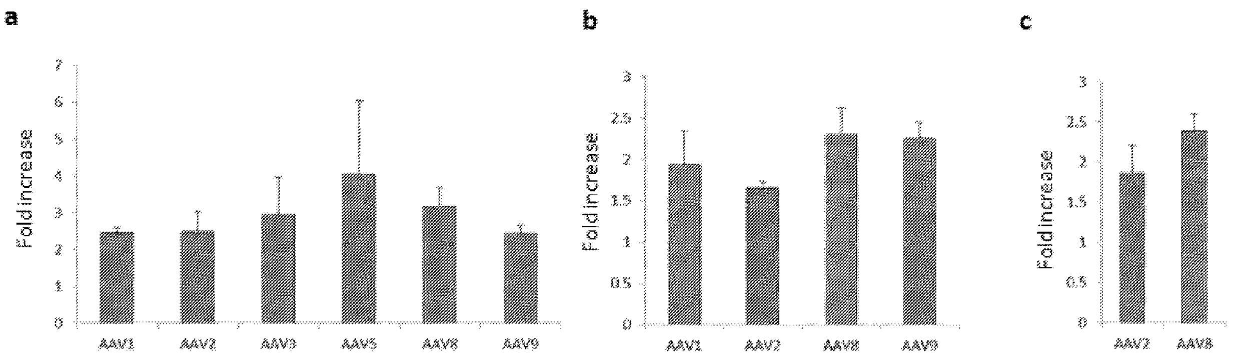


Fig. 10

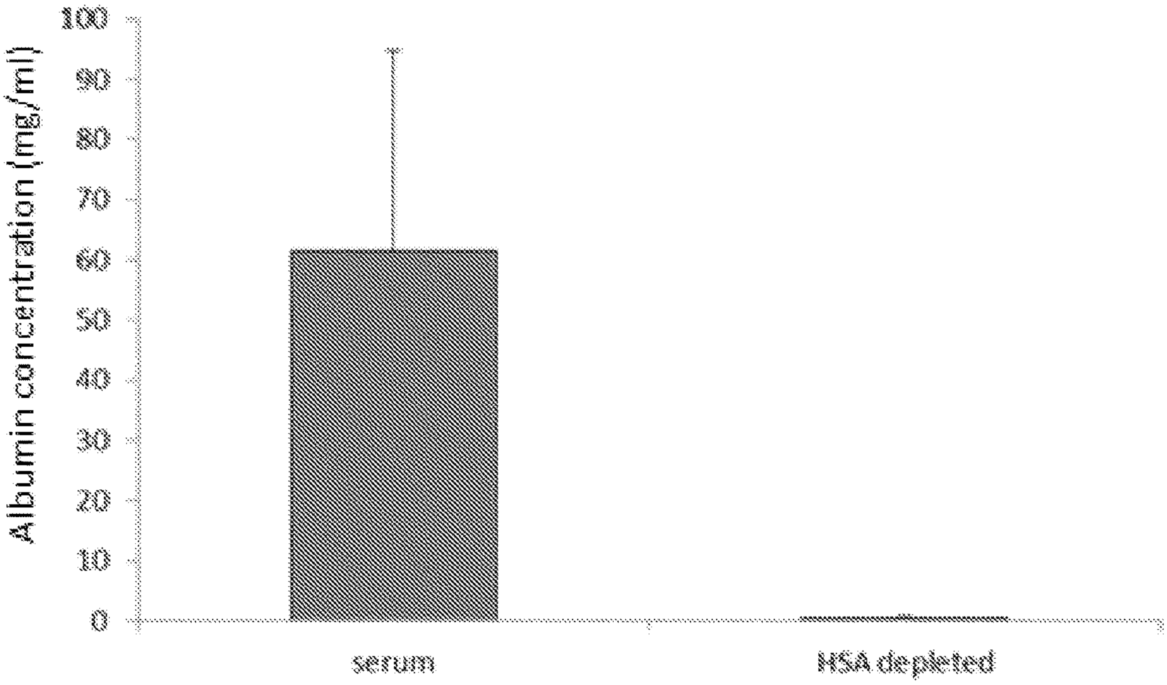
Albumin concentration (mg/ml)
100
90
80
70
60
50
40
30
20
10
0
serum
HSA depleted

Fig. 11

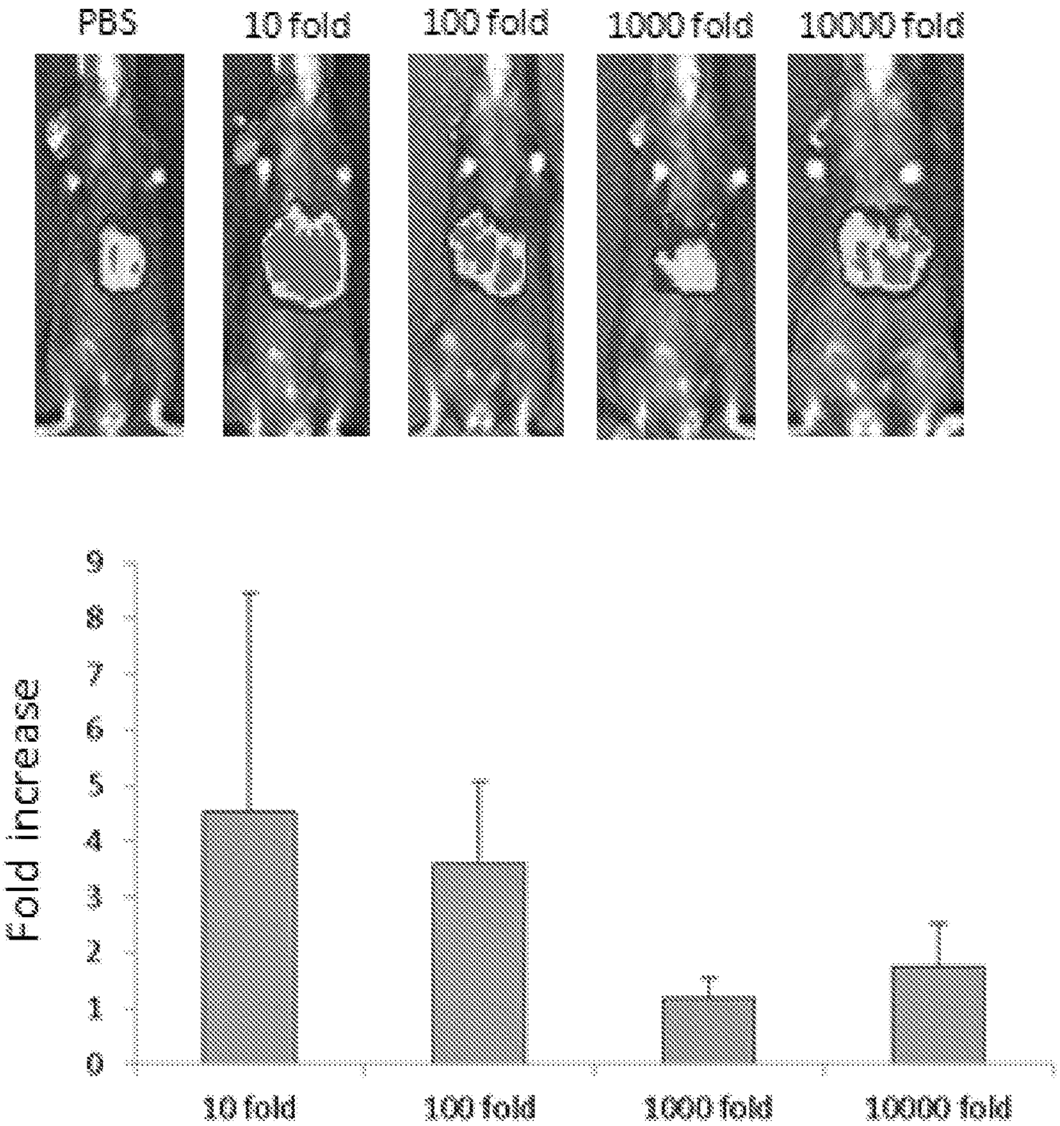
PBS
10 fold
100 fold
1000 fold
10000 fold
Fold increase
0
1
2
3
4
5
6
7
8
9
10 fold
100 fold
1000 fold
10000 fold

Fig. 12a

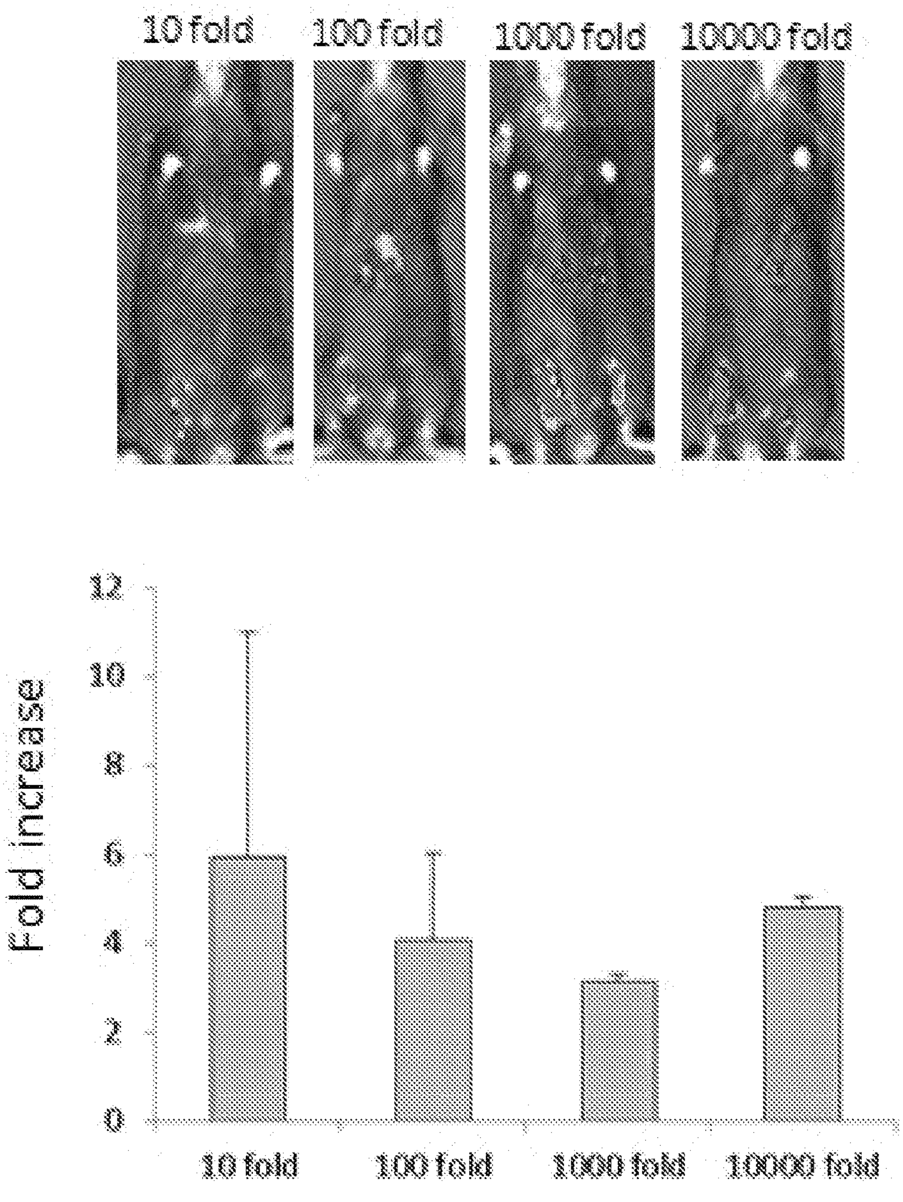
10 fold
100 fold
1000 fold
10000 fold
Fold increase
12
10
8
6
4
2
0
10 fold
100 fold
1000 fold
10000 fold

**Fig. 12b**

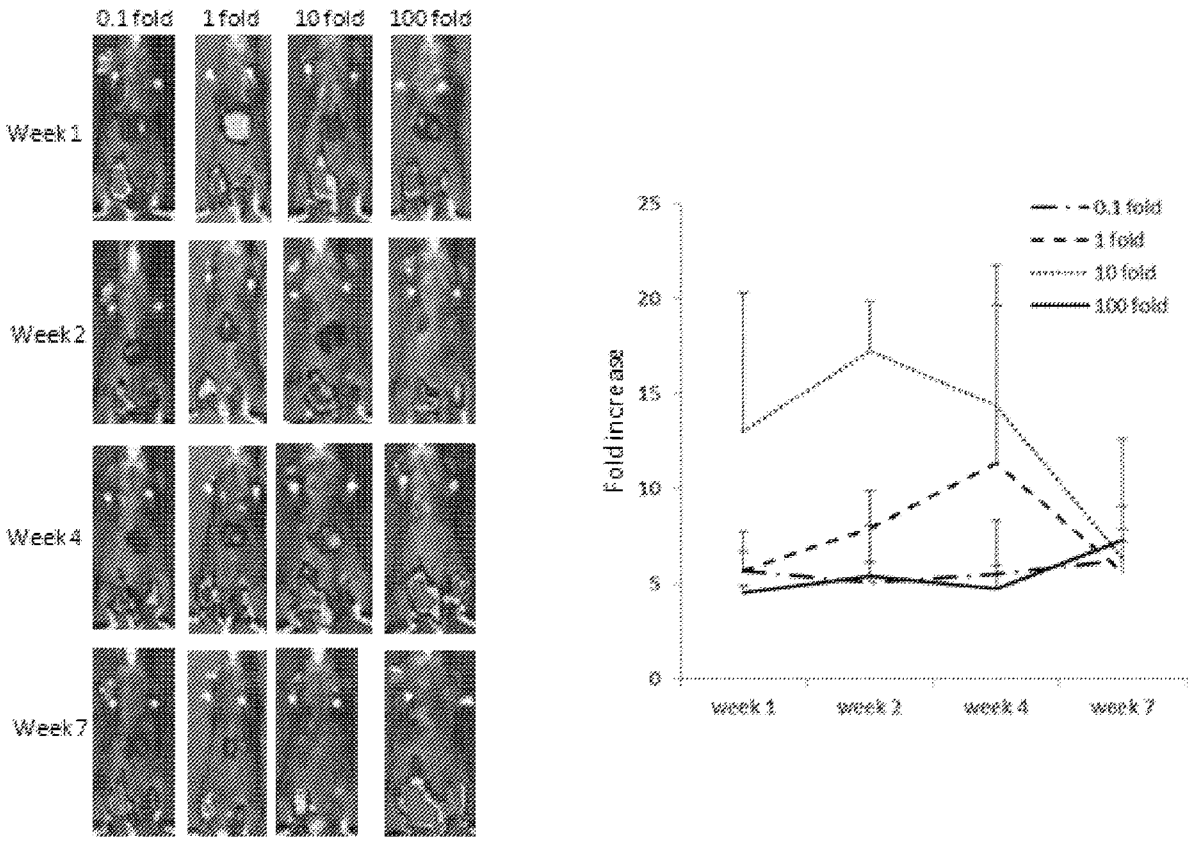
0.1 fold
1 fold
10 fold
100 fold
Week 1
Week 2
Week 4
Week 7
Fold increase
25
20
15
10
5
0
week 1
week 2
week 4
week 7
0.1 fold
1 fold
10 fold
100 fold

**Fig. 13**

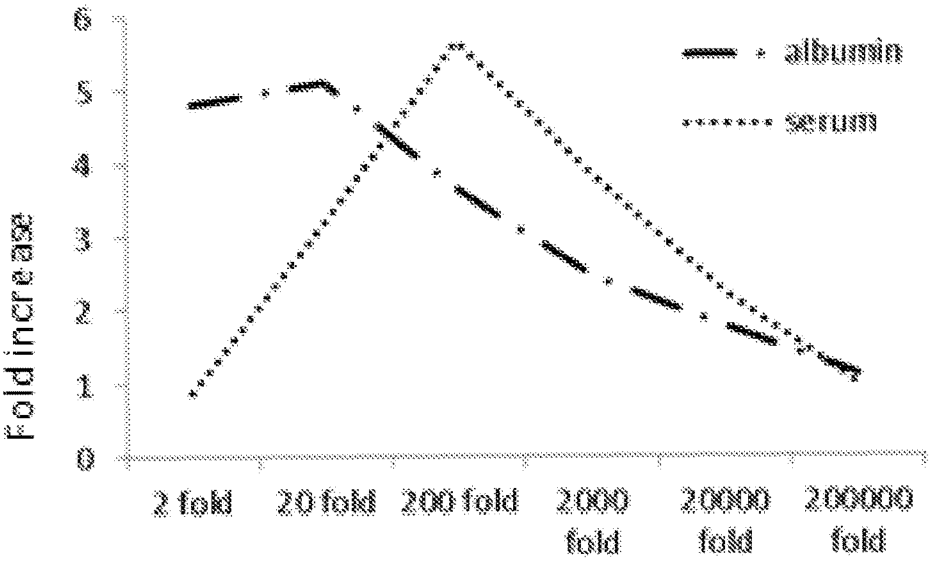


Fig. 14a

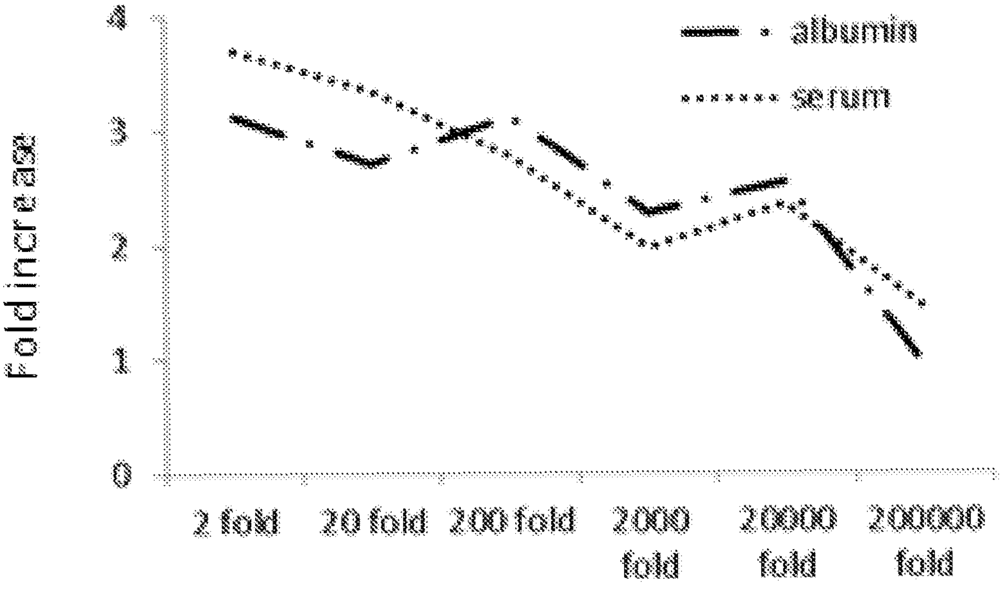


Fig. 14b

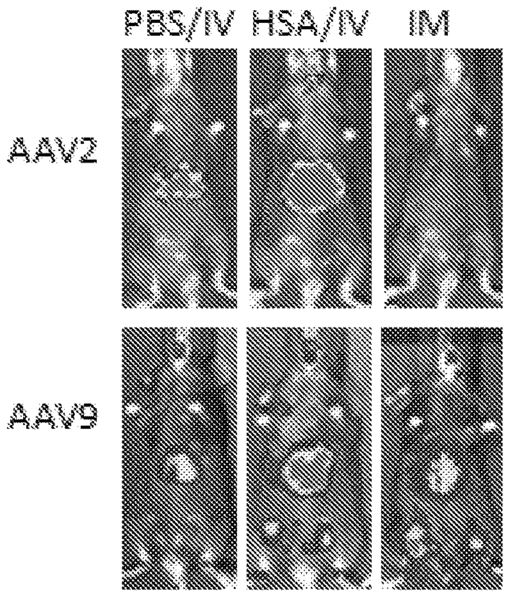


Fig. 14c

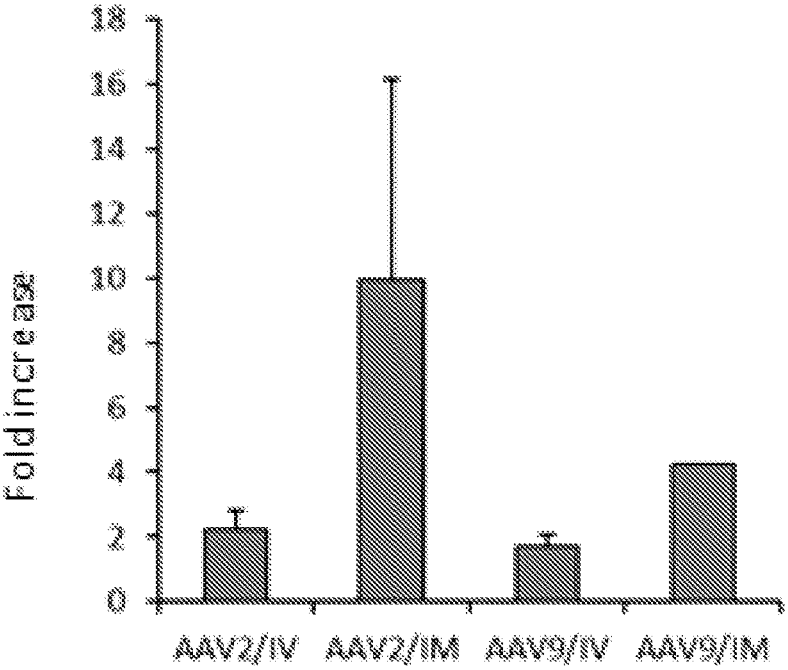


Fig. 14d

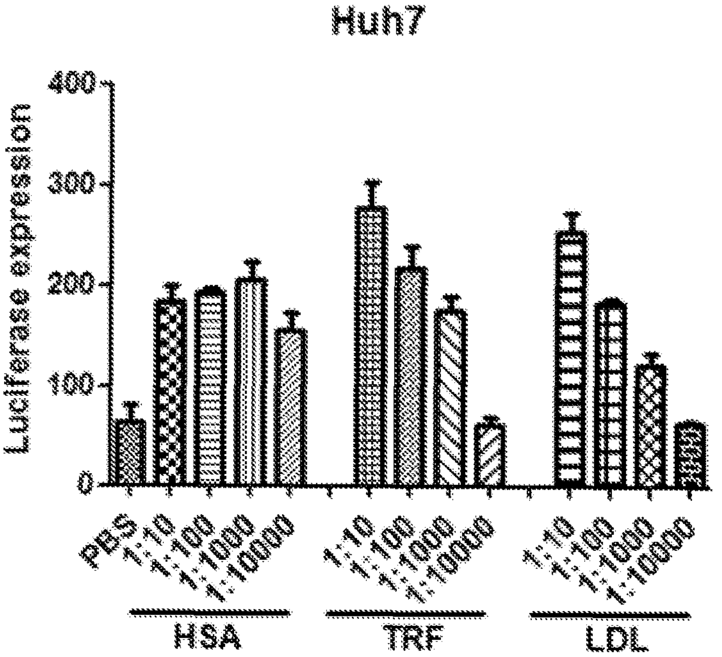


Fig. 15A

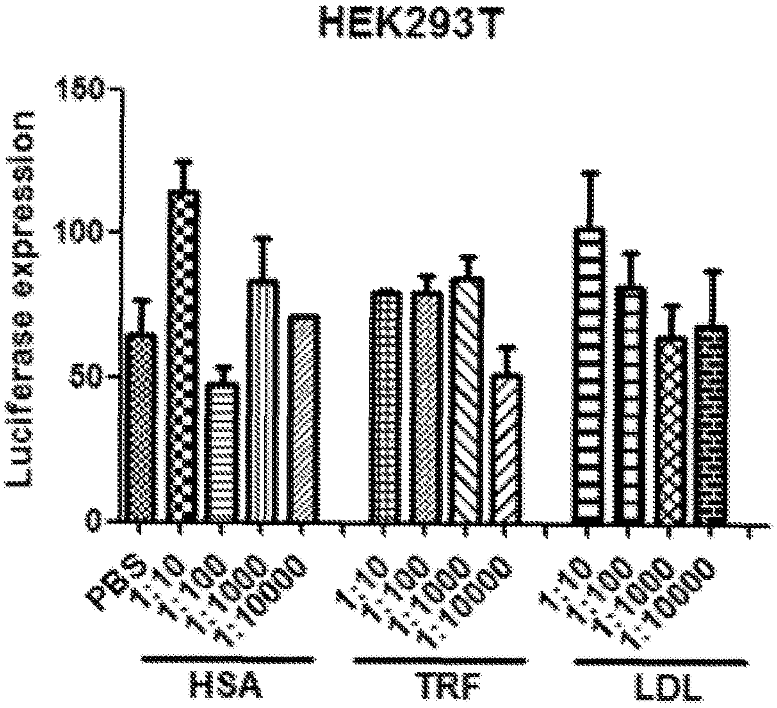


Fig. 15B

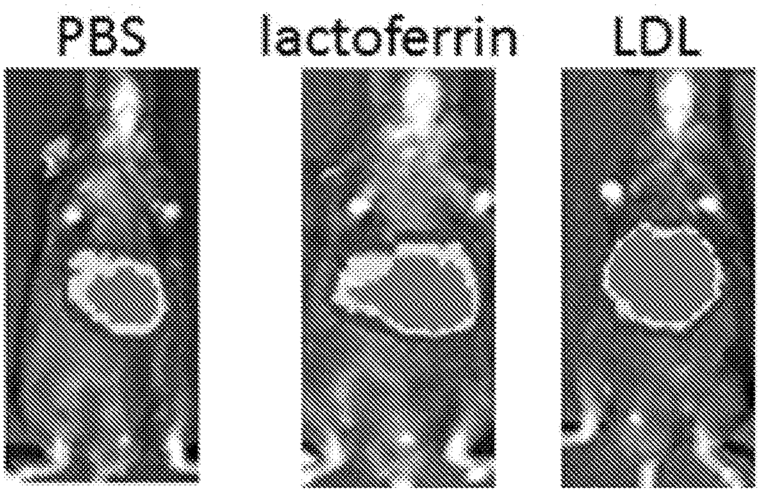


Fig. 16A

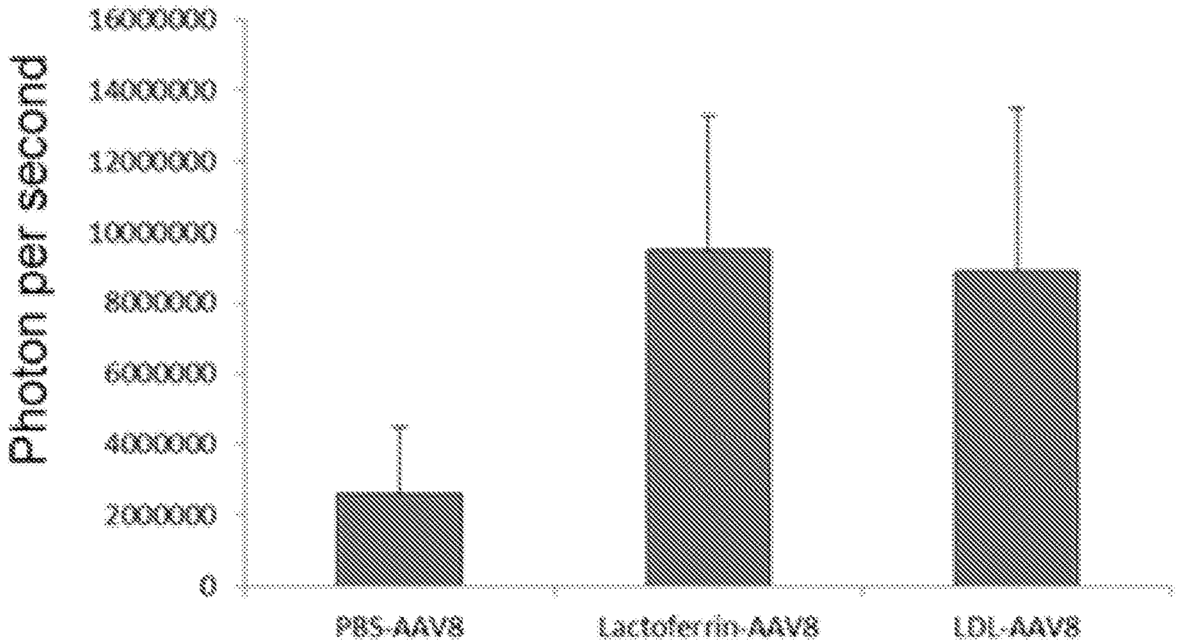


Fig. 16B

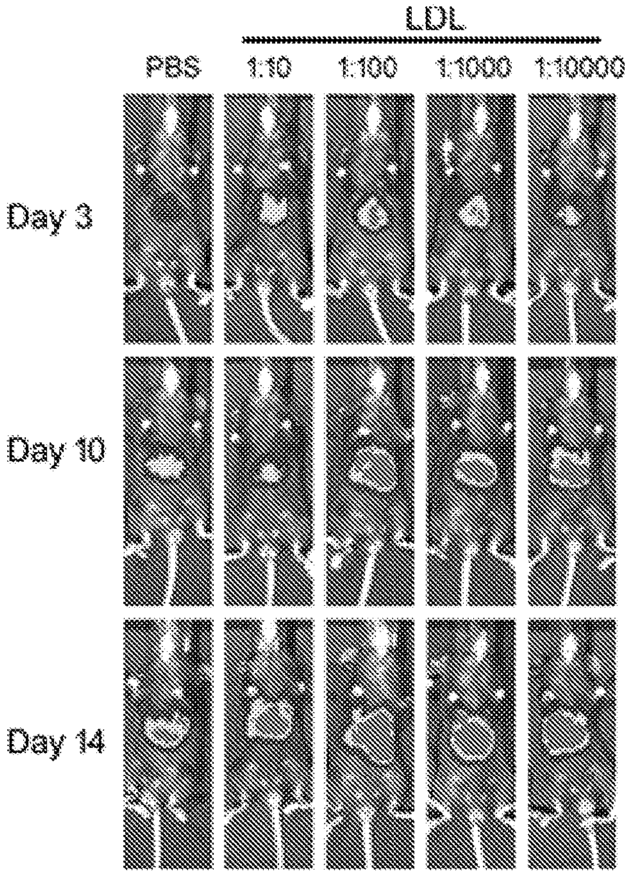


Fig. 17A

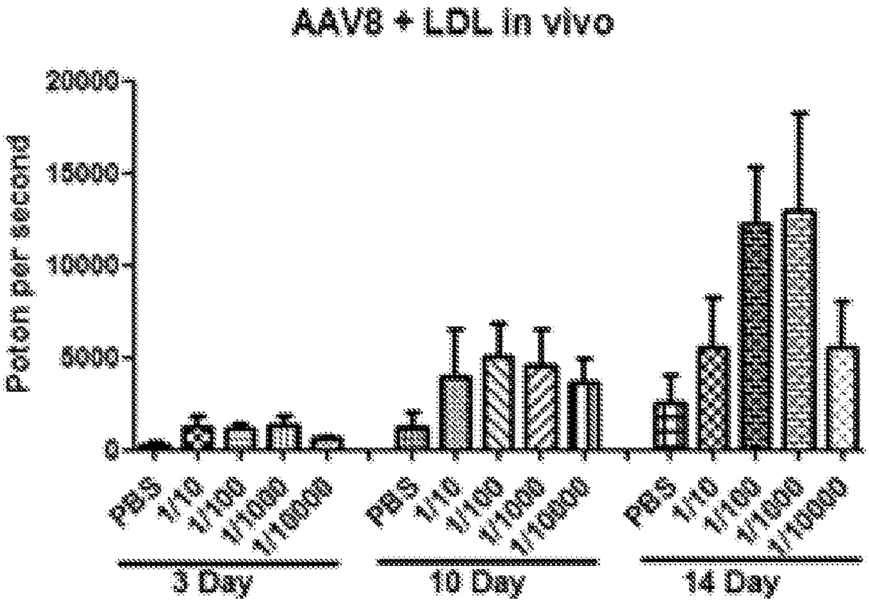


Fig. 17B

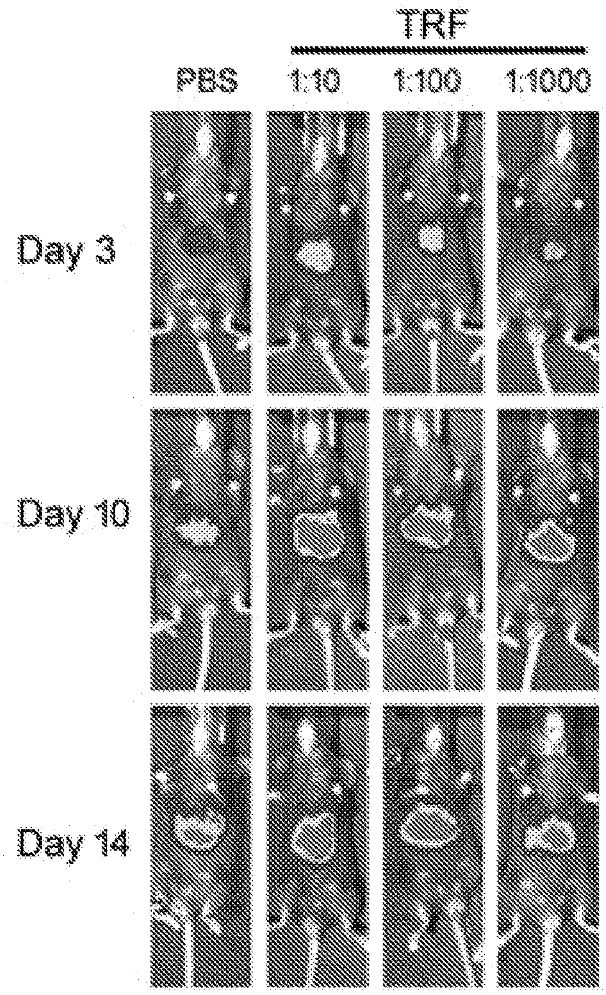


Fig. 17C

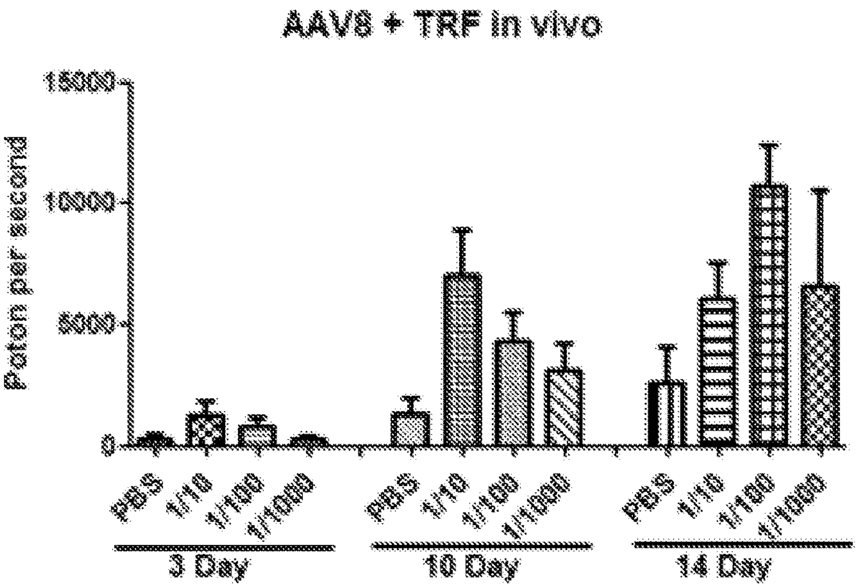


Fig. 17D

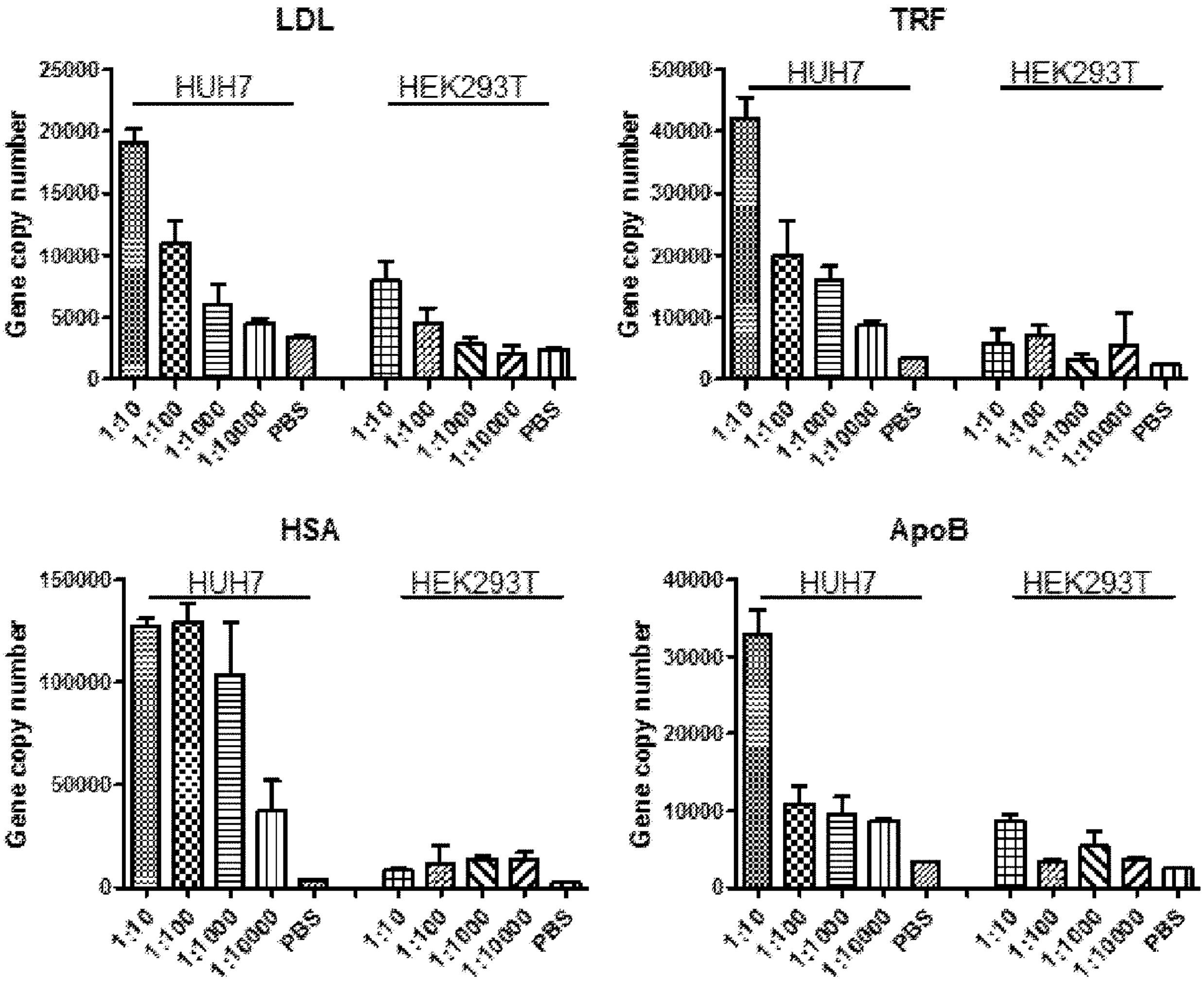
LDL
TRF
HSA
ApoB
HUH7
HEK293T
Gene copy number
1:10
1:100
1:1000
1:10000
PBS
25000
20000
15000
10000
5000
0
50000
40000
30000
20000
10000
150000
100000
50000
40000

Fig. 18

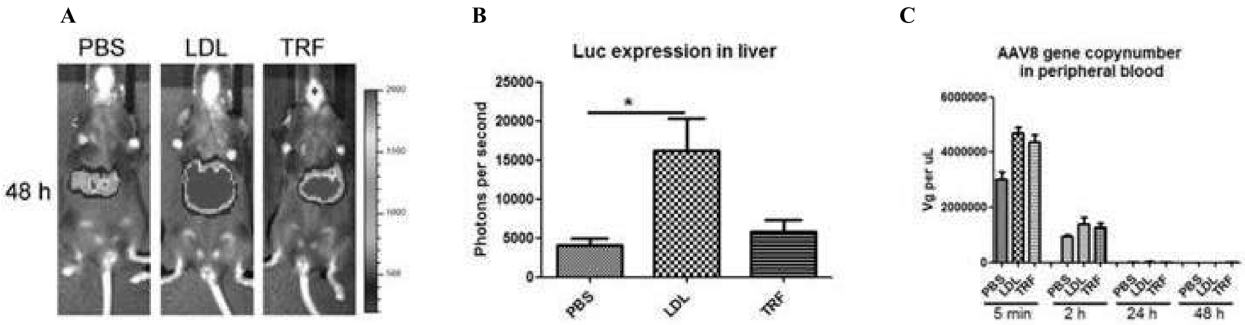
A
PBS
LDL
TRF
48 h
B
Luc expression in liver
Photons per second
25000
20000
15000
10000
5000
0
*
PBS
LDL
TRF
C
AAV8 gene copynumber
in peripheral blood
Vg per uL
6000000
4000000
2000000
0
PBS
LDL
TRF
5 min
2 h
24 h
48 h

Fig. 19

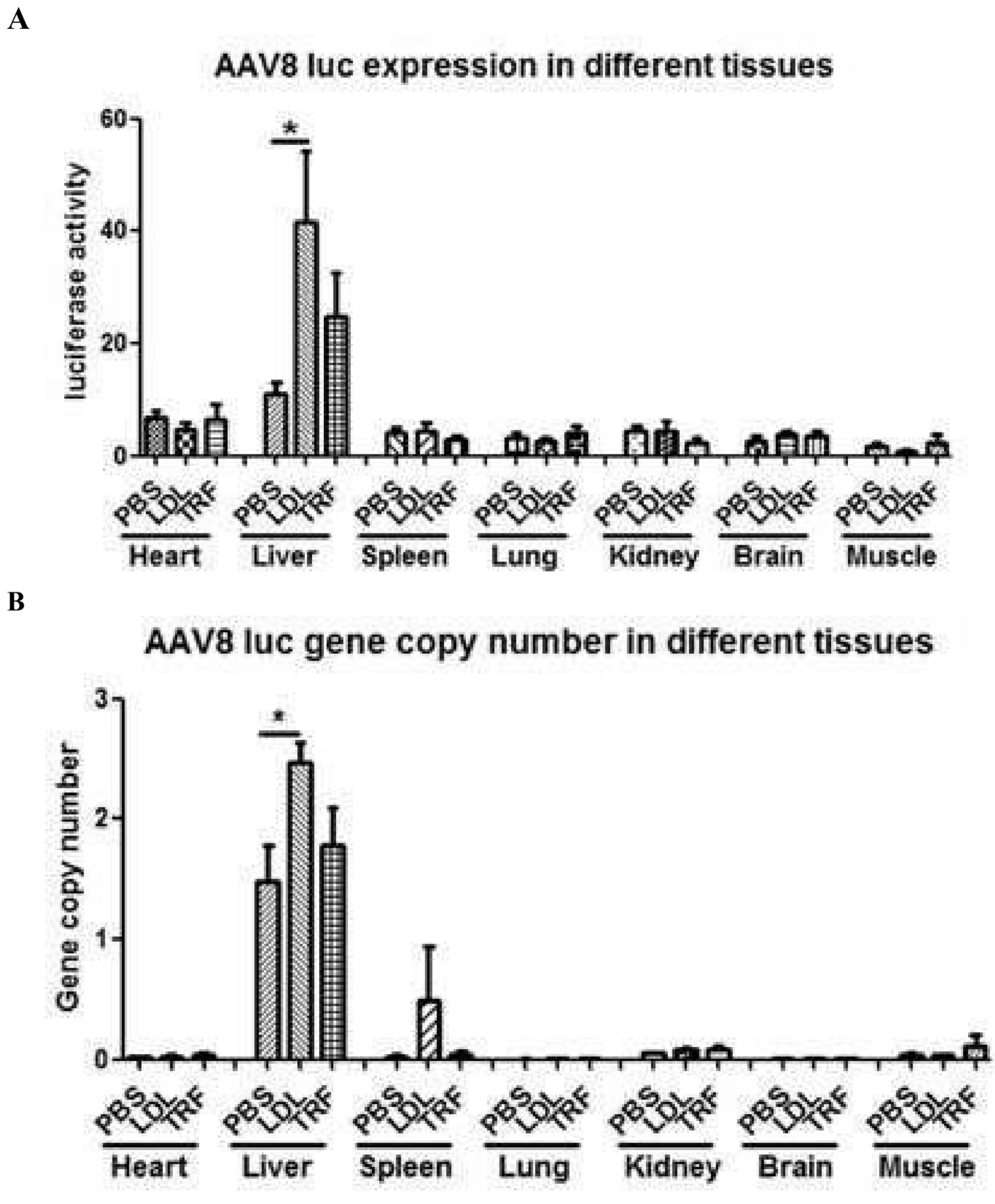
A
AAV8 luc expression in different tissues
luciferase activity
60
40
20
0
*
PBS LDL TRF
Heart
Liver
Spleen
Lung
Kidney
Brain
Muscle
B
AAV8 luc gene copy number in different tissues
Gene copy number
3
2
1
0
*
PBS LDL TRF
Heart
Liver
Spleen
Lung
Kidney
Brain
Muscle

Fig. 20

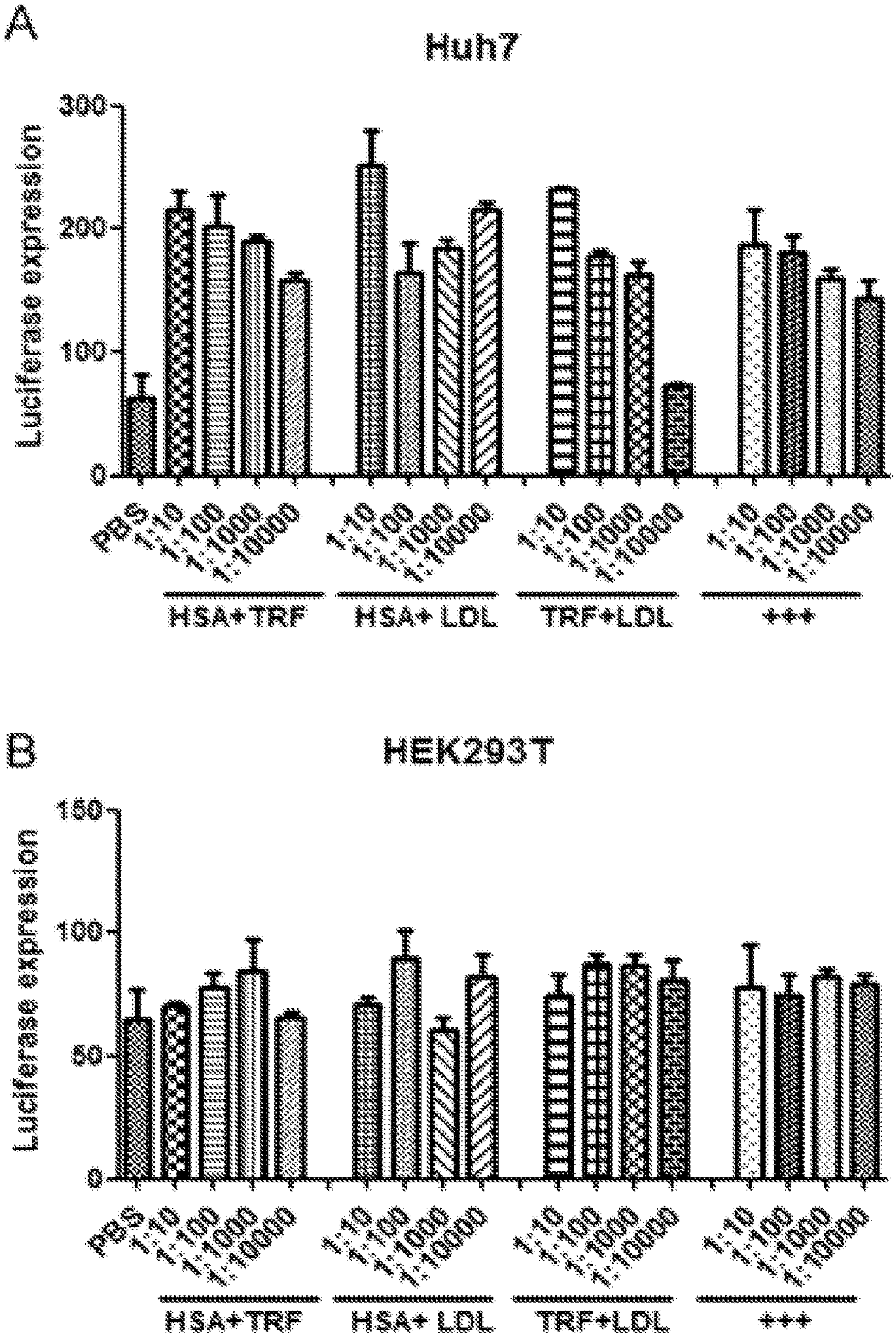
A
Huh7
Luciferase expression
300
200
100
0
PBS
1:10
1:100
1:1000
1:10000
HSA+TRF
HSA+ LDL
TRF+LDL
+++
B
HEK293T
Luciferase expression
150
100
50
0

Fig. 21

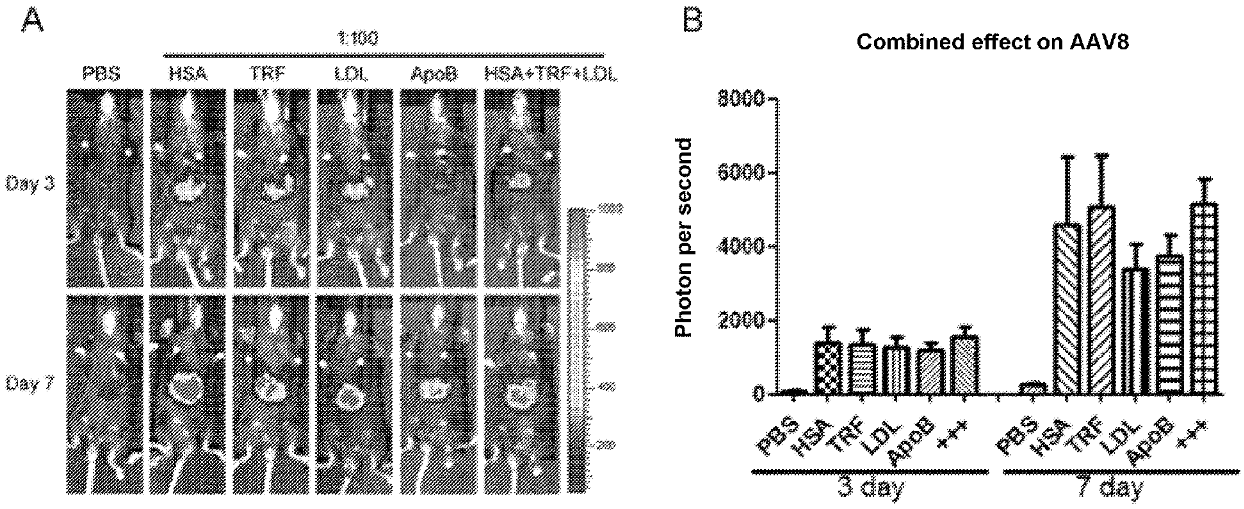
A
1:100
PBS
HSA
TRF
LDL
ApoB
HSA+TRF+LDL
Day 3
Day 7
B
Combined effect on AAV8
Photon per second
8000
6000
4000
2000
0
PBS
HSA
TRF
LDL
ApoB
+++
3 day
7 day

Fig. 22

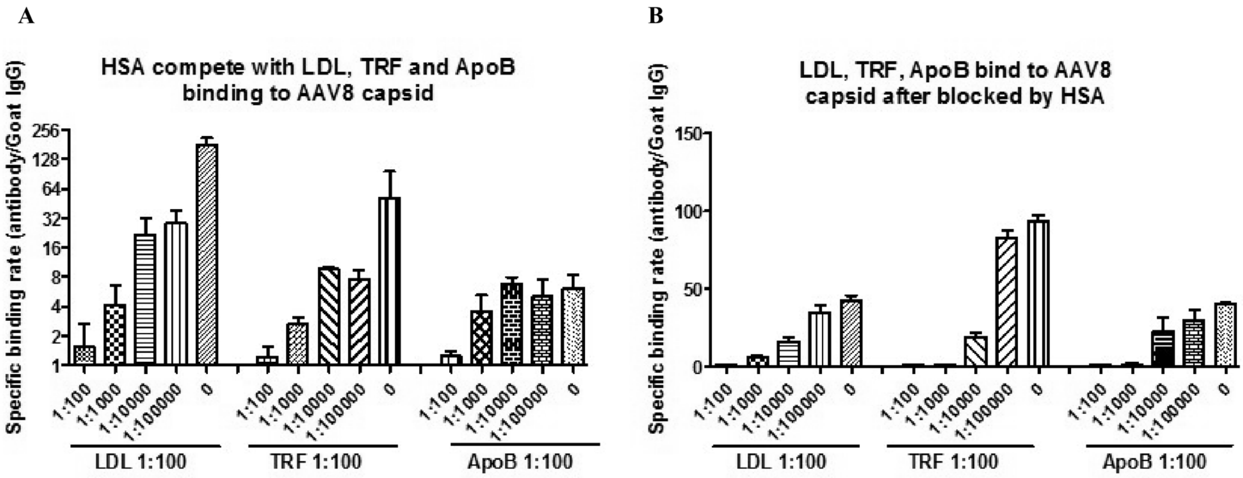
A
HSA compete with LDL, TRF and ApoB binding to AAV8 capsid
Specific binding rate (antibody/Goat IgG)
256
128
64
32
16
8
4
2
1
1:100
1:1000
1:10000
1:100000
0
LDL 1:100
TRF 1:100
ApoB 1:100
B
LDL, TRF, ApoB bind to AAV8 capsid after blocked by HSA
Specific binding rate (antibody/Goat IgG)
150
100
50
0
1:100
1:1000
1:10000
1:100000
0
LDL 1:100
TRF 1:100
ApoB 1:100

Fig. 23

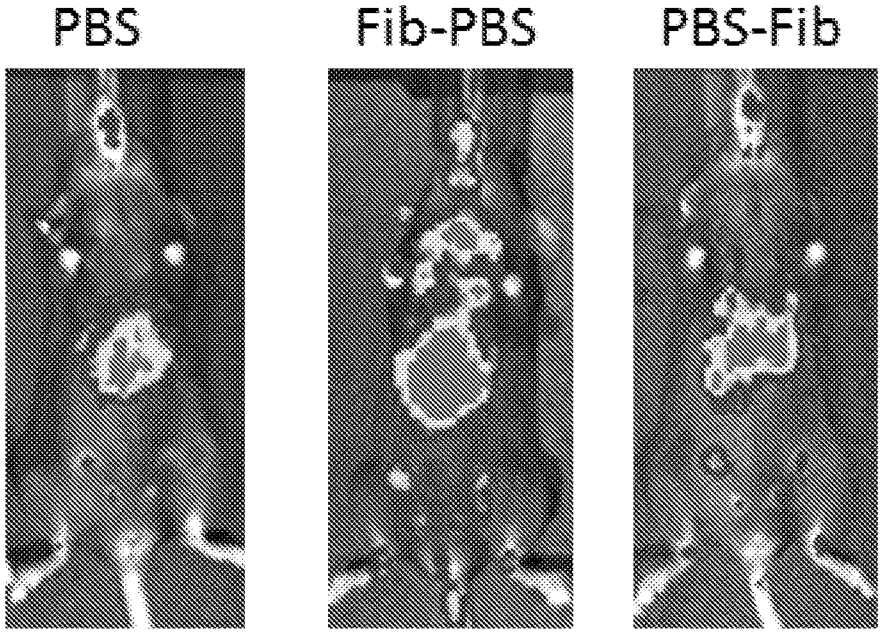


Fig. 24A

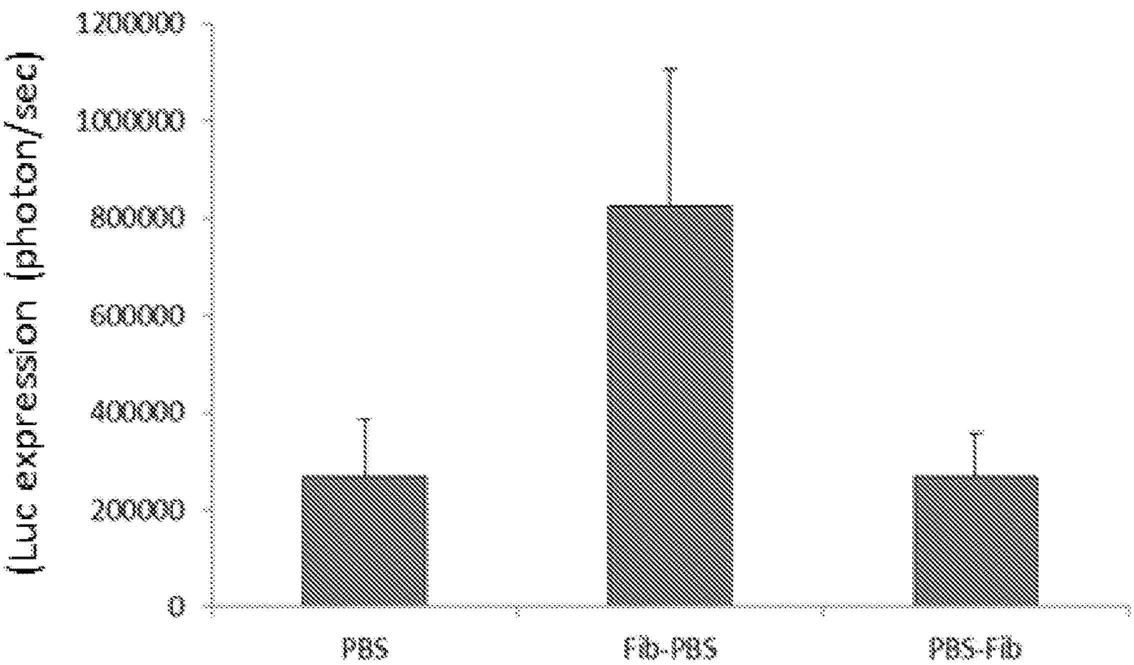


Fig. 24B

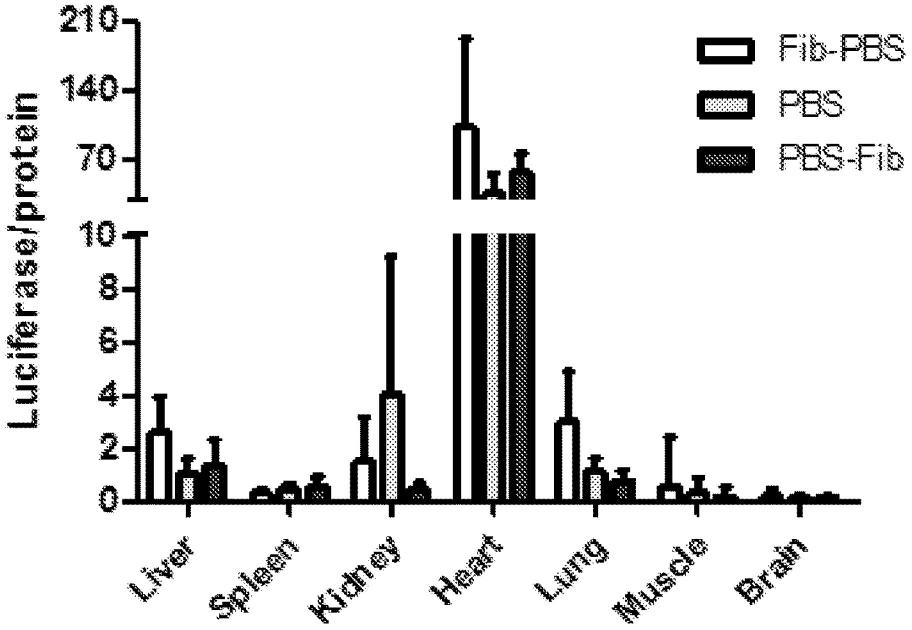


Fig. 25A

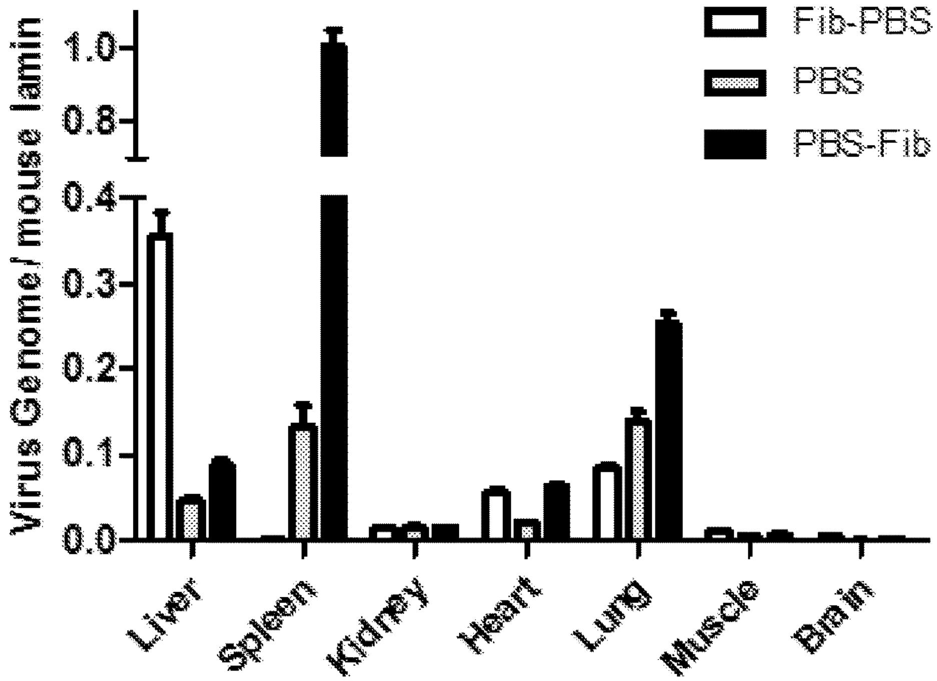


Fig. 25B

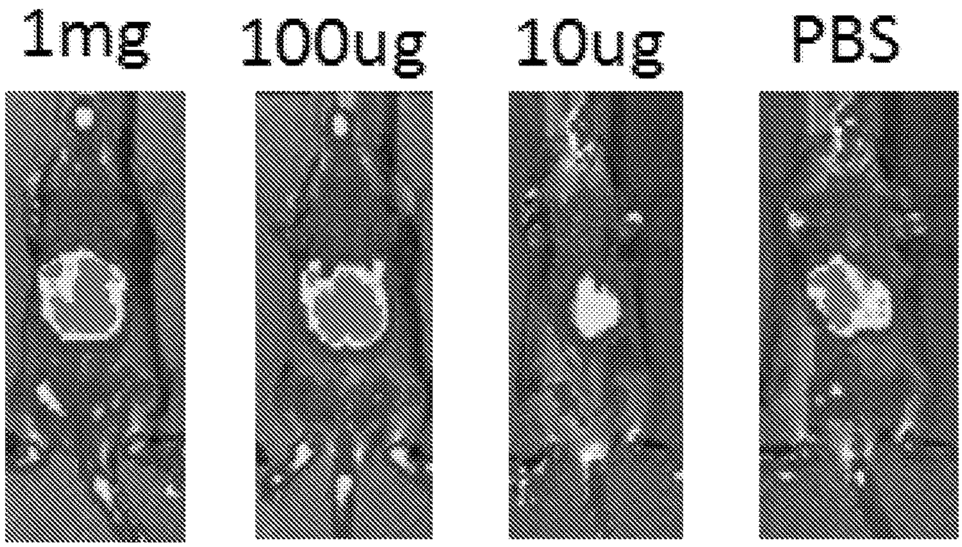


Fig. 26A

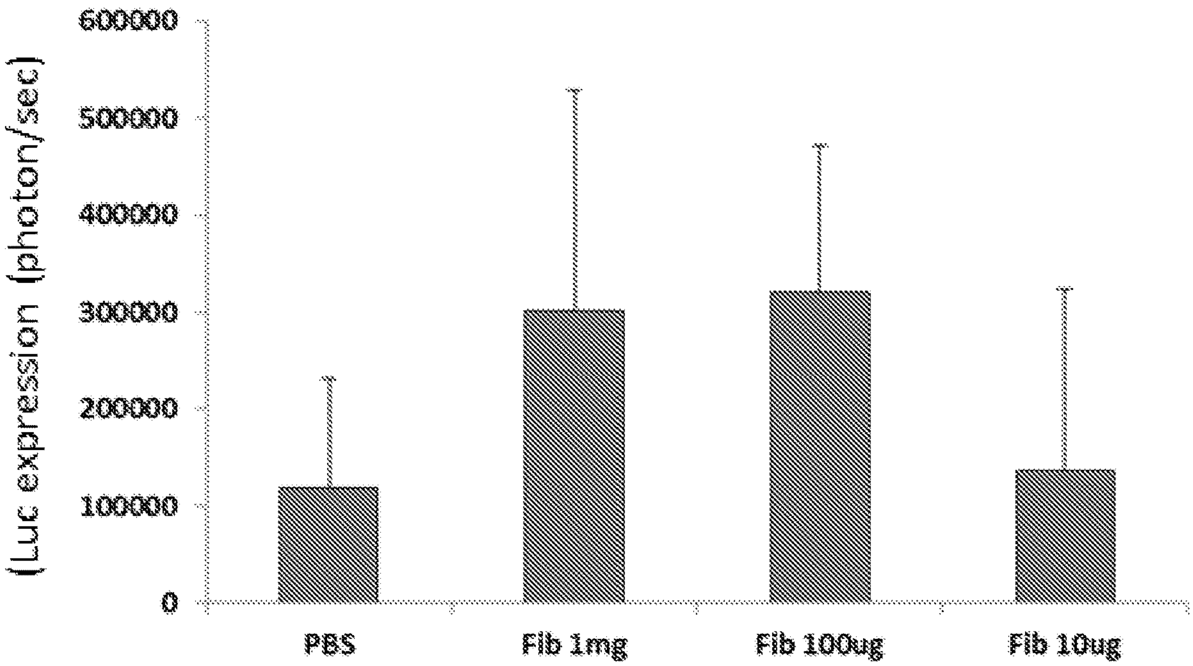


Fig. 26B

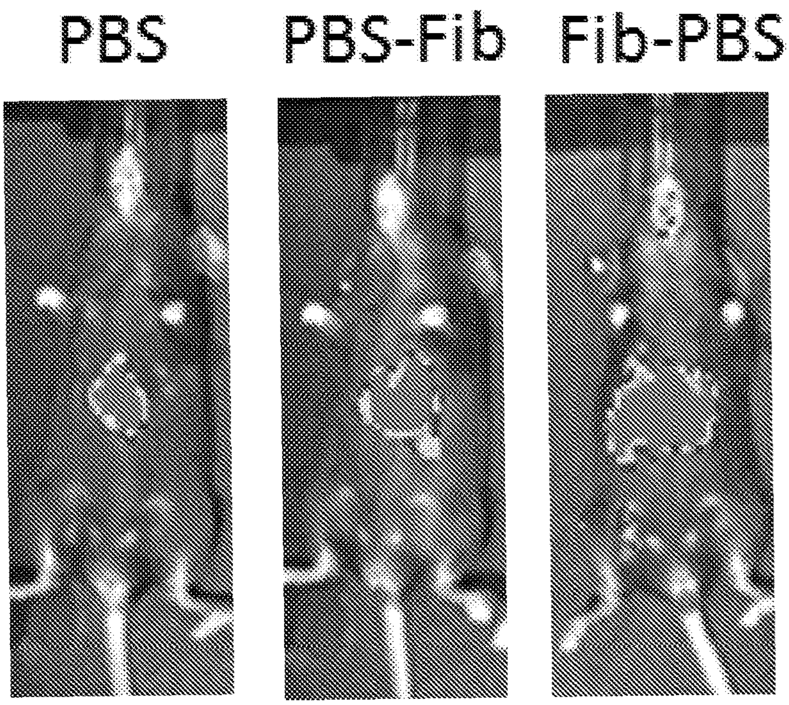


Fig. 27A

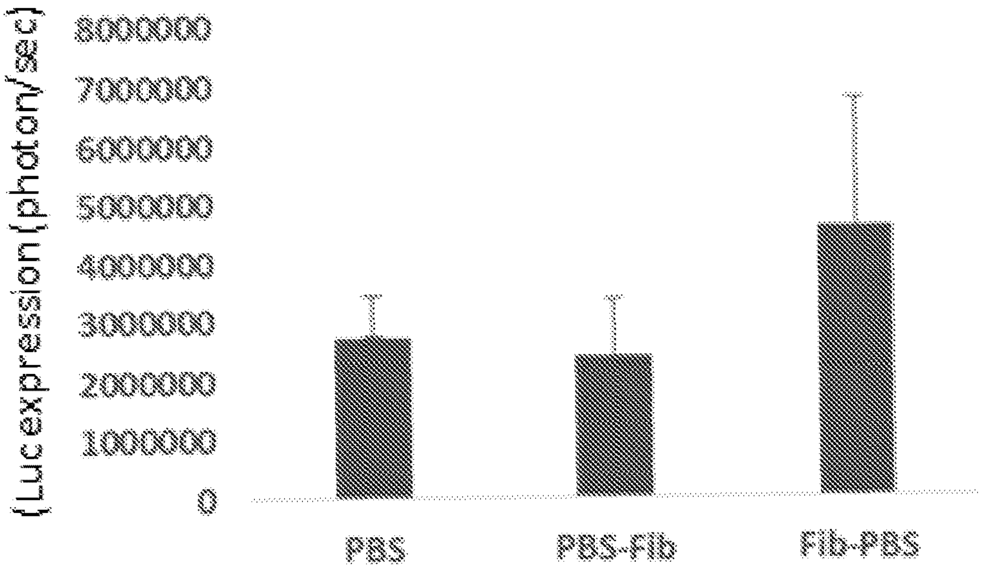


Fig. 27B

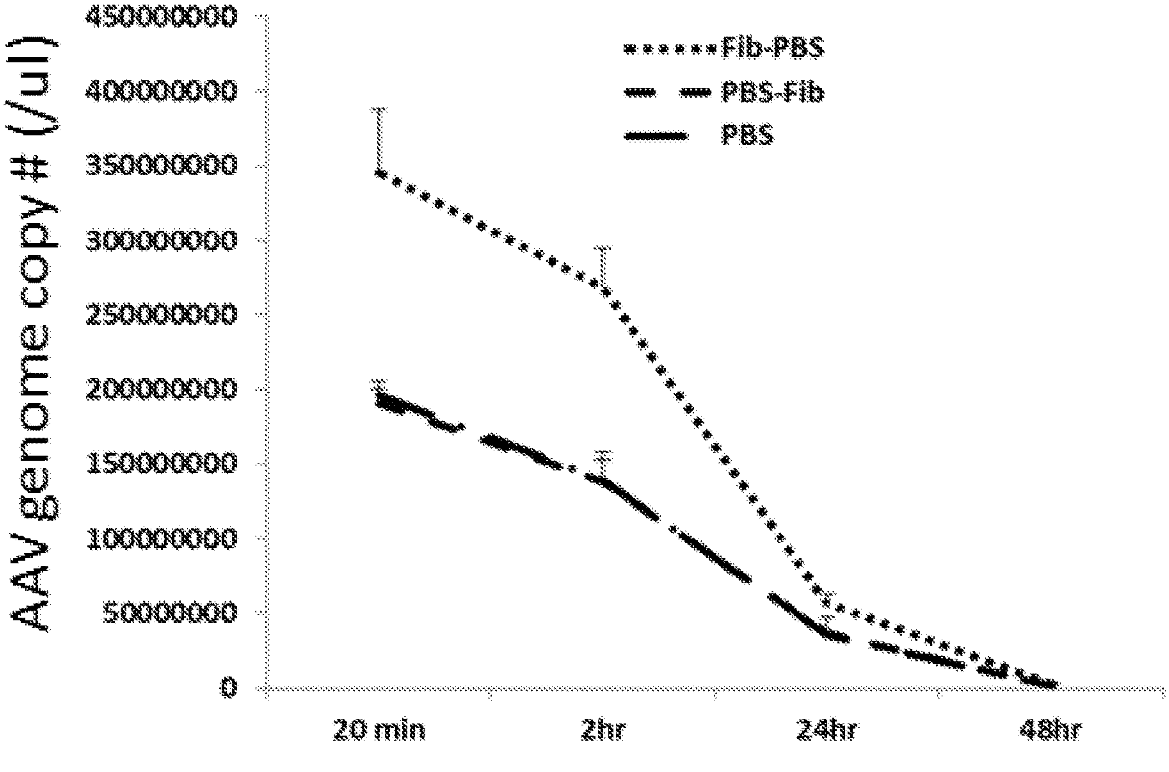
AAV genome copy # (/ul)
450000000
400000000
350000000
300000000
250000000
200000000
150000000
100000000
50000000
0
20 min
2hr
24hr
48hr
Fib-PBS
PBS-Fib
PBS

Fig. 27C

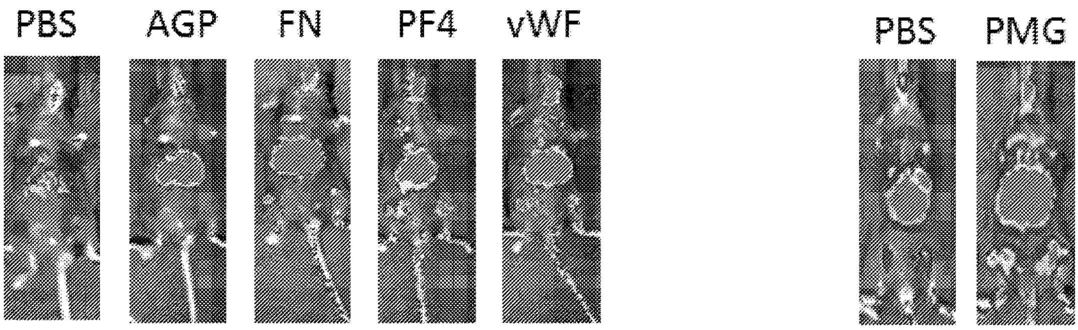


Fig. 28A

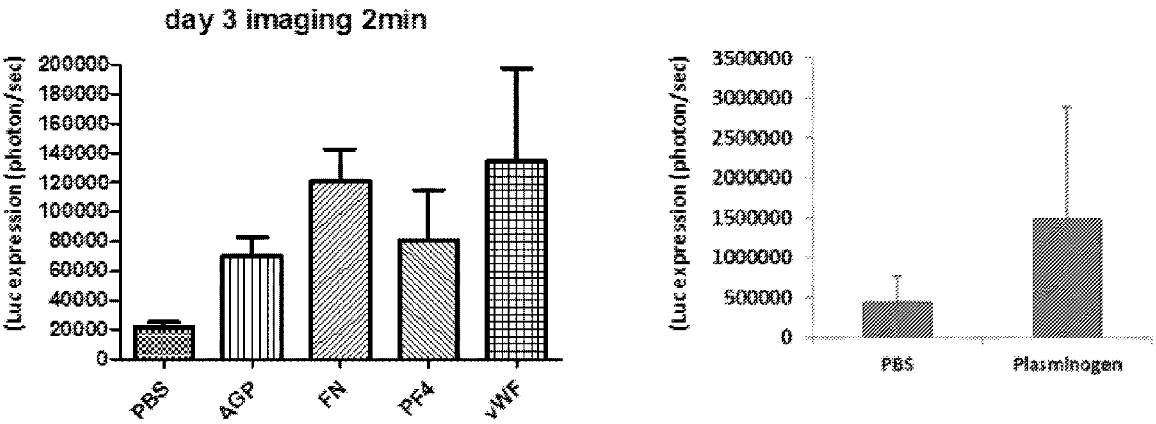


Fig. 28B

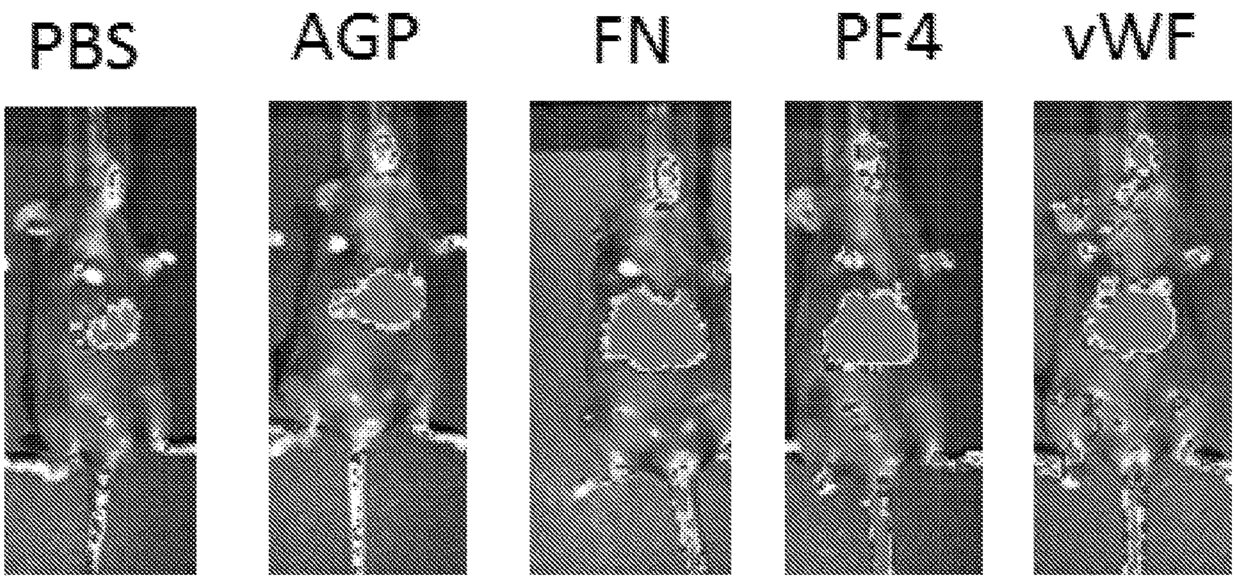


Fig. 29A

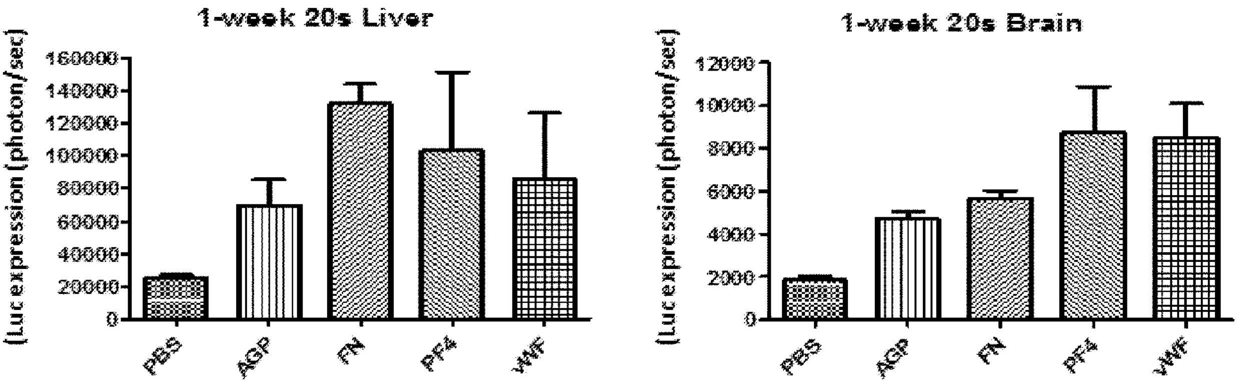


Fig. 29B

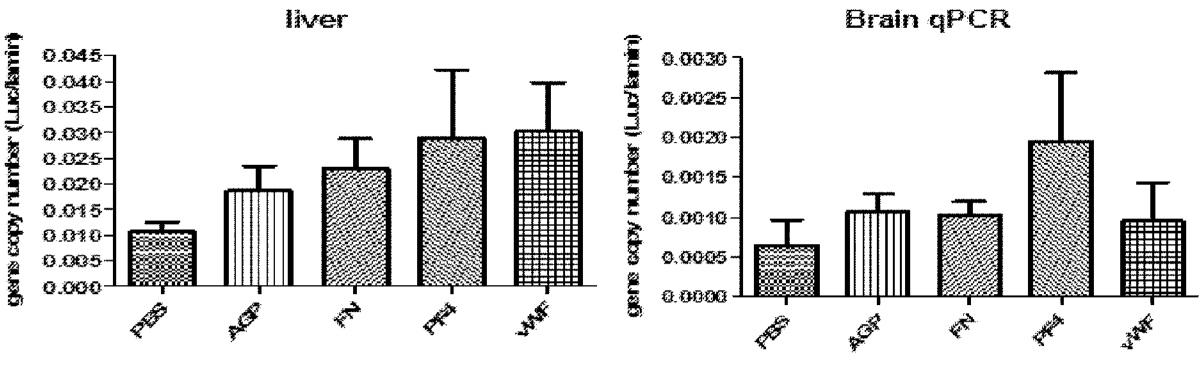


Fig. 29C

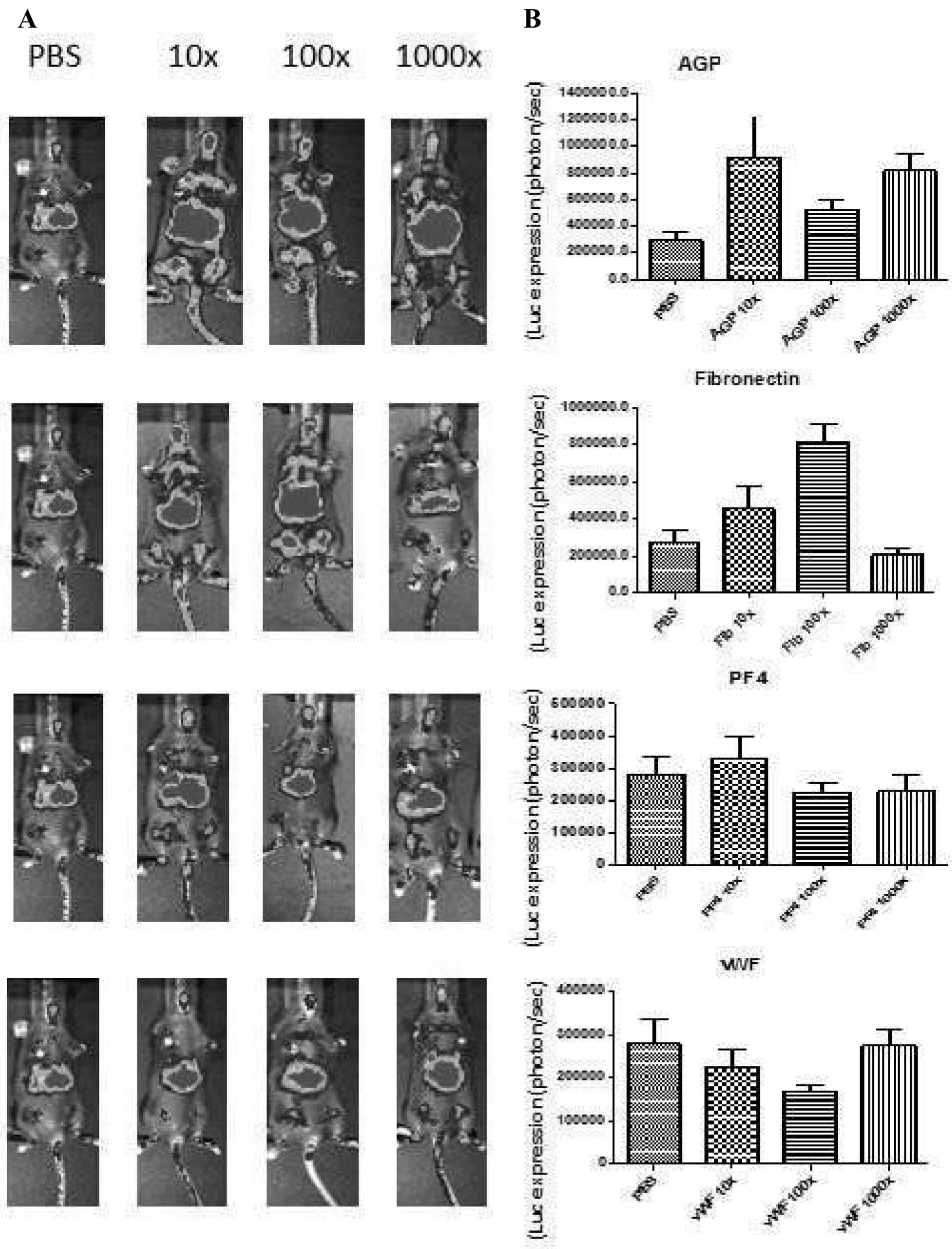
A
PBS
10x
100x
1000x
B
AGP
(Luc expression(photon/sec)
1400000.0
1200000.0
1000000.0
800000.0
600000.0
400000.0
200000.0
0.0
PBS
AGP 10x
AGP 100x
AGP 1000x
Fibronectin
(Luc expression(photon/sec)
1000000.0
400000.0
200000.0
0.0
PBS
Fib 10x
Fib 100x
Fib 1000x
PF4
(Luc expression(photon/sec)
500000
400000
300000
200000
100000
0
VWF
(Luc expression(photon/sec)
400000
100000
0
PBS
VWF 10x
VWF 100x
VWF 1000x

Fig. 30

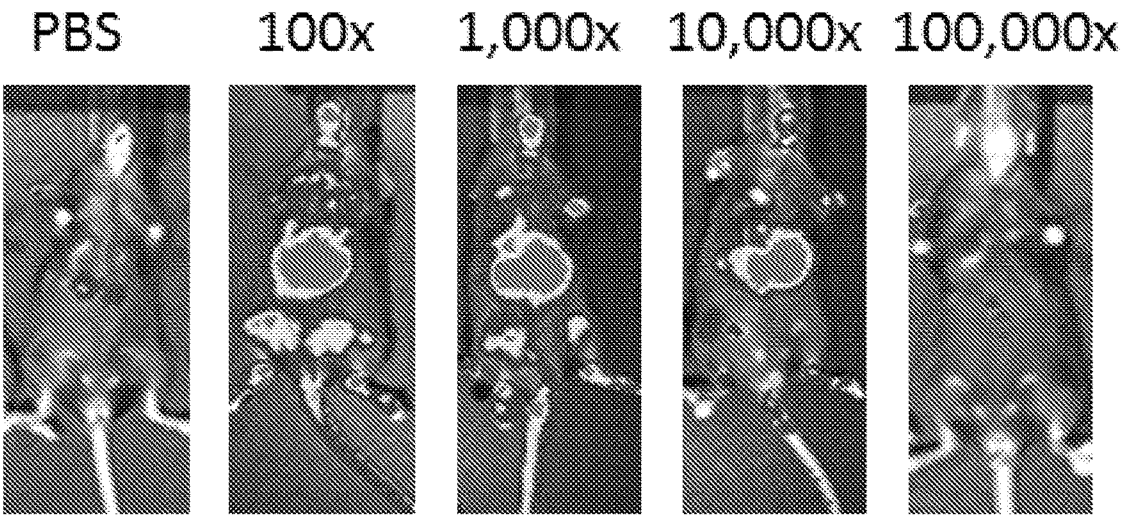


Fig. 31A

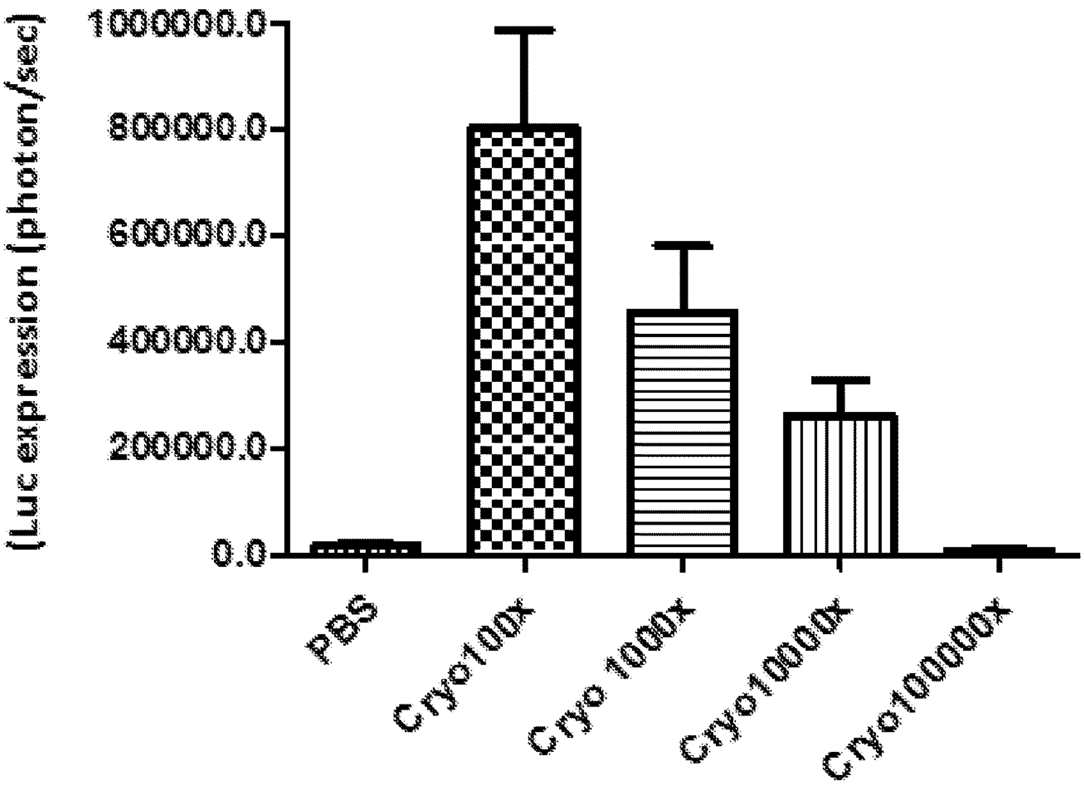


Fig. 31B

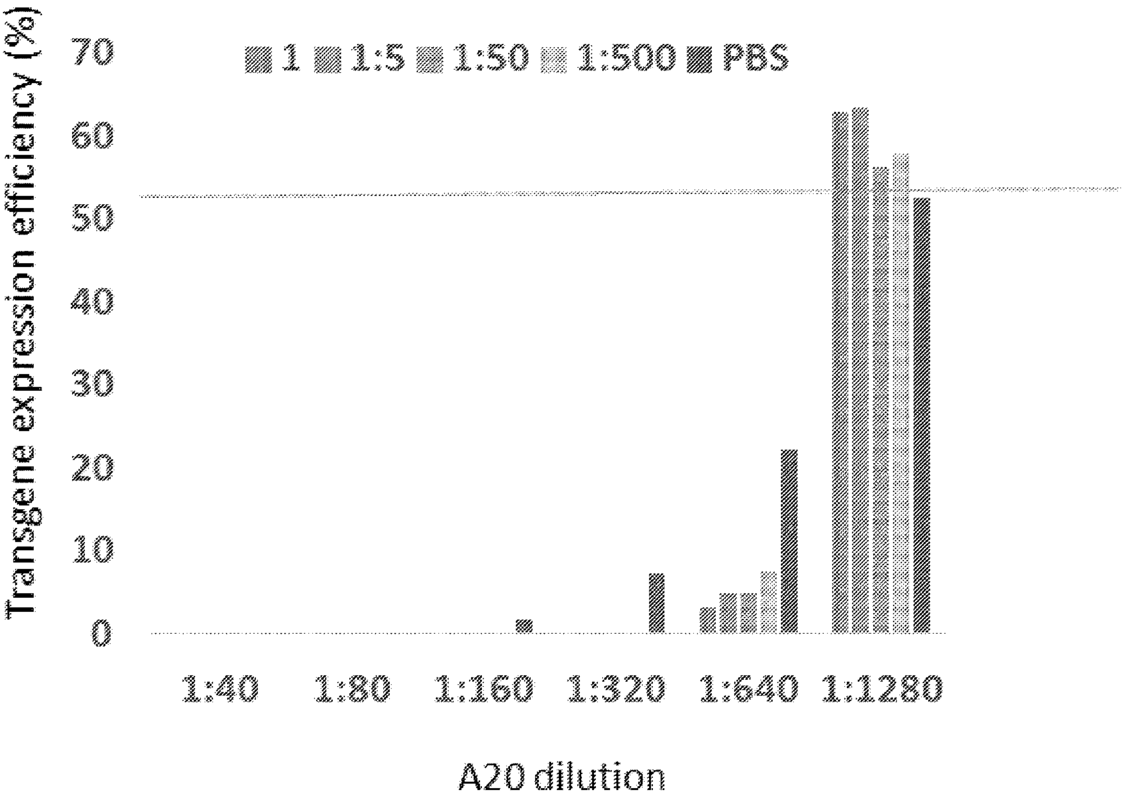
Transgene expression efficiency (%)
70
60
50
40
30
20
10
0
1 1:5 1:50 1:500 PBS
1:40 1:80 1:160 1:320 1:640 1:1280
A20 dilution

Fig. 32

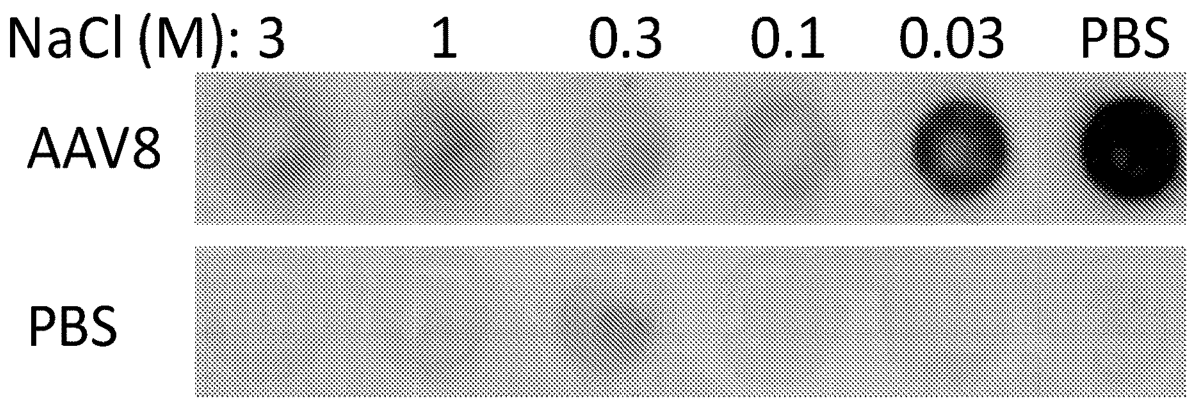


Fig. 33A

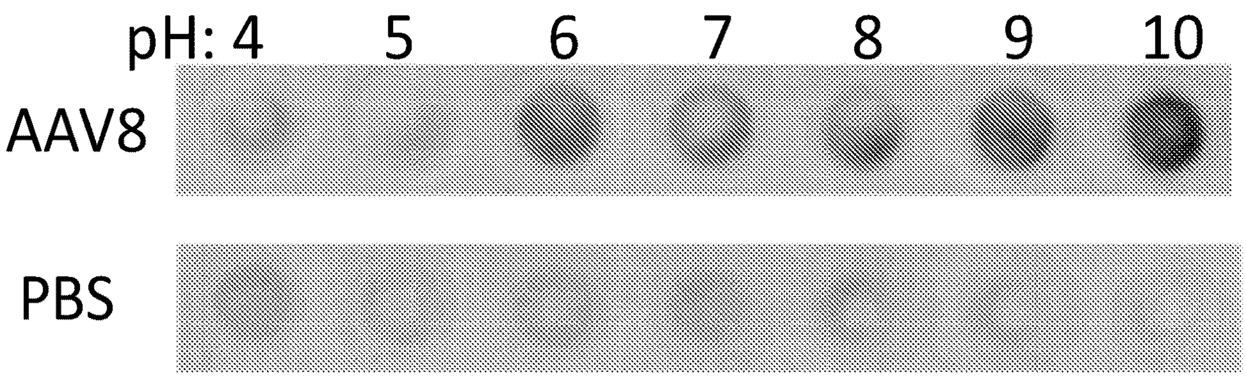


Fig. 33B

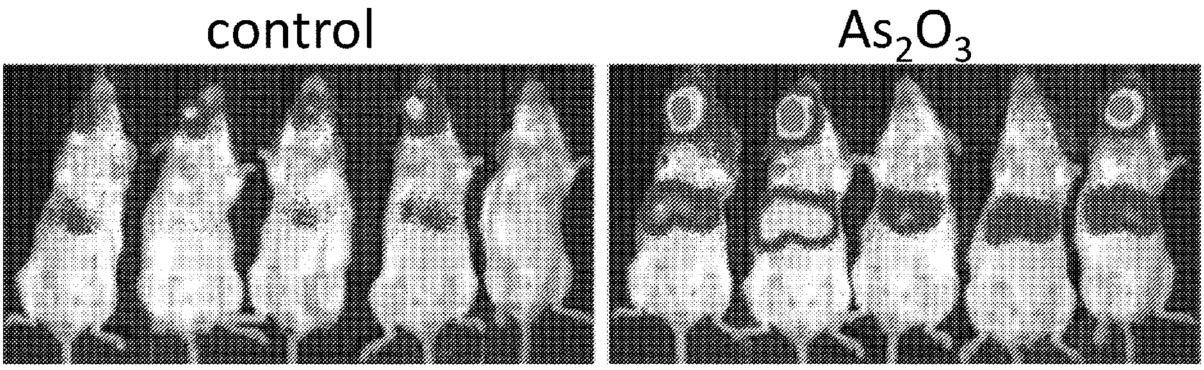


Fig. 34A

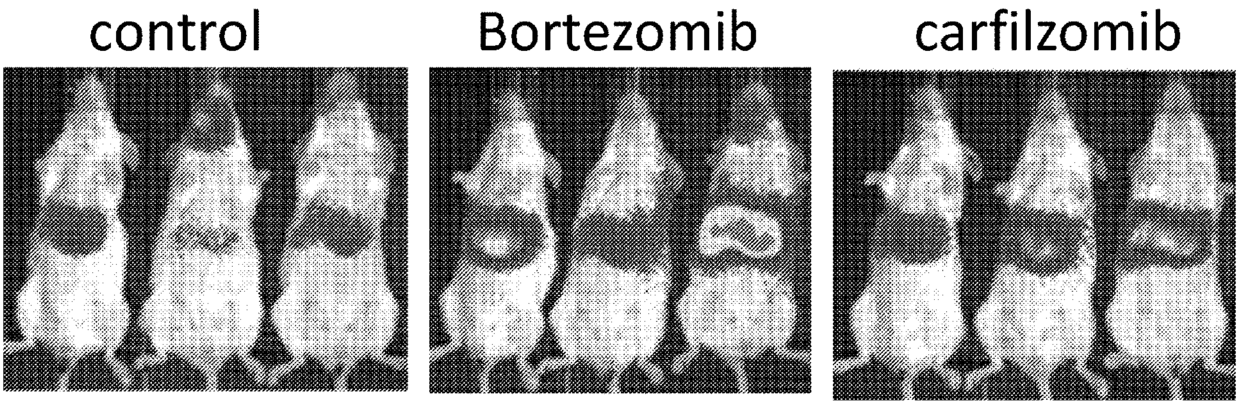


Fig. 34B

| Accession | Description | ΣCoverage | Σ# Proteins | Σ# Unique Peptides | Σ# Peptides |
|---|---|---|---|---|---|
| P02768 | Serum albumin | 52.55 | 13 | 34 | 36 |

Fig. 35A

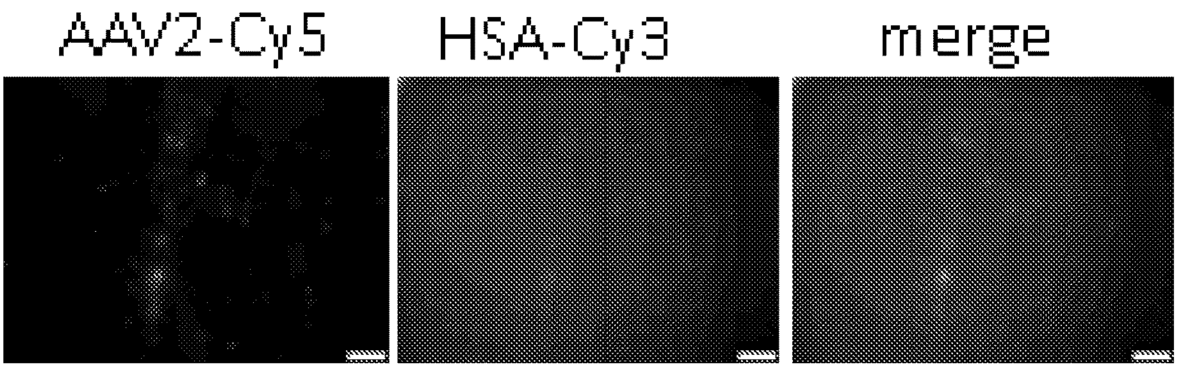


Fig. 35B

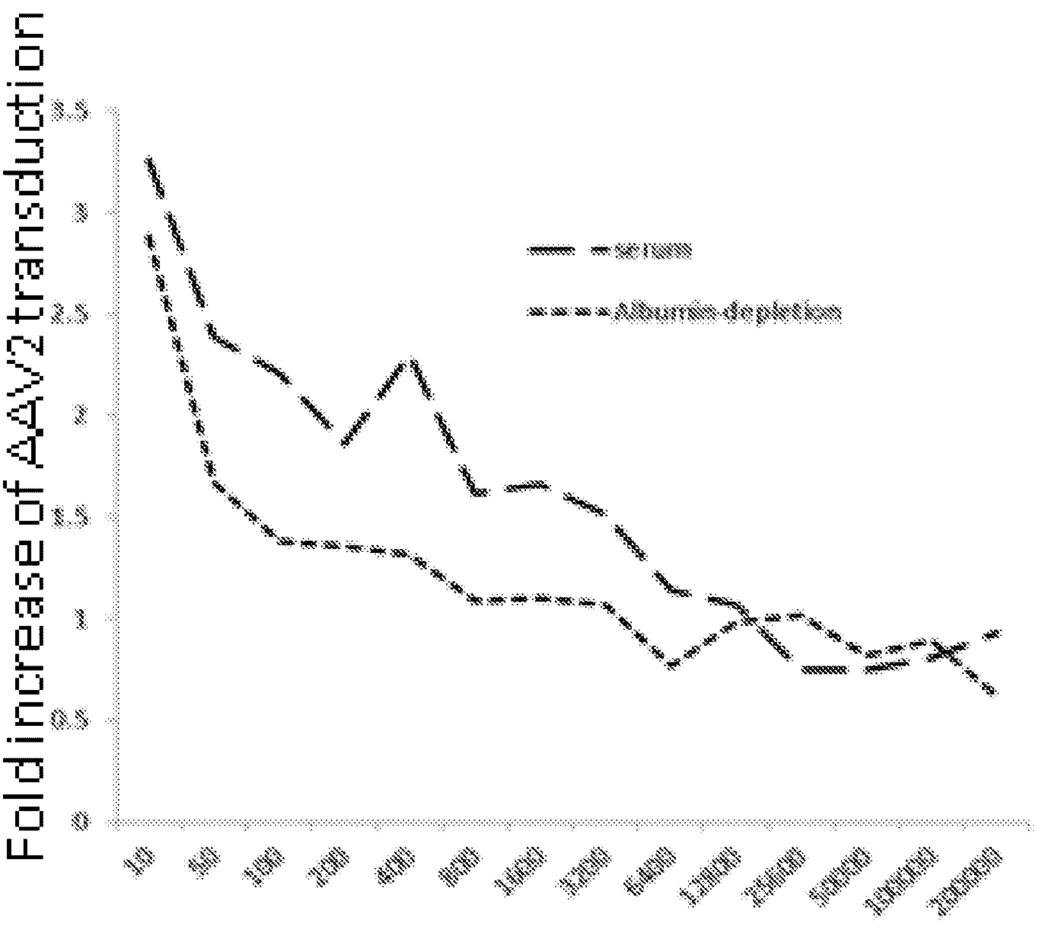
Fold increase of AAV2 transduction
3.5
3
2.5
2
1.5
1
0.5
0
serum
Albumin depletion

Fig. 35C

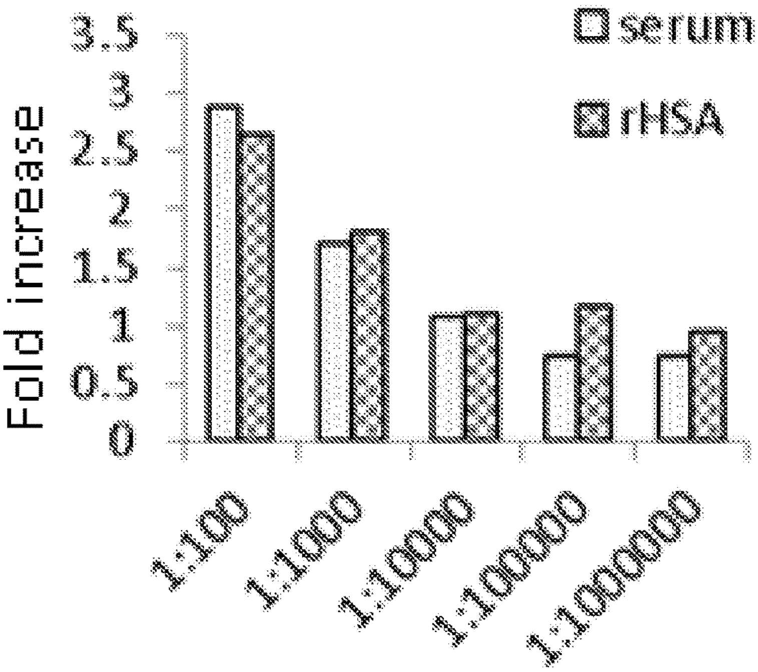


**Fig. 35D**

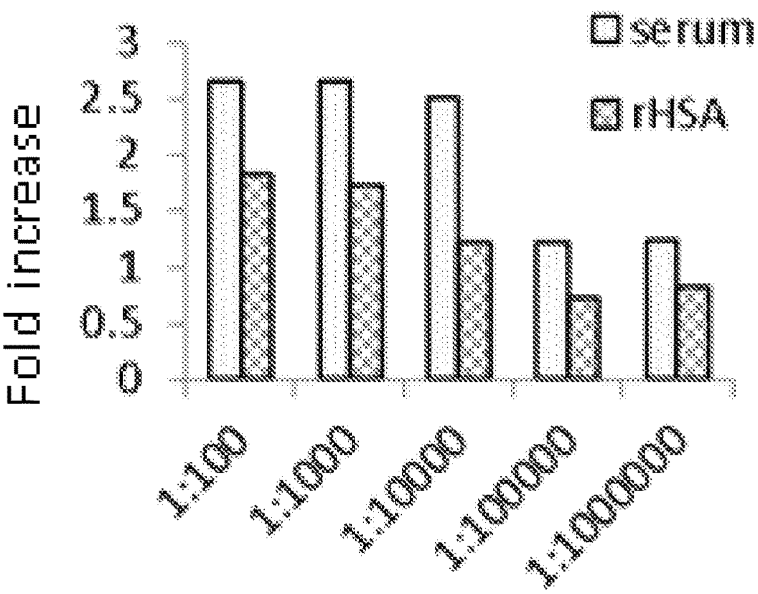


**Fig. 35E**

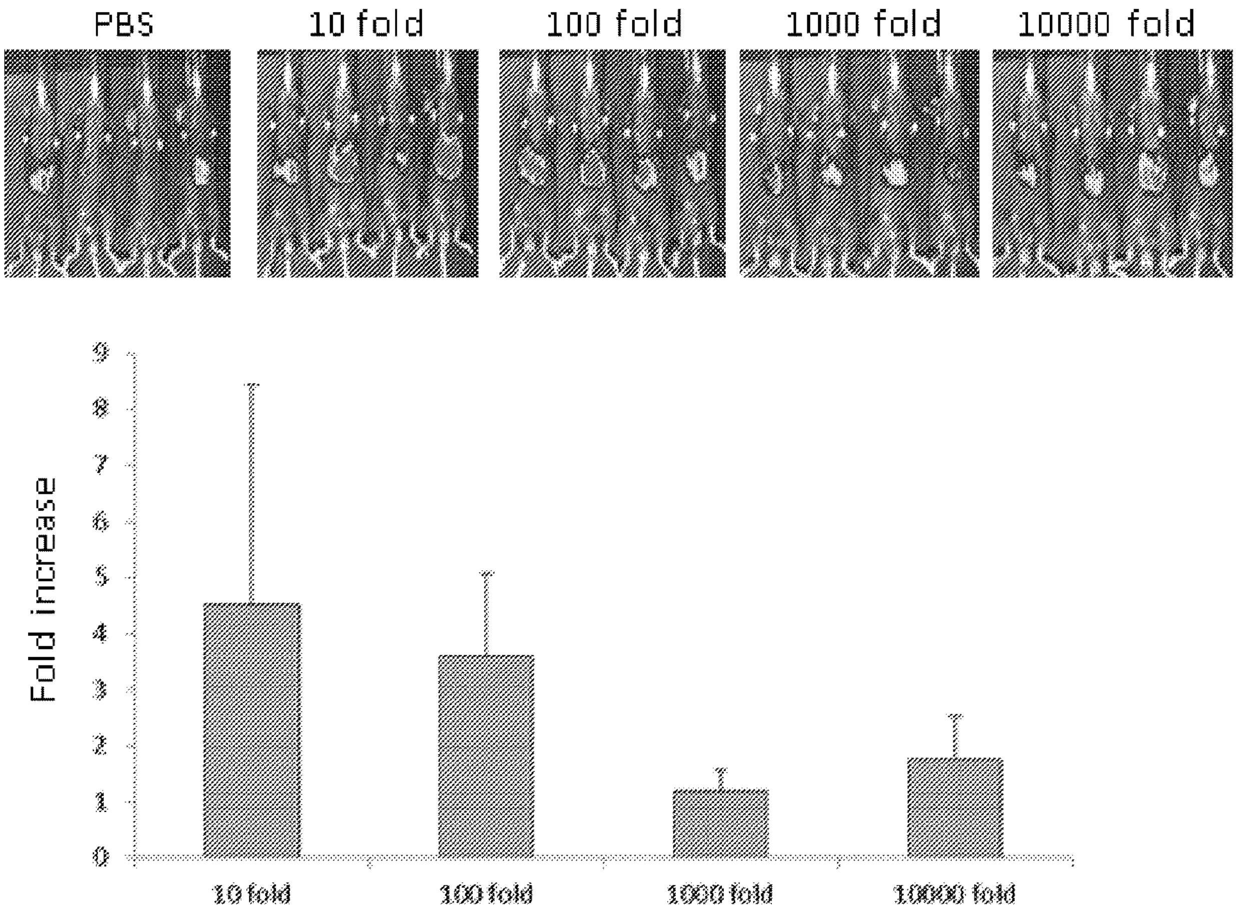
PBS
10 fold
100 fold
1000 fold
10000 fold
Fold increase
0
1
2
3
4
5
6
7
8
9
10 fold
100 fold
1000 fold
10000 fold

Fig. 35F

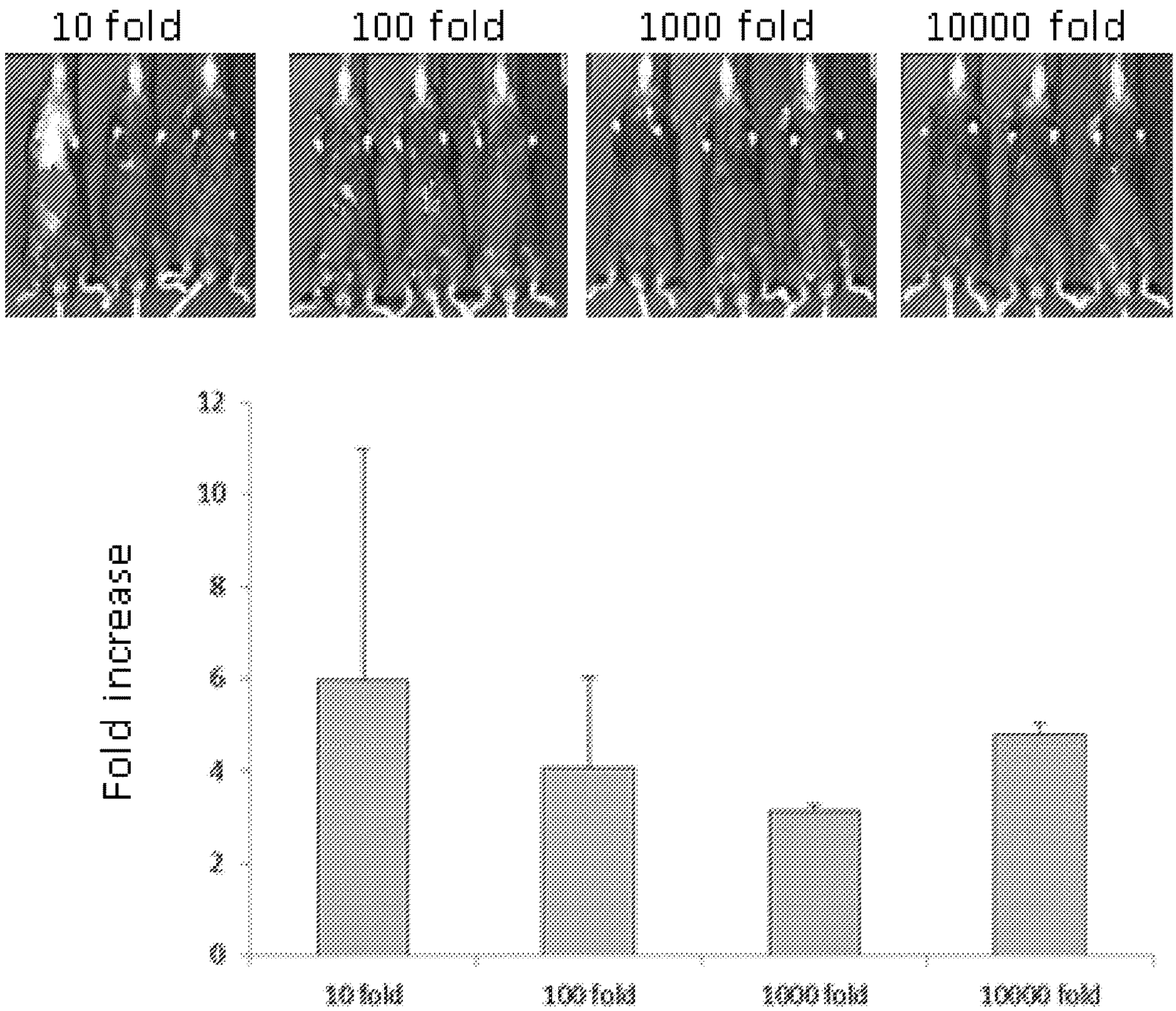
10 fold
100 fold
1000 fold
10000 fold
Fold increase
0
2
4
6
8
10
12
10 fold
100 fold
1000 fold
10000 fold

Fig. 35G

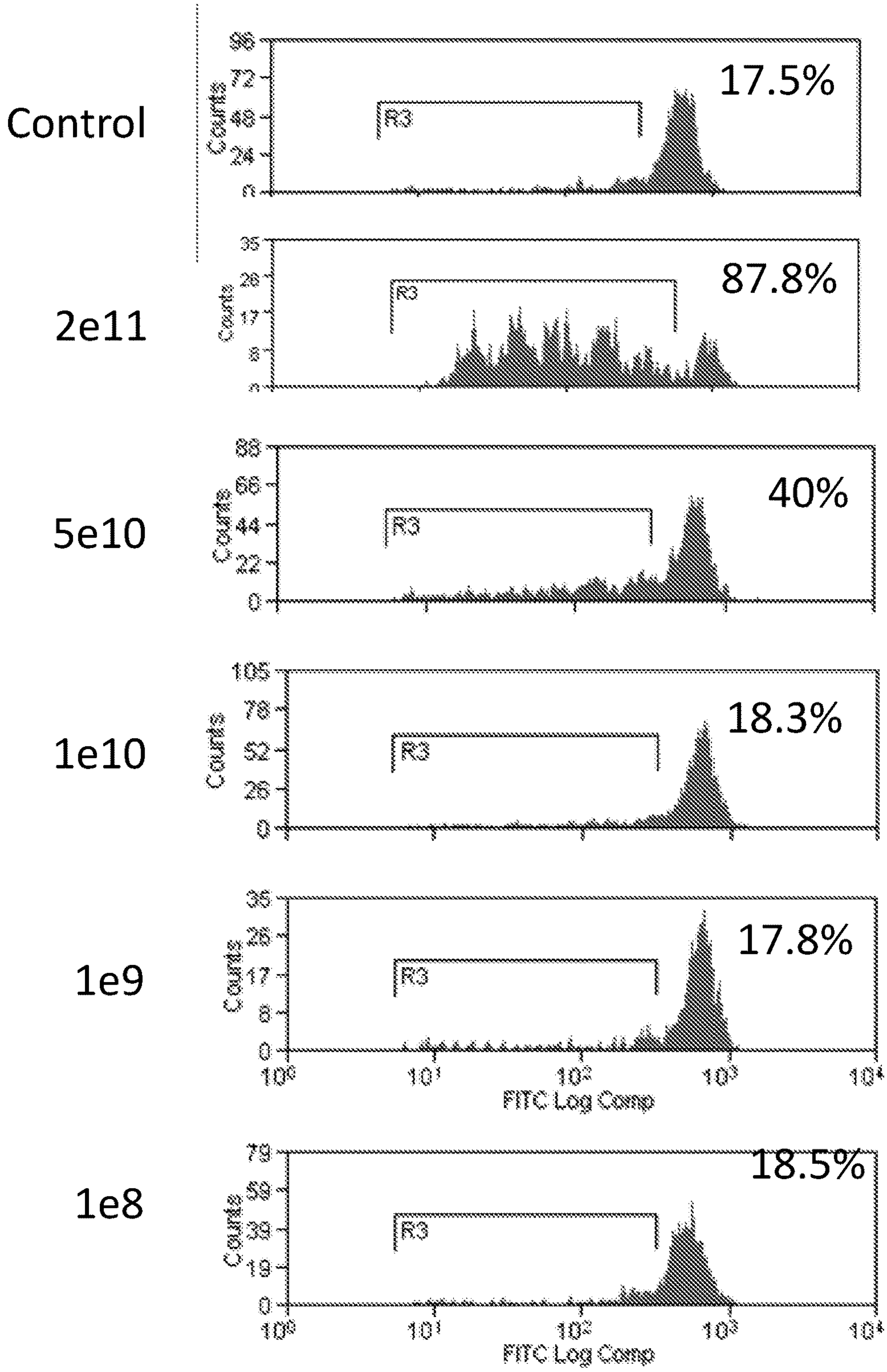
Control
17.5%
2e11
87.8%
5e10
40%
1e10
18.3%
1e9
17.8%
1e8
18.5%
R3
Counts
FITC Log Comp

Fig. 36A

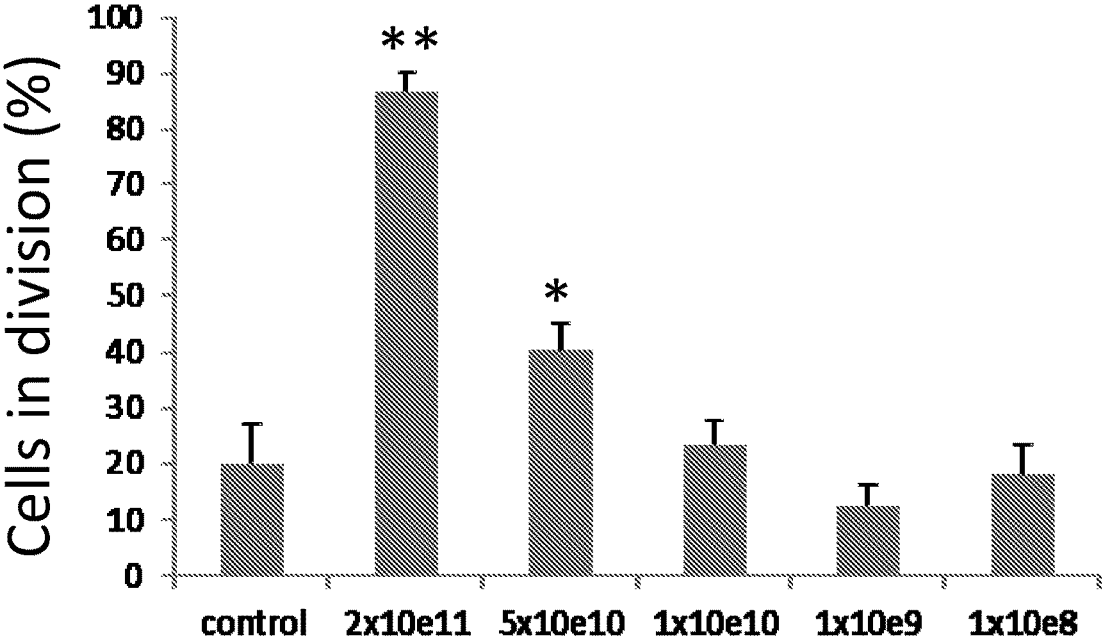


Fig. 36B

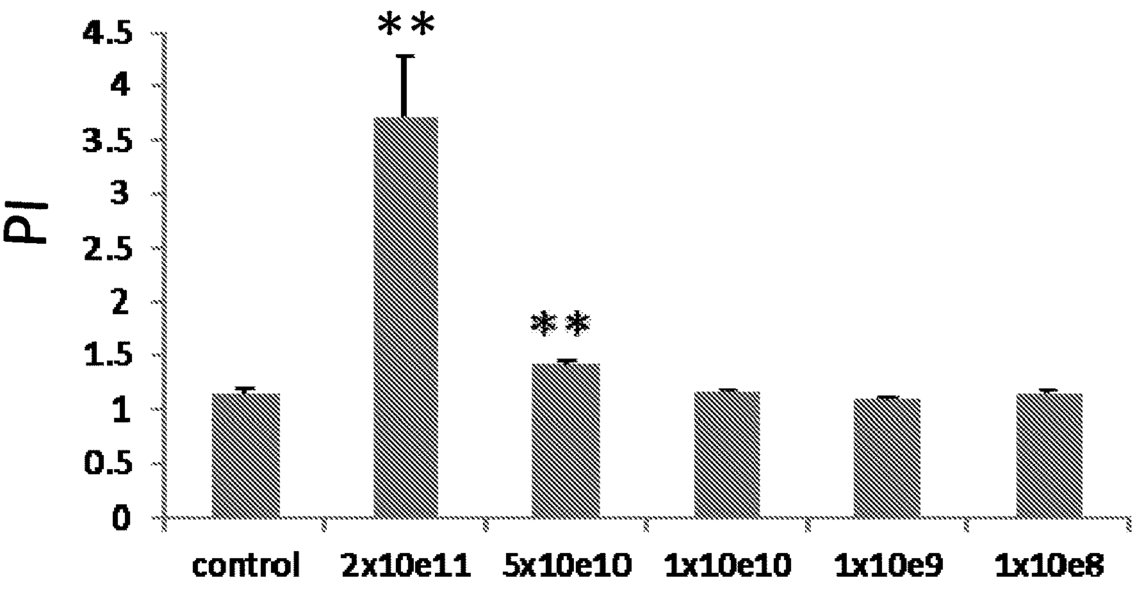


Fig. 36C

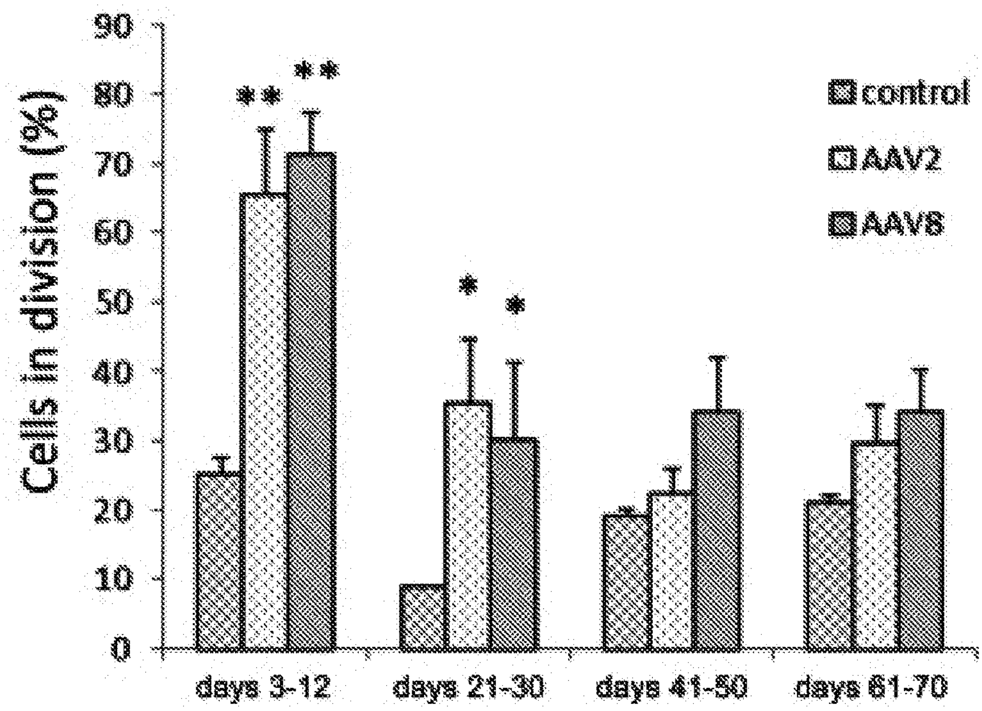


Fig. 37A

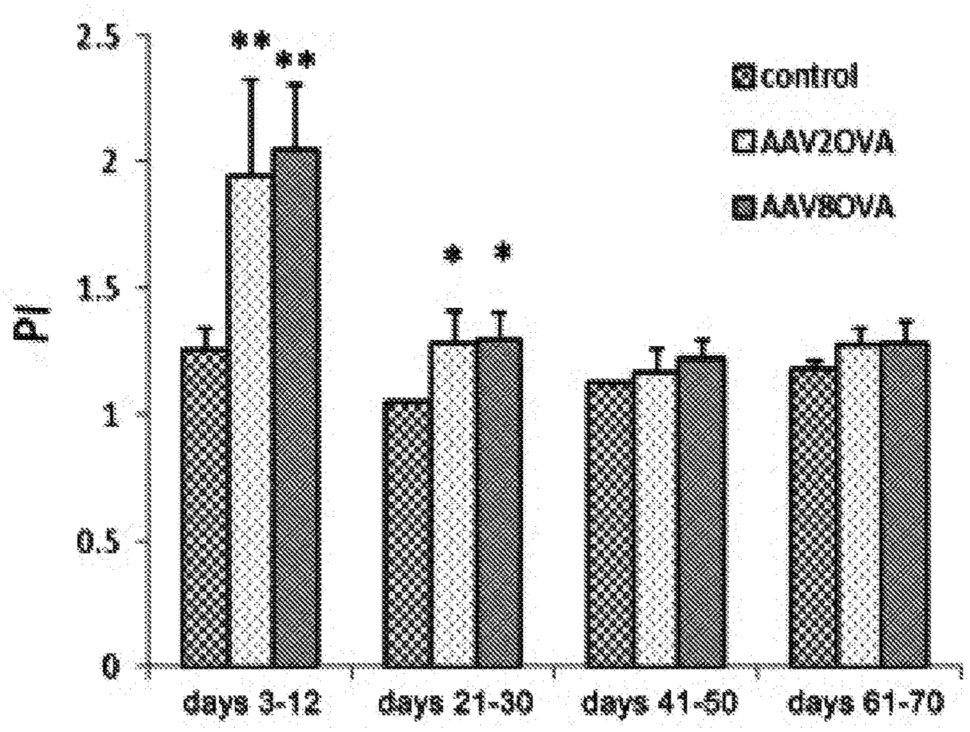


Fig. 37B

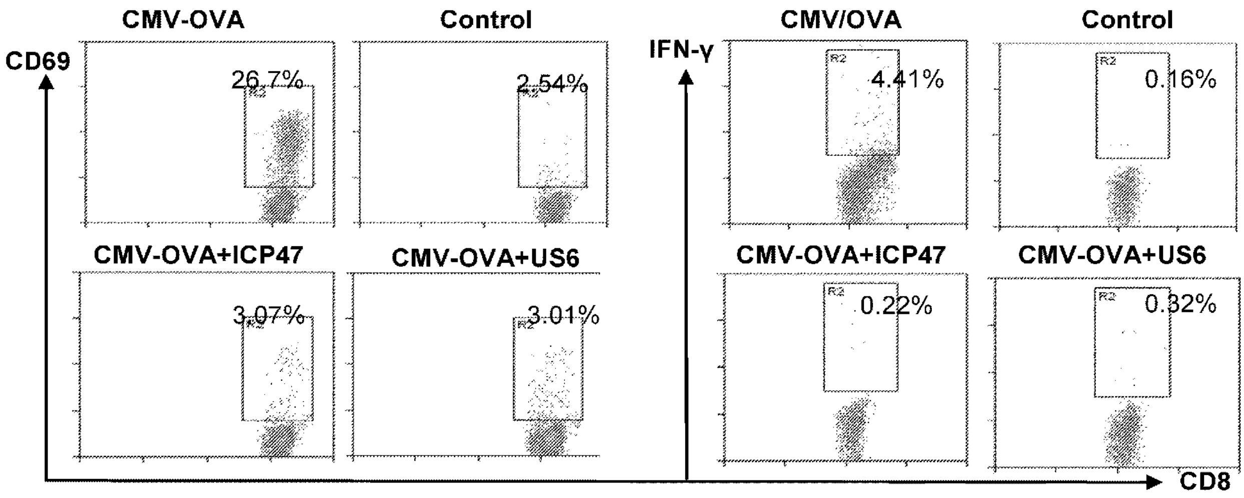
CMV-OVA
Control
CD69
26.7%
2.54%
CMV-OVA+ICP47
CMV-OVA+US6
3.07%
3.01%
CMV/OVA
Control
IFN-γ
4.41%
0.16%
CMV-OVA+ICP47
CMV-OVA+US6
0.22%
0.32%
CD8

Fig. 38

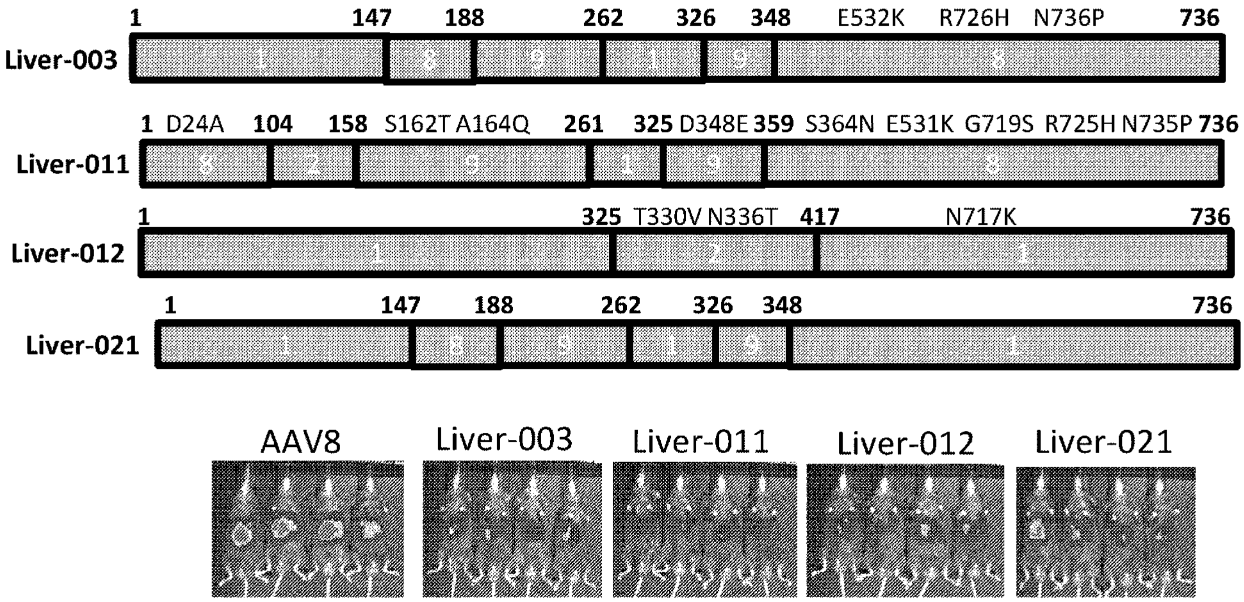
1 147 188 262 326 348 E532K R726H N736P 736
Liver-003
1 8 9 1 9 8
1 D24A 104 158 S162T A164Q 261 325 D348E 359 S364N E531K G719S R725H N735P 736
Liver-011
8 2 9 1 9 8
1 325 T330V N336T 417 N717K 736
Liver-012
1 2 1
1 147 188 262 326 348 736
Liver-021
1 8 9 1 9 1
AAV8
Liver-003
Liver-011
Liver-012
Liver-021

Fig. 39

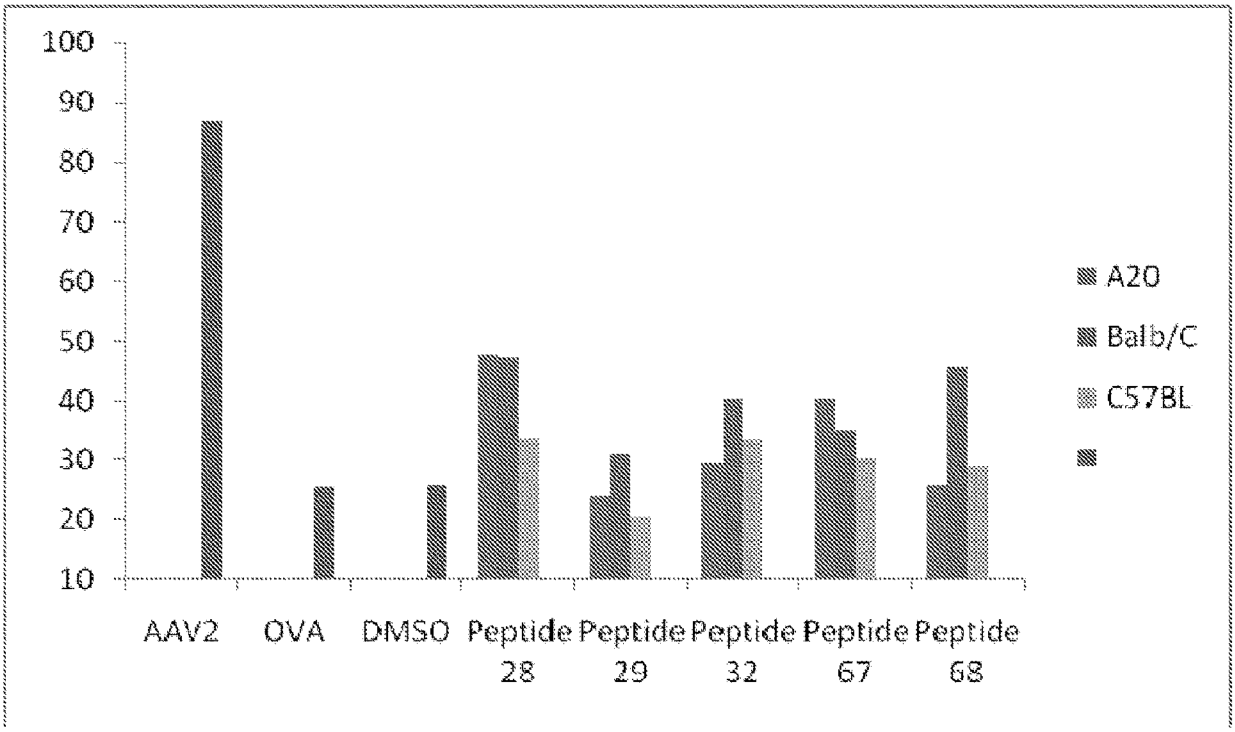
100
90
80
70
60
50
40
30
20
10
AAV2
OVA
DMSO
Peptide 28
Peptide 29
Peptide 32
Peptide 67
Peptide 68
A20
Balb/C
C57BL

**Fig. 40**

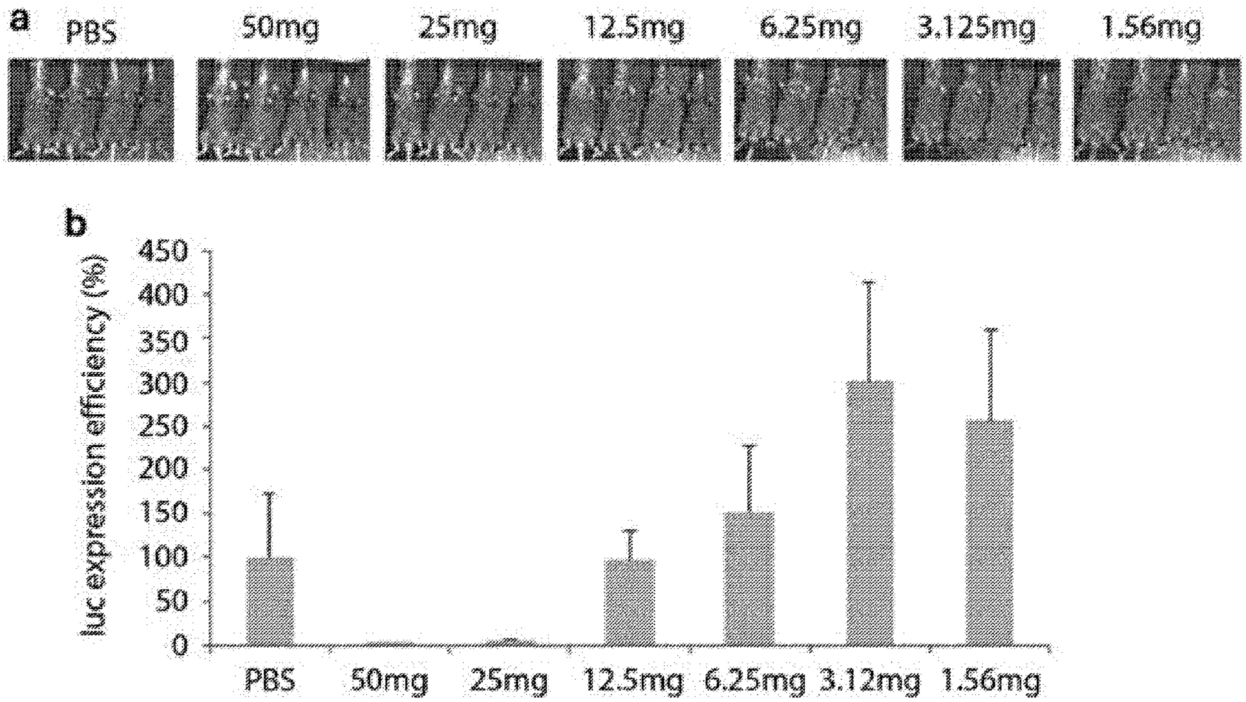
a
PBS
50mg
25mg
12.5mg
6.25mg
3.125mg
1.56mg
b
luc expression efficiency (%)
450
400
350
300
250
200
150
100
50
0
PBS
50mg
25mg
12.5mg
6.25mg
3.12mg
1.56mg

Fig. 41

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62471762
- US 62520901
- US 62630558
- WO 0028061 A
- WO 9961601 A
- WO 9811244 A
- US 6156303 A
- US 5478745 A, Samulski
- US 20140037585 A
- WO 0028004 A
- WO 0192551 A
- WO 2013158879 A1
- US 486254
- WO 2006066066 A
- WO 2009108274 A
- US 5863541 A
- US 7314912 B
- WO 2007089632 A
- US 6040183 A
- US 6093570 A
- WO 0017377 A
- US 2003017131 A
- WO 2008088895 A
- WO 2006029319 A
- WO 2007100465 A
- US 7071172 B
- JP 2006073052 A
- US 5877022 A
- US 6013487 A
- US 6083702 A
- US 5869248 A, Yuan
- US 5916563 A, Young
- US 5905040 A, Mazzara
- US 5882652 A
- US 4968603 A
- WO 9005142 A
- WO 2006119137 A
- WO 2003095647 A
- WO 2006021724 A
- US 5399346 A
- US 7201898 B
- US 20020192189 A
- US 20040013645 A
- US 4501729 A
- US 9441206 B


### Non-patent literature cited in the description


- **BERNARD N. FIELDS et al.** VIROLOGY. Lippincott-Raven Publishers, vol. 2
- **GAO et al.** *J. Virology*, 2004, vol. 78, 6381-6388
- **MORIS et al.** *Virology*, 2004, vol. 33, 375-383
- **SRIVISTAVA et al.** *J. Virology*, 1983, vol. 45, 555
- **CHIARINI et al.** *J. Virology*, 1998, vol. 71, 6823
- **CHIARINI et al.** *J. Virology*, 1999, vol. 73, 1309
- **BANTEL-SCHAAL et al.** *J. Virology*, 1999, vol. 73, 939
- **XIAO et al.** *J. Virology*, 1999, vol. 73, 3994
- **MURAMATSU et al.** *Virology*, 1996, vol. 221, 208
- **SHADE et al.** *J. Virol.*, 1986, vol. 58, 921
- **GAO et al.** *Proc. Nat. Acad. Sci. USA*, 2002, vol. 99, 11854
- **XIE et al.** *Proc. Nat. Acad. Sci.*, 2002, vol. 99, 10405-10
- **PADRON et al.** *J. Virol.*, 2005, vol. 79, 5047-58
- **WALTERS et al.** *J. Virol.*, 2004, vol. 78, 3361-71
- **XIE et al.** *J. Mol. Biol.*, 1996, vol. 6, 497-520
- **TSAO et al.** *Science*, 1991, vol. 251, 1456-64
- **MUZYCZKA**. *Curr. Topics Microbiol. Immunol.*, 1992, vol. 158, 97
- **CHAO et al.** *Molecular Therapy*, 2000, vol. 2, 619
- **WANG et al.** *Annu Rev Biophys Biomol Struct.*, 2006, vol. 35, 225-49
- **L LISOWSKI** ; **AP DANE** ; **K CHU** ; **Y ZHANG** ; **SC CUNNINGHAMM** ; **EM WILSON et al.** Selection and evaluation of clinically relevant AAV variants in a xenograft liver model.. *Nature*, 2014, vol. 506, 382-386
- **GRIMM D** ; **LEE JS** ; **WANG L** ; **DESAI T** ; **AKACHE B** ; **STORM TA** ; **KAY MA**. In vitro and in vivo gene therapy vector evolution via multispecies interbreeding and retargeting of adeno-associated viruses.. *J. Virol*, June 2008, vol. 82 (12), 5887-911

- **POWELL SK ; KHAN N ; PARKER CL ; SAMULSKI RJ ; MATSUSHIMA G ; GRAY SJ ; MCCOWN TJ**. Characterization of a novel adeno-associated viral vector with preferential oligodendrocyte tropism.. *Gene Ther.*, November 2016, vol. 23 (11), 807-814
- **TERVO DG ; HWANG BY ; VISWANATHAN S ; GAJ T ; LAVZIN M ; RITOLA KD ; LINDO S ; MICHAEL S ; KULESHOVA E ; OJALA D**. A Designer AAV Variant Permits Efficient Retrograde Access to Projection Neurons.. *Neuron.*, 19 October 2016, vol. 92 (2), 372-382
- **MARSIC D ; GOVINDASAMY L ; CURRLIN S ; MARKUSIC DM ; TSENG YS ; HERZOG RW ; AGBANDJE-MCKENNA M ; ZOLOTUKHIN S.** Vector design Tour de Force: integrating combinatorial and rational approaches to derive novel adeno-associated virus variants.. *Mol Ther.*, November 2014, vol. 22 (11), 1900-9
- **SALLACH J ; DI PASQUALE G ; LARCHER F ; NIEHOFF N ; RÜBSAM M ; HUBER A ; CHIORINI J ; ALMARZA D ; EMING SA ; ULUS H**. Tropism-modified AAV vectors overcome barriers to successful cutaneous therapy.. *Mol Ther.*, May 2014, vol. 22 (5), 929-39
- **DALKARA D ; BYRNE LC ; KLIMCZAK RR ; VISEL M ; YIN L ; MERIGAN WH ; FLANNERY JG ; SCHAFFER DV**. In vivo-directed evolution of a new adeno-associated virus for therapeutic outer retinal gene delivery from the vitreous.. *Sci Transl Med.*, 12 June 2013, vol. 5 (189), 189-76
- **ASURI P ; BARTEL MA ; VAZIN T ; JANG JH ; WONG TB ; SCHAFFER DV**. Directed evolution of adeno-associated virus for enhanced gene delivery and gene targeting in human pluripotent stem cells.. *Mol Ther.*, February 2012, vol. 20 (2), 329-38
- **JANG JH ; KOERBER JT ; KIM JS ; ASURI P ; VAZIN T ; BARTEL M ; KEUNG A ; KWON I ; PARK KI ; SCHAFFER DV.** An evolved adeno-associated viral variant enhances gene delivery and gene targeting in neural stem cells.. *Mol Ther.*, April 2011, vol. 19 (4), 667-75
- **GRAY SJ ; BLAKE BL ; CRISWELL HE ; NICOLSON SC ; SAMULSKI RJ ; MCCOWN TJ ; LI W.** Directed evolution of a novel adeno-associated virus (AAV) vector that crosses the seizure-compromised blood-brain barrier (BBB).. *Mol Ther.*, March 2010, vol. 18 (3), 570-8
- **MAGUIRE CA ; GIANNI D ; MEIJER DH ; SHAKET LA ; WAKIMOTO H ; RABKIN SD ; GAO G ; SENA-ESTEVES M.** Directed evolution of adeno-associated virus for glioma cell transduction.. *J. Neurooncol.*, February 2010, vol. 96 (3), 337-47
- **KOERBER JT ; KLIMCZAK R ; JANG JH ; DALKARA D ; FLANNERY JG ; SCHAFFER DV**. Molecular evolution of adeno-associated virus for enhanced glial gene delivery.. *Mol Ther.*, December 2009, vol. 17 (12), 2088-95
- **LI W ; ZHANG L ; JOHNSON JS ; ZHIJIAN W ; GRIEGER JC ; PING-JIE X ; DROUIN LM ; AGBANDJE-MCKENNA M ; PICKLES RJ ; SAMULSKI RJ**. Generation of novel AAV variants by directed evolution for improved CFTR delivery to human ciliated airway epithelium.. *Mol Ther.*, December 2009, vol. 17 (12), 2067-77
- **KLIMCZAK RR ; KOERBER JT ; DALKARA D ; FLANNERY JG ; SCHAFFER DV.** A novel adeno-associated viral variant for efficient and selective intravitreal transduction of rat Müller cells.. *PLoS One.*, 14 October 2009, vol. 4 (10), e7467
- **EXCOFFON KJ ; KOERBER JT ; DICKEY DD ; MURTHA M ; KESHAVJEE S ; KASPAR BK ; ZABNER J ; SCHAFFER DV**. Directed evolution of adeno-associated virus to an infectious respiratory virus.. *Proc Natl Acad Sci USA.*, 10 March 2009, vol. 106 (10), 3865-70
- **SELLNER L ; STIEFELHAGEN M ; KLEINSCHMIDT JA ; LAUFS S ; WENZ F ; FRUEHAUF S ; ZELLER WJ ; VELDWIJK MR**. Generation of efficient human blood progenitor-targeted recombinant adeno-associated viral vectors (AAV) by applying an AAV random peptide library on primary human hematopoietic progenitor cells.. *Exp Hematol.*, August 2008, vol. 36 (8), 957-64
- **LI W ; ASOKAN A ; WU Z ; VAN DYKE T ; DIPRIMIO N ; JOHNSON JS ; GOVINDASWAMY L ; AGBANDJE-MCKENNA M ; LEICHTLE S ; REDMOND DE JR**. Engineering and selection of shuffled AAV genomes: a new strategy for producing targeted biological nanoparticles.. *Mol Ther.*, July 2008, vol. 16 (7), 1252-60
- **CHARBEL ISSA P ; DE SILVA SR ; LIPINSKI DM ; SINGH MS ; MOURAVLEV A ; YOU Q**. Assessment of tropism and effectiveness of new primate-derived hybrid recombinant AAV serotypes in the mouse and primate retina.. *PLoS ONE.*, 2013, vol. 8, e60361
- **HUANG W ; MCMURPHY T ; LIU X ; WANG C ; CAO L**. Genetic Manipulation of Brown Fat Via Oral Administration of an Engineered Recombinant Adeno-associated Viral Serotype Vector.. *Mol. Ther.*, June 2016, vol. 24 (6), 1062-9
- **CRONIN T ; VANDENBERGHE LH ; HANTZ P et al.** Efficient transduction and optogenetic stimulation of retinal bipolar cells by a synthetic adeno-associated virus capsid and promoter.. *EMBO Mol. Med.*, 2014, vol. 6, 1175-1190
- **CHOUDHURY SR ; FITZPATRICK Z ; HARRIS AF ; MAITLAND SA ; FERREIRA JS ; ZHANG Y ; MA S ; SHARMA RB ; GRAY-EDWARDS HL ; JOHNSON JA**. In Vivo Selection Yields AAV-B1 Capsid for Central Nervous System and Muscle Gene Therapy.. *Mol Ther.*, August 2016, vol. 24 (7), 1247-57

- **DEVERMAN BE** ; **PRAVDO PL** ; **SIMPSON BP** ; **KUMAR SR** ; **CHAN KY** ; **BANERJEE A** ; **WU WL** ; **YANG B** ; **HUBER N** ; **PASCA SP**. Cre-dependent selection yields AAV variants for widespread gene transfer to the adult brain.. *Nat Biotechnol.*, February 2016, vol. 34 (2), 204-9
- **PULICHERLA N** ; **SHEN S** ; **YADAV S** ; **DEBBINK K** ; **GOVINDASAMY L** ; **AGBANDJE-MCKENNA M** ; **ASOKAN A**. Engineering liver-detargeted AAV9 vectors for cardiac and musculoskeletal gene transfer.. *Mol Ther.*, June 2011, vol. 19 (6), 1070-8
- **YANG L** ; **JIANG J** ; **DROUIN LM** ; **AGBANDJE-MCKENNA M** ; **CHEN C** ; **QIAO C** ; **PU D** ; **HU X** ; **WANG DZ** ; **LI J**. A myocardium tropic adeno-associated virus (AAV) evolved by DNA shuffling and in vivo selection.. *Proc Natl Acad Sci USA.*, 10 March 2009, vol. 106 (10), 3946-51
- **KÖRBELIN J** ; **SIEBER T** ; **MICHELFELDER S** ; **LUNDING L** ; **SPIES E** ; **HUNGER A** ; **ALAWI M** ; **RAPTI K** ; **INDENBIRKEN D** ; **MÜLLER OJ**. Pulmonary Targeting of Adeno-associated Viral Vectors by Next-generation Sequencing-guided Screening of Random Capsid Displayed Peptide Libraries.. *Mol Ther.*, June 2016, vol. 24 (6), 1050-61
- **GEOGHEGAN JC** ; **KEISER NW** ; **OKULIST A** ; **MARTINS I** ; **WILSON MS** ; **DAVIDSON BL**. Chondroitin Sulfate is the Primary Receptor for a Peptide-Modified AAV That Targets Brain Vascular Endothelium In Vivo.. *Mol Ther Nucleic Acids.*, 14 October 2014, vol. 3, e202
- **VARADI K** ; **MICHELFELDER S** ; **KORFF T** ; **HECKER M** ; **TREPEL M** ; **KATUS HA** ; **KLEINSCHMIDT JA** ; **MÜLLER OJ**. Novel random peptide libraries displayed on AAV serotype 9 for selection of endothelial cell-directed gene transfer vectors.. *Gene Ther.*, August 2012, vol. 19 (8), 800-9
- **MICHELFELDER S** ; **VARADI K** ; **RAUPP C** ; **HUNGER A** ; **KÖRBELIN J** ; **PAHRMANN C** ; **SCHREPFER S** ; **MÜLLER OJ** ; **KLEINSCHMIDT JA** ; **TREPEL M.** Peptide ligands incorporated into the threefold spike capsid domain to re-direct gene transduction of AAV8 and AAV9 in vivo.. *PLoS One.*, 2011, vol. 6 (8), e23101
- **YU CY** ; **YUAN Z** ; **CAO Z** ; **WANG B** ; **QIAO C** ; **LI J** ; **XIAO X**. A muscle-targeting peptide displayed on AAV2 improves muscle tropism on systemic delivery.. *Gene Ther.*, August 2009, vol. 16 (8), 953-62
- **MICHELFELDER S** ; **LEE MK** ; **DELIMA-ILAHN E** ; **WILMES T** ; **KAUL F** ; **MÜLLER O** ; **KLEINSCHMIDT JA** ; **TREPEL M**. Vectors selected from adeno-associated viral display peptide libraries for leukemia cell-targeted cytotoxic gene therapy.. *Exp Hematol.*, December 2007, vol. 35 (12), 1766-76
- **MÜLLER OJ** ; **KAUL F** ; **WEITZMAN MD** ; **PASQUALINI R** ; **ARAP W** ; **KLEINSCHMIDT JA** ; **TREPEL M**. Random peptide libraries displayed on adeno-associated virus to select for targeted gene therapy vectors.. *Nat Biotechnol.*, September 2003, vol. 21 (9), 1040-6
- **GRIFMAN M** ; **TREPEL M** ; **SPEECE P** ; **GILBERT LB** ; **ARAP W** ; **PASQUALINI R** ; **WEITZMAN MD**. Incorporation of tumor-targeting peptides into recombinant adeno-associated virus capsids.. *Mol Ther.*, June 2001, vol. 3 (6), 964-75
- **ANNE GIROD** ; **MARTIN RIED** ; **CHRISTIANE WOBUS** ; **HARALD LAHM** ; **KRISTIN LEIKE** ; **JÜRGEN KLEINSCHMIDT** ; **GILBERT DELÉAGE** ; **MICHAEL HALLEK**. Genetic capsid modifications allow efficient re-targeting of adeno-associated virus type 2.. *Nature Medicine*, 1999, 1052-1056
- **BELLO A** ; **CHAND A** ; **AVILES J** ; **SOULE G** ; **AURICCHIO A** ; **KOBINGER GP**. Novel adeno-associated viruses derived from pig tissues transduce most major organs in mice.. *Sci Rep.*, 22 October 2014, vol. 4, 6644
- **GAO G** ; **VANDENBERGHE LH** ; **ALVIRA MR** ; **LU Y** ; **CALCEDO R** ; **ZHOU X** ; **WILSON JM.** Clades of Adeno-associated viruses are widely disseminated in human tissues.. *J. Virol.*, June 2004, vol. 78 (12), 6381-8
- **ARBETMAN AE** ; **LOCHRIE M** ; **ZHOU S** ; **WELLMAN J** ; **SCALLAN C** ; **DOROUDCHI MM et al.** Novel caprine adeno-associated virus (AAV) capsid (AAV-Go.1) is closely related to the primate AAV-5 and has unique tropism and neutralization properties.. *J. Virol.*, 2005, vol. 79, 15238-15245
- **LOCHRIE MA** ; **TATSUNO GP** ; **ARBETMAN AE** ; **JONES K** ; **PATER C** ; **SMITH PH et al.** Adeno-associated virus (AAV) capsid genes isolated from rat and mouse liver genomic DNA define two new AAV species distantly related to AAV-5.. *Virology.*, 2006, vol. 353, 68-82
- **SCHMIDT M** ; **KATANO H** ; **BOSSIS I** ; **CHIORINI JA.** Cloning and characterization of a bovine adeno-associated virus.. *J. Virol.*, 2004, vol. 78, 6509-6516
- **BOSSIS I** ; **CHIORINI JA.** Cloning of an avian adeno-associated virus (AAAV) and generation of recombinant AAAV particles.. *J. Virol.*, 2003, vol. 77, 6799-6810
- **CHEN CL** ; **JENSEN RL** ; **SCHNEPP BC** ; **CONNELL MJ** ; **SHELL R** ; **SFERRA TJ** ; **BARTLETT JS** ; **CLARK KR** ; **JOHNSON PR**. Molecular characterization of adeno-associated viruses infecting children.. *J. Virol.*, December 2005, vol. 79 (23), 14781-92

- **SEN D** ; **GADKARI RA** ; **SUDHA G** ; **GABRIEL N** ; **KUMAR YS** ; **SELOT R** ; **SAMUEL R** ; **RAJALINGAM S** ; **RAMYA V** ; **NAIR SC**. Targeted modifications in adeno-associated virus serotype 8 capsid improves its hepatic gene transfer efficiency in vivo.. *Hum Gene Ther Methods.*, April 2013, vol. 24 (2), 104-16
- **LI B** ; **MA W** ; **LING C** ; **VAN VLIET K** ; **HUANG LY** ; **AGBANDJE-MCKENNA M** ; **SRIVASTAVA A** ; **ASLANIDI GV.** Site-Directed Mutagenesis of Surface-Exposed Lysine Residues Leads to Improved Transduction by AAV2, But Not AAV8, Vectors in Murine Hepatocytes In Vivo.. *Hum Gene Ther Methods.*, December 2015, vol. 26 (6), 211-20
- **GABRIEL N** ; **HAREENDRAN S** ; **SEN D** ; **GADKARI RA** ; **SUDHA G** ; **SELOT R** ; **HUSSAIN M** ; **DHAKSNAMOORTHY R** ; **SAMUEL R** ; **SRINIVASAN N et al.** Bioengineering of AAV2 capsid at specific serine, threonine, or lysine residues improves its transduction efficiency in vitro and in vivo.. *Hum Gene Ther Methods.*, April 2013, vol. 24 (2), 80-93
- **ZINN E** ; **PACOURET S** ; **KHAYCHUK V** ; **TURUNEN HT** ; **CARVALHO LS** ; **ANDRES-MATEOS E** ; **SHAH S** ; **SHELKE R** ; **MAURER AC** ; **PLOVIE E**. In Silico Reconstruction of the Viral Evolutionary Lineage Yields a Potent Gene Therapy Vector.. *Cell Rep.*, 11 August 2015, vol. 12 (6), 1056-68
- **SHEN S** ; **HOROWITZ ED** ; **TROUPES AN** ; **BROWN SM** ; **PULICHERLA N** ; **SAMULSKI RJ** ; **AGBANDJE-MCKENNA M** ; **ASOKAN A**. Engraftment of a galactose receptor footprint onto adeno-associated viral capsids improves transduction efficiency.. *J Biol Chem.*, 04 October 2013, vol. 288 (40), 28814-23
- **LI C** ; **DIPRIMIO N** ; **BOWLES DE** ; **HIRSCH ML** ; **MONAHAN PE** ; **ASOKAN A** ; **RABINOWITZ J** ; **AGBANDJE-MCKENNA M** ; **SAMULSKI RJ**. Single amino acid modification of adeno-associated virus capsid changes transduction and humoral immune profiles.. *J. Virol.*, August 2012, vol. 86 (15), 7752-9
- **BOWLES DE** ; **MCPHEE SW** ; **LI C** ; **GRAY SJ** ; **SAMULSKI JJ** ; **CAMP AS** ; **LI J** ; **WANG B** ; **MONAHAN PE** ; **RABINOWITZ JE et al.** Phase 1 gene therapy for Duchenne muscular dystrophy using a translational optimized AAV vector.. *Mol. Ther.*, February 2012, vol. 20 (2), 443-55
- **MESSINA EL** ; **NIENABER J** ; **DANESHMAND M** ; **VILLAMIZAR N** ; **SAMULSKI J** ; **MILANO C** ; **BOWLES DE**. Adeno-associated viral vectors based on serotype 3b use components of the fibroblast growth factor receptor signaling complex for efficient transduction.. *Hum. Gene Ther.*, October 2012, vol. 23 (10), 1031-42
- **ASOKAN A** ; **CONWAY JC** ; **PHILLIPS JL** ; **LI C** ; **HEGGE J** ; **SINNOTT R** ; **YADAV S** ; **DIPRIMIO N** ; **NAM HJ** ; **AGBANDJE-MCKENNA M**. Reengineering a receptor footprint of adeno-associated virus enables selective and systemic gene transfer to muscle.. *Nat Biotechnol.*, January 2010, vol. 28 (1), 79-82
- **VANCE M** ; **LLANGA T** ; **BENNETT W** ; **WOODARD K** ; **MURLIDHARAN G** ; **CHUNGFAT N** ; **ASOKAN A** ; **GILGER B** ; **KURTZBERG J** ; **SAMULSKI RJ**. AAV Gene Therapy for MPS1-associated Corneal Blindness.. *Sci Rep.*, 22 February 2016, vol. 6, 22131
- **ZHONG L** ; **LI B** ; **MAH CS** ; **GOVINDASAMY L** ; **AGBANDJE-MCKENNA M** ; **COOPER M** ; **HERZOG RW** ; **ZOLOTUKHIN I** ; **WARRINGTON KH JR** ; **WEIGEL-VAN AKEN KA**. Next generation of adeno-associated virus 2 vectors: point mutations in tyrosines lead to high-efficiency transduction at lower doses.. *Proc Natl Acad Sci USA.*, 03 June 2008, vol. 105 (22), 7827-32
- **PETRS-SILVA H** ; **DINCULESCU A** ; **LI Q** ; **MIN SH** ; **CHIODO V** ; **PANG JJ** ; **ZHONG L** ; **ZOLOTUKHIN S** ; **SRIVASTAVA A** ; **LEWIN AS**. High-efficiency transduction of the mouse retina by tyrosine-mutant AAV serotype vectors.. *Mol. Ther.*, March 2009, vol. 17 (3), 463-71
- **QIAO C** ; **ZHANG W** ; **YUAN Z** ; **SHIN JH** ; **LI J** ; **JAYANDHARAN GR** ; **ZHONG L** ; **SRIVASTAVA A** ; **XIAO X** ; **DUAN D**. Adeno-associated virus serotype 6 capsid tyrosine-to-phenylalanine mutations improve gene transfer to skeletal muscle.. *Hum Gene Ther.*, October 2010, vol. 21 (10), 1343-8
- **CARLON M** ; **TOELEN J** ; **VAN DER PERREN A** ; **VANDENBERGHE LH** ; **REUMERS V** ; **SBRAGIA L** ; **GIJSBERS R** ; **BAEKELANDT V** ; **HIMMELREICH U** ; **WILSON JM**. Efficient gene transfer into the mouse lung by fetal intratracheal injection of rAAV2/6.2.. *Mol. Ther.*, December 2010, vol. 18 (12), 2130-8
- **OPIE et al.** *J. Viral.*, 2003, vol. 77, 6995-7006
- **SMITH** ; **WATERMAN**. *Adv. Appl. Math.*, 1981, vol. 2, 482
- **NEEDLEMAN** ; **WUNSCH**. *J. Mol. Biol.*, 1970, vol. 48, 443
- **PEARSON** ; **LIPMAN**. *Proc. Natl. Acad. Sci. USA*, 1988, vol. 85, 2444
- **DEVEREUX et al.** *Nucl. Acid Res.*, 1984, vol. 12, 387-395
- **ALTSCHUL et al.** *J. Mol. Biol.*, 1990, vol. 215, 403-410
- **KARLIN et al.** *Proc. Natl. Acad. Sci. USA*, 1993, vol. 90, 5873-5787
- **ALTSCHUL et al.** *Methods in Enzymology*, 1996, vol. 266, 460-480
- **ALTSCHUL et al.** *Nucleic Acids Res.*, 1997, vol. 25, 3389-3402

- **BROWN et al.** *Virology*, 1994, vol. 198, 477-488
- **HAUCK et al.** *J. Virology*, 2003, vol. 77, 2768-2774
- **SHI et al.** *Human Gene Therapy*, 2006, vol. 17, 353-361
- **GRIFMAN et al.** *Molecular Therapy*, 2001, vol. 3, 964-975
- **GREG T.** Hermanson, Bioconjugate Techniques. Academic Press, 1996
- **BROWN et al.** *Science*, 1993, vol. 262, 114
- **AGBANDJE-MCKENNA et al.** *Virology*, 1994, vol. 203, 106
- **CHAPMAN et al.** *Virology*, 1993, vol. 194, 419
- **CHIPMAN et al.** *Proc. Nat. Acad. Sci. USA*, 1996, vol. 93, 7502
- **CLEVES**. *Current Biology*, 1997, vol. 7, R318
- **MÜLLER et al.** *Nature Biotechnology*, 2003, vol. 21, 1040-1046
- **WORK et al.** *Molecular Therapy*, 2006, vol. 13, 683-693
- **HAJITOU et al.** *TCM*, 2006, vol. 16, 80-88
- **KOIVUNEN et al.** *J. Nucl. Med.*, 1999, vol. 40, 883-888
- **NEWTON** ; **DEUTSCHER**. Phage Peptide Display in Handbook of Experimental Pharmacology. Springer-Verlag, 2008, 145-163
- **ZHONG et al.** *Virology*, 2008, vol. 381, 194-202
- *Proc. Nat. Acad. Sci.*, 2008, vol. 105, 7827-32
- **CLEMENT** ; **GRIEGER**. Manufacturing of recombinant adeno-associated viral vectors for clinical trials. *Mol. Ther. Methods Clin Dev.*, 2016, vol. 3, 16002
- **GRIEGER et al.** Production of recombinant adeno-associated virus vectors using suspension HEK293 cells and continuous harvest of vector from the culture media for GMP FIX and FLT1 clinical vector. *Mol Ther*, 2016, vol. 24 (2), 287-297
- **MARGOLSKI**. *Curr. Top. Microbiol. Immun.*, 1992, vol. 158, 67
- **PALOMBO et al.** *J. Virology*, 1998, vol. 72, 5025
- **FERRARI et al.** *Nature Med.*, 1997, vol. 3, 1295
- **ZHANG et al.** *Gene Ther.*, 2001, vol. 18, 704-12
- **CONWAY et al.** *Gene Therapy*, 1999, vol. 6, 986
- **URABE et al.** *Human Gene Therapy*, 2002, vol. 13, 1935-43
- **ZOLOTUKHIN et al.** *Gene Therapy*, 1999, vol. 6, 973
- **VINCENT et al.** *Nature Genetics*, 1993, vol. 5, 130
- **WANG et al.** *Proc. Natl. Acad. Sci. USA*, 2000, vol. 97, 13714-13719
- **GREGOREVIC et al.** *Mol. Ther.*, 2008, vol. 16, 657-64
- **TINSLEY et al.** *Nature*, 1996, vol. 384, 349
- **FANG et al.** *Nature Biotechnology*, 2005, vol. 23, 584-590
- **PUTTARAJU et al.** *Nature Biotech.*, 1999, vol. 17, 246
- **SHARP et al.** *Science*, 2000, vol. 287, 2431
- **GORMAN et al.** *Proc. Nat. Acad. Sci. USA*, 1998, vol. 95, 4929
- **ANDINO et al.** *J. Gene Med.*, 2008, vol. 10, 132-142
- **LI et al.** *Acta Pharmacol Sin.*, 2005, vol. 26, 51-55
- **HOSHIJIMA et al.** *Nat. Med.*, 2002, vol. 8, 864-871
- **MIYAMURA et al.** *Proc. Nat. Acad. Sci USA*, 1994, vol. 91, 8507
- **S.A. ROSENBERG**. *Immunity*, 1991, vol. 10, 281
- **KAWAKAMI et al.** *Proc. Natl. Acad. Sci. USA*, 1994, vol. 91, 3515
- **KAWAKAMI et al.** *J. Exp. Med.*, 1994, vol. 180, 347
- **KAWAKAMI et al.** *Cancer Res.*, 1994, vol. 54, 3124
- **BRICHARD et al.** *J. Exp . Med.*, 1993, vol. 178, 489
- **LEVINE**. *Ann. Rev. Biochem.*, 1993, vol. 62, 623
- **ROSENBERG**. *Ann. Rev. Med.*, 1996, vol. 47, 481-91
- Immunophysiology: Cell Function and Cellular Interactions in Antibody Formation. **HERBERT B. HERSCOWITZ**. IMMUNOLOGY: BASIC PROCESSES. 1985, vol. 117
- **ARRUDA et al.** *Blood*, 2005, vol. 105, 3458-3464